# EUROPEAN PATENT APPLICATION

(11) **EP 4 364 732 A2**
(43) Date of publication of application: **08.05.2024**
(21) Application number: 24165278.3
(22) Date of filing: 29.07.2021
(51) Int. Cl.: A61K 31/22

(54) **FUNCTIONALIZED ISONITRILES AND PRODUCTS, PREPARATION AND USES THEREOF**

(30) Priority: 29.07.2020 EP 20382684
(62) Divisional of application: 21755721.4
(71) Applicant: Universidade de Santiago de Compostela, 15782 Santiago de Compostela, La Coruña (ES)
(72) Inventor: SOTELO PÉREZ, Eddy, 15706 Santiago de Compostela - A Coruña (ES); AZUAJE GUERRERO, Jhonny Alberto, 15706 Santiago de Compostela - A Coruña (ES); MAJELLARO, María, 15706 Santiago de Compostela - A Coruña (ES)
(74) Representative: ABG Intellectual Property Law, S.L.

(57) **Abstract**

The present invention relates to functionalized isonitrile compounds, formed by means of multicomponent environmentally friendly reactions, suitable for coupling to functional molecules such as biomolecules, APIs, chromophore and fluorophore molecules. The invention also relates to conjugates between said isonitrile compounds and functional molecules, which are useful in the synthesis of pharmaceutic drugs or tools, fluorescent molecules and labels, polymeric smart materials. The invention furthermore provides a kit for the in-vitro preparation of the functionalized isontrile compounds and conjugates. The invention also contemplates the medical use of said compounds and conjugates.

## Description

### FIELD OF INVENTION

The present invention is related to functionalized isonitriles, products obtained therefrom, preparation methods and uses thereof. The present invention further relates to kits comprising precursor isonitrile molecules for bioassays.

### BACKGROUND OF THE INVENTION

Isonitriles (also called isocyanides, general formula R-NC) constitute a family of organic compounds that stand out for their ability of creating chemical diversity, particularly in multicomponent reactions and especially in the Ugi reaction. Despite its potential, the chemistry of isonitriles remains poorly explored and the structural diversity and commercial availability of isonitriles is very limited. Most of the work in this field use fairly simple isonitriles (typically: benzylisonitrile, cyclohexylisonitrile or tert-butylisonitrile). The intense and repulsive odor of many isonitriles (Ugi, I., Fetzer, U., Knupfer, H., Offerman, K., Angew. Chem. Int. Ed., 1965, 4, 472-484) as well as the short supply of precursor molecules could explain the latter.

Generally, isonitriles are synthesized by employing a primary amine or an alkyl halide as key precursors. Starting from a primary amine, two synthetic methods are known: 1) formylation and subsequent dehydration of the obtained formamide, by treatment with phosphorous oxychloride, phosgene, triphosgene or a sulfonyl chloride (Ugi, I., Meyr, R., Chem. Ber., 1960, 93, 239-248); and 2) Hofmann's method, which consists of treating the primary amine with a haloform in the presence of an excess of a strong base such as NaOH or NaH (Hofmann, AW Ann. 1868, 146, 107 and Gokel, GW; Widera, RP; Weber, WP Organic Syntheses. 1988, 55, 232). Alternatively, the isonitriles can be prepared from a halogenated derivative, which is treated with silver cyanide and a phase transfer catalyst (80-100 °C) for 1-6 hours (El Kaim, L., Grimaud, L., Schiltz, A., Tetrahedron Lett., 2009, 50, 5235-5237).

The extensive application of isonitrile chemistry in different research areas demands the development of more complex isonitriles. In this regard, functional isonitriles are especially interesting; that is, those that contain structural elements that provide the new compound (isonitrile) with specific properties and/or characteristics (such as optical activity, affinity for a biological target, chelating effect of metals, fluorescence, among others). The following scheme shows representative examples of how isonitriles are obtained.

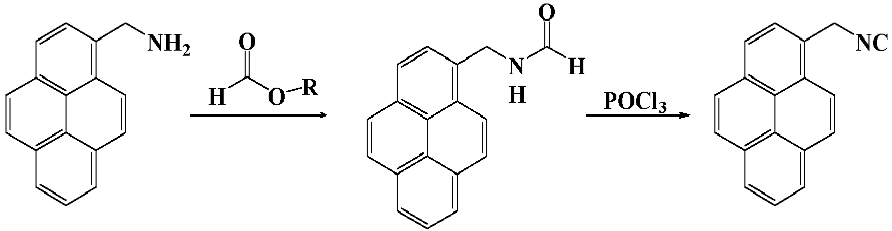

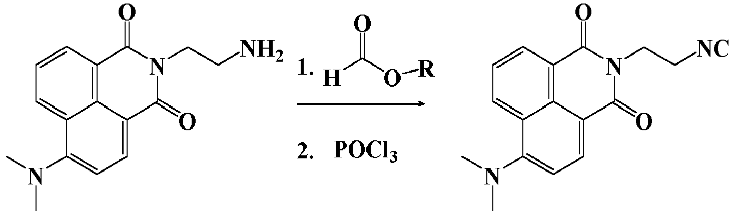

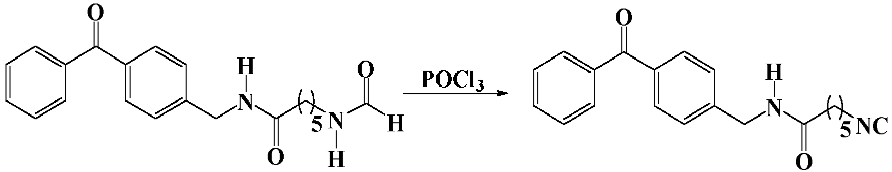

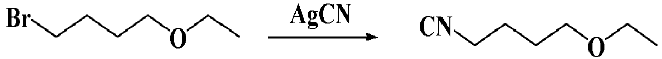

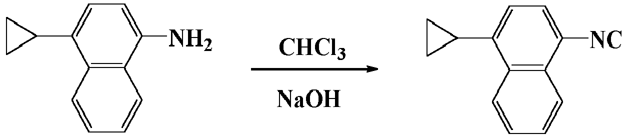

Despite their satisfactory yields (50-90%), such synthesis methods generally require drastic reaction conditions (strong acidic or basic media) that limit the preparation of complex isonitriles. The polyfunctional nature of the required precursors usually generates chemoselectivity problems and/or promotes the epimerization of the chiral centers present in the precursor molecular structure. These factors complicate synthetic strategies, in many cases requiring the extensive use of protecting groups. Although they guarantee the chemoselectivity of the transformation, the use of protecting groups entails an increase in the number of steps leading to longer, more difficult and expensive synthesis.

US 2004/102608 A1 discloses peptidic copolymers, US 2003/138432 A1 refers to multifunctional prodrugs. WO 03/074005 A2 discloses polypeptides and compositions for targeting primary receptors on endothelial cells for VEGF. None of these documents disclose the compounds of the invention.

Therefore, there is still a great need for providing reagents which possess novel, tuneable properties and whose use results in crucial improvements in biologically relevant products and in synthetic products. It would furthermore be desirable to also make this technology available to users who do not have their own special laboratory equipment at their disposal or do not have any access to specialist laboratories.

The purpose of the present invention is to provide new functional isonitriles and conjugates and methods of obtaining them, as well as kits comprising the necessary tools for the preparation of the compounds of the invention.

### SUMMARY OF INVENTION

The inventors have used new methods for the synthesis of functionalized isonitriles. The isonitriles of the invention are particularly useful for the study and manipulation of biological systems and further development of new functional materials.

In a first aspect, the invention is directed at a compound of formula (**Ia**),

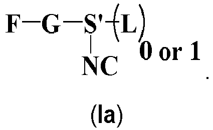

In a second aspect, the invention is directed at a compound of formula (**I**), 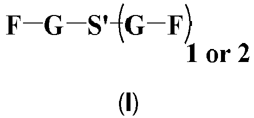 or a salt or solvate thereof.

In a third aspect, the invention is directed at a method for the preparation of a compound of formula (**Ia**), or a salt or solvate thereof.

In a fourth aspect, the invention is directed at a method for the preparation of a compound of formula (**I**), or a salt or solvate thereof.

In a fifth aspect, the invention is directed at a pharmaceutical composition comprising at least one compound of formula (**I**), or of formula (**Ia**), and a pharmaceutically acceptable excipient.

In an sixth aspect, the invention relates to the use of the compounds of the invention, preferably of compounds of formula (**Ia**), in the preparation of a compound suitable for being used in chemical sensors, diagnostic methods, bioanalytical methods, chemical tests and as chelating agents.

In an seventh aspect, the invention relates to the use of the compounds of the invention, preferably of compounds of formula (**I**), in sensors, diagnostic methods, bioanalytical methods, chemical tests and as chelating agents, wherein the use is not practiced on the human or animal body.

In a eighth aspect, the invention relates to a compound of formula (**I**), or of a compound of formula (**Ia**), or of a pharmaceutical composition as defined in aspect seven, for use as a medicament.

In an ninth aspect, the invention is addressed to the compounds of the invention, or to the pharmaceutical composition of the invention, for use in the treatment and/or prevention of a heavy metal poisoning, cardiovascular diseases, neurodevelopment disorders, immune system disorders, metabolic diseases or disorders, neurodegenerative diseases, diabetes, respiratory diseases and cancer.

The invention is also directed at the of the isonitrile compounds of the invention in the preparation of final products.

The invention is also directed at a kit for the preparation of a compound of formula (**Ia**) and (**I**), respectively.

### DETAILED DESCRIPTION OF INVENTION

The purpose of the present invention is to provide new functional isonitriles and conjugates and methods of obtaining them, as well as kits comprising the necessary tools for the preparation of the compounds of the invention. The intermediate compounds of the invention (of formula (**Ia**)) have at least one isonitrile group in their structure, which allows them to undergo isonitrile-representative reactions. The intermediate compounds of the invention are also characterized by containing functionalized chains, more specifically they contain at least one unit derived from glycine, which facilitates the conjugation (or integration) with natural structures (such as proteins or biological membranes) and allow modulating the structural parameters that condition the affinity of the resulting compounds for their biological targets. As a further advantage, the intermediate compounds of the invention may further possess other functional elements (and/or groups) such as label or reporter molecules including dyes, fluorophores or chromophores.

The present invention provides functional isonitriles and bis-isonitriles that are assembled by various reactions based on isonitrile chemistry. During the synthesis of the compounds of the invention, precursor molecules are used which can be grouped into three main categories, depending on their function in the product obtained: functional elements (F), connecting elements (G), and bis-isonitrile or tris-isonitrile spacer elements (S'). These elements can also be thought of as "residues" comprised in the compounds of the invention.

### Compounds of formula (Ia)

In a first aspect, the invention is directed at a compound of formula (**Ia**), 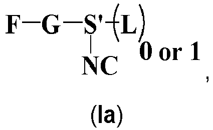
or a salt or solvate thereof,
wherein **F** and **G** are as described below,
**L,** when present, is selected from **G-F** or -NC,
wherein **S'** is a moiety of formula **(IV),** 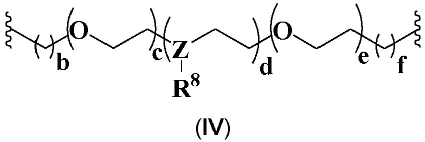 wherein,
   **b** is an integer comprised between 1 and 3;
   **c** is an integer comprised between 0 and 20;
   **d** is an integer comprised between 0 and 1;
   **e** is an integer comprised between 0 and 20;
   **f** is an integer comprised between 0 and 3;
   **Z** is selected from C, N, -C₃-C₁₀ cycloalkyl, (5- to 10-membered)-C₂₋₉ heterocyclyl, -C₆-C₁₂ aryl, or (5- to 10-membered)-C₁₋₉ heteroaryl; and
   **R⁸** = H, C₁-C₆ optionally substituted alkyl, or a moiety of formula (V) 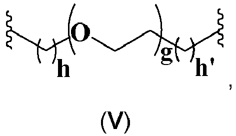 wherein,
      - **g** is selected from an integer comprised between 0 and 20; and
      - h and h' are each independently selected from an integer comprised between 0 and 3. In a particular embodiment of the compound of formula (**Ia**), the variable L is present. In a particular embodiment, when a compound of formula (**Ia**) comprises two isonitrile groups, then L is -NC and **d** is 1, where **R⁸** is a moiety of formula (**V**).

The compounds of formula (**Ia**) are particularly relevant as precursor compounds to, for example, compounds of formula (**I**).

### Exemplary compounds of formula (Ia)

Particularly preferred compounds of formula (**Ia**) are those depicted in the Example section below. The following compounds are similarly preferred compounds of formula (**Ia**) and are depicted as follows: 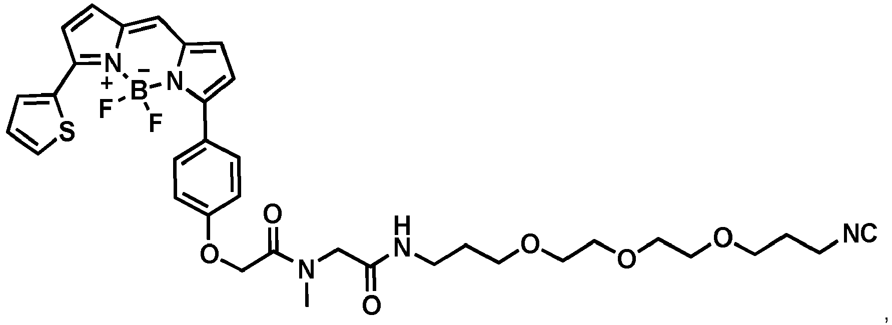 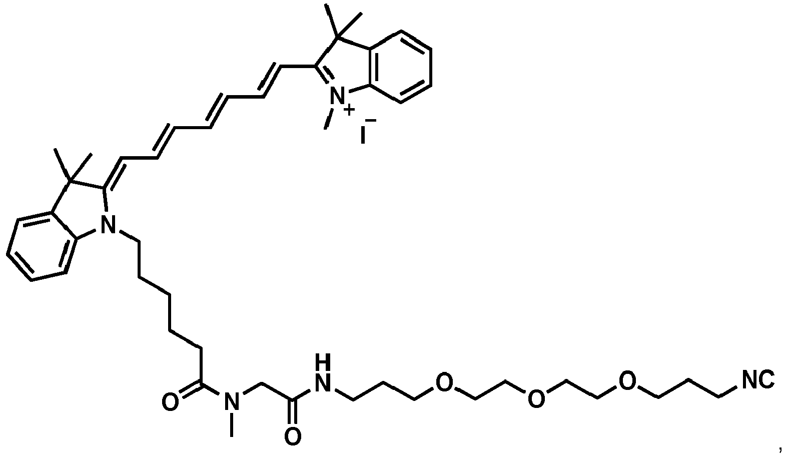 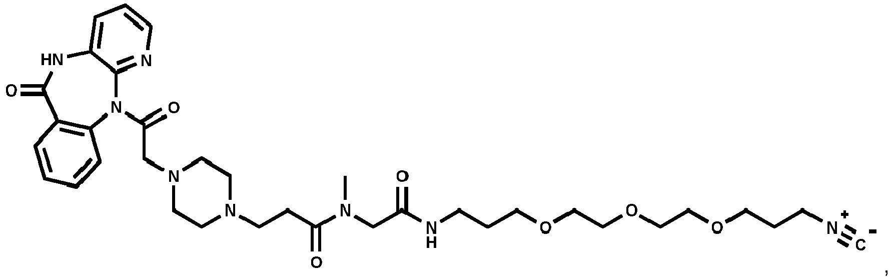 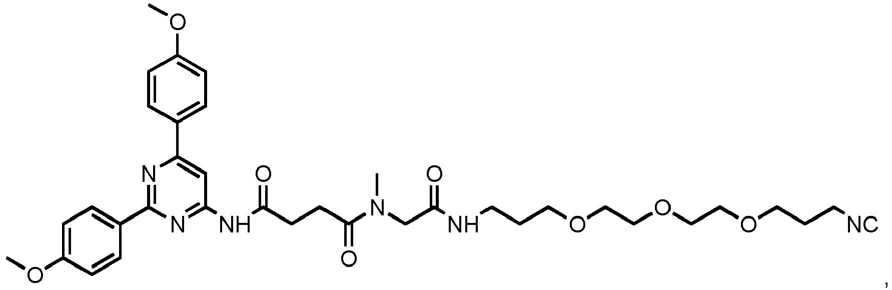 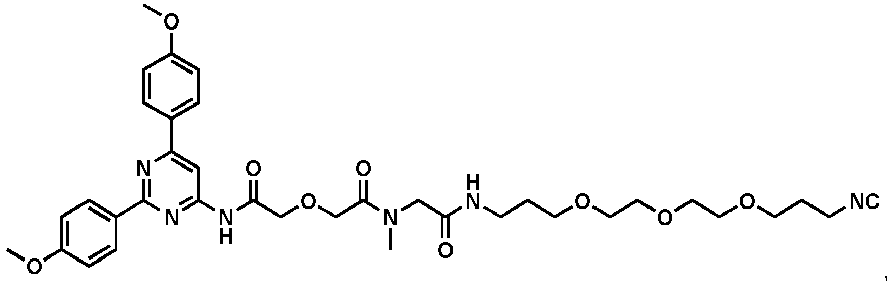 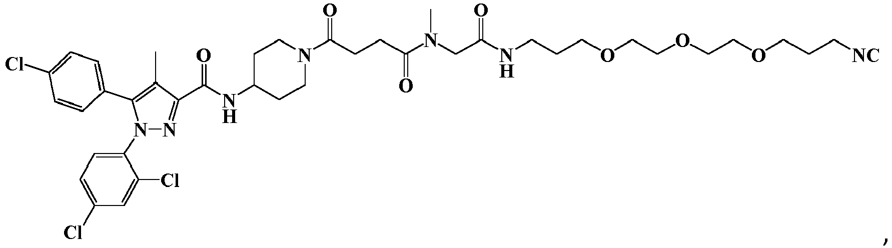 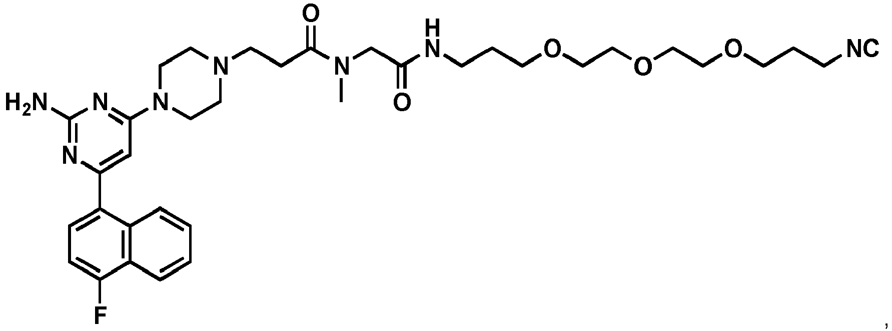 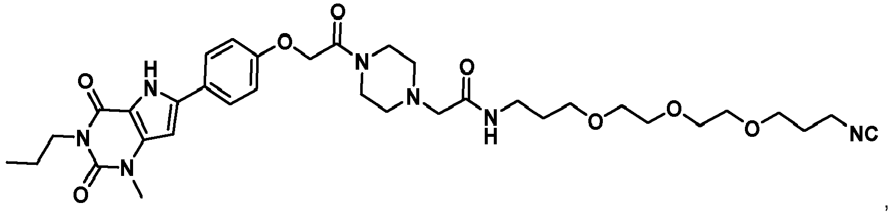 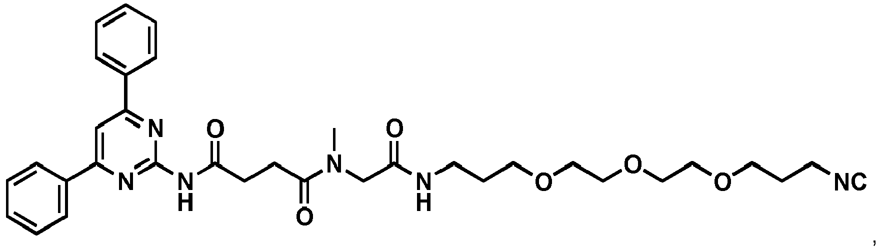 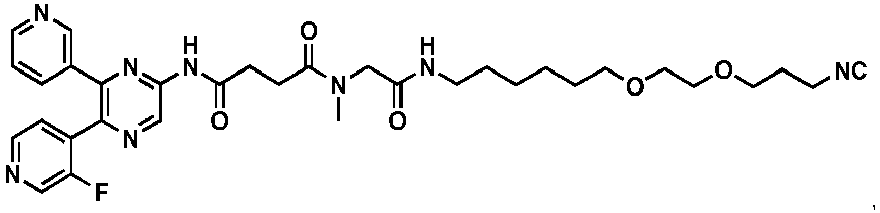 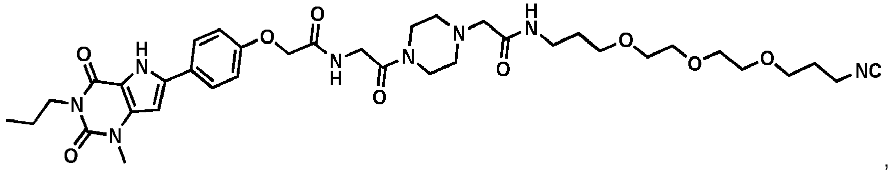 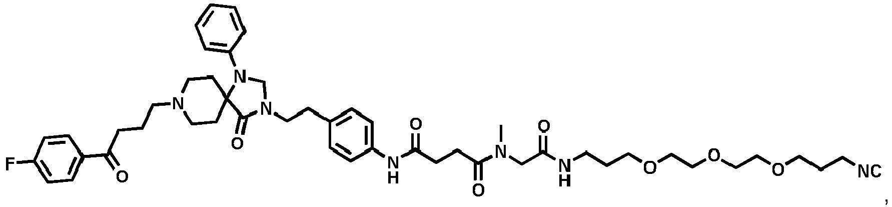 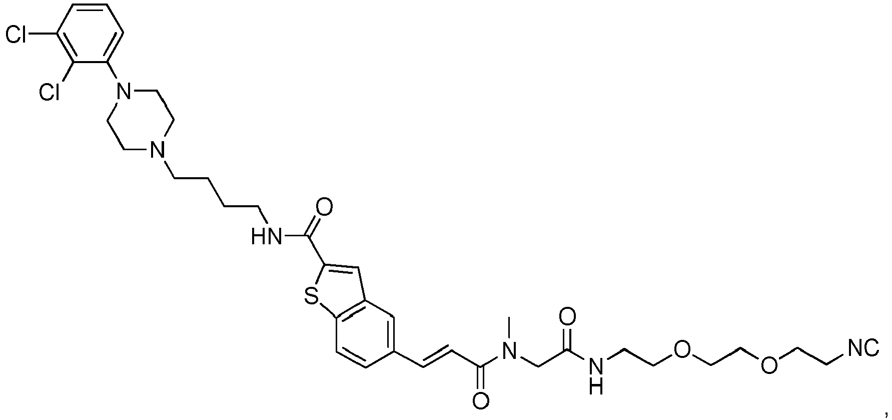 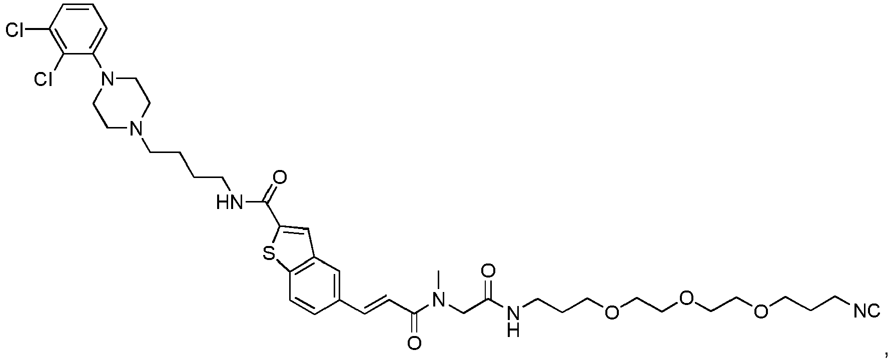 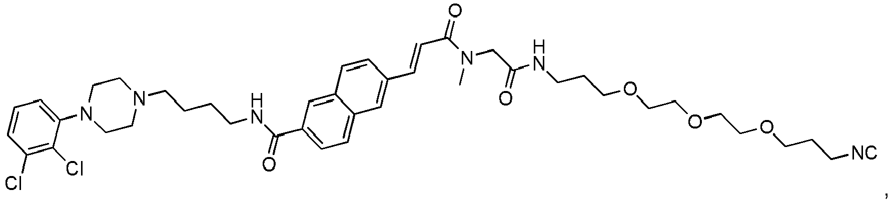 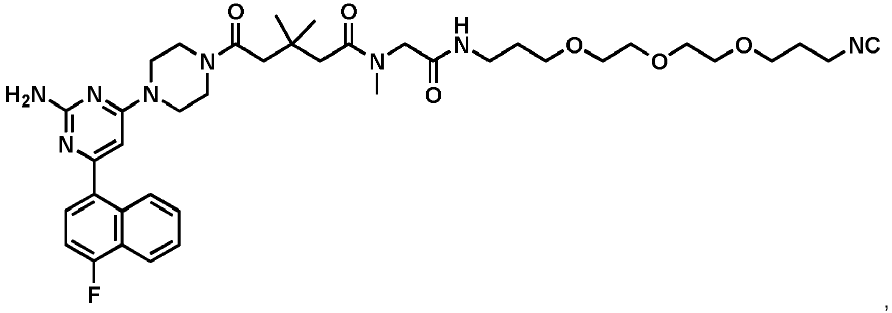 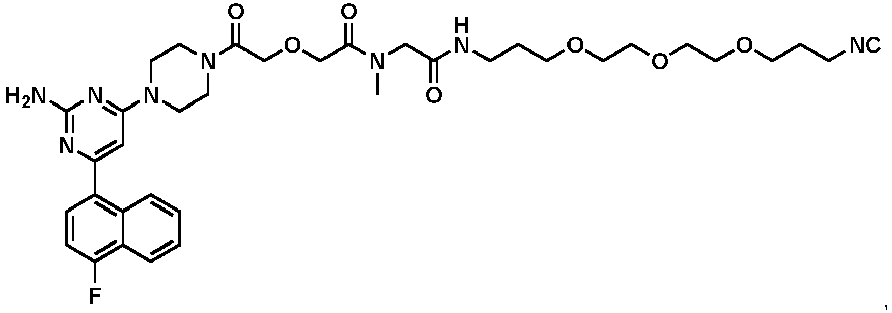 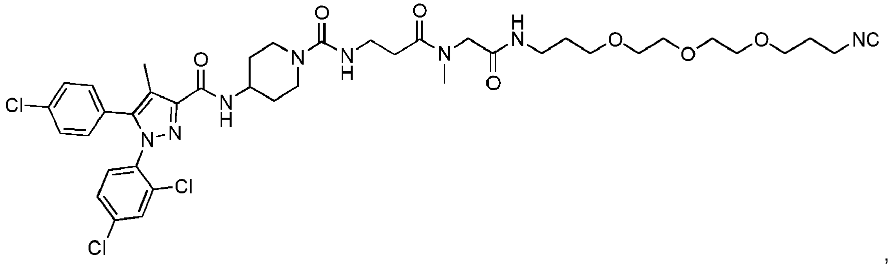 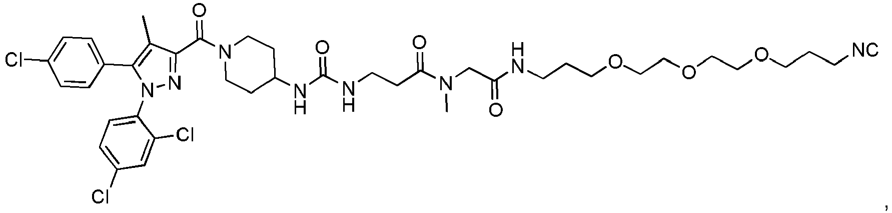 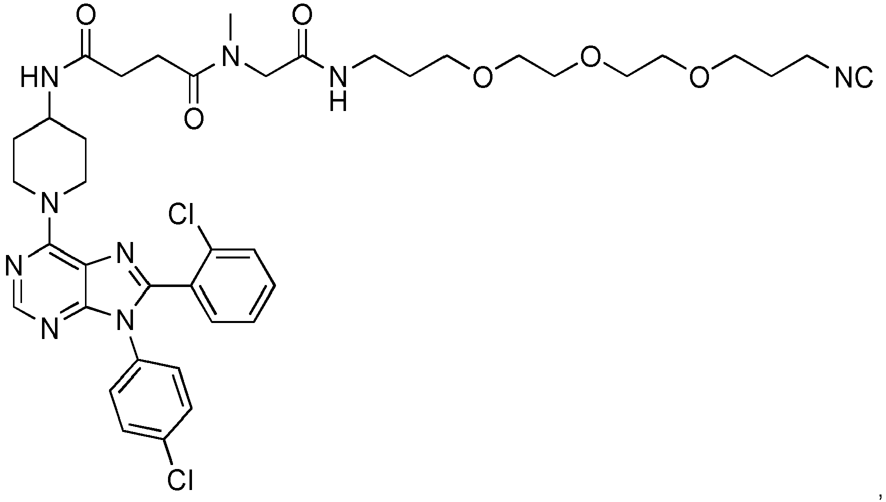 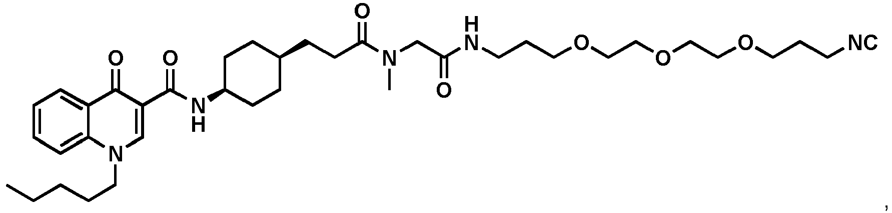 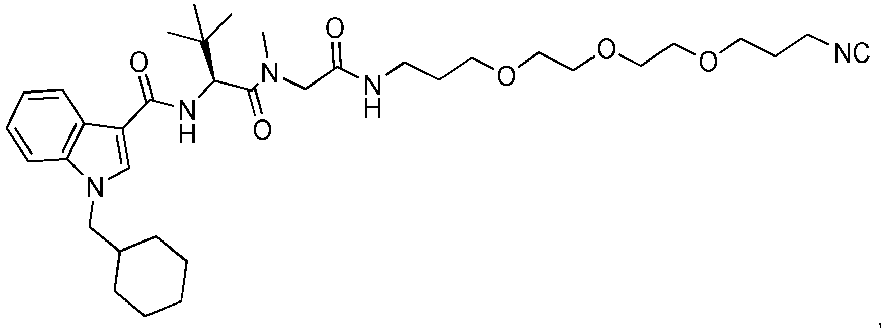 and

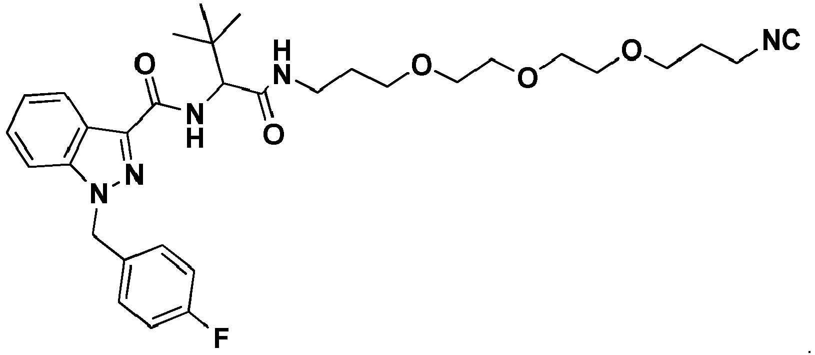

### Compounds of formula (I)

In a second aspect, the invention is directed at a compound of formula (**I**), 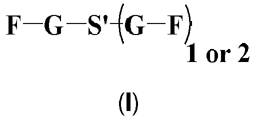 or a salt or solvate thereof.

The invention also provides "salts" of the compounds described herein. By way of illustration, said salts can be acid addition salts, base addition salts or metal salts, and can be synthesized from the parent compounds containing a basic or acid moiety by means of conventional chemical processes known by the person skilled in the art. See, generally, G. S. Paulekuhn, et al., "Trends in Active Pharmaceutical Ingredient Salt Selection based on Analysis of the Orange Book Database", J. Med. Chem., 2007, 50: 6665-72, S. M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, pyrosulfate, bisulfate, sulfite, bisulfite, nitrate, phosphate, monohydrogen-phosphate, dihydrogenphosphate, meta-phosphate, pyrophosphate, etc., organic acid addition salts such as, for example, acetate, maleate, fumarate, citrate, oxalate, succinate, tartrate, malate, mandelate, methanesulfonate, p-toluenesulfonate, camphorsulfonate, propionates, decanoates, caprylates, acrylates, formates, isobutyrates, caproates, heptanoates, propiolates, malonates, succinates, suberates, sebacates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, lactates, y-hydroxybutyrates, glycolates, propanesulfonates, naphthalene-1-sulfonates, naphthalene-2-sulfonates, etc.

Illustrative examples of base addition salts include inorganic base salts such as, for example, ammonium salts and organic base salts such as, for example, ethylenediamine, ethanolamine, *N,N*-dialkylenethanolamine, triethanolamine, glutamine, amino acid basic salts, etc. Illustrative examples of metal salts include, for example, sodium, potassium, calcium, magnesium, aluminum and lithium salts.

The compounds of the invention may be in the form of salts or solvates, preferably pharmaceutically acceptable salts or solvates.

The term "solvate" according to this invention is to be understood as meaning any form of the active compound according to the invention which has another molecule (most likely a polar solvent) attached to it via non-covalent bonding. Examples of solvates include hydrates and alcoholates. Solvation methods are generally known in the state of the art.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans.

The compounds of the invention may have asymmetric centers and therefore exist in different enantiomeric or diastereomeric forms. Thus, any given compound referred to herein is intended to represent any one of a racemate, one or more enantiomeric forms, one or more diastereomeric forms. Compounds referred to herein may also exist as atropisomers. All the stereoisomers including enantiomers, diastereoisomers and atropisomers of the compounds referred to herein, and mixtures thereof, are considered within the scope of the present invention.

### Functional element F

**F** is independently selected from a moiety comprising a photoactive or a biologically active moiety.

For the purposes of the present invention, **F** represents a "functional element" or "functional moiety" or **"F** moiety" or **"F** residue" and is to be understood as a compound or as a moiety within a larger molecule with a structure providing active properties to the compounds of the invention. Such active properties are either photo- or biologically-active properties. In the present invention, **F** is represented as a moiety in the compounds of the invention and also as a reagent. As a reagent, the functional element (**F**), prior to the reaction that leads to the compounds of the invention, contains a reactive group (typically a hydroxyl, carboxylic acid, amine or aldehyde) that allows covalent bond formation to the structure of the isonitrile or bis-isonitrile precursor. Typical functional elements **F** include pharmacophores (molecules capable of supramolecular interaction with a biological target and to trigger or block its biological response such as bioactive drugs or ligands), photoactive moieties, as well as reactive groups or structural elements that can be biologically recognized, interact/fixate on biological media or polymeric materials (organic or inorganic in nature). The pharmacophore feature of the compounds is defined by a set of chemical structure patterns having the active site of drug like molecules and includes structural features like hydrophobic, aromatic, hydrogen bond acceptor, hydrogen bond donor, negative and positive functional groups, to name a few. **F** may represent any photoactive moiety capable of being optically recognized. Examples of photoactive moieties are those found in chromophores (colourants, pigments, dyes), luminescent compounds (including fluorescent or phosphorescence) and photosensitizers, particularly useful for diagnostic purposes.

In the context of the present invention, when **F** is a photoactive moiety it can be any moiety capable of absorbing UV, visible or IR light and transforming said energy into heat or light that can be used for monitoring purposes or generating singlet oxygen transient species.

When **F** is a chromophore moiety, any chromophore molecule can be selected including colourants, dyes or pigments. Dye moiteies to be applied as labels can be prepared by a large number of methods which are already known; see for example: Venkataraman (Ed.): "The Chemistry of Synthetic Dyes", Academic Press, New York, Vol. I (1952), II (1952), III (1970), IV (1971), V (1971), VI (1972), VII (1974), VIII (1978); and Harms, 1979 in: Banks (Ed.): "Organofluorine Chemicals and their Industrial Applications", Ellis Horwood Ltd., Chichester; pp. 188-204). The following are exemplary chromophore molecules contemplated within the present invention: bilirubin, urobilin, porphyrins, chlorophyll pigments, carotenes, xanthophyll, phaeophytins, phenolphthalein, Bromophenol Blue, Alizarine Pure Blue B, Acid red 88, indigo, safranin, eosin, fuchsins, crystal violet, congo red, methylene blue, anthraquinones, coumarin, acridines, arylmethane dyes, azo dyes, quinones, indamins, indophenol dyes, oxazin and oxazone dyes, thiazine dyes, thiazole dyes, xanthene dyes and fluorene. Typical dyes include Alcian Blue 8GX, Alcian yellow GXS, Alizarin, Alizarin Red S, Alizarin yellow GG, Alizarin yellow R, Azophloxin, Bismarck brown R, Bismarck brown Y, Brilliant cresyl blue, Chrysoidine R, Chrysoidine Y, Congo red, Crystal violet, Ethyl Green, Fuchsin acid, Gentian violet, Janus green, Lissamine fast yellow, Malachite green, Martius yellow, Meldola blue, Metanil yellow, Methyl orange, Methyl red, Naphthalene black 12B, Naphthol green B, Naphthol yellow S, Orange G, Purpurin, Rose bengal, Sudan II, Titan yellow, Tropaeolin O, Tropaeolin OO, Tropaeolin OOO, Victoria blue 4R, Victoria blue B, Victoria blue R, Xylene cyanol FF.

When **F** is a fluorescent molecule, any fluorescent molecule can be selected. The following are exemplary fluorescent molecules contemplated within the present invention: xanthene derivatives such as fluorescein, rhodamine, oregon green, eosin, and texas red; cyanine derivatives such as cyanine, indocarbocyanine, oxacarbocyanine, thiacarbocyanine, and merocyanine; squaraine derivatives and ring-substituted squaraines, including seta and square dyes; squaraine rotaxane derivatives such as SeTau dyes; naphthalene derivatives (dansyl and prodan derivatives); coumarin derivatives such as Hydroxycoumarin, Aminocoumarin and Methoxycoumarin; oxadiazole derivatives such as pyridyloxazole, nitrobenzoxadiazole and benzoxadiazole; anthracene derivatives such as anthraquinones, including DRAQ5, DRAQ7 and CyTRAK Orange; pyrene derivatives such as cascade blue; oxazine derivatives such as Nile red, Nile blue, cresyl violet, oxazine 170; acridine derivatives such as proflavin, acridine orange, acridine yellow; arylmethine derivatives such as auramine, crystal violet, malachite green; tetrapyrrole derivatives such as porphin, phthalocyanine, bilirubin and dipyrromethene derivatives such as BODIPY and aza-BODIPY. Other fluorescent molecules include Pacific Blue, Pacific Orange, Lucifer yellow, NBD-TMA ([2-(4-nitro-2,1,3-benzoxadiazol-7-yl)aminoethyl]trimethylammonium), R-Phycoerythrin, PE-Cy5 conjugates, PE-Cy7 conjugates, Red 613, PerCP, TruRed, FluorX, BODIPY-FL, G-Dye100, G-Dye200, G-Dye300, G-Dye400, Cy2, Cy3, Cy3B, Cy3.5, Cy5, Cy5.5, Cy7, TRITC, X-Rhodamine, Lissamine Rhodamine B, Allophycocyanin and APC-Cy7 conjugates. As for fluorescent dyes based on nuclei acids, these include Hoechst 33342, DAPI, Hoechst 33258, SYTOX Blue, Chromomycin A3, Mithramycin, YOYO-1, Ethidium Bromide, Acridine Orange, SYTOX Green, TOTO-1, TO-PRO-1, TO-PRO: Cyanine Monomer, Thiazole Orange, CyTRAK Orange, Propidium Iodide, LDS 751, 7-AAD, SYTOX Orange, TOTO-3, TO-PRO-3, DRAQ5 and DRAQ7. Fluorescent molecules of particular interest for cellular probing are Indo-1, Fluo-3, Fluo-4, DCFH, DHR and SNARF. Fluorecent proteins are also contemplated as F fluorescent groups for the purposes of the invention.

Another example of **F** functional elements are photosensitizers, particularly useful in photodynamic therapy, including transition metal complexes (Jie Yange, et al., Two photoactive Ru (II) compounds based on tetrazole ligands for photodynamic therapy, Journal of Inorganic Biochemistry, Volume 210, 2020).

As already mentioned above, **F** may also represent a biologically active moiety, as well as any biologically active or relevant moiety such as biomolecules or active pharmaceutical ingredients (APIs), particulary useful for biological assays, biosensors or for therapeutic purposes.

In a preferred embodiment, the compounds of formula (**I**) and/or (**Ia**) comprise at least one **F** moiety which is a biologically active moiety.

When **F** is a biologically active moiety, any molecule will be adequate as long as it is capable of binding to a particular biological target or has a particular biologically effect for example, a molecule or moiety which can bind to a functional group which is present in a target active compound, for example a binding group which is able to bind to an amino group, a thiol group, a carboxyl group, a guanidine group, a carbonyl group, a hydroxyl group, a heterocycle, in particular containing N as the heteroatom (e.g. in histidine residues), a C-nucleophilic group, a C-electrophilic group, a phosphate, a sulfate, chelates, complexes with metals, e.g. at surfaces or with radioisotopes, as well as bonds to siliconcontaining surfaces, are also possible. An API (or drug), antibiotic, a minor or major groove binder, a biotinyl residue, a streptavidin residue, an intercalating residue, an alkylating residue, a steroid, a lipid, a polyamine, a folate, folic acid, a receptor agonist or receptor antagonist, an enzyme inhibitor, a peptide, an antibody or an antibody fragment, an amino sugar, a saccharide or oligosaccharide, dexamethasone, alkylglycosides, glycosides, an antisense polymer, a succinate or aconitate are suitable examples of biologically active moieties. Naturally, the skilled person readily understands that for the purposes of obtaining a compound of formula (**I**) or (**Ia**), such biologically active compounds are moieties in the sense that they comprise at least one reactive group capable of yielding a covalent bond to the rest of the molecule of the invention. In this sense, unless stated or depicted otherwise, such biologically active compounds may require a modification to provide such reactive group.

In a particular embodiment, at least one **F** moiety is a biologically active moiety with the proviso that said at least one biologically active moiety does not consist of an amino acid sequence comprising from 1 to 3 aminoacids, preferably, from 2 to 6 aminoacids. More preferably, at least one **F** moiety is a biologically active moiety with the proviso that said at least one biologically active moiety does not consist of an amino acid sequence comprising from 2 to 12 aminoacids and even more preferably, at least one **F** moiety is a biologically active moiety with the proviso that said biologically active moiety does not consist of an amino acid sequence.

Particularly interesting adenosine antagonists are selected from the group consisting of Acefylline, Aminophylline, ATL-444 (of IUPAC name (1S,3R)-1-[2-(6-amino-9-prop-2-ynylpurin-2-yl)ethynyl]-3-methylcyclohexan-1-ol), Bamifylline, Cafedrine, Caffeine, Caffeine citrate, Cartazolate, CGS-15943 (of IUPAC name 9-chloro-2-(furan-2-yl)-[1,2,4]triazolo[1,5-c]quinazolin-5-amine), 8-Chlorotheophylline, Choline theophyllinate, 8-Cyclopentyl-1,3-dimethylxanthine, Dipropylcyclopentylxanthine, Dimethazan, Diprophylline, DMPX (3,7-dimethyl-1-propargylxanthine), Enprofylline, Etanautine, Etazolate, Fenethylline, IBMX (3-isobutyl-1-methylxanthine), Istradefylline, KF-26777 (of IUPAC name (2-(4-bromophenyl)-7,8-dihydro-4-propyl-1H-imidazo[2,1-i]purin-5(4H)-one), MRS-1706 (of IUPAC name N-(4-Acetylphenyl)-2-[4-(2,3,6,7-tetrahydro-2,6-dioxo-1,3-dipropyl-1H-purin-8-yl)phenoxy]acetamide), Paraxanthine, Pentoxifylline, 8-Phenyltheophylline, Preladenant, Propentofylline, Proxyphylline, (8R)-ethyl-4-methyl-2-(2,3,5-trichlorophenyl)-4,5,7,8-tetrahydro-1H-imidazo[2,1-i]purin-5-one (preferably known as PSB-10), Reversine, Rolofylline, 2-(Furan-2-yl)-7-[3-(4-methoxyphenyl)propyl]-7H-pyrazolo[4,3-e][1,2,4]triazolo[1,5-c]pyrimidin-5-amine (also known as SCH-442,416), SCH-58261 (of CAS number 160098-96-4), Theacrine, Theobromine, Theodrenaline, Theophylline, Tracazolate, 4-(2-{[7-Amino-2-(furan-2-yl)[1,2,4]triazolo[1,5-a][1,3,5]triazin-5-yl]amino}ethyl)phenol (also known as ZM-241,385) and derivatives therefrom.

Particularly interesting adenosine agonists are selected from the group consisting of Adenosine and phosphates thereof, regadenoson, dipyridamole, capadenoson, selodenoson, tecadenoson, GS 9667 (CVT-3619 or 2-{6-[((1R,2R)-2-hydroxycyclopentyl)-amino]purin-9-yl}(4S,5S,2R,3R)-5-[(2-fluorophenylthio)methyl]-oxolane-3,4-diol), Lexiscan, Apadenoson, Sonedenoson, Binodenoson, 2-[[6-Amino-3,5-dicyano-4-[4-(cyclopropylmethoxy)phenyl]-2-pyridinyl]thio]-acetamide (BAY 60-6583), piclidenoson, namodenoson and derivatives therefrom.

Particularly interesting dopamine antagonists are selected from the group consisting of Acepromazine, Aceprometazine, Acetophenazine, Alizapride, Amisulpride, Amoxapine, Aripiprazole, AS-8112 (of IUPAC name 5-Bromo-N-[(6R)-1-ethyl-4-methyl-1,4-diazepan-6-yl]-2-methoxy-4-(methylamino)benzamide), Asenapine, Azaperone, Benperidol, Blonanserin, Bromopride, Buspirone, Carphenazine, Chlorpromazine, Chlorprothixene, Clebopride, Clopenthixol, Clozapine, Domperidone, Droperidol, Ecopipam, Eticlopride, Fallypride, Flupentixol, Fluphenazine, Fluspirilene, Haloperidol, Iloperidone, JNJ-37822681 (of IUPAC name N-[1-(3,4-difluorobenzyl)piperidin-4-yl]-6-(trifluoromethyl)pyridazin-3-amine), Lisuride, Loxapine, Lumateperone, Lurasidone, Melperone, Mesoridazine, Methotrimeprazine, Methylergometrine, Metoclopramide, Mianserin, Molindone, Nafadotride, Nemonapride, Nortriptyline, Olanzapine, Paliperidone, Penfluridol, Perazine, Periciazine, Perospirone, Perphenazine, Pimozide, Pipamperone, Pipotiazine, Prochlorperazine, Promazine, Promethazine, Propiomazine, Quetiapine, Raclopride, Remoxipride, Risperidone, Sertindole, Spiperone, Sulpiride, Sultopride, Tetrahydropalmatine, Thiethylperazine, Thioproperazine, Thioridazine, Thiothixene, Tiapride, Trifluoperazine, Trifluperidol, Triflupromazine, YKP-1358, Yohimbine, Ziprasidone, Zotepine, Zuclopenthixol and derivatives therefrom. Particularly interesting dopamine agonists are selected from the group consisting of Amphetamine , Apomorphine , Aripiprazole , Brexpiprazole, Bromocriptine , Bupropion, Cabergoline , Cathinone, Ciladopa , Cocaine , DAR-0100A, dexmethylphenidate , Dexpramipexole, dextroamphetamine, Dihydrexidine , Dihydroergocornine, Dihydroergocryptine, Dihydroergotamine, Dihydroergotoxine, Dinapsoline , Dopexamine, Doxanthrine , Epicriptine , Ergoloid mesylate, fenoldopam, Ibopamine, Lisdexamfetamine, Lisuride , Lumateperone, Metergoline, Methamphetamine, Methylphenidate , Pergolide , Phencyclidine , Phenethylamine, Piribedil , Pramipexole , Propylnorapomorphine , p-Tyramine , Quinagolide , Quinpirole , Ropinirole , Rotigotine , Roxindole , Salvinorin A, Sumanirole and derivatives therefrom.

Particularly interesting serotonin antagonists are selected from the group consisting of Acepromazine, Agomelatine, Alosetron, Alprenolol, Alverine, Amisulpride, Amitriptyline, Amoxapine, Amperozide, APD791, Apomorphine, Aripiprazole, Aripiprazole lauroxil, Asenapine, Blonanserin, Brexpiprazole, Butriptyline, Captodiame, Carbinoxamine, Cariprazine, Cerlapirdine, Chlorpromazine, Chlorprothixene, Cilansetron, Cinitapride, Citalopram, Clomipramine, Clozapine, Cyproheptadine, Deramciclane, Desipramine, Dolasetron, Dosulepin, Dotarizine, Doxepin, Epinastine, Eplivanserin, Esmirtazapine, Etoperidone, Flibanserin, Fluoxetine, Flupentixol, Fluspirilene, Granisetron, Hydroxyzine, Idalopirdine, Iferanserin, Iloperidone, Imipramine, Iprazochrome, Ketanserin, Ketanserin, Ketotifen, Lisuride, Lorpiprazole, Loxapine, Lumateperone, Lurasidone, Lysergic acid diethylamide, Mesoridazine, Metergoline, Methdilazine, Methotrimeprazine, Methysergide, Mianserin, Minaprine, Mirtazapine, Molindone, Naftidrofuryl, Nefazodone, Nortriptyline, Olanzapine, Ondansetron, Oxatomide, Oxetorone, Paliperidone, Palonosetron, Penbutolol, Pindolol, Pipamperone, Pirlindole, Pizotifen, Promazine, Promethazine, Propiomazine, Propranolol, PRX-08066, Quetiapine, Ramosetron, Renzapride, Risperidone, Ritanserin, Sarpogrelate, Sertindole, Spiperone, Tegaserod, Thioproperazine, Thioridazine, Thiothixene, Tramadol, Trazodone, Triflupromazine, Trimipramine, Tropisetron, Vortioxetine, Wortmannin, YKP-1358, Yohimbine, Ziprasidone, Zotepine, Zuclopenthixol and derivatives therefrom.

Particularly interesting serotonin agonists are selected from the group consisting of 2,5-Dimethoxy-4-ethylthioamphetamine, 4-Bromo-2,5-dimethoxyamphetamine, 5-methoxy-N,N-dimethyltryptamine, Almotriptan, Aripiprazole, Asenapine, Brexpiprazole, Bromocriptine, Cabergoline, Cannabidiol, Cariprazine, Cinitapride, Cisapride, Dihydroergotamine, Dimethyltryptamine, Eletriptan, Eltoprazine, Ergotamine, Fenfluramine, Flibanserin, Frovatriptan, Gepirone, Lisuride, Lofexidine, Lorcaserin, Lysergic acid diethylamide, m-Chlorophenylpiperazine, Metergoline, Midomafetamine, MK-212, MMDA, Mosapride, N-(2-hydroxybenzyl)-2,5-dimethoxy-4-cyanophenylethylamine, Naluzotan, Naratriptan, Naronapride, Pergolide, Piclozotan, Prucalopride, Renzapride, Rizatriptan, Rotigotine, Serotonin, Sumatriptan, Tandospirone, TD-8954, Tetrahydrocannabivarin, Urapidil, Vabicaserin, Vilazodone, Vortioxetine, Ziprasidone, Zolmitriptan and derivatives therefrom.

Particularly interesting cannabinoid antagonists are selected from the group consisting of Ibipinabant, Otenabant, Rimonabant and derivatives therefrom, SLV319, Surinabant, Taranabant, WIN 55212-2 and derivatives therefrom.

Particularly interesting cannabinoid agonists are selected from the group consisting of dronabinol, nabilone, Sativex^{®} and derivatives therefrom.

Particularly interesting muscarinic antagonists are selected from the group consisting of (R)-Hexbutinol, 3-Quinuclidinyl benzilate, 4-DAMP, Aclidinium, Aclidinium bromide, AF-DX 250, AF-DX 384, Amisulpride, Amitriptyline, Amoxapine, Anisotropine methylbromide, AQ-RA 741, Aripiprazole, Asenapine, Atropine, Atropine, Atropine methonitrate, Atropine (D/L-Hyoscyamine), Benactyzine, Benzatropine, Benzquinamide, Benztropine, Biperiden, Bornaprine, Bromperidol, Brompheniramine, Buclizine, Bupropion, Butylscopolamine, Camylofin, Chlorphenamine (chlorpheniramine), Chlorphenoxamine, Chlorpromazine, Chlorprothixene, cis-Thiothixene, Citalopram, Clidinium, Clozapine, Cocaine, Cyamemazine (cyamepromazine), Cyclopentolate, Cycrimine, Cyproheptadine, Darifenacin, Desipramine, Desmethylcitalopram, Desmethyldesipramine, Desvenlafaxine, Dexetimide, Dicyclomine, Difemerine, Dimenhydrinate, Dimetindene, Diphenhydramine, Diphenidol, Disopyramide, Dosulepin, Doxacurium, Doxepin, Doxylamine, Emepronium, Escitalopram, Etoperidone, Femoxetine, Fesoterodine, Flavoxate, Fluoxetine, Flupentixol, Fluperlapine, Fluphenazine, Fluvoxamine, Gallamine triethiodide, Glycopyrronium, Haloperidol, Hexahydrodifenidol, Hexahydrosiladifenidol, Hexocyclium, Himbacine, Homatropine, Homatropine methylbromide, Hydroxyzine, Hyoscyamine, Iloperidone, Imidafenacin, Imipramine, Ipratropium, Ipratropium bromide, Isopropamide, Lamotrigine, Lofepramine, Loxapine, Maprotiline, Mebeverine, Melperone, Mepenzolate, Mequitazine, Mesoridazine, Methantheline, Methoctramine, Methotrimeprazine, Methscopolamine bromide, Metixene, Metocurine, Mivacurium, Molindone, N-Desmethylclozapine, Nicardipine, N-Methylscopolamine, Norfluoxetine, Nortriptyline, Olanzapine, Orphenadrine, Otenzepad (AF-DX 116), Otilonium, Oxybutynin, Oxyphencyclimine, Oxyphenonium, Pancuronium, Paroxetine, Perphenazine, Pimozide, Pipecuronium, Pirenzepine, Pizotifen, Procyclidine, Profenamine, Promazine, Promethazine, Propantheline, Propiomazine, Propiverine, Quetiapine, Quinidine, Rapacuronium, Remoxipride, Revefenacin, Rilapine, Risperidone, Rocuronium, Scopolamine, Sertindole, Sertraline, Silahexacyclium, Solifenacin, Tenilapine, Terfenadine, Thioridazine, Thiothixene, Thonzylamine, Tianeptine, Timepidium, Tiospirone, Tiotropium, Tiquizium, Tolterodine, Tramadol, Trazodone, Tridihexethyl, Trifluoperazine, Triflupromazine, Trihexyphenidyl, Tripitamine (tripitramine), Tropicamide, Trospium, Umeclidinium, Venlafaxine, Zamifenacin, Ziprasidone, Zotepine and derivatives therefrom.

Particularly interesting muscarinic agonists are selected from the group consisting of Bethanechol, Cevimeline, Methacholine, NGX267, Pilocarpine, Xanomeline and derivatives therefrom.

A particularly interesting biologically active moiety is a protein-binding moiety capable of engaging an E3 ubiquitin ligase, so that if the compounds of the invention comprise at least one of such protein-binding moieties and at least a second protein-binding moiety capable of binding to a target protein meant for degradation, the resulting compound of the invention can act as a proteolysis targeting chimera (PROTAC) ligand. Non-limiting examples of E3 ligases include pVHL, Mdm2, beta-TrCP1, cereblon, c-IAP1, pomalidomide, thalidomide and lenalidomide.

As already described above, the above identified biologically active compounds may require a chemical modification to provide a reactive group capable of yielding a covalent bond to the rest of the molecule of the invention. In a preferred embodiment, a derivative of such biologically active moiety is a product resulting from a chemical modification to said biologically active compound.

For the purposes of the present invention, the general term **"F"** shown in general formula (**I**) and (**Ia**) does not represent a fluorine atom "F". The functional element **"F"** is a variable representing multiple alternatives of functional elements and is not to be mistaken with the F atomic symbol, which is only used and depicted in the examples section further below. If F is represented in a specific individual molecule, it represents a fluorine atom. If **F** is represented elsewhere, it is a functional element as defined herein.

In a preferred embodiment, the compounds of the invention comprise a total of **F** moieties between 1 and 4, more preferably between 2 and 3.

In another preferred embodiment, the compounds of the invention comprise at least two **F** moieties. The skilled person will readily understand that said at least two **F** moieties may be structurally identical or different. Therefore, the invention contemplates compounds of formula (**I**) and/or (**Ia**) wherein at least two **F** moieties are either identical or different. In a particular embodiment, the compounds of formula (**I**) and/or (**Ia**) comprise at least two **F** identical moieties. In another particular embodiment, the compounds of formula (**I**) and/or (**Ia**) comprise two F identical moieties.

Preferably, the compounds of formula (**I**) and/or (**Ia**) comprise at least one occurrence of **F** which is different from any other occurrences of **F.** In other words, the compounds of the invention preferably comprise at least two different **F** moieties. In a preferred embodiment of the latter, at least one is a biologically active moiety. One example of such preferred embodiment would be a compound comprising two pharmacophore moieties, each with a different molecular structure. Another example would be a compound comprising one photoactive moiety and one pharmacophore. Another example would be a compound comprising one photoactive moiety and two pharmacophores or a compound comprising two photoactive moieties and one pharmacophore.

Even more preferably, the compounds of the invention are such that each occurrence of **F** is different from any other occurrences of **F.** In other words, all **F** moieties comprised in the compounds of the invention are different. A particularly preferred embodiment is that where the compounds of the present invention comprise two different **F** moieties. Non-limiting examples of **F** moieties are described as follows. In a preferred embodiment of the compounds of the present invention, i.e., those of formula (**I**) and (**Ia**), each occurrence of **F** is independently selected from the group consisting of formulae **F¹** to **F¹²⁰**: 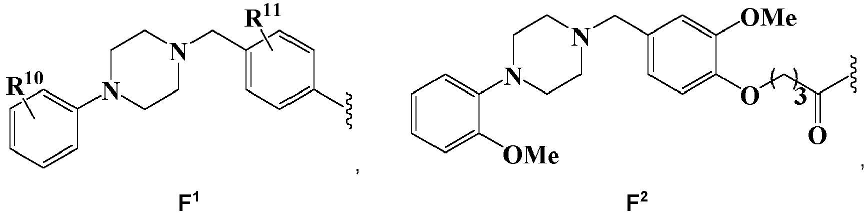 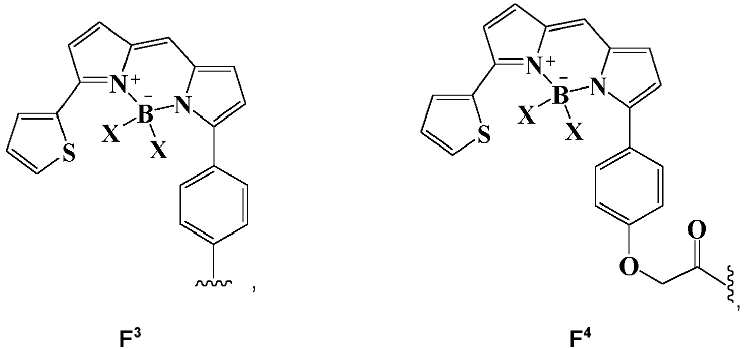 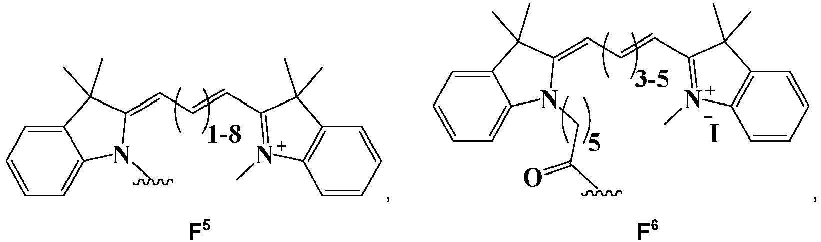 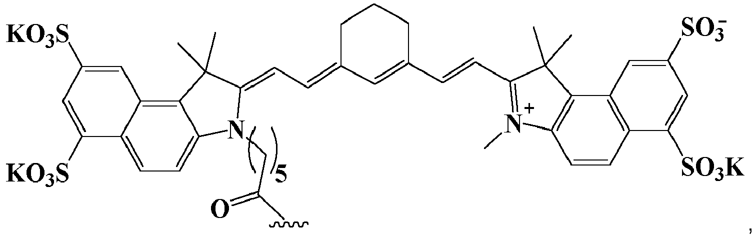 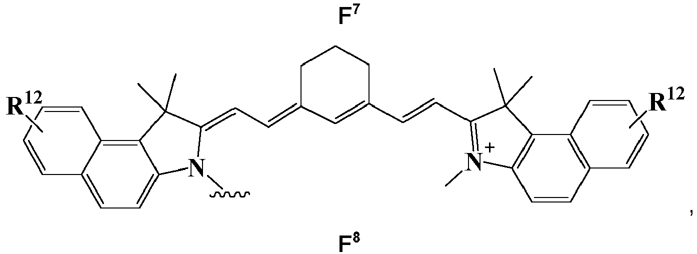 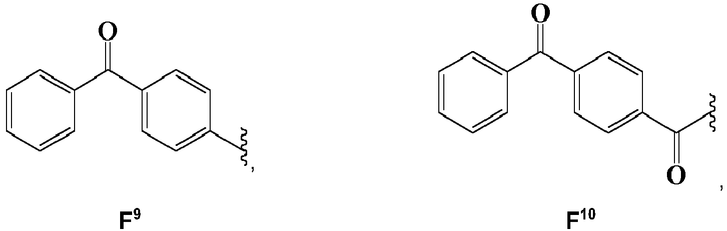 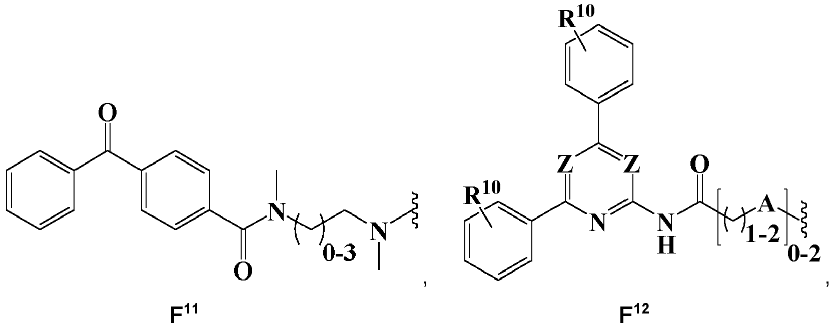 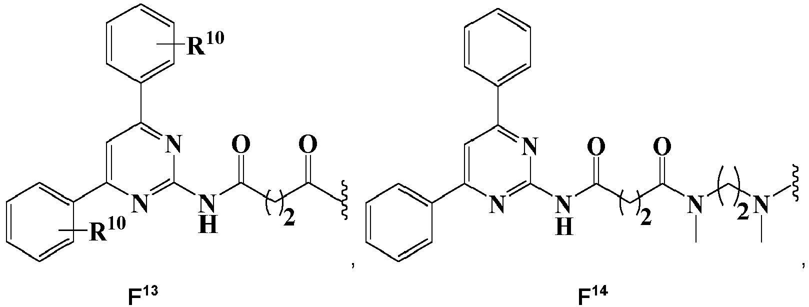 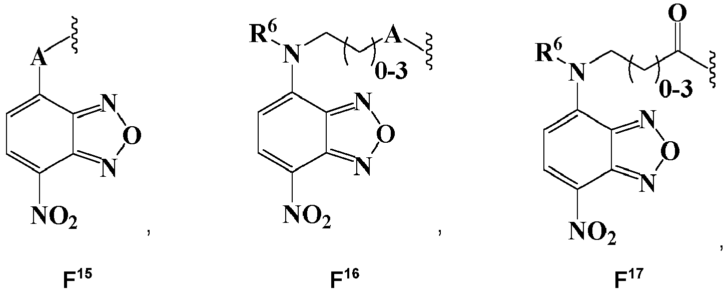 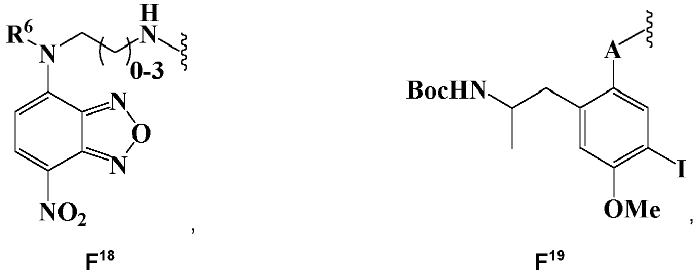 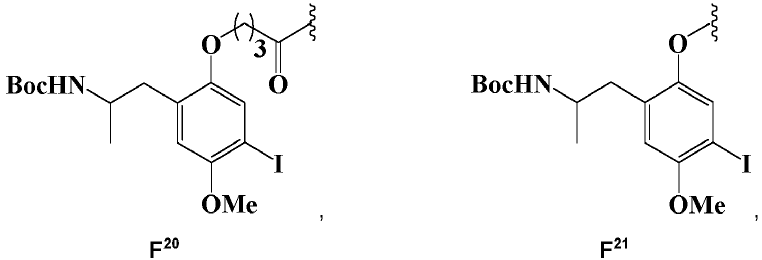 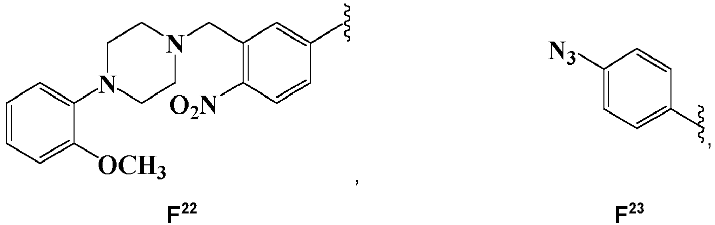 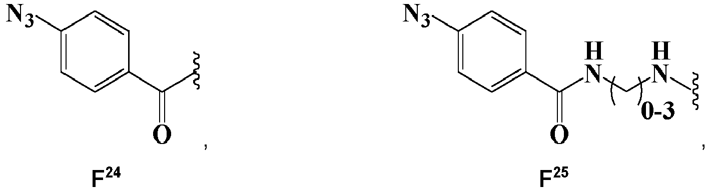 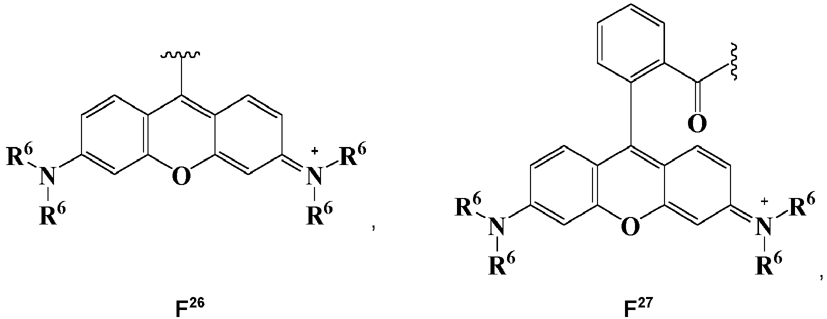 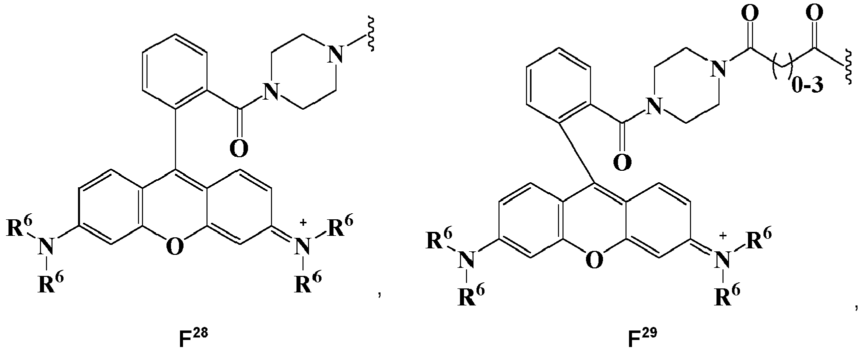 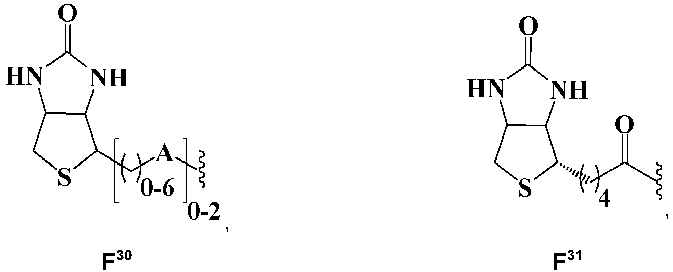 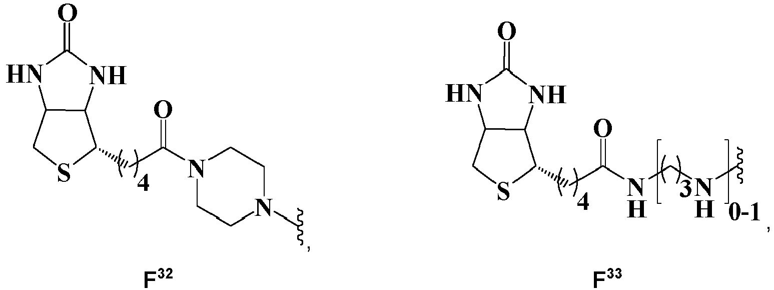 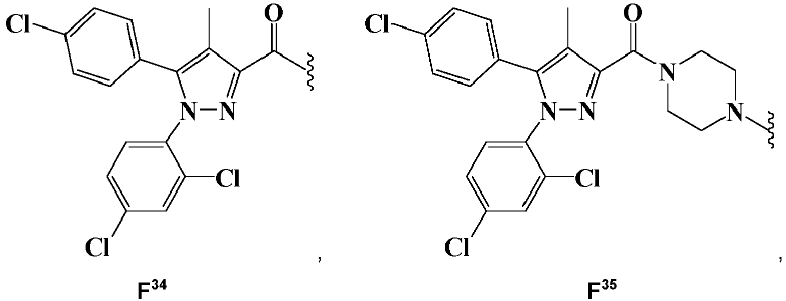 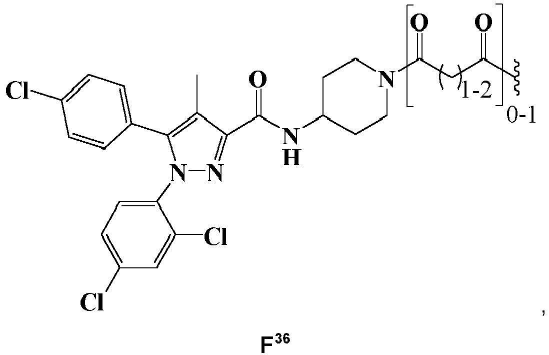 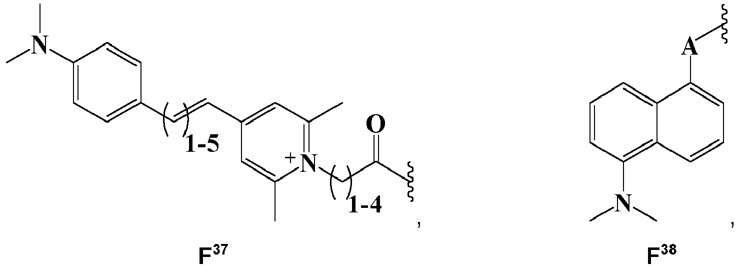 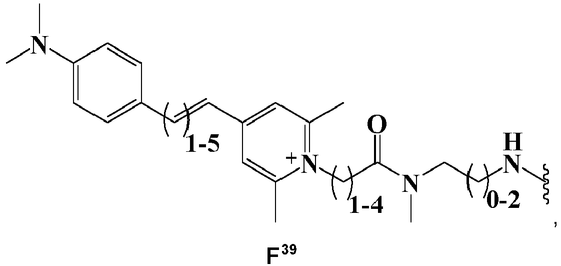 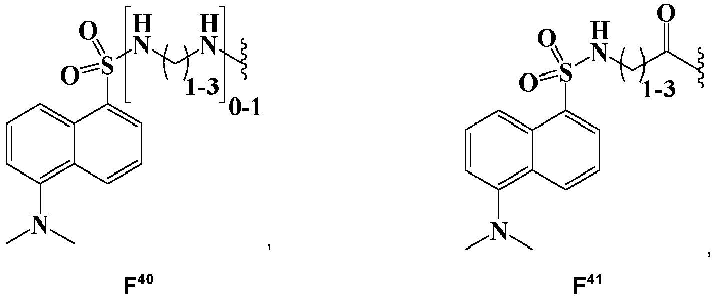 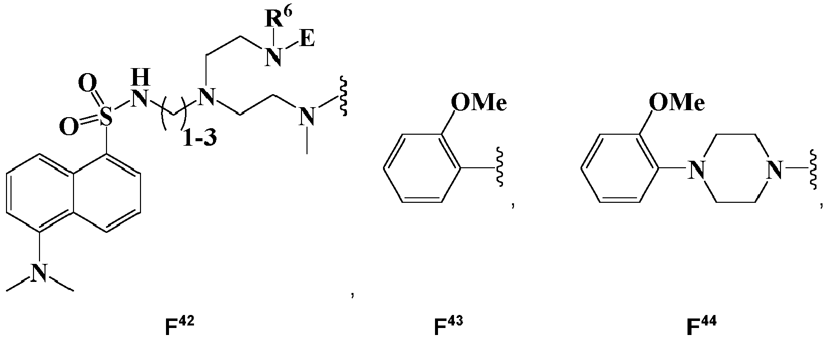 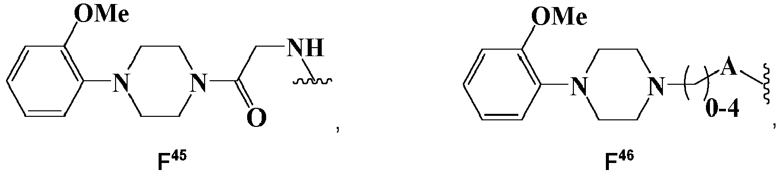 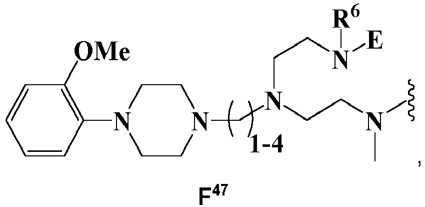 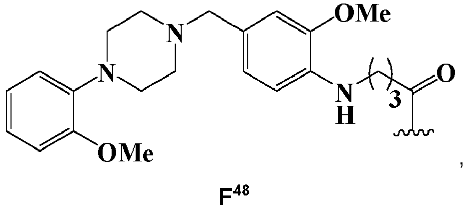 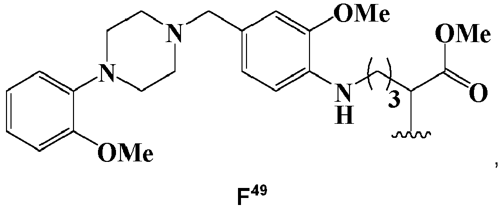 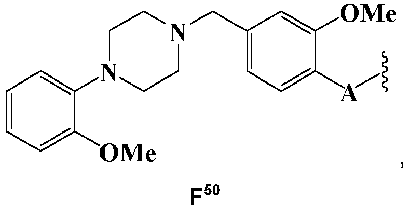 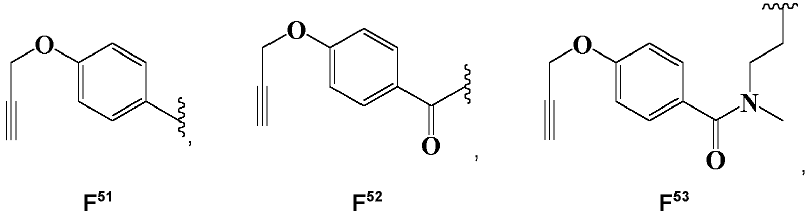 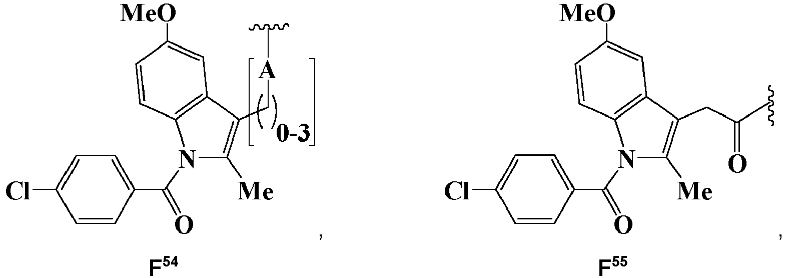 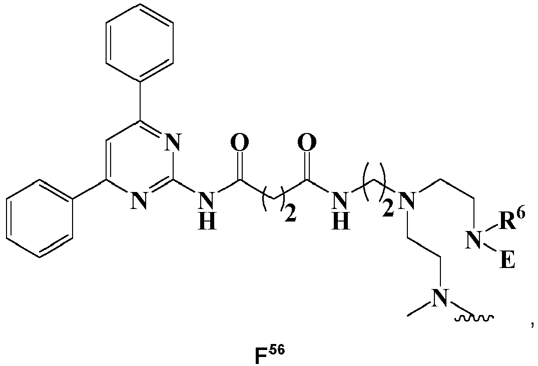 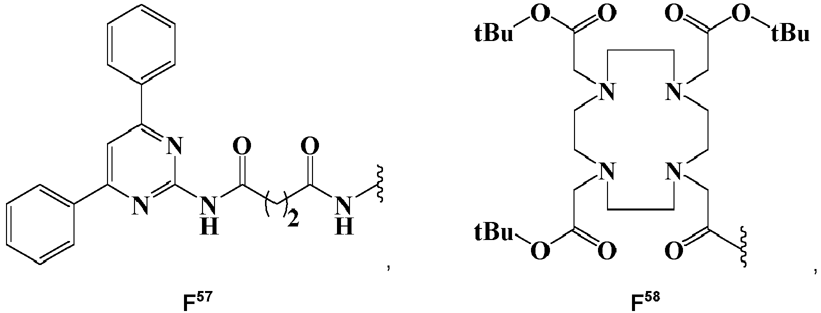 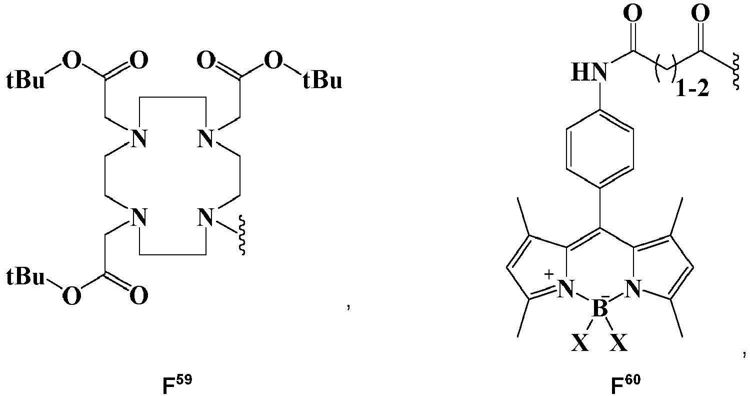 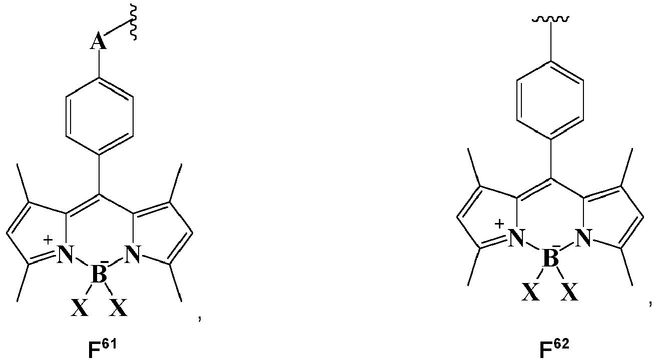 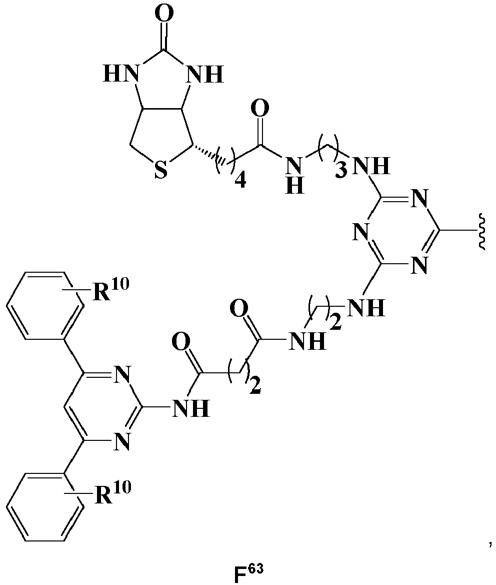 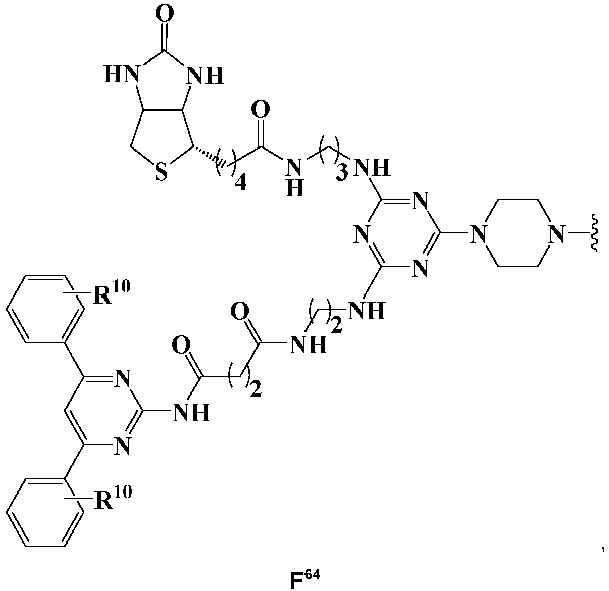 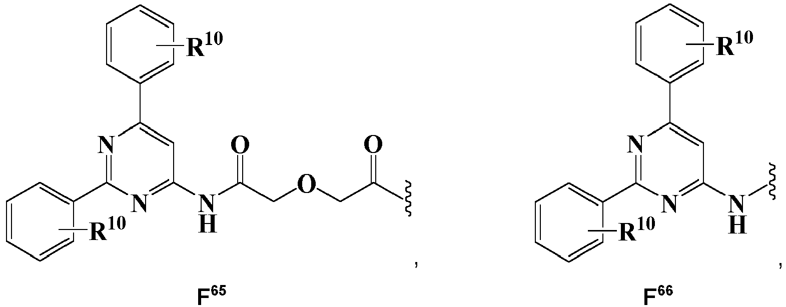 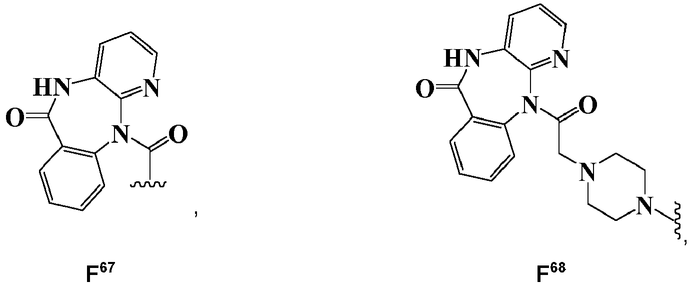 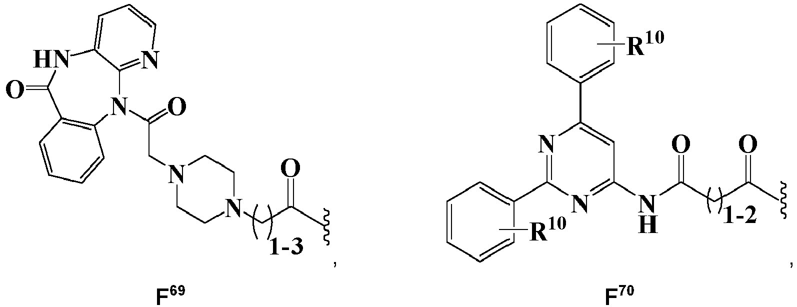 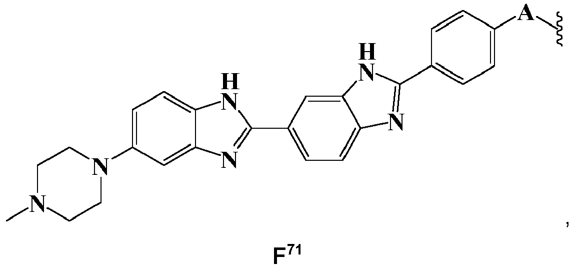 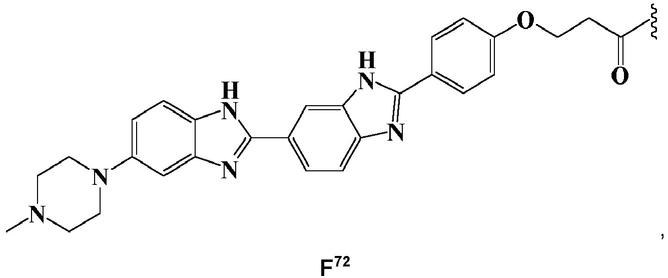 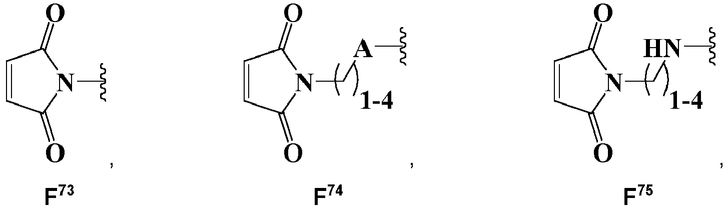 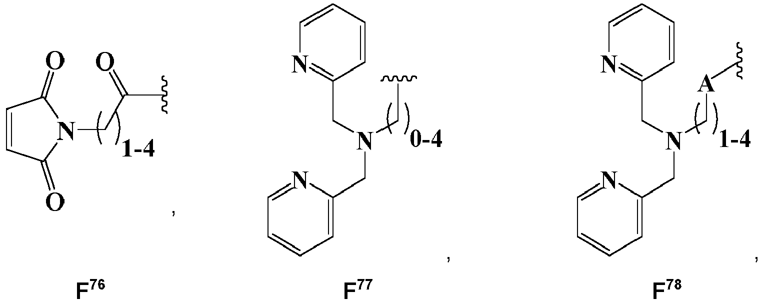 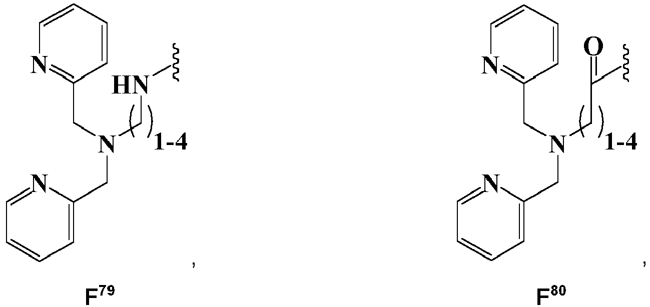 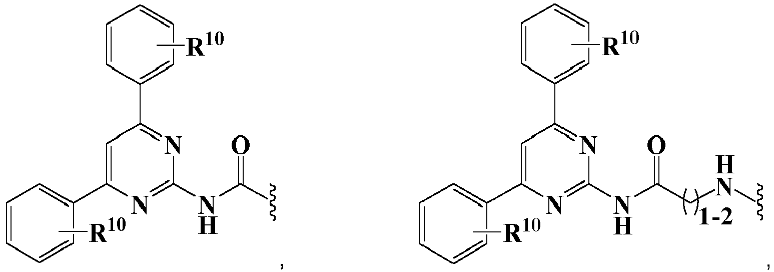 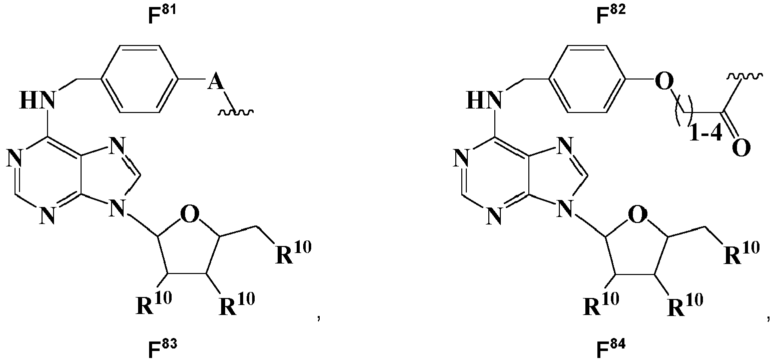 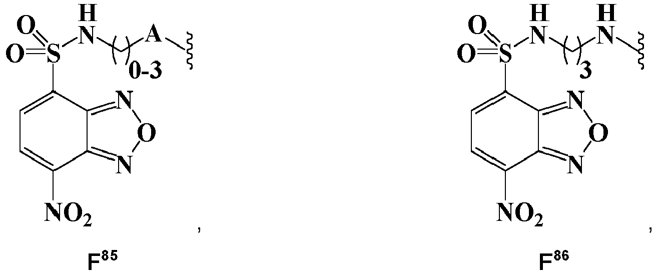 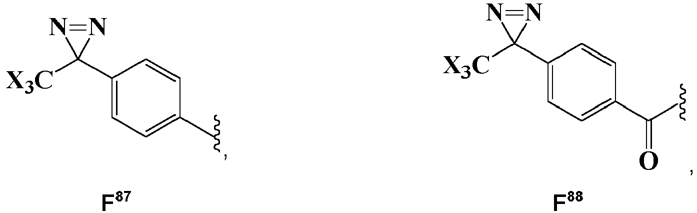 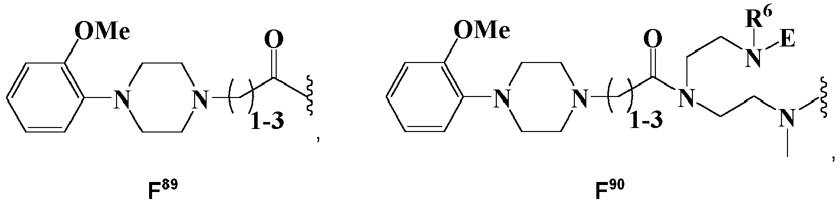 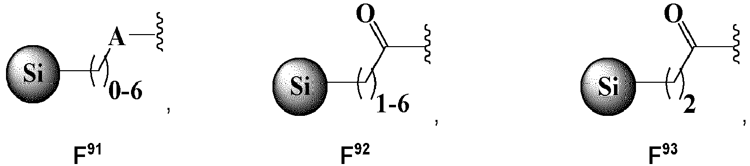 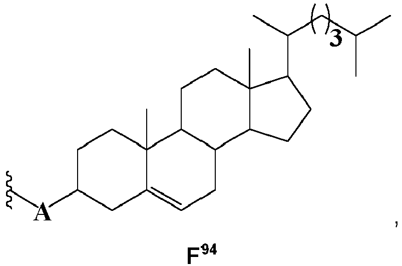 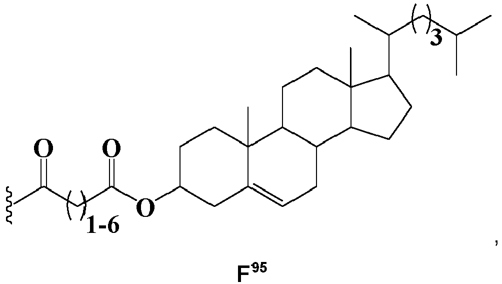 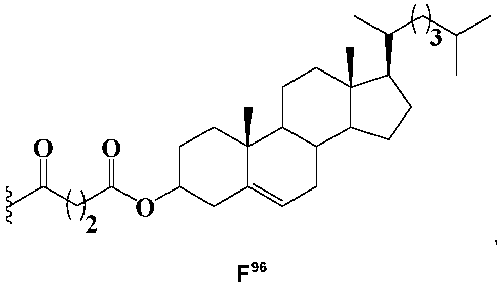 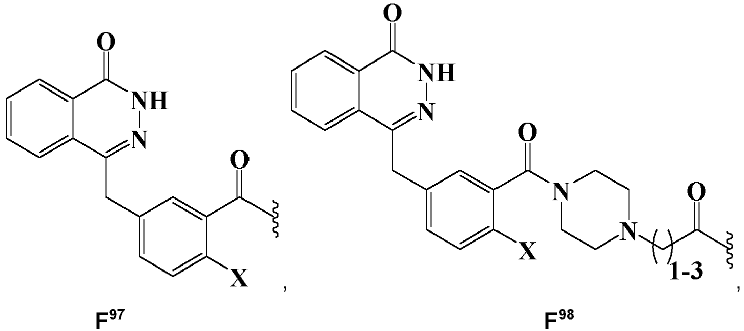 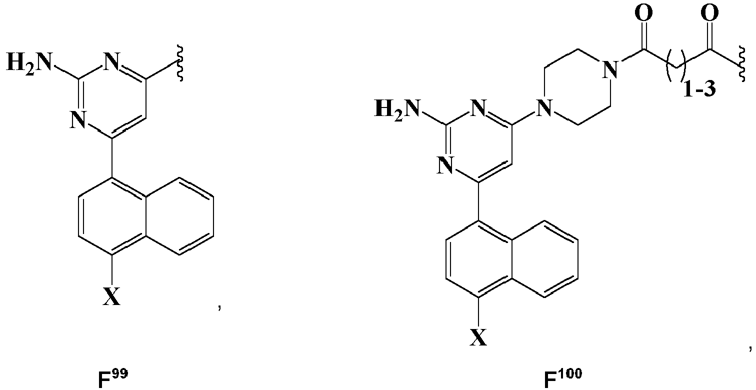 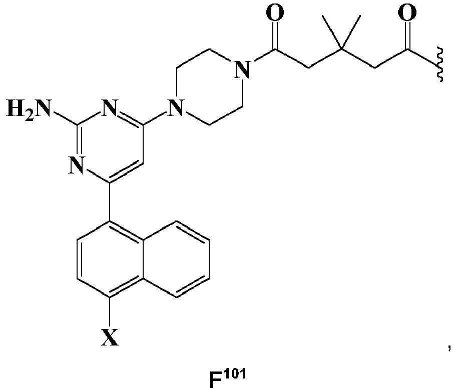 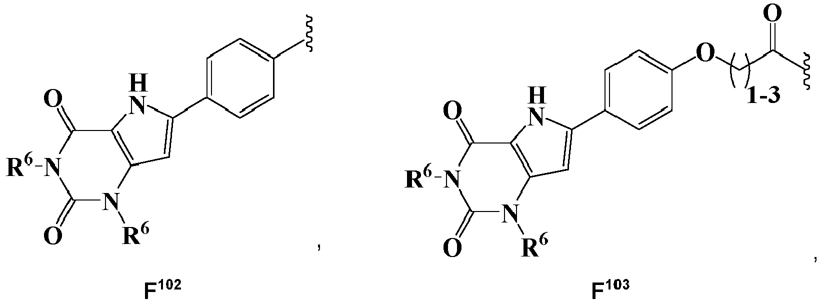 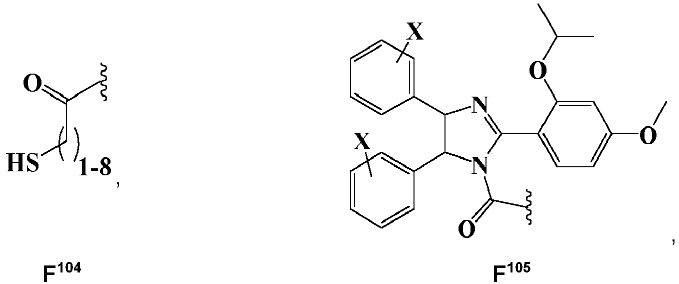 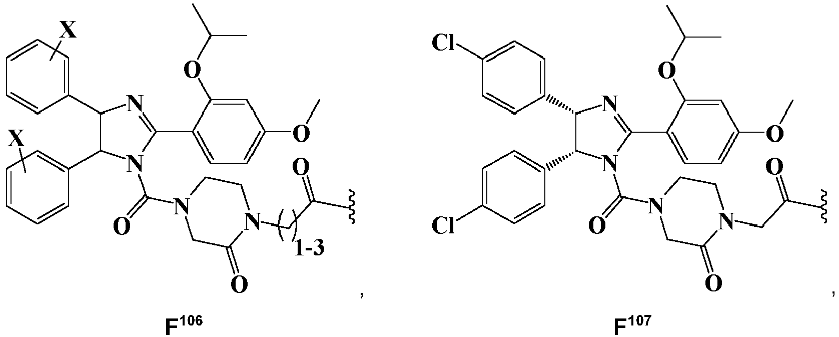 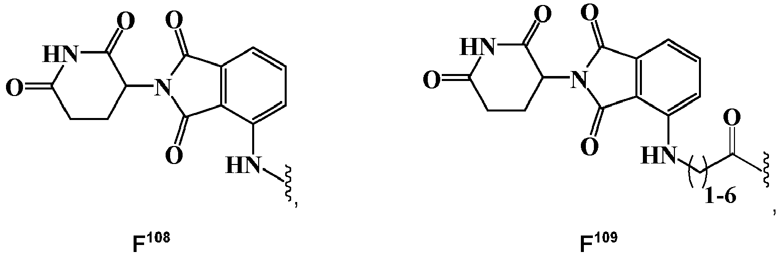 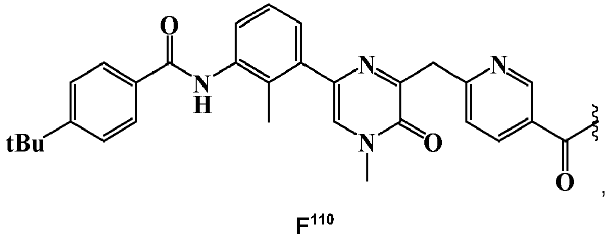 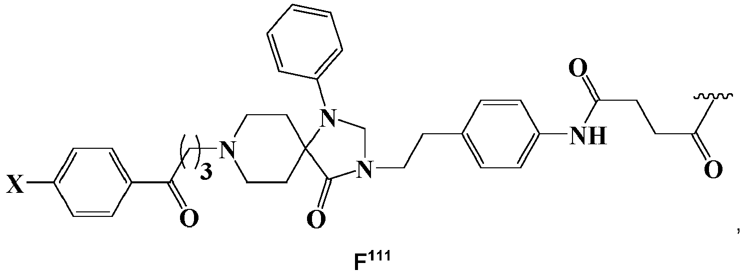 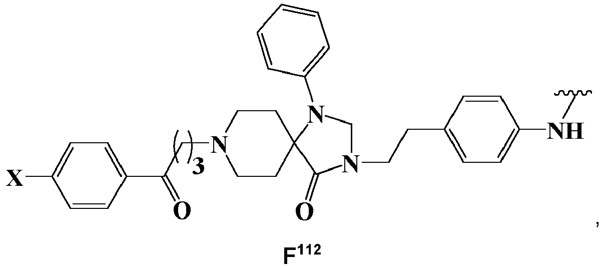 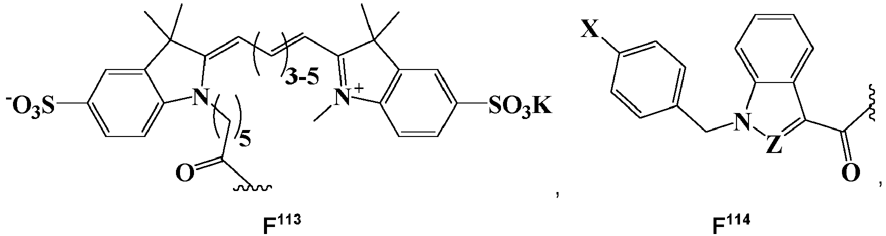 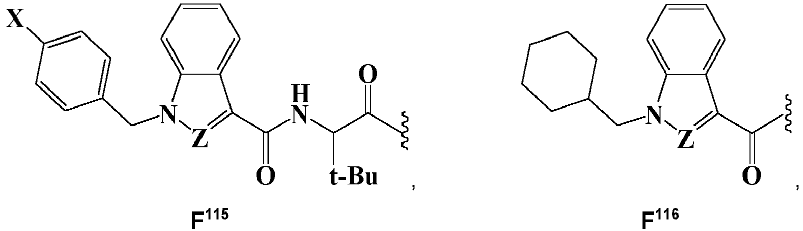 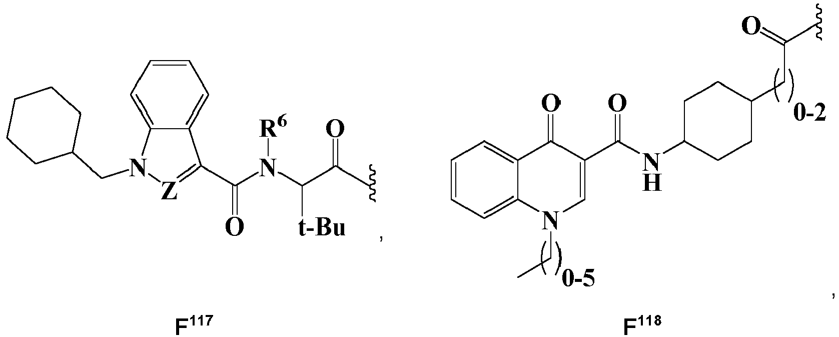 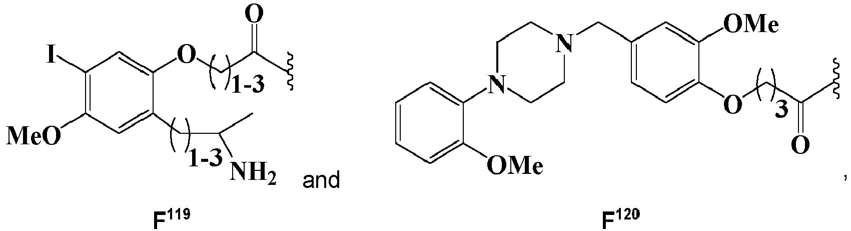 wherein
**R⁶** is as defined below, preferably selected from H, methyl or ethyl;
**R¹⁰** represents at least one substituent, each independently selected from H, -OH or - OMe;
**R¹¹** represents at least one substituent, each independently selected from H, -OMe or - NO₂;
**R¹²** represents at least one substituent, each independently selected from H or -SO₃⁻;
**X** is a halogen atom, preferably a chlorine or a fluorine atom;
**Z** is each independently selected from C or N atoms;
**A** is each independently selected from the group consisting of -O-, -OCH₂C(=O)-, - C(=O)-, -NH-, -N(CH₃)-, -C(=O)NH-, -NHC(=O)- and -S(O)₂-;
**E** is as defined below, preferably selected from H, methyl, ethyl or **-F;** and
**Si** represents any silicon-based support.

The wavy line depicted in moieties **F¹** to **F¹²⁰** represents
- the point of connection to the rest of the molecule of the compounds of formula (**I**) or (**Ia**); or,
- if **F** is a reagent in a reaction of the invention that produces the compounds of formula (**I**) or (**Ia**), then said wavy line is a connection to -H, -NH₂, -OH or -COOH. Such reagents could also be identified as a moiety **F** and at least one reactive group, wherein said at least one reactive group is preferably selected from an amine, hydroxyl, aldehyde or acid reactive group.

In the context of the present invention, silicon-based support is to be interpreted as any support (a 2D surface or a 3D particle) to which the compounds of the present invention can be attached by means of silane chemistry. Preferably, Si represents a silica-based support, a silicone-based support or a silane-based support.

### Connecting element G

For the purposes of the present invention, "connecting element" **G** is to be understood as a moiety or residue with a structure that allows covalent bonding between the functional element F and the spacer element **S'.** In the compounds of the invention, **G** is independently selected from a moiety of formula (**II**) or (**III**): 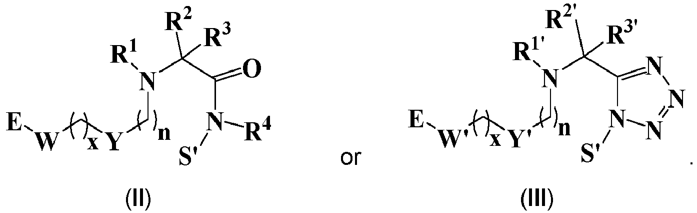 where **S'** is merely depicted to indicate the connection point to the rest of the molecule. In formula (**II**) or (**III**), each occurrence of **R¹, R^{1'}, R², R^{2'}, R³, R^{3'}** or **R⁴** is independently selected from H, -COO**R⁹**, -C(=O)H, -CS**R⁹**, -C₁₋₁₂ alkyl, -C₁₋₆ alkyl-O-C₁₋₆ alkyl, **F,** -C₁₋₁₂ alkyl-**F,** -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, - C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkylheteroaryl, heterocyclyl and alkylheterocyclyl groups are optionally substituted with 1, 2 or 3 substituents each independently selected from the group consisting of halogen, (=O), -O**R⁹**, -COO**R⁹**, -OC(=O)**R⁹**, -C(=O)H, -CN, -S**R⁹**, - N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom.

**R¹** and **R²,** or **R^{1'}** and **R^{2'},** together with the nitrogen and carbon atoms to which they are attached, can form an optionally substituted 5- to 10-membered heterocyclic ring, wherein said heterocyclic ring optionally contains 1, 2, or 3 additional heteroatoms selected from the group consisting of N, S, or O; **R¹,** or **R^{1'},** and **R⁵**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O, as long as **W** or **W'** is not a bond; and **R¹,** or **R^{1'},** and **R⁶**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O.

In a particular embodiment, **R¹** or **R^{1'}** is each independently selected from H, -COO**R⁹**, - C(=O)H, -CS**R⁹**, -C₁₋₁₂ optionally substituted alkyl, **F,** -C₁₋₁₂ optionally substituted alkyl-**F**, -C₁₋₆ alkyl-O-C₁₋₆ alkyl-**F**, -C₃₋₁₀ cycloalkyl, -C₆₋₁₂ aryl, -C₁₋₄ alkyl-C₆₋₁₀ aryl, (5- to 10-membered)-C₁₋₉ heteroaryl, (5- to 10-membered)-C₂₋₉ heterocyclyl.

In a particular embodiment, **R¹** and **R²,** or **R^{1'}** and **R^{2'},** together with the nitrogen and carbon atoms to which they are attached, can also form an optionally substituted 5- to 10-membered heterocyclic ring, wherein said heterocyclic ring optionally contains 1, 2, or 3 additional heteroatoms selected from the group consisting of N, S, or O; **R¹,** or **R^{1'},** and **R⁵**, together with the nitrogen atoms to which they are attached, can also form an optionally substituted 5- to 10-membered heterocyclic ring, wherein said heterocyclic ring optionally contains 1, 2, or 3 additional heteroatoms selected from the group consisting of N, S, or O, as long as **W or W'** is not a bond; and **R¹,** or **R^{1'},** and **R⁶**, together with the nitrogen atoms to which they are attached, can also form an optionally substituted 5- to 10-membered heterocyclic ring, wherein said heterocyclic ring optionally contains 1, 2, or 3 additional heteroatoms selected from the group consisting of N, S, or O.

In another particular embodiment, **R¹** or **R^{1'}** is each independently selected from H, - COO**R⁹**, -C(=O)H, -CS**R⁹**, -C₁-C₆ optionally substituted alkyl, -C₁₋₆ optionally substituted alkyl-F, optionally substituted 5- to 6-membered heterocyclic ring with **R²** or **R^{2'},** respectively, optionally substituted 6- to 10-membered heterocyclic ring with **R⁵** or optionally substituted 6- to 10-membered heterocyclic ring with **R⁶**.

In yet another particular embodiment, **R¹** or **R^{1'}** is each independently selected from H, - COOH, -C(=O)H or -CSH, -C₁-C₃ optionally substituted alkyl, -C₁₋₆ optionally substituted alkyl-F, optionally substituted 5- to 6-membered heterocyclic ring with **R²** or **R^{2'},** respectively, optionally substituted 6- to 10-membered heterocyclic ring with **R⁵**, or optionally substituted 6- to 10-membered heterocyclic ring with **R⁶**.

Preferably, **R¹** or **R^{1'}** is each independently selected from H, -C₁-C₃ alkyl, -C₁₋₃ alkyl-F, 5-membered heterocyclic ring with **R²** or **R^{2'},** respectively, 6- to 7-membered heterocyclic ring with **R⁵**, or optionally substituted 6- to 7-membered heterocyclic ring with **R⁶**. In a preferred embodiment, when **R¹** or **R^{1'}** forms a heterocyclic ring with **R²** or **R^{2'},** respectively, said ring is a pyrrolidine ring.

In a particular embodiment, any of **R², R^{2'}, R³ or R^{3'}** is each independently selected from H, -COO**R⁹**, -C(=O)H, -CS**R⁹**, -C₁₋₁₂ alkyl, **F,** -C₁₋₁₂ alkyl-**F,** -C₁₋₆ alkyl-O-C₁₋₆ alkyl-F, -C₃₋₁₀ cycloalkyl, -C₁₋₄ alkyl-C₃₋₁₀ cycloalkyl, -C₆₋₁₀ aryl, -C₁₋₄ alkyl-C₆₋₁₀ aryl, (5- to 6-membered)-C₁₋₅ heteroaryl, -C₁₋₄ alkyl-(5- to 6-membered)-C₁₋₅ heteroaryl, (5- to 7-membered)-C₂₋₆ heterocyclyl, or -C₁₋₄ alkyl-(5- to 7-membered)-C₂₋₆ heterocyclyl.

In another particular embodiment, any of **R², R^{2'}, R³ or R^{3'}** is each independently selected from H, -COOH, -C(=O)H, -CSH, -C₁₋₆ alkyl, **F,** -C₁₋₆ alkyl-**F**, -C₁₋₃ alkyl-O-C₁₋₃ alkyl-**F**, - C₆ aryl, -C₁₋₄ alkyl-C₆ aryl, (5- to 7-membered)-C₂₋₆ heterocyclyl, or -C₁₋₄ alkyl-(5- to 7-membered)-C₂₋₆ heterocyclyl.

In a preferred embodiment, **R²** or **R^{2'}** is each independently selected from H, -C₁-C₃ optionally substituted alkyl, -C₁₋₃ alkyl-**F**, **-F,** or 5-membered heterocyclic ring with **R¹** or **R^{1'},** respectively, preferably H, -C₁-C₃ alkyl or 5-membered heterocyclic ring with **R¹** or **R^{1'},** respectively. In a preferred embodiment, **R²** or **R^{2'}** is each independently selected from H, -C₁-C₃ unsubstituted alkyl, -C₁₋₃ unsubstituted alkyl-**F**, **or -F.**

In a preferred embodiment, **R³** or **R^{3'}** is each independently selected from H or -C₁-C₃ optionally substituted alkyl.

In a preferred embodiment, if any of **R², R^{2'}, R³** or **R^{3'}** is alkyl substituted with OH, then **F** cannot be fluorene. Fluorene is a fluorophore of CAS number 86-73-7.

In another preferred embodiment, **R²** and **R³** do not comprise an ester group at the terminal end, more preferably **R²** and **R³** do not comprise an ester group.

In another preferred embodiment, **R^{2'}** and **R^{3'}** do not comprise an ester group at the terminal end, more preferably **R^{2'}** and **R^{3'}** do not comprise an ester group.

In a preferred embodiment, **R⁴** is each independently selected H or C₁-C₆ optionally substituted alkyl, preferably H or C₁-C₃ optionally substituted alkyl.

In a preferred embodiment, when any of **R¹, R^{1'}, R², R^{2'}, R³, R^{3'}** or **R⁴** is said alkyl, cycloalkyl, alkylcycloalkyl, aryl, alkylaryl, heteroaryl, alkylheteroaryl, heterocyclyl and alkylheterocyclyl groups, these are optionally substituted with 1 to 2 substituents each independently selected from the group consisting of halogen, (=O), -O**R⁹**, -COO**R⁹**, - OC(=O)**R⁹**, -C(=O)H, -S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom.

In formulas (**II**) and (**III**), **n** and **x** are integers each independently selected from 0 to 6. In a particular embodiment, when **G** is a moiety of formula (**II**), **n** is selected from 0 to 6, particulary **n** is greater than 0, than 1, than 2, or greater than 3. Also in particular, n is lower than 6, than 5 or lower than 4.

In a particular embodiment, when **G** is a moiety of formula (**II**), x is selected from 0 to 6, particulary **x** is greater than 0, than 1, than 2, or greater than 3. Also in particular, **x** is lower than 6, than 5 or lower than 4.

In a particular embodiment, when **G** is a moiety of formula (**III**), **n** is selected from 0 to 6, particulary **n** is greater than 0, than 1, than 2, or greater than 3. Also in particular, **n** is lower than 6, than 5 or lower than 4.

In a particular embodiment, when **G** is a moiety of formula (**III**), **x** is selected from 0 to 6, particulary **x** is greater than 0, than 1, than 2, or greater than 3. Also in particular, **x** is lower than 6, than 5 or lower than 4.

In formulas (**II**) and (**III**), **Y** and **Y'** are each independently selected from single bond, - CH(COO**R⁹**)-, -C(=O)-, -O-, -N(**R⁵**)- or optionally substituted phenyl. Preferably, **Y** and **Y'** are each independently selected from single bond, -CH(COOH)-, -CH(COOMe)-, - C(=O)-, -O-, -N(**R⁵**)- or -phenyl-. More preferably, **Y** and **Y'** are each independently selected from single bond, -CH(COOH)-, -C(=O)-, -O-, -N(H)-, -N(C₁₋₁₂ alkyl)-, -N(-F)-, - N(C₁₋₁₂ alkyl-**F**)-, or-phenyl-. Said phenyl and alkyl groups are optionally substituted with 1, 2 or 3 substituents each independently selected from the group consisting of halogen, (=O), -O**R⁹**, -COO**R⁹**, -OC(=O)**R⁹**, -C(=O)H, -CN, -S**R⁹**, -N(**R⁹**)₂, -N(**R⁹**)C(=O)H, - C(=O)N(**R⁹**)₂, -ON(**R⁹**)₂, and (5- to 10-membered)-C₂₋₉ heterocyclyl with at least one N atom.

In formulas (**II**) and (**III**), **R⁵** is each independently selected from H, -COO**R⁹**, -C(=O)H, - CS**R⁹**, -C₁₋₁₂ alkyl, -C₁₋₆ alkyl-O-C₁₋₆ alkyl, **-F,** -C₁₋₁₂ alkyl-**F**, -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, -C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkylheteroaryl, heterocyclyl and alkylheterocyclyl groups are optionally substituted with 1, 2 or 3 substituents each independently selected from the group consisting of halogen, (=O), - O**R⁹**, -COO**R⁹**, -OC(=O)**R⁹**, -C(=O)H, -CN, -S**R⁹**, -N(**R⁹**)₂, -N(**R⁹**)C(=O)H, -C(=O)N(**R⁹**)₂, - ON(**R⁹**)₂, and (5- to 10-membered)-C₂₋₉ heterocyclyl with at least one N atom.

For the sake of clarity, the skilled person readily understands that **R⁵** may be selected from different groups of alternatives depending on if it is in a **G** moiety of formula (**II**) or (**III**).

In a particular embodiment, **R⁵** is each independently selected from H, -C₁₋₆ optionally substituted alkyl, **-F,** -C₁₋₆ optionally substituted alkyl-**F**, or a 6- to 7-membered heterocyclic ring with **R¹,** or **R^{1'}**.

Therefore, in a particular embodiment of the moiety formula (**II**), **R⁵** is each selected from H, -C₁₋₆ optionally substituted alkyl, -F, -C₁₋₆ optionally substituted alkyl-F or a 6- to 7-membered heterocyclic ring with **R¹**. Also, in a particular embodiment of the moiety of formula (**III**), **R⁵** is each selected from H, -C₁₋₆ optionally substituted alkyl, **-F,** -C₁₋₆ optionally substituted alkyl-F or a 6- to 7-membered heterocyclic ring with **R^{1'}**.

In formula (**II**), **W** is each independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁷**)-, -OC(=O)(**R⁷**)-, -C(=O)CH₂O-, - CH₂OC(=O)-, -C(=O)-, -O-, -C₆₋₁₂ aryl-, or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), -O**R⁹**, -COO**R⁹**, -OC(=O)**R⁹**, -C(=O)H, -CN, -S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom. Preferably, **W** is each independently selected from single bond, -N(**R⁶**)-, - N(**R⁶**)C(=O)-, -N(**R⁶**)-CH₂O-, -ON(**R⁶**)-, -C(=O)N(**R⁶**)-, -C(=O)O(**R⁷**)-, -OC(=O)(**R**⁷)-, - C(=O)CH₂O-, -CH₂OC(=O)-, -C(=O)-, -O-, -C₆ aryl-, or -(6-membered)-C₂₋₅ heterocyclyl with at least one N atom-, even more preferably single bond, -N(**R⁶**)-, -N(**R⁶**)C(=O)-, - N(**R⁶**)-CH₂O-, -ON(**R⁶**)-, -C(=O)N(**R⁶**)-, piperazine or piperidine.

In formula (**III**), **W'** is independently selected from single bond, -N(**R⁶**)-, -N(**R⁶**)C(=O)-, - N(**R⁶**)-CH₂O-, -ON(**R⁶**)-, -C(=O)N(**R⁶**)-, -C(=O)O(**R⁶**)-, -OC(=O)(**R⁶**)-, -C(=O)-, -C(=O)O-, -O-, -C₆₋₁₂ aryl- or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), - O**R⁹**, -COO**R⁹**, -OC(=O)**R⁹**, -C(=O)H, -CN, -S**R⁹**, -N(**R⁹**)₂, -N(**R⁹**)C(=O)H, -C(=O)N(**R⁹**)₂, - ON(**R⁹**)₂, and (5- to 10-membered)-C₂₋₉ heterocyclyl with at least one N atom. Preferably, **W'** is each independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁶**)-, -OC(=O)(**R⁶**)-, -C(=O)CH₂O-, - CH₂OC(=O)-, -C(=O)-, -O-, -C₆ aryl-, or -(6-membered)-C₂₋₅ heterocyclyl with at least one N atom-, even more preferably single bond, -N(**R⁶**)-, -N(**R⁶**)C(=O)-, -N(**R⁶**)-CH₂O-, - ON(**R⁶**)-, -C(=O)N(**R⁶**)-, piperazine or piperidine.

**R⁶** is independently selected from H, -C₁₋₆ alkyl or-C₆₋₁₂ aryl, wherein said alkyl and aryl groups are optionally substituted with 1, 2 or 3 substituents each independently selected from the group consisting of halogen, (=O), -O**R⁹**, -COO**R⁹**, -OC(=O)**R⁹**, -C(=O)H, -CN, - S**R⁹**, -N(**R⁹**)₂, -N(**R⁹**)C(=O)H, -C(=O)N(**R⁹**)₂, -ON(**R⁹**)₂, and -(5- to 10-membered)-C₂₋₉ heterocyclyl with at least one N atom.

For the sake of clarity, the skilled person readily understands that **R⁶** may be selected from different groups of alternatives depending on if it is in a **G** moiety of formula (**II**) or (**III**).

In a particular embodiment of a moiety of formula (**II**), **R⁶** is H, -C₁₋₆ alkyl, -C₆₋₁₂ aryl, or optionally substituted 5- to 10-membered heterocyclic ring with **R¹,** wherein said alkyl and aryl groups are optionally substituted with halogen, (=O), -OH, or -COOH.

In a particular embodiment of a moiety of formula (**III**), **R⁶** is H, -C₁₋₆ alkyl, -C₆₋₁₂ aryl, or optionally substituted 5- to 10-membered heterocyclic ring with **R^{1'},** wherein said alkyl and aryl groups are optionally substituted with halogen, (=O), -OH, or -COOH.

In another particular embodiment, **R¹** forms a ring with **R⁶**. In another particular embodiment, **R¹** forms a piperazine ring with **R⁶**. In a preferred embodiment, the piperazine ring is not substituted by a carbonyl group (=O), preferably the piperazine ring is unsubstituted.

In another particular embodiment, **R^{1'}** forms a ring with **R⁶**. In another particular embodiment, **R^{1'}** forms a piperazine ring with **R⁶**. In a preferred embodiment, the piperazine ring is not substituted by a carbonyl group (=O), preferably the piperazine ring is unsubstituted.

**R⁷** is independently selected from -C₂-C₁₂ alkyl, optionally substituted with 1, 2 or 3 substituents each independently selected from the group consisting of halogen, (=O), - O**R⁹**, -COO**R⁹**, -OC(=O)**R⁹**, -C(=O)H, -CN, -S**R⁹**, -N(**R⁹**)₂, -N(**R⁹**)C(=O)H, -C(=O)N(**R⁹**)₂, - ON(**R⁹**)₂, and -(5- to 10-membered)-C₂₋₉ heterocyclyl with at least one N atom. Preferably, **R⁷** is C₂-C₁₀ alkyl optionally substituted with halogen, (=O), -OH, or -COOH, preferably C₂-C₈ alkyl optionally substituted with halogen, (=O), -OH, or -COOH.

In a particular embodiment, **E** is each independently selected from -**R⁶** or **-F.** Preferably, **E** is each independently selected from H, C₁-C₃ alkyl **or -F.**

When **E** is an **F** moiety, then that **F** moiety is the same **F** moiety as the one depicted in formula (**I**) or (**Ia**), in other words, when **E** is an **F** moiety, **E** represents a connection of G to the rest of the molecule of formula (**I**) or (**Ia**)**.**

**R⁹** is independently selected from H or C₁-C₄ alkyl.

In view of the above particular embodiments, **G** can be a moiety of formula (**II**) or (**III**) which connects to more than one **F** functional elements. Preferably, at least one of **E, R¹, R^{1'}, R², R^{2'}** and **R⁵** comprises one **F** functional element so that **G** is connected to at least one F functional element. In a particular embodiment, at least two of **E, R¹, R^{1'}, R², R^{2'}** and **R⁵** comprise one **F** functional element so that **G** is connected to at least two **F** functional elements.

In a preferred embodiment, each occurrence of **G,** which allows covalent bonding between the functional element F and the spacer element **S',** is independently selected from the group consisting of formulae **G¹** to **G²⁷**: 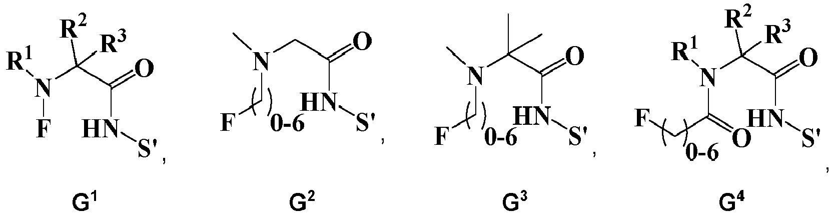 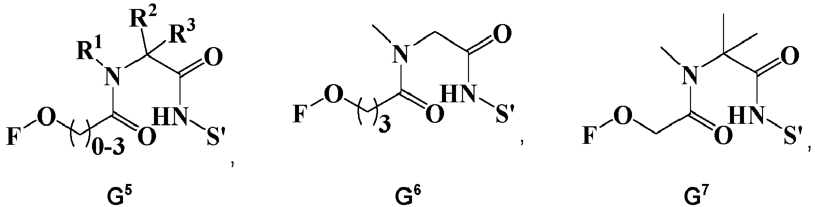 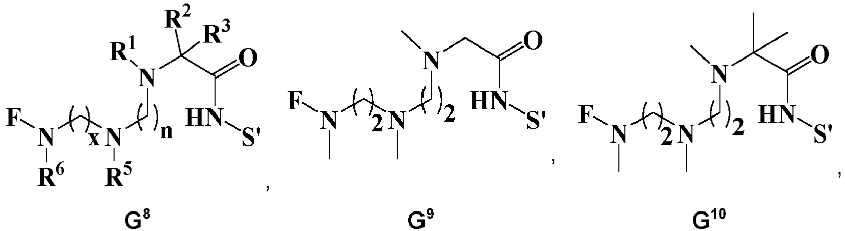 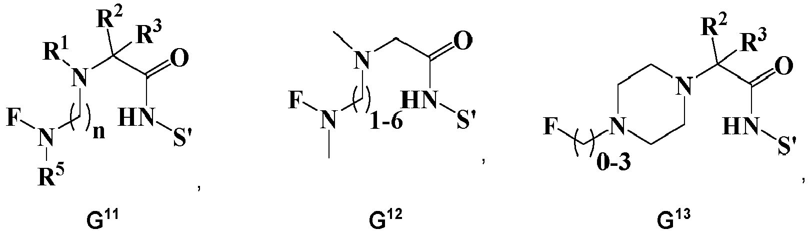 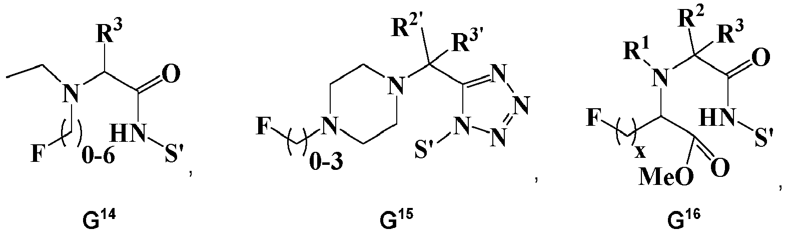 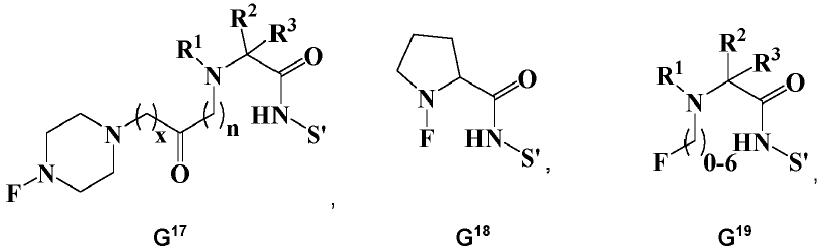 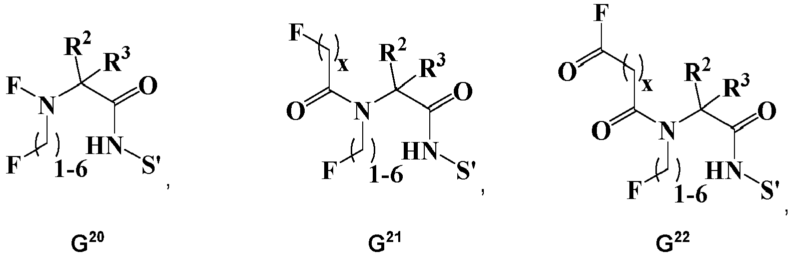 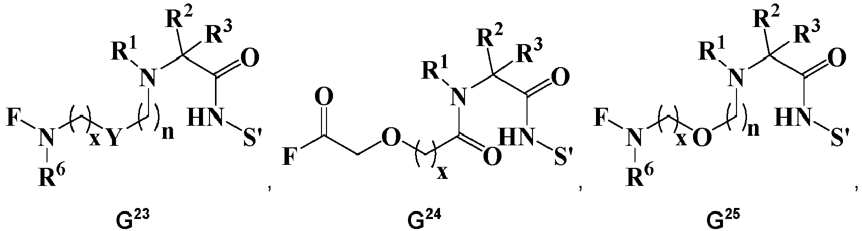 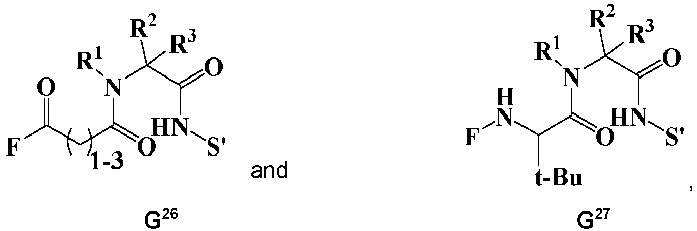 wherein,
**R¹, R³** and **R^{3'}** are as defined above, preferably each is independently selected from the group consisting of H, methyl and ethyl;
**R²** and **R^{2'}** are as defined above, preferably each is independently selected from the group consisting of H, methyl, ethyl, isopropyl and tert-butyl;
**R⁵** is as defined above, preferably each is independently selected from the group consisting of H, methyl, **-F** and -C₁₋₆ alkyl-F;
**R⁶** is as defined above, preferably each is independently selected from the group consisting of H and methyl; and
**x** and **n** are as defined above.

As in the case described above concerning the **G** moieties of formula (**II**) and (**III**), the skilled person will readily understand that in the above formulae **G¹** to **G²⁷**, the **F** and **S'** moieties represented therein are depicted with the purpose of showing the possible points of connection to the rest of the molecule of the compounds of the invention.

### Spacer element S'

In the compounds of the present invention, **S'** is a moiety of formula (IV),

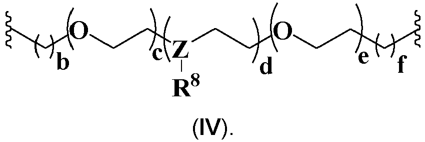

For the purposes of the present invention, **S'** represents a "spacer element", "spacer", **"S'** moiety" or **"S'** residue", and is to be understood as a linear or branched alkyl residue, optionally intercalated by oxygen atoms and/or one nitrogen atom.

The inventors have found that the spacer element affects the binding of the compounds of the invention to a given target. As shown in the examples section below, by tuning the spacer, the affinity of the compounds of the invention towards a given receptor is improved.

In the compounds of the invention of formula (**Ia**), the **S'** residue is found between at least one **G** moiety and one isonitrile group. In the compounds of the invention of formula (**I**), the **S'** residue is found between two or three **G** moieties.

In the precursor reagent compounds of the method of the invention, the **S'** residue may be found as a spacer between two or three isonitrile groups, or as a spacer between one isonitrile group and a **G** moiety i.e., the **S'** residue is found in an isonitrile, a bis-isonitrile or a tris-isonitrile reagent, respectively. In the compounds of formula (**I**) and (**Ia**), there is at least one **F** moiety attached to a **G** moiety which in turn is bonded to an **S'** moiety. Therefore, at least one isonitrile group attached to an **S'** moiety is required for bonding to the functional element **F,** via **G.**

In the **S'** moiety: **b** is an integer comprised between 1 and 3, preferably 2 or 3, and more preferably 2; **c** is an integer comprised between 0 and 20, preferably comprised between 1 and 10, more preferably between 1 and 5; **d** is an integer comprised between 0 and 1; **e** is an integer comprised between 0 and 20, preferably comprised between 1 and 10, more preferably between 1 and 5; and **f** is an integer comprised between 0 and 3, preferably between 1 and 3, preferably 2 or 3, and more preferably 2.

In a particular embodiment, **d** is 0. In another particular embodiment, **d** is 1.

In a particular embodiment, if **d** is 0, then **c** + **e** is at least 1.

In another particular embodiment, if **d** is 1, then **c** + **e** is comprised between 0 and 10, preferably between 0 and 5, more preferably 0.

In a preferred embodiment, **c** + **e** is at least 1, except when **d** is 1.

In a particular embodiment, **c** + **e** is at least 1. In a preferred embodiment, **c** + **e** is at least 2 and more preferably at least 3. In another particular embodiment, **c** + **e** is not higher than 30, preferably 20, more preferably 10, even more preferably 5.

In a particular embodiment of the **S'** moiety, **c** + **e** is 0 and:
**b** is an integer comprised between 1 and 3, preferably 2 or 3, and more preferably 2;
**c** is an integer comprised between 0 and 20, preferably comprised between 1 and 10, more preferably between 0 and 5;
**d** is an integer comprised between 0 and 1, preferably 1;
**e** is an integer comprised between 0 and 20, preferably comprised between 1 and 10, more preferably between 0 and 5; and
**f** is an integer comprised between 0 and 3, preferably between 1 and 3, preferably 2 or 3, and more preferably 2.

In the **S'** moiety, **Z** is selected from C, N, -C₃-C₁₀ cycloalkyl, (5- to 10-membered)-C₂₋₉ heterocyclyl, -C₆-C₁₂ aryl, or (5- to 10-membered)-C₁₋₉ heteroaryl. In a particular embodiment, said cycloalkyl, aryl, heteroaryl, and heterocyclyl groups are optionally substituted with 1, 2 or 3 substituents each independently selected from the group consisting of halogen, (=O), -O**R⁹**, -COO**R⁹**, -OC(=O)**R⁹**, -C(=O)H, -S**R⁹**, -N(**R⁹**)₂, - N(**R⁹**)C(=O)H, -C(=O)N(**R⁹**)₂ and -ON(**R⁹**)₂. Preferably, **Z** is selected from C or N, more preferably N.

In the **S'** moiety, if **d** is 1, then **R⁸** is selected from H, -C₁-C₆ alkyl, or a moiety of formula **(V)** 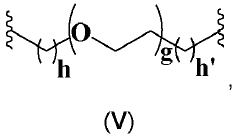 wherein said alkyl is optionally substituted with 1, 2 or 3 substituents each independently selected from the group consisting of halogen, (=O), -O**R⁹**, -COO**R⁹**, -OC(=O)**R⁹**, - C(=O)H, -CN, -S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom, preferably (=O), -O**R⁹**, -COO**R⁹**, - OC(=O)**R⁹**, -C(=O)H, -CN, -S**R⁹**, -N(**R⁹**)₂, -N(**R⁹**)C(=O)H, -C(=O)N(**R⁹**)₂ and -ON(**R⁹**)₂.

In the **S'** moiety, **g** is selected from an integer comprised between 0 and 20, preferably between 1 and 5.

In the **S'** moiety, **h and h'** are each independently selected from an integer comprised between 0 and 3. In a particular embodiment, **h** is 2. In another particular embodiment, **h'** is 2.

### Compounds excluded from the scope of the invention

The compounds of formula (**I**) are as defined above and any possible and chemically coherent combination of particular and preferred embodiments is contemplated within the scope of the invention.

However, the following conditions are excluded:
When the compounds comprise only two biologically active **F** moieties, which are identical, and **G** = formula (**II**),
**R²** = methyl, **R¹** = **R³** = **R⁴** = H, and **n** = **x** = 0, and **Y** = CO and **W** = bond;
**R²** = -CH₂COOH, **R**¹ = **R³** = **R⁴** = H, and **n** = **x** = 0, and **Y** = **W** = bond;
**R¹** forms a 2-oxopiperazine ring with **R⁵** or **R⁶**;
**R²** = diphenylmethyl, unsubstituted phenyl or CH₂-OH; or
**R²** = **R³** = **R⁴** = H, **R¹** forms a piperazine ring with **R⁶** and **F** = quinolone.

In a preferred embodiment, the following conditions are excluded when the compounds comprise only two **F** moieties, which are identical, and **G** = formula (**II**),
**R²** = methyl, **R¹** = **R³** = **R⁴** = H, and **n** = **x** = 0, and **Y** = CO and **W** = bond;
**R²** = -CH₂COOH, **R¹** = **R³** = **R⁴** = H, and **n** = **x** = 0, and **Y** = **W** = bond;
**R¹** forms a 2-oxopiperazine ring with **R⁵** or **R⁶**;
**R²** = diphenylmethyl, unsubstituted phenyl or CH₂-OH; or
**R²** = **R³** = **R⁴** = H, **R¹** forms a piperazine ring with **R⁶** and **F** = quinolone.

In a preferred embodiment, the following conditions are excluded when the compounds comprise at least two identical **F** moieties, and **G** = formula (**II**),
**R²** = methyl, **R¹** = **R³** = **R⁴** = H, and **n** = **x** = 0, and **Y** = CO and **W** = bond;
**R²** = -CH₂COOH, **R¹ = R³** = **R⁴** = H, and **n** = **x** = 0, and **Y** = **W** = bond;
**R¹** forms a 2-oxopiperazine ring with **R⁵** or **R⁶**;
**R²** = diphenylmethyl, unsubstituted phenyl or CH₂-OH; or
**R²** = **R³** = **R⁴** = H, **R¹** forms a piperazine ring with **R⁶** and F = quinolone.

### Exemplary compounds of formula (I)

Particularly preferred compounds of formula (**I**) are those depicted in the Example section below. The following compounds are also preferred compounds of formula (**I**) and are depicted as follows: 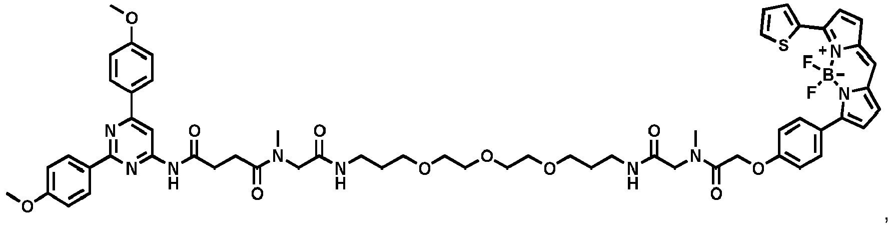 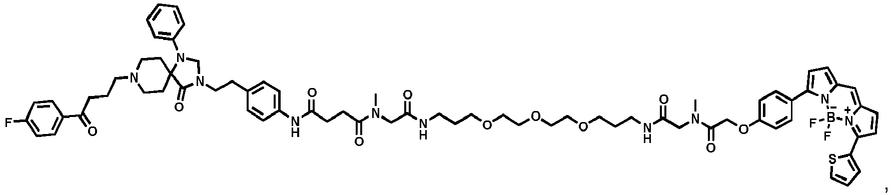 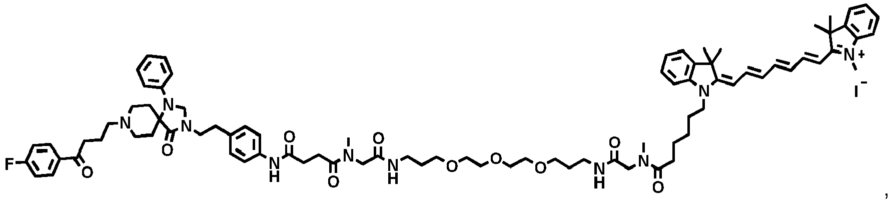 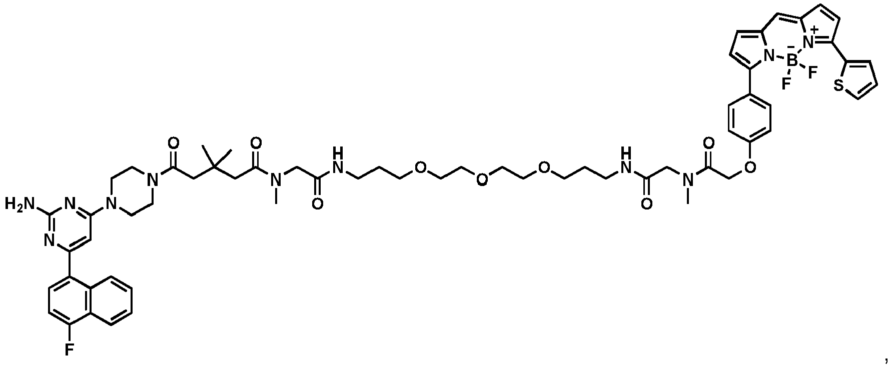 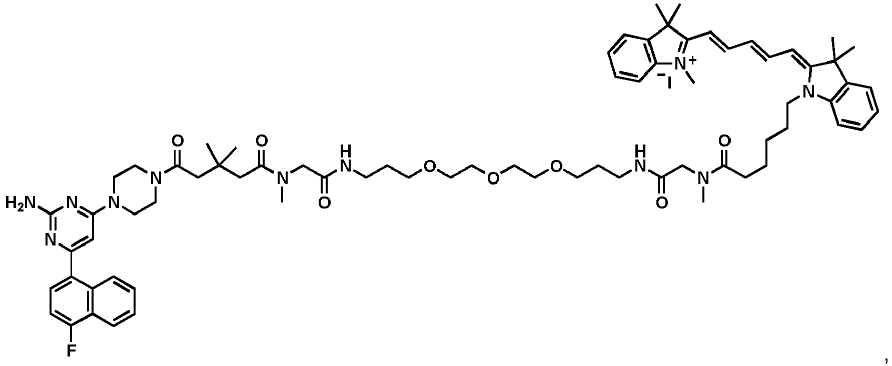 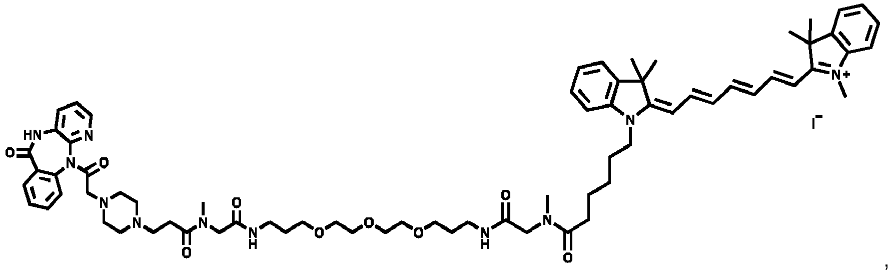 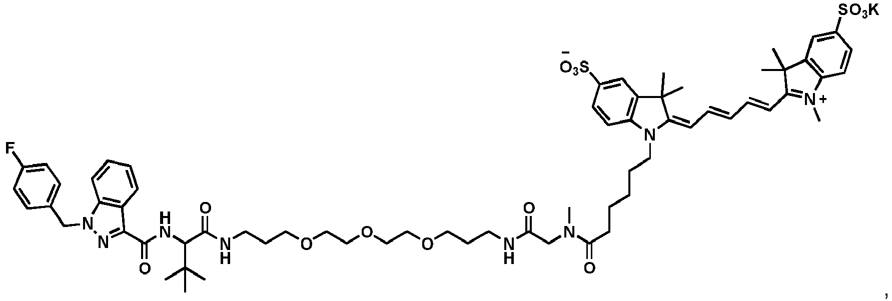 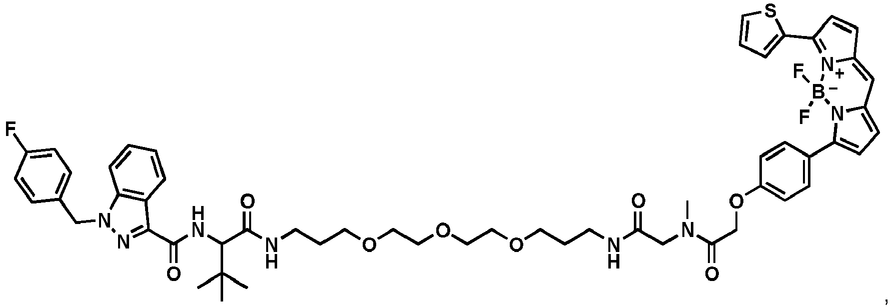 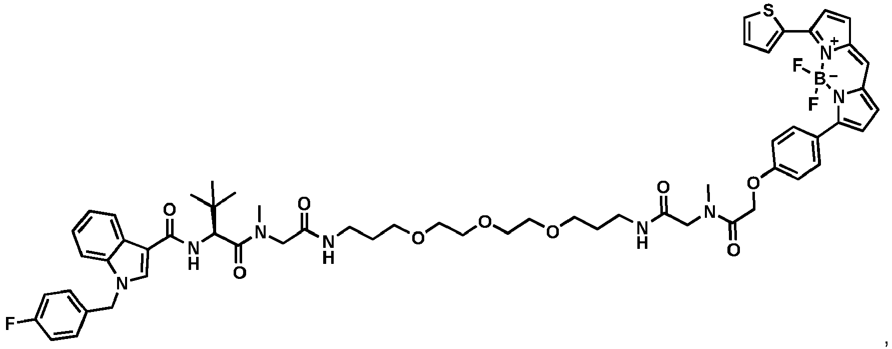 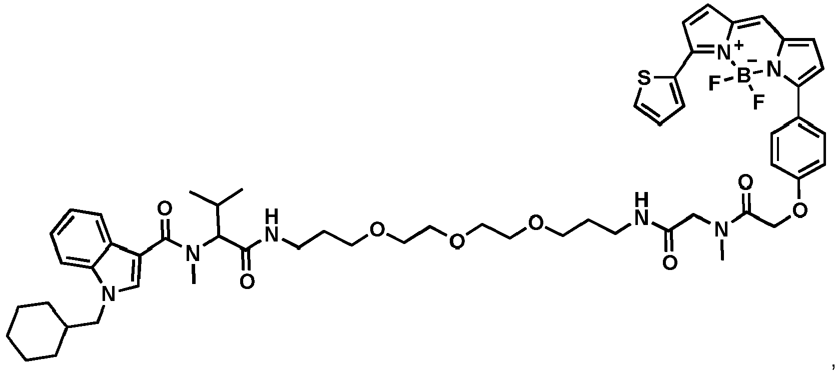 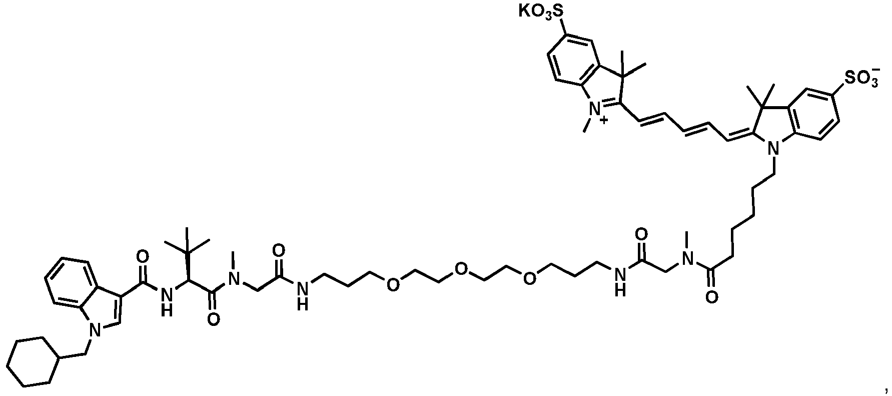 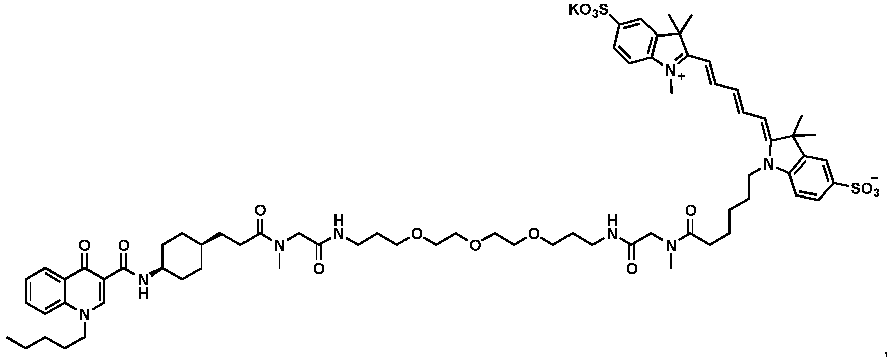 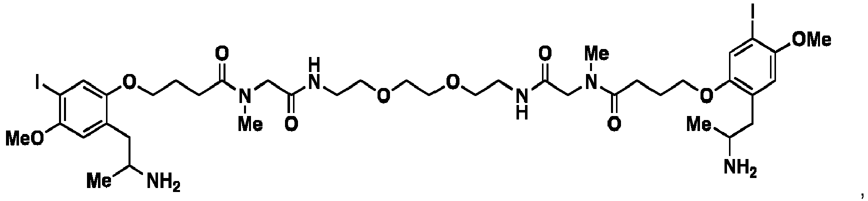 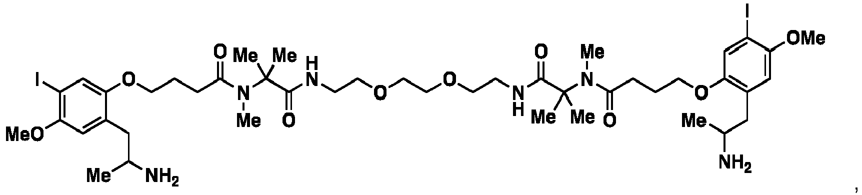 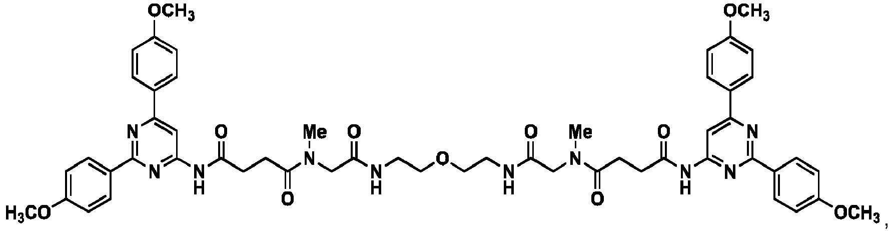 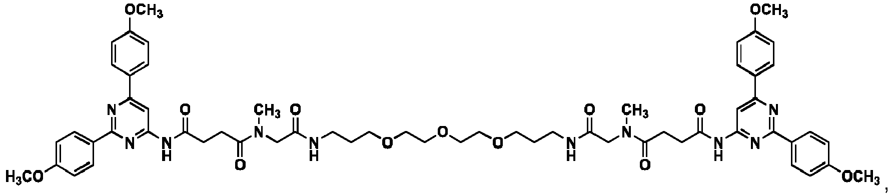 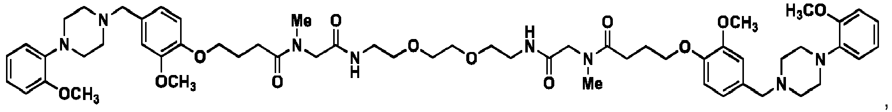 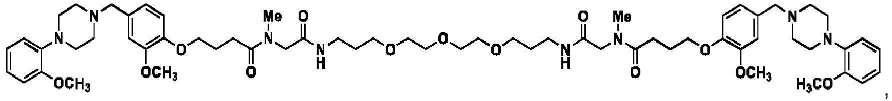 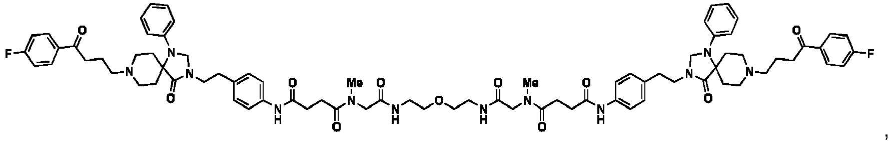 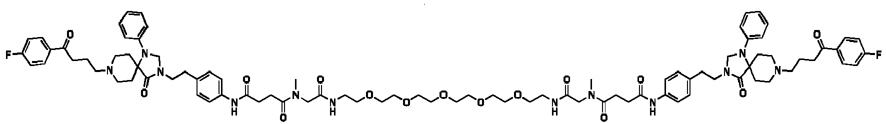 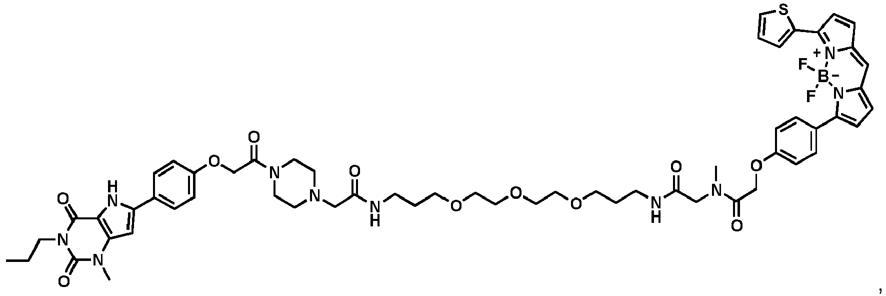 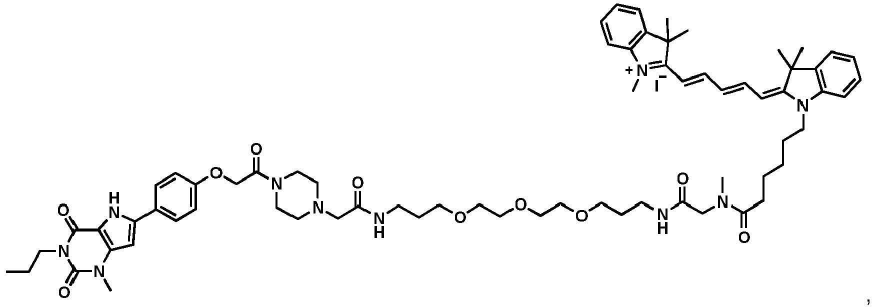 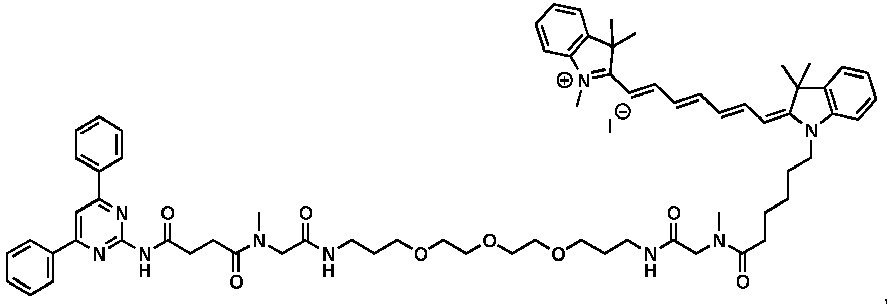 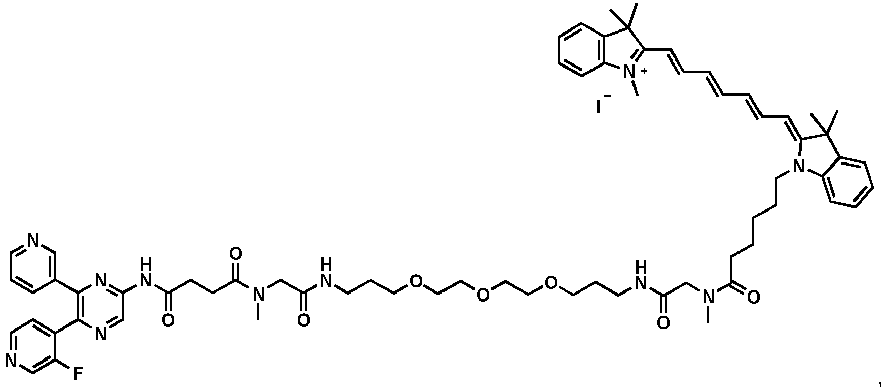 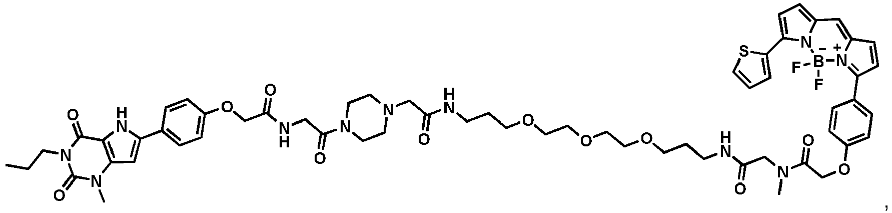 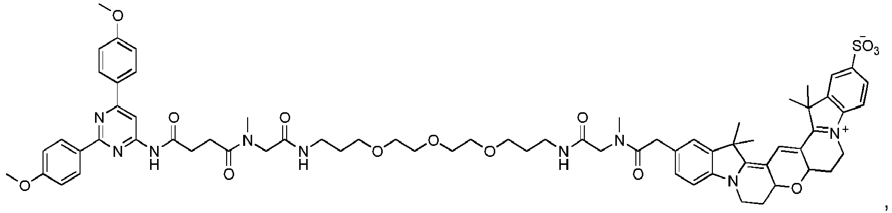 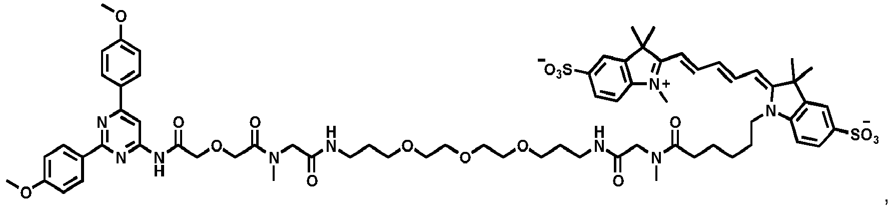 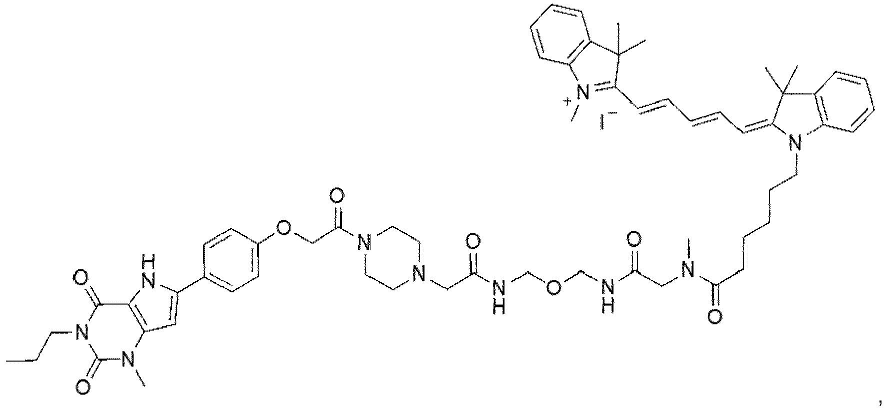 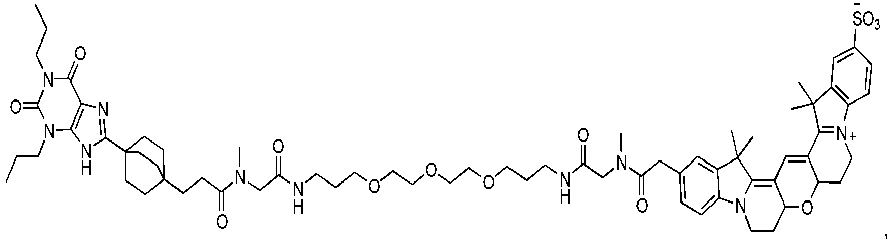 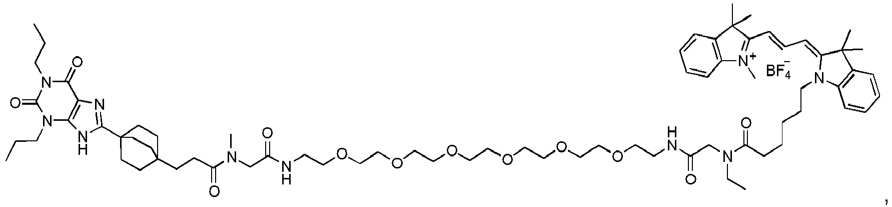 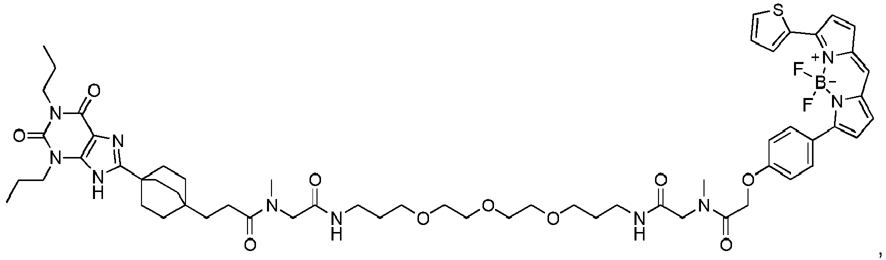 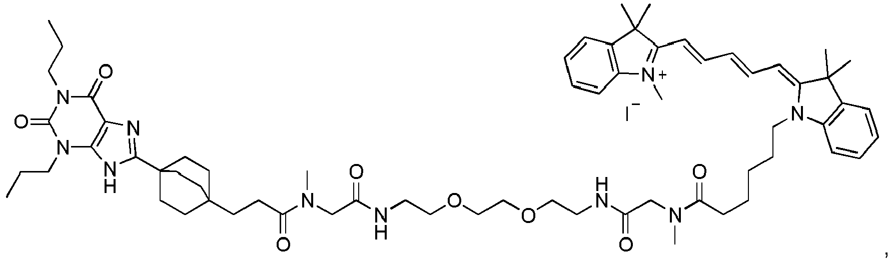 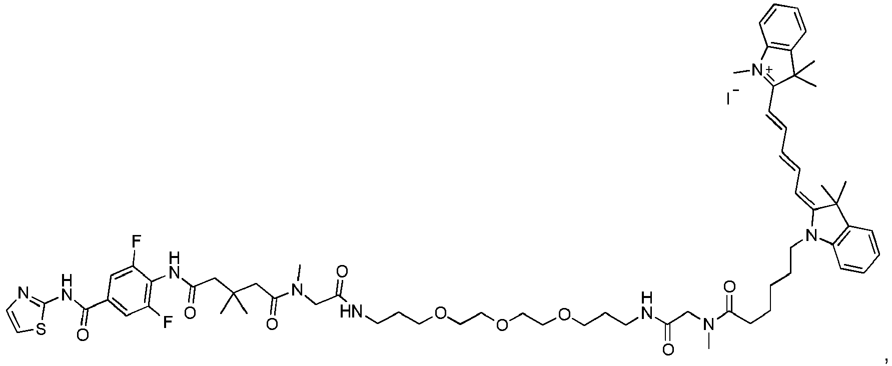 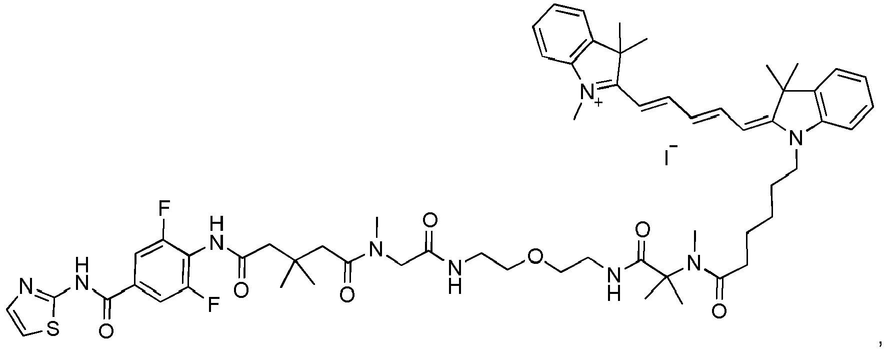 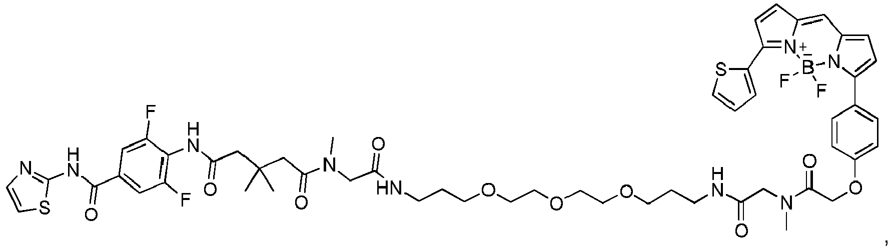 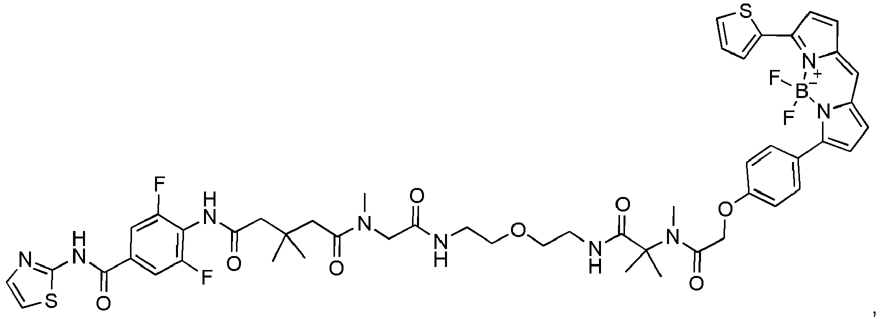 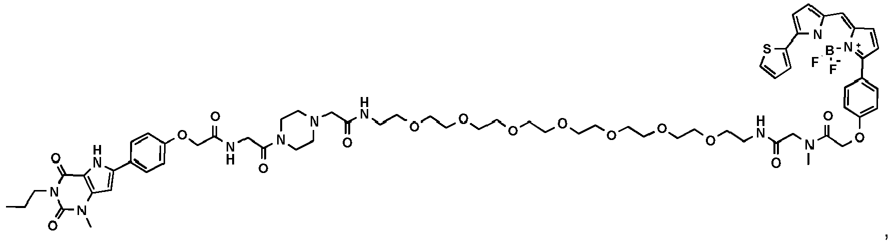 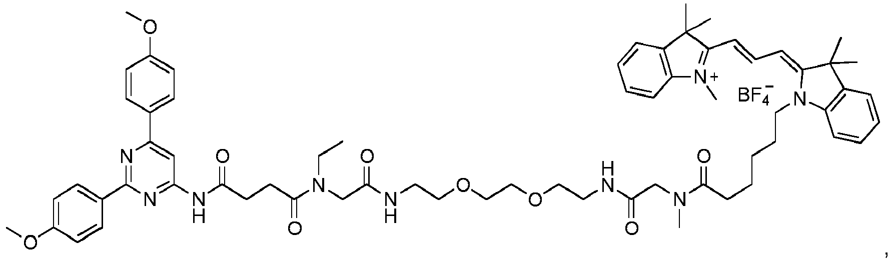 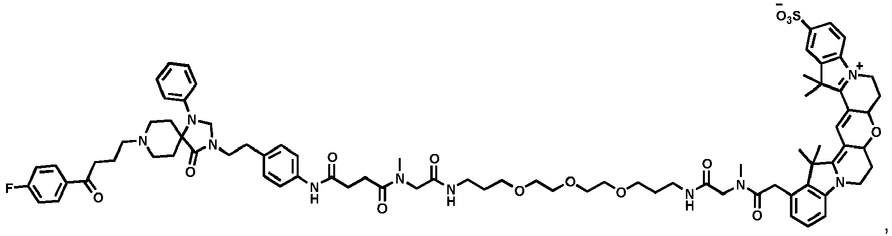 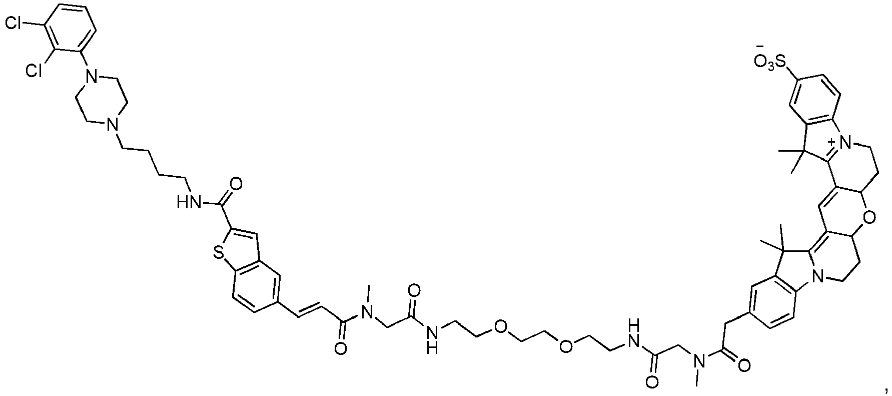 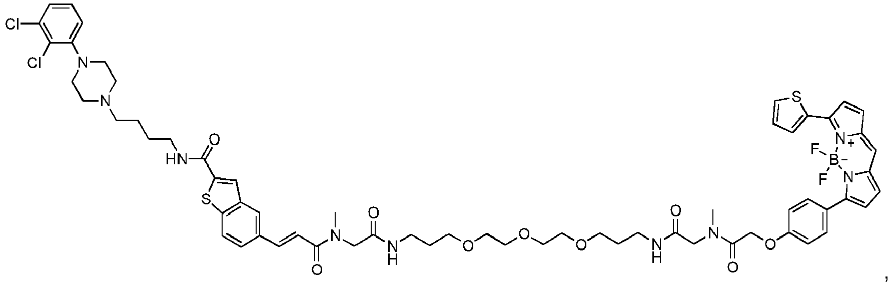 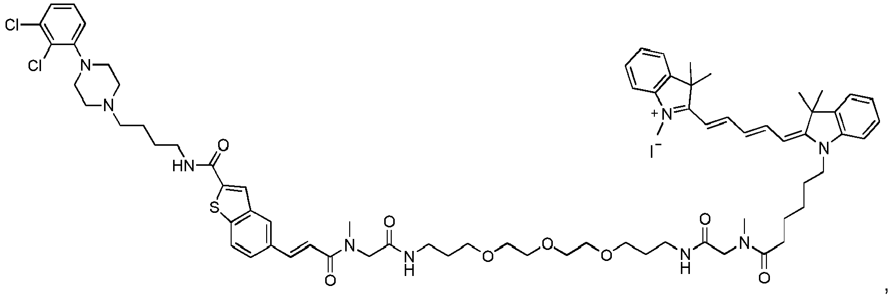 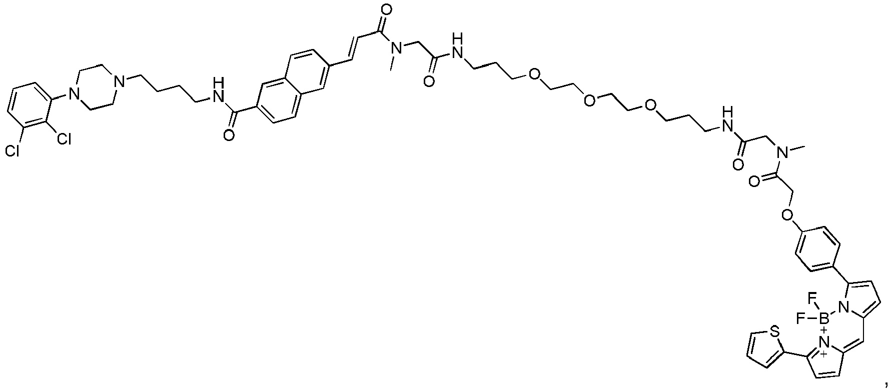 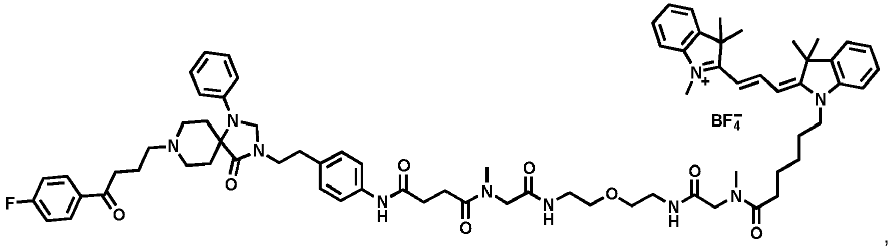 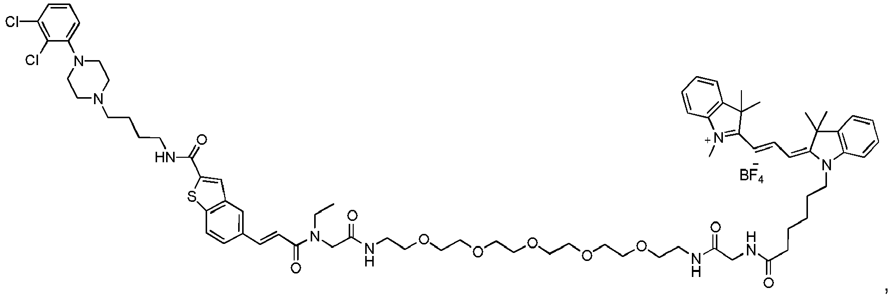 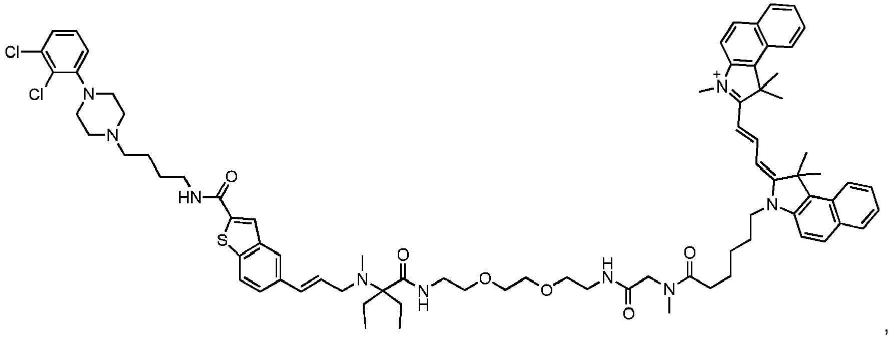 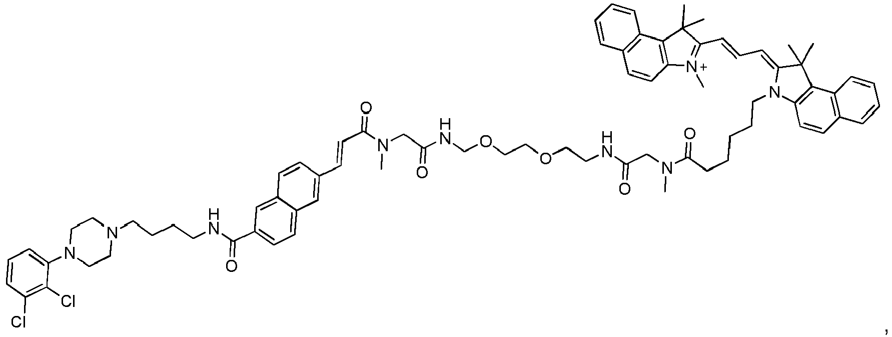 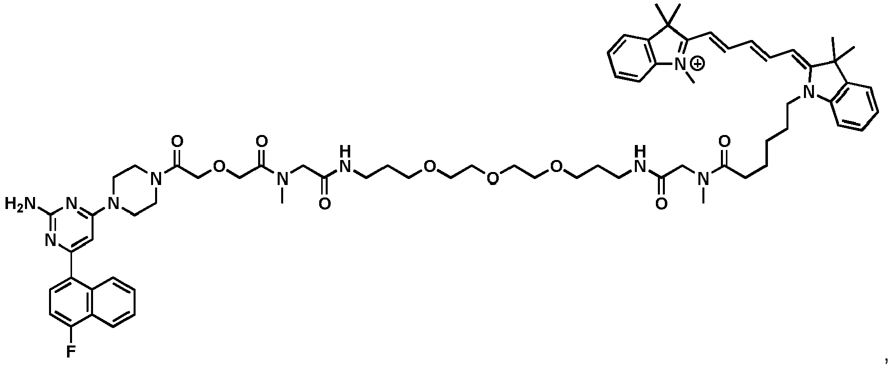 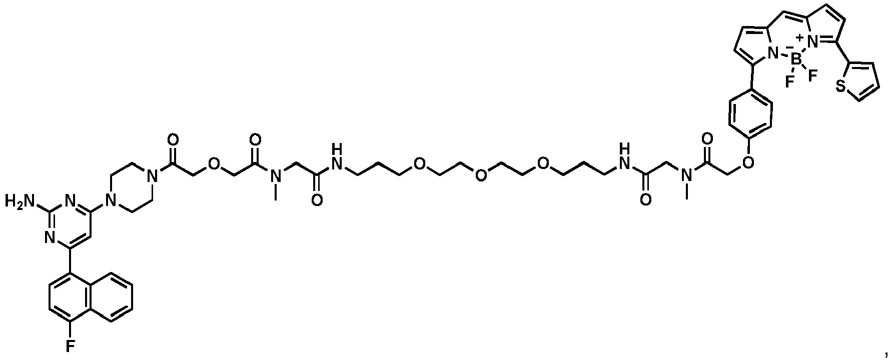 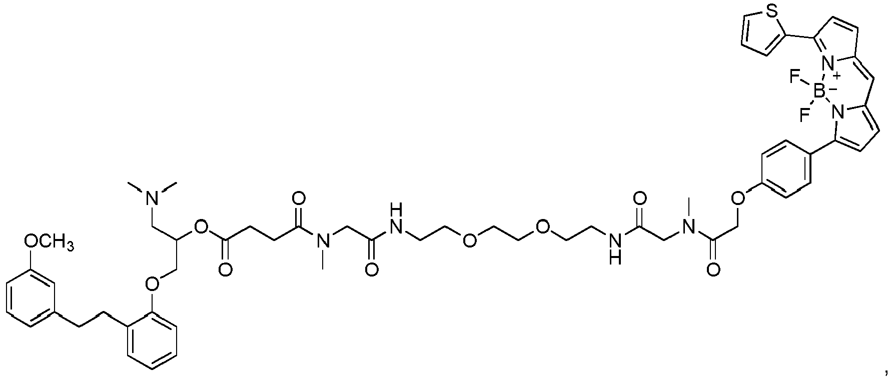 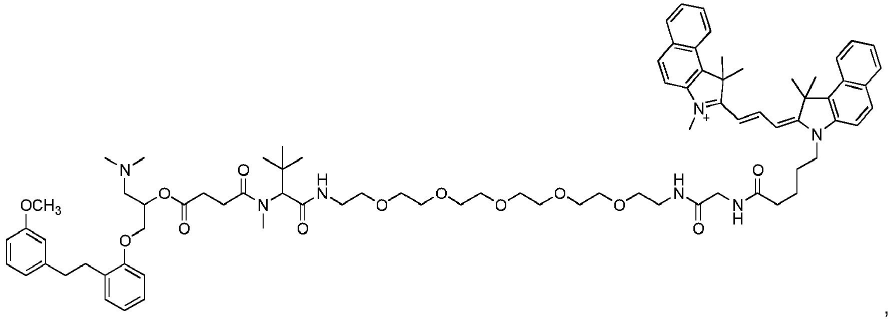 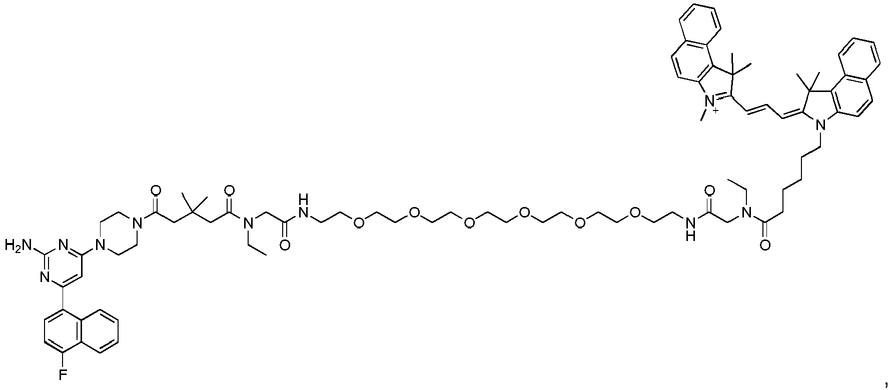 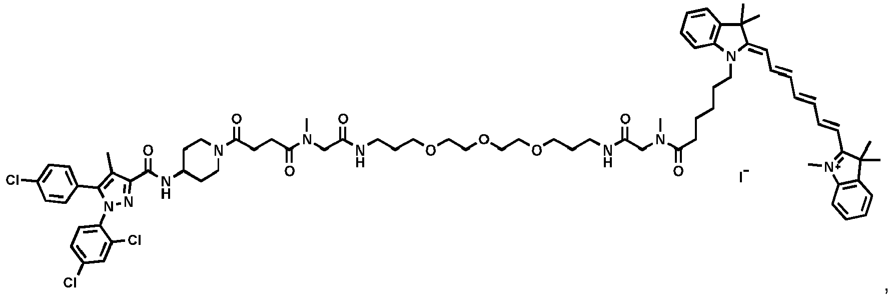 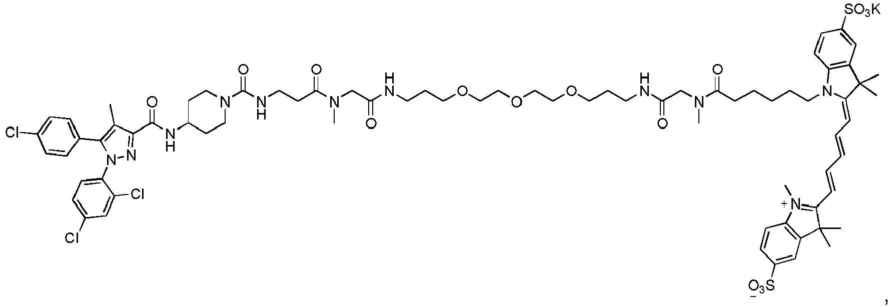 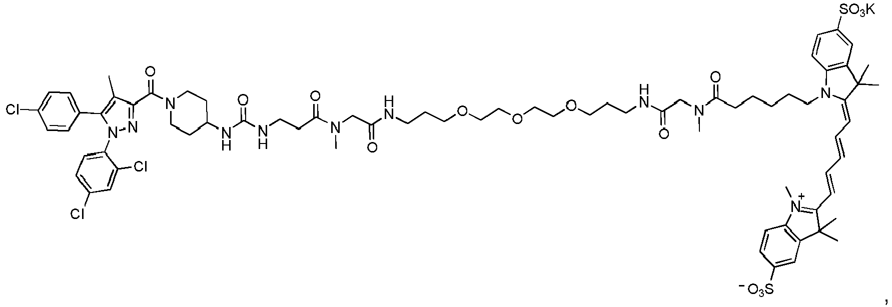 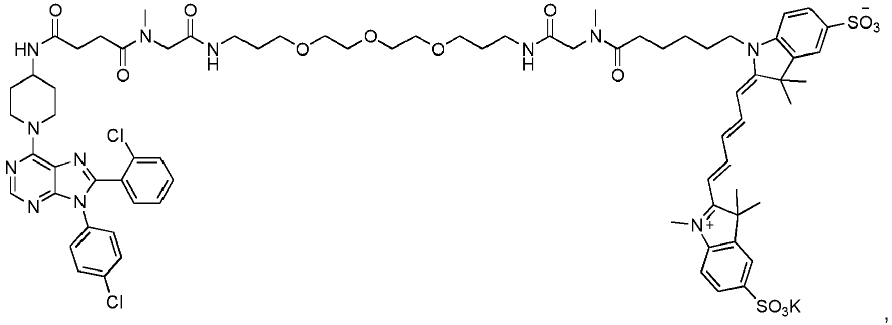 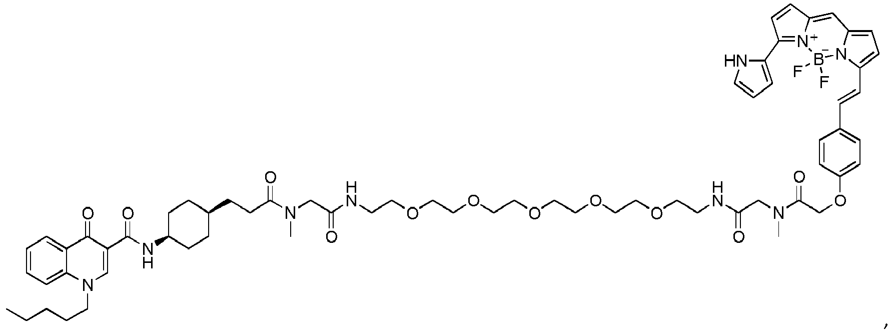 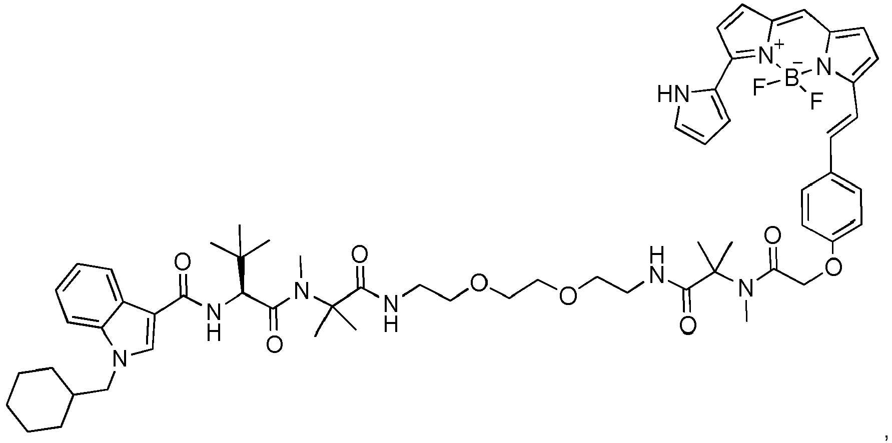 and

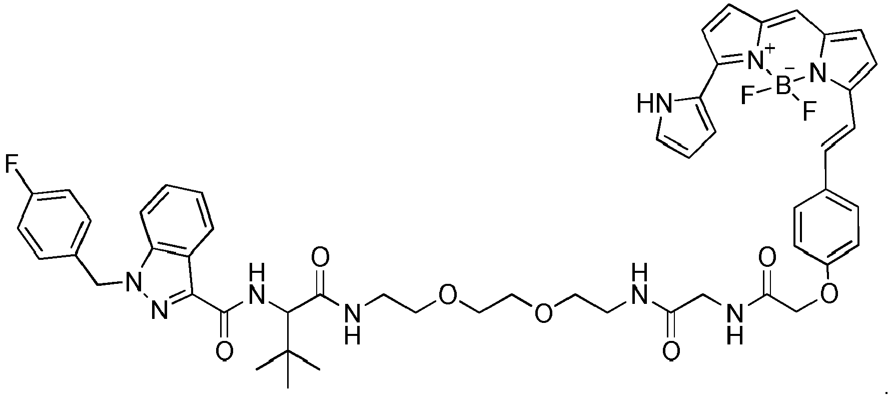

### General Process of preparation of the compounds of formula (Ia)

The intermediate compounds of the present invention can be prepared by means of coupling reactions comprising the mixing of at least three reagents, one of which is an isonitrile. Therefore, in a third aspect, the present invention relates to a method for the preparation of a compound of formula (**Ia**), or a salt or solvate thereof, comprising the mixing of:
a) - at least one compound of general formula (**G1**) 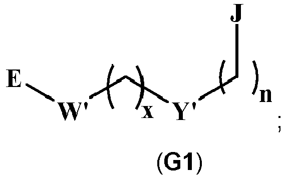 and/or
   - a combination of at least one compound of general formula (G2) 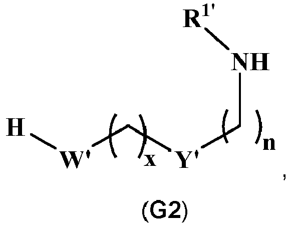 and at least one compound comprising a moiety **F** and at least one reactive group, wherein said at least one reactive group is preferably selected from an amine, hydroxyl, aldehyde or acid reactive group;
b) optionally, at least one compound of formula **R**^{**2**'}-C(=O)-**R**^{**3**'} or **R**^{**1**'}-NH-(CH₂)₀₋₆-C(**R**^{**2**'})(**R**^{**3**'})-COOH and
c) at least one compound of general formula (**S'1**) 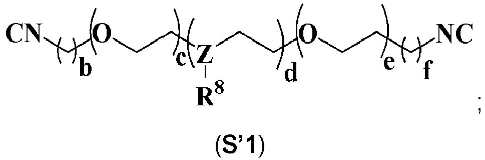 wherein **R⁸** = H, C₁-C₆ optionally substituted alkyl, or a moiety of formula (V1) 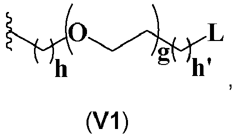 wherein,
   **J** is COOH or NH**R¹**,
   **E, F, W', x, Y', n, R^{1'}, R^{2'}, R^{3'}** and **R⁸**, **Z** and **b-h'** are as defined in any of the particular embodiments above and wherein L, when present, is selected from **G-**F or -NC.

In a particular embodiment, the method for the preparation of a compound of formula (**Ia**) requires the presence of at least one compound of formula **R**^{**2**'}-C(=O)-**R**^{**3**'}.

In a particular embodiment of the method for the preparation of a compound of formula (**Ia**), the compound of formula (**G2**) and at least one compound comprising a moiety **F** and at least one reactive group are present. In another particular embodiment, the compound of formula (**G2**) and at least one compound comprising a moiety **F** and at least one acid or amine reactive group are present. In another particular embodiment of the method for the preparation of a compound of formula (**Ia**), **G2** is an alkyl-monoamine, an alkyl-diimine or an alkyl-triimine, preferably wherein the alkyl is C₁₋₆ alkyl or (5- to 6-membered)-C₂₋₅ heterocyclyl. In a particular embodiment, the (5- to 6-membered)-C₂₋₅ heterocyclyl is piperazine. In a particular embodiment, said alkyl-amine **G2** compounds are optionally substituted with methyl or ethyl.

In a particular embodiment, the method for the preparation of a compound of formula (**Ia**) requires the presence of at least one compound of formula **R^{1'}**-NH-(CH₂)₀₋₆-C(**R^{2'}**)(**R**^{**3**'})-COOH. The latter compound is an aminoacid and in a preferred embodiment, **R^{1'}** is H. Preferably, said aminoacid has the formula N(H₂)-C(**R^{2'}**)(**R**^{**3**'})-COOH.

In a particular embodiment, the method for the preparation of a compound of formula (**Ia**) further comprises an aminoborane compound, a trimethylsilyl azide or phenylphosphinic acid.

In a particular embodiment, the method for the preparation of a compound of formula (**Ia**) is a Ugi reaction, a Ugi-N-Split reaction or a Ugi-Smile reaction.

In a particular embodiment of the method for the preparation of a compound of formula (**Ia**), the compound of formula (**S'1**) is present at a molar excess, preferably at an amount comprised between 2 and 6, more preferably between 3 and 5, even more preferably 4 times the molar amount of **G1** or **G2.**

### General method for the preparation of the compounds of formula (I)

The compounds of the present invention can be prepared by means of reactions comprising the mixing of at least three reagents, one of which is an isonitrile comprising an **F** moiety. Therefore, in a fourth aspect, the present invention relates to a method for the preparation of a compound of formula (**I**), or a salt or solvate thereof, comprising the mixing of at least three precursors selected from:
a) - at least one compound of general formula (**G1**) 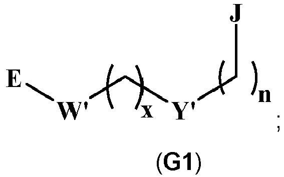 and/or
   - a combination of at least one compound of general formula (G2) 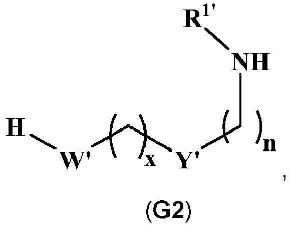
   and at least one compound comprising a moiety **F** and at least one reactive group, wherein said at least one reactive group is preferably selected from an amine, hydroxyl, aldehyde or acid reactive group;
b) optionally, at least one compound of formula **R²**-C(=O)-**R³** or **R^{1'}**-NH-(CH₂)₀₋₆-C(**R^{2'}**)(**R^{3'}**)-COOH; and
c) at least one compound of general formula (**Ia**) 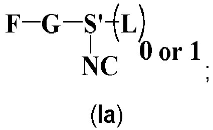 wherein
   J is COOH or NH**R¹**,
   **E, F, W', x, Y', n, R^{1'}, R^{2'}** and **R**^{**3**'}, **G** and **S'** are as defined above and wherein **L,** when present, is **G-F.**

In a particular embodiment, the method for the preparation of a compound of formula (**I**) requires the presence of at least one compound of formula **R^{2'}**-C(=O)-**R^{3'}**.

In a particular embodiment of the method for the preparation of a compound of formula (**I**), the compound of formula (**G2**) and at least one compound comprising a moiety **F** and at least one reactive group are present. In another particular embodiment, the compound of formula (**G2**) and at least one compound comprising a moiety **F** and at least one acid or amine reactive group are present. In another particular embodiment of the method for the preparation of a compound of formula (**I**), **G2** is an alkyl-monoamine, an alkyl-diimine or an alkyl-triimine, preferably wherein the alkyl is C₁₋₆ alkyl or (5- to 6-membered)-C₂₋₅ heterocyclyl. In a particular embodiment, the (5- to 6-membered)-C₂₋₅ heterocyclyl is piperazine. In a particular embodiment, said alkyl-amine **G2** compounds are optionally substituted with methyl or ethyl.

In a particular embodiment, the method for the preparation of a compound of formula (**I**) requires the presence of at least one compound of formula **R^{1'}**-NH-(CH₂)₀₋₆-C(**R**^{**2**'})(**R^{3'}**)-COOH. The latter compound is an aminoacid and in a preferred embodiment, **R^{1'}** is H. Preferably, said aminoacid has the formula N(H₂)-C(**R**^{**2**'})(**R**^{**3**'})-COOH.

In a particular embodiment, the method for the preparation of a compound of formula (**I**) further comprises an aminoborane compound, a trimethylsilyl azide or phenylphosphinic acid.

In a particular embodiment, the method for the preparation of a compound of formula (**I**) is a Ugi reaction, a Ugi-N-Split reaction or a Ugi-Smile reaction.

In a particular embodiment of the method for the preparation of a compound of formula (**I**), the compound of formula (**S'1**) is present at a molar excess, preferably at an amount comprised between 2 and 6, more preferably between 3 and 5, even more preferably 4 times the molar amount of **G1** or **G2.**

In a particular embodiment, when a compound of formula (**I**) which comprises two identical **F** moieties is desired, then an excess of **F**-comprising compounds is required.

### Kits of the invention

The compounds of the present invention may be provided as final products but also in the form of a "kit of parts". In said kit, the necessary reagents are provided along with instructions for conducting the preparation of the desired products. The kit may comprise a single variety of reactive **F**-comprising molecules or a plurality of said **F**-comprising molecules.

Therefore, the invention is also related to a kit for the preparation of a compound of formula (**Ia**) and (**I**), respectively.

All of the particular and preferred embodiments concerning the methods of the invention described above apply without exceptions to the kits of the invention.

### Pharmaceutical compositions

The compounds of formula (**I**) comprise pharmacophore moieties, label moieties or both. Therefore, the compounds may be used in the context of a pharmaceutical composition. Therefore, the invention further provides a fifth aspect consisting of a pharmaceutical composition comprising at least one compound of formula (**I**), or of formula (**Ia**), and a pharmaceutically acceptable excipient.

The term "pharmaceutically acceptable excipient" refers to a vehicle, diluent, or adjuvant that is administered with the active ingredient, comprised in a compound of formula (**I**). Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Fauli i Trillo, Luzán 5, S.A. de Ediciones, 1993.

### Uses

The versatility of the method of the invention allows the preparation of a large variety of compounds comprising functional elements **F.** These functional elements can be used for both medical and industrial purposes. For example, they may be biologically relevant such as pharmacophores and label moieties or chemically relevant such as ligands and label moieties. Therefore, an sixth aspect of the invention relates to the use of the compounds of the invention, preferably of compounds of formula (**I**), in sensors, diagnostic methods, bioanalytical methods, chemical tests and as chelating agents, wherein the use is not practiced on the human or animal body. Exemplary uses include test strips and immunoassays, among others.

The compounds of the invention allow for the qualitative and quantitative determination of an immunochemically reactive component such as a hapten, antigen or antibody. Apart from reading off with the naked eye, physical methods can also be used, such as spectrophotometry, fluorimetry, nephelometry and electron-dark field microscopy. These methods can be combined with the use of a label or tracer **F** moiety, present in the compounds of the invention. Instead of detecting the actual immune complex, a label **F,** coupled with a biologically active moiety **F,** is then detected, so that a considerably lower detection limit can be attained.

Further uses contemplated in the present invention include in vitro/ex vivo diagnosis of diseases, contrast agents for imaging purposes and in chelation therapy.

### Medical uses

Since the compounds of the invention may comprise active pharmacological ingredients or residues that bind, selectively or not, to biological molecules within a living being, then the invention further contemplates the medical use of the compounds of the invention, preferably of the compounds of formula (**I**).

In this way, a seventh aspect of the invention relates to a compound of formula (**I**), or of a compound of formula (**Ia**), or of a pharmaceutical composition as described above, for use as a medicament.

A eighth aspect of the invention is addressed to the compounds of the invention as described above, or a pharmaceutical composition such as has been defined above for use in the treatment and/or prevention of a heavy metal poisoning, cardiovascular diseases, neurodevelopment disorders, immune system disorders, metabolic diseases or disorders, neurodegenerative diseases, diabetes, respiratory diseases and cancer.. The compounds of the present invention may also be used in photodynamic therapy. This aspect may also be formulated as the use of the compounds of the invention, in the manufacture of a medicament for the treatment and/or prevention of a heavy metal poisoning, cardiovascular diseases, neurodevelopment disorders, immune system disorders, metabolic diseases or disorders, neurodegenerative diseases, diabetes, respiratory diseases and cancer..

This aspect may also be formulated as a method of treating and/or preventing heavy metal poisoning, cardiovascular diseases, neurodevelopment disorders, immune system disorders, metabolic diseases or disorders, neurodegenerative diseases, diabetes, respiratory diseases and cancer., comprising administering to a subject in need of such treatment the compounds of the invention as described above, or a pharmaceutical composition as defined above.

Non-limiting examples of cardiovascular diseases (CVDs), include coronary artery diseases (CAD) such as angina and myocardial infarction (commonly known as a heart attack). Other CVDs include stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, and venous thrombosis.

Neurodevelopment disorders include autism, ADHD, mood affective disorders including anxiety, depressive disorders and bipolar disorders.

Immune system disorders include allergic diseases and autoimmune diseases. Non-limiting examples of allergic diseases are hay fever, food allergies, atopic dermatitis, allergic asthma, anaphylaxis, food intolerances and food poisoning. Non-limiting examples of autoimmune diseases include lupus, celiac disease, diabetes mellitus type, Graves' disease, inflammatory bowel disease, multiple sclerosis, psoriasis, rheumatoid arthritis, and systemic lupus erythematosus.

Metabolic diseases or disorders can happen when abnormal chemical reactions in the body alter the normal metabolic process and include gestational diabetes, type 1 or type 2 diabetes, acid-base imbalance, metabolic brain diseases, disorders of calcium metabolism, dna repair-deficiency disorders, glucose metabolism disorders, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis deficiencies, metabolic skin diseases, wasting syndrome and water-electrolyte imbalance.

Neurodegenerative diseases include amyotrophic lateral sclerosis, Parkinson's disease, Alzheimer's disease, fatal familial insomnia, Batten disease and Huntington's disease.

Respiratory diseases are conditions of the respiratory tract including the trachea, bronchi, bronchioles, alveoli, pleurae, pleural cavity, and the nerves and muscles of respiration. Respiratory diseases include respiratory tract infections such as pneumonia including bacterial pneumonia. Other respiratory diseases include pulmonary embolism, asthma, chronic bronchitis, bronchiectasis, chronic obstructive pulmonary disease, severe acute respiratory syndromes or acute respiratory distress syndrome.

Cancer include carcinoma, such as breast, prostate, lung, pancreas and colon cancer; sarcoma, including bone, cartilage, fat and nerve cancer; lymphoma and leukemia; germ cell tumor such as testicle or ovary cancer; and blastoma.

Further uses contemplated in the present invention include in vitro/ex vivo diagnosis of diseases, contrast agents for imaging purposes and in chelation therapy.

The terms "treat" and "treatment", as used herein, mean reversing, alleviating, inhibiting progression of the disease or condition to which said term or one or more symptoms of said disease or condition applies.

The terms "prevent" and "prevention," as used herein, mean the inhibition of the occurrence of the disease or condition to which this term applies or one or more symptoms of such disease or condition.

### Particular embodiments of the invention

Embodiment 1. A compound of formula (**I**), 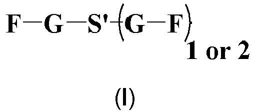 or a salt or solvate thereof,
wherein each occurrence of,
- **F** is independently selected from a moiety comprising a photoactive or a biologically active moiety; and
- **G** is independently selected from a moiety of formula (**II**) or (**III**), 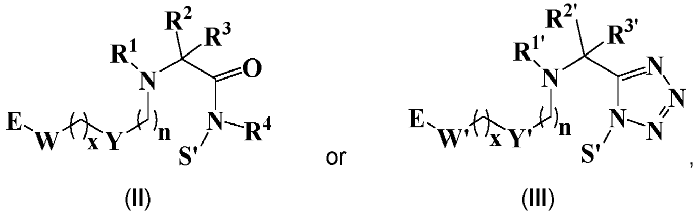 wherein each occurrence of,
   - **R¹, R^{1'}, R², R^{2'}, R³, R^{3'}** or **R⁴** is independently selected from H, -COO**R⁹**, - C(=O)H, -CS**R⁹**, -C₁₋₁₂ alkyl, -C₁₋₆ alkyl-O-C₁₋₆ alkyl, **F,** -C₁₋₁₂ alkyl-**F**, -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, -C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkyl heteroaryl, heterocyclyl and alkylheterocyclyl groups are optionally substituted with 1, 2 or 3 substituents each independently selected from the group consisting of halogen, (=O), -O**R⁹**, -COO**R⁹**, - OC(=O)**R⁹**, -C(=O)H, -CN, -S**R⁹**, -N(**R⁹**)₂, -N(**R⁹**)C(=O)H, -C(=O)N(**R⁹**)₂, - ON(**R⁹**)₂, and (5- to 10-membered)-C₂₋₉ heterocyclyl with at least one N atom;
   - **n** and **x** are integers independently selected from 0 to 6;
   - **Y** and **Y'** are independently selected from single bond, -CH(COO**R⁹**)-, -C(=O)-, -O-, -N(**R⁵**)- or optionally substituted phenyl;
   - **R⁵** is independently selected from H, -COO**R⁹**, -C(=O)H, -CS**R⁹**, -C₁₋₁₂ alkyl, -C₁₋₆ alkyl-O-C₁₋₆ alkyl, **-F,** -C₁₋₁₂ alkyl-**F**, -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, -C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkylheteroaryl, heterocyclyl and alkylheterocyclyl groups are optionally substituted with 1, 2 or 3 substituents each independently selected from the group consisting of halogen, (=O), -O**R⁹**, -COO**R⁹**, -OC(=O)**R⁹**, - C(=O)H, -CN, -S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom;
   - **W** is independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁷**)-, -OC(=O)(**R⁷**)-, -C(=O)CH₂O-, - CH₂OC(=O)-, -C(=O)-, -O-, -C₆₋₁₂ aryl-, or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), -O**R⁹**, -COO**R⁹**, - OC(=O)**R⁹**, -C(=O)H, -CN, -S**R⁹**, -N(**R⁹**)₂, -N(**R⁹**)C(=O)H, -C(=O)N(**R⁹**)₂, - ON(**R⁹**)₂, and (5- to 10-membered)-C₂₋₉ heterocyclyl with at least one N atom;
   - **W'** is independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁶**)-, -OC(=O)(**R⁶**)-, -C(=O)-, - C(=O)O-, -O-, -C₆₋₁₂ aryl- or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), -O**R⁹**, -COO**R⁹**, -OC(=O)**R⁹**, -C(=O)H, -CN, - S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom;
   - **R⁶** is independently selected from H, -C₁₋₆ alkyl or -C₆₋₁₂ aryl, wherein said alkyl and aryl groups are optionally substituted with 1, 2 or 3 substituents each independently selected from the group consisting of halogen, (=O), -O**R⁹**, - COO**R⁹**, -OC(=O)**R⁹**, -C(=O)H, -CN, -S**R⁹**, -N(**R⁹**)₂, -N(**R⁹**)C(=O)H, - C(=O)N(**R⁹**)₂, -ON(**R⁹**)₂, and -(5- to 10-membered)-C₂₋₉ heterocyclyl with at least one N atom;
   - **R⁷** is independently selected from -C₂-C₁₂ alkyl, optionally substituted with 1, 2 or 3 substituents each independently selected from the group consisting of halogen, (=O), -O**R⁹**, -COO**R⁹**, -OC(=O)**R⁹**, -C(=O)H, -CN, -S**R⁹**, -N(**R⁹**)₂, - N(**R⁹**)C(=O)H, -C(=O)N(**R⁹**)₂, -ON(**R⁹**)₂, and -(5- to 10-membered)-C₂₋₉ heterocyclyl with at least one N atom;
   - **E** is independently selected from -**R⁶** or **-F;**
   - **R⁹** is independently selected from H or C₁-C₄ alkyl; and
      wherein,
   - **R¹** and **R²**, or **R^{1'}** and **R^{2'},** together with the nitrogen and carbon atoms to which they are attached, can form an optionally substituted 5- to 10-membered heterocyclic ring, wherein said heterocyclic ring optionally contains 1, 2, or 3 additional heteroatoms selected from the group consisting of N, S, or O;
   - **R¹,** or **R^{1'},** and **R⁵**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O, as long as W or W' is not a bond; and
   - **R¹,** or **R^{1'},** and **R⁶**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O;
   and wherein **S'** is a moiety of formula (IV), 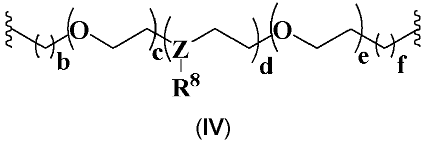 wherein,
   **b** is an integer comprised between 1 and 3;
   **c** is an integer comprised between 0 and 20;
   **d** is an integer comprised between 0 and 1;
   **e** is an integer comprised between 0 and 20;
   **f** is an integer comprised between 0 and 3;
   **Z** is selected from C, N, -C₃-C₁₀ cycloalkyl, (5- to 10-membered)-C₂₋₉ heterocyclyl, - C₆-C₁₂ aryl, or (5- to 10-membered)-C₁₋₉ heteroaryl; and
   **R⁸** is selected from H, C₁-C₆ optionally substituted alkyl, or a moiety of formula (V) 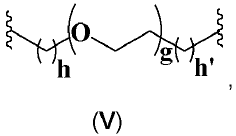 wherein,
      - **g** is selected from an integer comprised between 0 and 20; and
      - **h** and **h'** are each independently selected from an integer comprised between 0 and 3;
with the proviso that the following conditions are excluded when the compounds comprise only two **F** moieties, which are identical, and **G** = formula (**II**),
**R²** = methyl, **R¹** = **R³** = **R⁴** = H, and **n** = **x** = 0, and **Y** = CO and **W** = bond;
**R²** = -CH₂COOH, **R¹** = **R³** = **R⁴** = H, and **n** = **x** = 0, and **Y** = **W** = bond;
**R¹** forms a 2-oxopiperazine ring with **R⁵** or **R⁶**;
**R²** = diphenylmethyl, unsubstituted phenyl or CH₂-OH; or
**R²** = **R³** = **R⁴** = H, **R¹** forms a piperazine ring with **R⁶** and **F** = quinolone.

Embodiment 2. The compound according to embodiment 1, comprising at least two different **F** moieties.

Embodiment 3. The compound according to any one of embodiments 1 or 2, wherein **R¹** or **R^{1'}** is each independently selected from H, -C₁-C₃ alkyl, -C₁₋₃ alkyl-**F**, 5-membered heterocyclic ring with **R²** or **R^{2'},** respectively, 6- to 7-membered heterocyclic ring with **R⁵**, respectively, or optionally substituted 6- to 7-membered heterocyclic ring with **R⁶**.

Embodiment 4. The compound according to any one of embodiments 1 to 3, wherein **R²** or **R^{2'}** is each independently selected from H, -C₁-C₃ optionally substituted alkyl, -C₁₋₃ alkyl-**F**, **-F,** or 5-membered heterocyclic ring with **R¹** or **R^{1'},** respectively, preferably H, - C₁-C₃ alkyl or 5-membered heterocyclic ring with **R¹** or **R^{1'},** respectively.

Embodiment 5. The compound according to any one of embodiments 1 to 4, wherein **R³** or **R^{3'}** is each independently selected from H or -C₁-C₃ optionally substituted alkyl.

Embodiment 6. The compound according to any one of embodiments 1 to 5, wherein each occurrence of **G** is independently selected from the group consisting of formulae **G¹** to **G²⁷**: 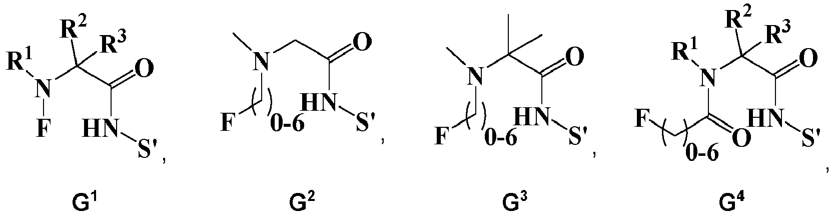 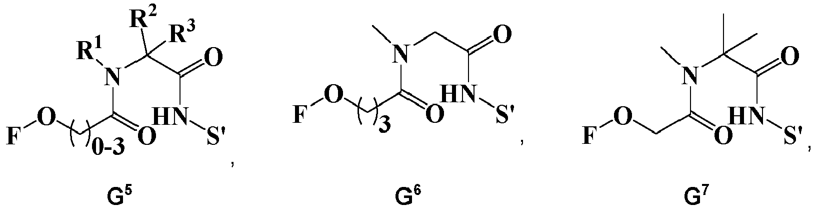 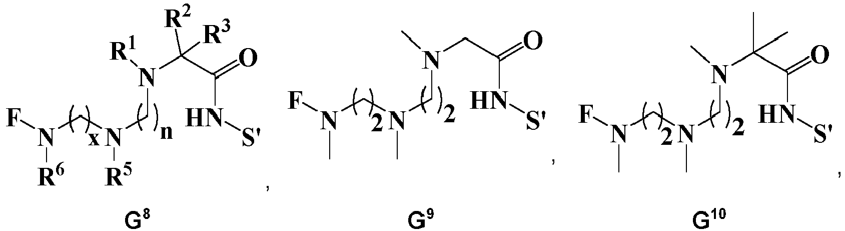 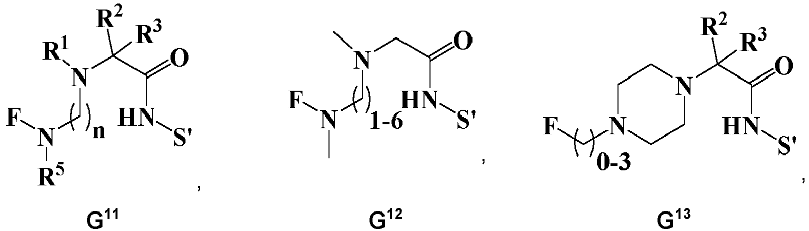 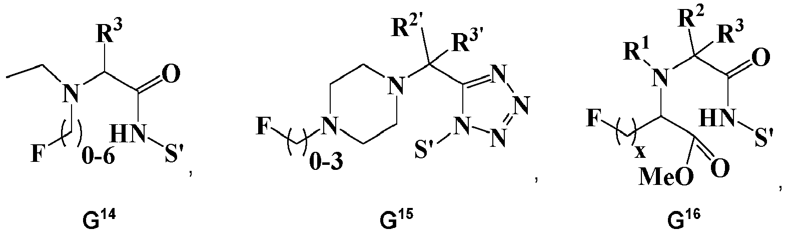 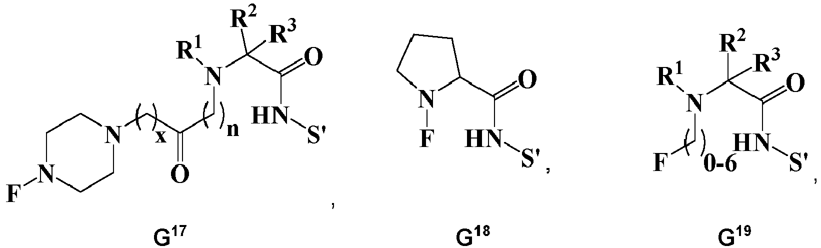 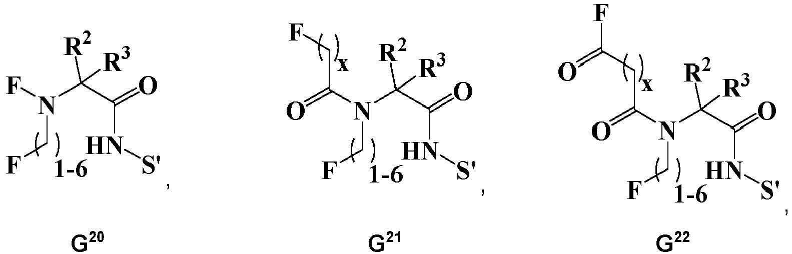 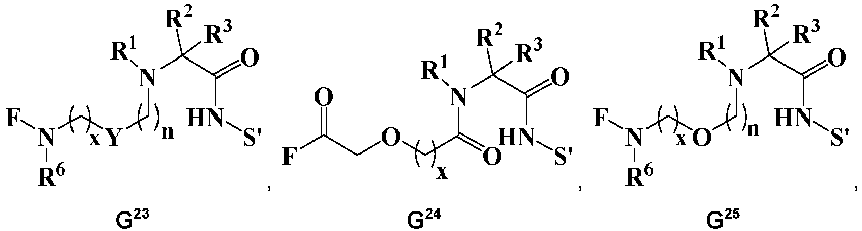 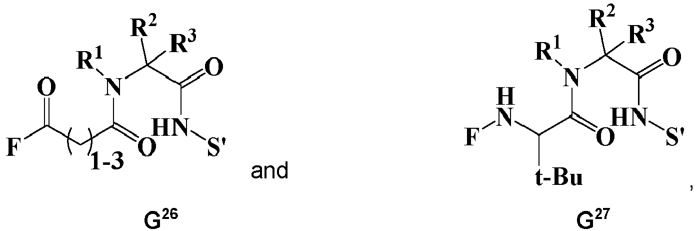 wherein,
**R¹, R³** and **R^{3'}** are as defined in any one of embodiments 1 to 5, preferably each is independently selected from the group consisting of H, methyl and ethyl;
**R²** and **R^{2'}** are as defined above, preferably each is independently selected from the group consisting of H, methyl, ethyl, isopropyl and *tert*-butyl;
**R⁵** is as defined in any one of embodiments 1 to 5, preferably each is independently selected from the group consisting of H, methyl, **-F** and -C₁₋₆ alkyl-**F**;
**R⁶** is as defined in any one of embodiments 1 to 5, preferably each is independently selected from the group consisting of H and methyl; and
**x** and **n** are as defined in any one of embodiments 1 to 5.

Embodiment 7. A compound of formula (**Ia**), 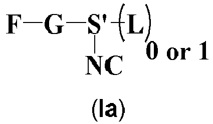 or a salt or solvate thereof,
wherein **F** and **G** are as defined in any one of embodiments 1 to 6,
**L,** when present, is selected from **G-F** or -NC,
wherein **S'** is a moiety of formula (IV), 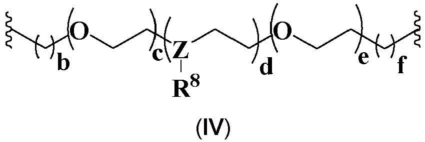 wherein,
   **b** is an integer comprised between 1 and 3;
   **c** is an integer comprised between 0 and 20;
   **d** is an integer comprised between 0 and 1;
   **e** is an integer comprised between 0 and 20;
   **f** is an integer comprised between 0 and 3;
   **Z** is selected from C, N, -C₃-C₁₀ cycloalkyl, (5- to 10-membered)-C₂₋₉ heterocyclyl, - C₆-C₁₂ aryl, or (5- to 10-membered)-C₁₋₉ heteroaryl; and
   **R⁸** = H, C₁-C₆ optionally substituted alkyl, or a moiety of formula (V) 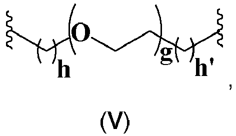 wherein,
      - **g** is selected from an integer comprised between 0 and 20; and
      - **h** and **h'** are each independently selected from an integer comprised between 0 and 3.

Embodiment 8. A method for the preparation of a compound of formula (**Ia**), or a salt or solvate thereof, according to embodiment 7, comprising the mixing of:
a) at least one compound of general formula (**G1**) 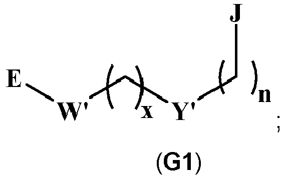 and/or a combination of at least one compound of general formula (**G2**) 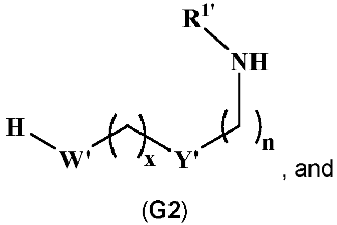 at least one compound comprising a moiety **F** and at least one reactive group, wherein said at least one reactive group is preferably selected from an amine, hydroxyl, aldehyde or acid reactive group;
b) optionally, at least one compound of formula **R^{2'}**-C(=O)-R³' or **R^{1'}**-NH-(CH₂)₀₋₆-C(**R^{2'}**)(**R^{3'}**)-COOH; and
c) at least one compound of general formula (**S'1**) 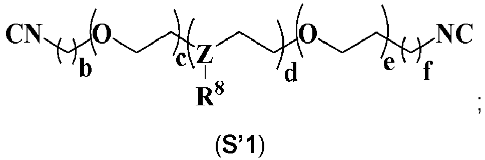 wherein **R⁸** = H, C₁-C₆ optionally substituted alkyl, or a moiety of formula (V1) 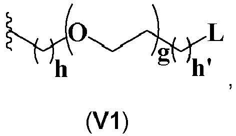 wherein,
   J is COOH or NH**R¹**,
   **E, F, W', x, Y', n, R^{1'}, R^{2'}**, **R^{3'}** and **R⁸**, **Z** and **b-h'** are as defined in any one of embodiments 1 to 7 and wherein L, when present, is selected from **G-F** or -NC.

Embodiment 9. A method for the preparation of a compound of formula (**I**), or a salt or solvate thereof, according to any one of embodiments 1 to 6, comprising the mixing of at least three precursors selected from:
a) at least one compound of general formula (G1) 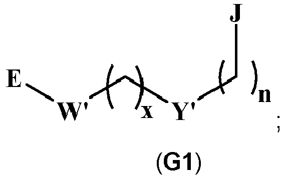 and/or a combination of at least one compound of general formula (**G2**) 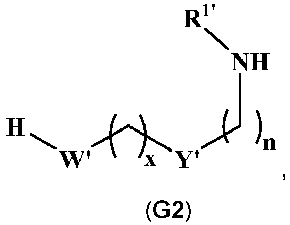 and at least one compound comprising a moiety F and at least one reactive group, wherein said at least one reactive group is preferably selected from an amine, hydroxyl, aldehyde or acid reactive group;
b) optionally, at least one compound of formula **R²**-C(=O)-**R³** or **R^{1'}**-NH-(CH₂)₀₋₆-C(**R^{2'}**)(**R^{3'}**)-COOH; and
c) at least one compound of general formula (**Ia**) 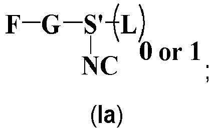 wherein
   **J** is COOH or NH**R¹**,
   **E, F, W', x, Y', n, R^{1'}, R^{2'}** and **R**^{**3**'}, **G** and **S'** are as defined in any one of embodiments 1 to 6 and wherein **L,** when present, is **G-F.**

Embodiment 10. A kit for the preparation of a compound of formula (**Ia**), or a salt or solvate thereof, according to embodiment 7, comprising:
a) at least one compound of general formula (**G1**) 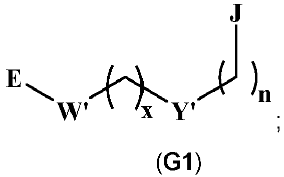 and/or at least one compound of general formula (**G2**) 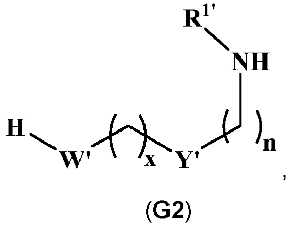 and at least one compound comprising a moiety **F** and at least one reactive group, wherein said at least one reactive group is preferably selected from an amine, hydroxyl, aldehyde or acid reactive group;
b) optionally, at least one compound of formula **R²**-C(=O)-**R³** or **R^{1'}**-NH-(CH₂)₀₋₆-C(**R^{2'}**)(**R^{3'}**)-COOH; and
c) at least one compound of general formula (**S'1**) 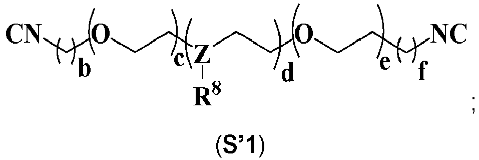 wherein **R⁸** = H, C₁-C₆ optionally substituted alkyl, or a moiety of formula (V1) 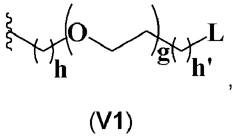 wherein
   **J** is COOH or NH**R¹**,
   **E, F, W', x, Y', n, R^{1'}, R^{2'}, R^{3'}** and **R⁸**, **Z** and **b-h'** are as defined in any one of embodiments 1 to 7 and wherein L, when present, is selected from **G-F** or -NC, further comprising instructions for obtaining the compound of formula (**Ia**).

Embodiment 11. A kit for the preparation of a compound of formula (**I**), or a salt or solvate thereof, according to any one of embodiments 1 to 6, comprising:
a) at least one compound of general formula (**G1**) 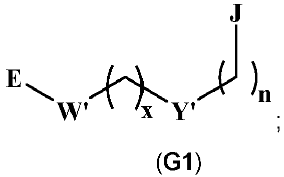 and/or at least one compound of general formula (**G2**) 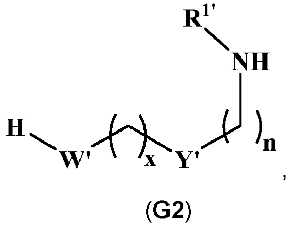 and at least one compound comprising a moiety **F** and at least one reactive group, wherein said at least one reactive group is preferably selected from an amine, hydroxyl, aldehyde or acid reactive group;
b) optionally, at least one compound of formula **R²**-C(=O)-**R³** or **R^{1'}**-NH-(CH₂)₀₋₆-C(**R^{2'}**)(**R^{3'}**)-COOH; and
c) at least one compound of general formula (**Ia**) 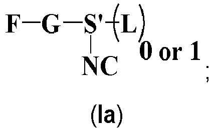 wherein
   **J** is COOH or NH**R¹**,
   **E, F, W', x, Y', n, R^{1'}, R^{2'}** and **R**^{**3**'}, **G** and **S'** are as defined in any one of embodiments 1 to 6 and wherein **L,** when present, is **G-F,**
   further comprising instructions for obtaining the compound of formula (**I**).

Embodiment 12. A pharmaceutical composition comprising a compound of formula (**I**) as defined in any one of embodiments 1 to 6, or a compound of formula (**Ia**) as defined in embodiment 7 and a pharmaceutically acceptable excipient.

Embodiment 13. Use of a compound of formula (**I**) as defined in any one of embodiments 1 to 6, or of a compound of formula (**Ia**) as defined in embodiment 7, in sensors, diagnostic methods, bioanalytical methods, chemical tests and as chelating agents, wherein the use is not practiced on the human or animal body.

Embodiment 14. A compound of formula (**I**) as defined in any one of embodiments 1 to 6, or of a compound of formula (**Ia**) as defined in embodiment 7, or a pharmaceutical composition as defined in embodiment 12, for use as a medicament.

Embodiment 15. A compound of formula (**I**) as defined in any one of embodiments 1 to 6, or of a compound of formula (**Ia**) as defined in embodiment 7, or a pharmaceutical composition as defined in embodiment 12, for use in the treatment and/or prevention of a heavy metal poisoning, cardiovascular diseases, neurodevelopment disorders, immune system disorders, metabolic diseases or disorders, neurodegenerative diseases, diabetes, respiratory diseases and cancer.

The following non-limiting examples are intended to illustrate the present invention and should not be considered as limitations of the scope of the same. Unless stated otherwise, the products whose synthesis is described in the following examples are also to be understood as part of the invention.

### Examples

¹H-NMR analysis. Nuclear magnetic resonance analyses were recorded in CDCl₃, MeOD, DMSO-*d₆* in a Varian Mercury 300 MHz spectrometer equipped with a broadband probe ATB 1H/19F/,31P,13C of 5mm. Spectra were acquired dissolving 1-2 mg of sample in 0.5 mL of deuterated solvent.

Mass spectrometry data were obtained using either a Varian MAT-711 instrument or a Bruker Microtof ESI-TOF.

Melting points were determined on a Gallenkamp melting point apparatus and are uncorrected.

All reagents and solvents were from obtained from commercially available sources.

### General procedures for obtaining the compounds of the invention

### General method 1: An F-carboxylic acid moiety, a bis-isonitrile, an amine and a carbonyl.

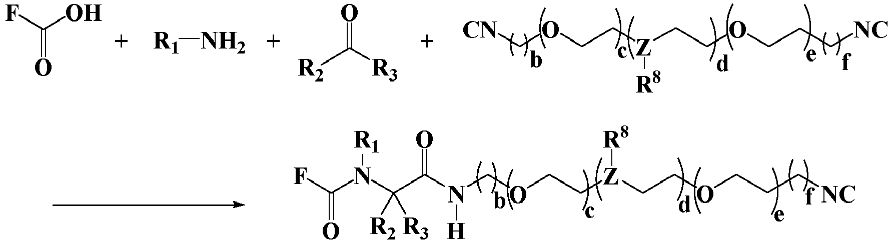

**F, R¹, R², R³, R⁸**, **Z** and **b-f** are as defined in the detailed description of the invention. Generally, a functional element **F** comprising a reactive carboxylic acid group, an isonitrile molecule, a primary amine and a carbonyl compound are mixed in an organic solvent. Any relative molar amounts of the compounds can be chosen. The isonitrile molecule is preferably in excess, whereas a 2-6 molar excess of the isonitrile can be added to the mixture. Preferably, the reaction is conducted at a molar ratio of **F:** isonitrile:amine:carbonyl compound of 1:4:1.2:1.

Any solvent can be chosen for the reaction, as long as it does not lead to unwanted secondary reactions. A particular choice of solvent is an organic solvent, preferably protic solvents such as methanol, ethanol or isopropanol.

The reaction can be conducted at a temperature comprised between 10 °C and 150 °C (the maximum being dependent on the boiling point of the solvent). The reaction is preferably conducted at room temperature or higher. When conducted at room temperature, the reaction is complete within 36-72 h.

If desired, upon isolating the resulting isonitrile product, it may be further purified by common purification techniques such as chromatographic techniques, particularly column chromatography.

### General method 2: An F-carboxylic acid moiety, a bis-isonitrile, a diamine and a carbonyl.

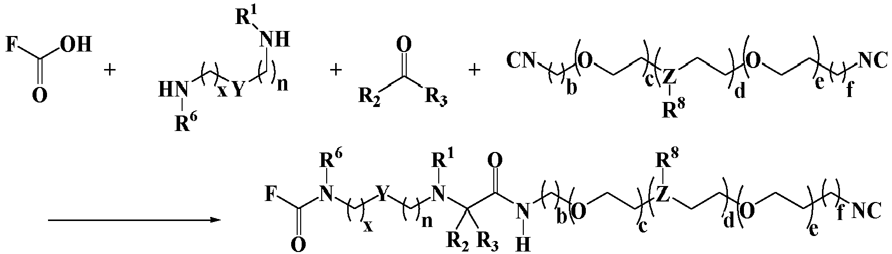

F, **R¹, R², R³, R⁶**, **R⁸**, x, **n, Y, Z** and **b-f** are as defined in the detailed description of the invention.

General method 2 can be conducted under the same conditions as general method 1, but in this case the reaction is preferably conducted at 40 °C or higher, more preferably at 60 °C. When conducted at 60 °C, the reaction is complete within 36-72 h.

### General method 3: An F-hydroxy moiety, a bis-isonitrile, an amine and a carbonyl.

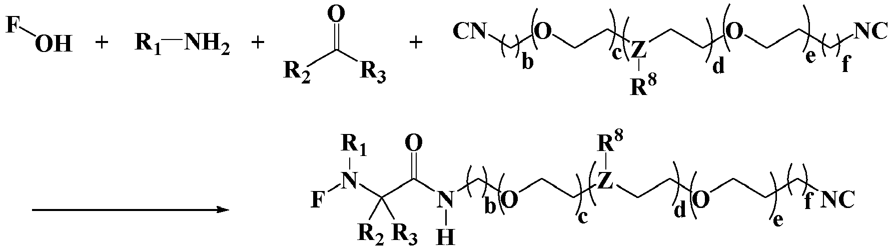

**F, R¹, R², R³, R⁸**, **Z** and **b-f** are as defined in the detailed description of the invention. General method 3 can be conducted under the same conditions as general method 1 or 2 but in this case the reaction is preferably conducted at 30 °C or higher, more preferably at 40 °C. When conducted at 40 °C, the reaction is complete within 36-72 h.

### General method 4: An F-amine moiety, a bis-isonitrile, a carbonyl and an aminoborane.

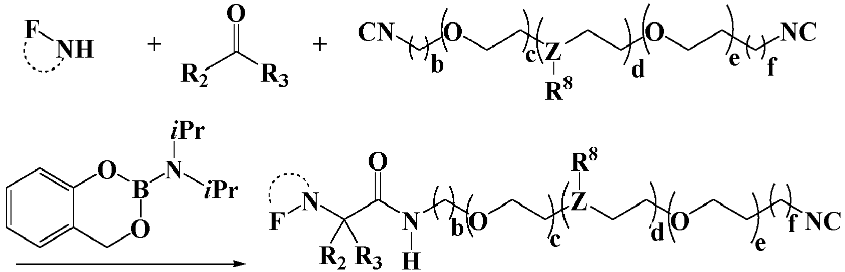

**F, R², R³, R⁸**, **Z** and **b-f** are as defined in the detailed description of the invention.

Generally, a functional element F comprising a reactive secondary amine group, a bis-isonitrile molecule, a carbonyl compound and an aminoborane are mixed in an organic solvent. Any relative molar amounts of the compounds can be chosen. The isonitrile molecule is preferably in excess, whereas a 2-6 molar excess of the isonitrile can be added to the mixture. Preferably, the reaction is conducted at a molar ratio of F: isonitrile:carbonyl:aminoborane compound of 1.2:4:1.2:1.

Any solvent can be chosen for the reaction, as long as it does not lead to unwanted secondary reactions. A particular choice of solvent is an organic solvent, preferably THF. The reaction can be conducted at a temperature comprised between 10 °C and 150 °C (the maximum being dependent on the boiling point of the solvent). The reaction is preferably conducted at room temperature or higher. When conducted at room temperature, the reaction is complete within 36-72 h.

If desired, upon isolating the resulting isonitrile product, it may be further purified by common purification techniques such as chromatographic techniques, particularly column chromatography.

### General method 5: An F-amine moiety, a bis-isonitrile, a carbonyl and phenylphosphinic acid.

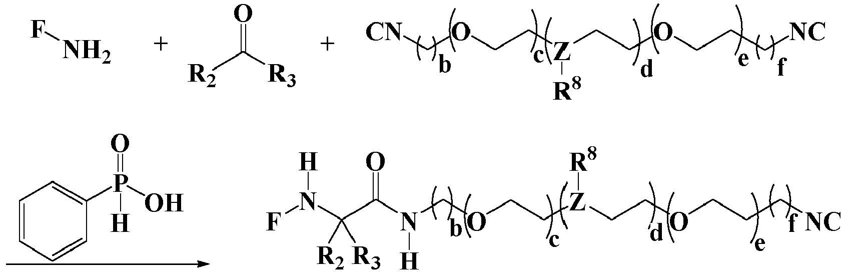

**F, R²**, **R³**, **R⁸**, **Z** and **b-f** are as defined in the detailed description of the invention.

Generally, a functional element **F** comprising a reactive primary amine group, a bis-isonitrile molecule, a carbonyl compound and phenyphosphinic acid are mixed in an organic solvent. Any relative molar amounts of the compounds can be chosen. The isonitrile molecule is preferably in excess, whereas a 2-6 molar excess of the isonitrile can be added to the mixture. Preferably, the reaction is conducted at a molar ratio of **F**: isonitrile:carbonyl:phenyphosphinic acid of 1.2:4:1:1.

Any solvent can be chosen for the reaction, as long as it does not lead to unwanted secondary reactions. A particular choice of solvent is an organic solvent, preferably toluene.

The reaction can be conducted at a temperature comprised between 10 °C and 150 °C (the maximum being dependent on the boiling point of the solvent). The reaction is preferably conducted at 100 °C or higher. When conducted at 130 °C, the reaction is complete within 12-24 h.

If desired, upon isolating the resulting isonitrile product, it may be further purified by common purification techniques such as chromatographic techniques, particularly column chromatography.

### General method 6: An F-amine moiety, a bis-isonitrile, a carbonyl and trimethylsilyl azide.

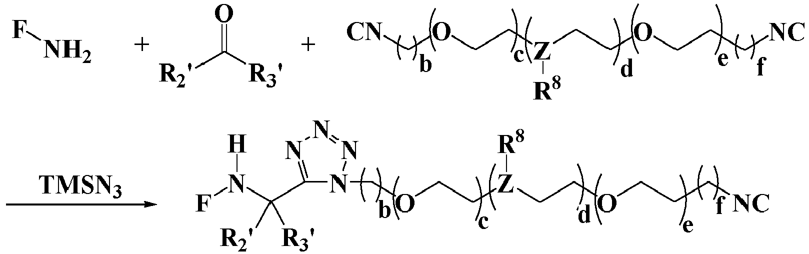

**F, R^{2'}**, **R^{3'}**, **R⁸**, **Z** and **b-f** are as defined in the detailed description of the invention.

General method 6 can be conducted under the same conditions as general method 1 but in this case the reaction uses an **F**-amine moiety and a trimethylsilyl azide (TMSN₃) instead of an **F**-carboxylic acid and a primary amine. The same amounts as those in general method 1 may be chosen, preferably the reaction is conducted at a molar ratio of **F**: isonitrile:TMSN₃:carbonyl compound of 1.2:4:1:1. When conducted at room temperature, the reaction is complete within 24-48 h.

### General method 7: An F-aldehyde moiety, a bis-isonitrile, and an aminoacid.

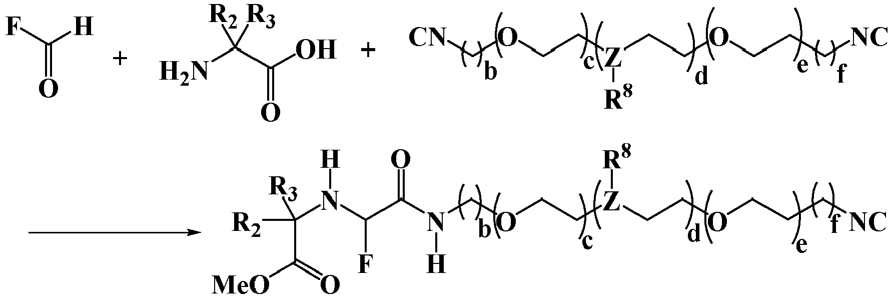

**F**, **R²**, **R³**, **R⁸**, **Z** and **b-f** are as defined in the detailed description of the invention. General method 7 can be conducted under the same conditions as general method 1.

### General method 8: An F-amine moiety, a bis-isonitrile, a carbonyl and an aminoacid.

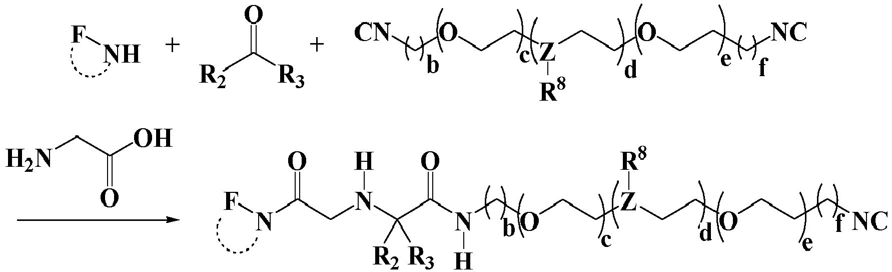

**F**, **R²**, **R³**, **R⁸**, **Z** and **b-f** are as defined in the detailed description of the invention. General method 8 can be conducted under the same conditions as general method 1.

### Exemplary synthesis of compounds of formula (Ia)

### Example 1.

Compound **1** was obtained by following the procedure described above in General method 1.

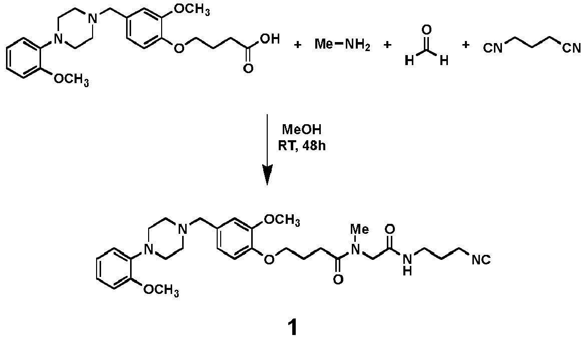

"**RT**" stands for room temperature. Yield: 35%. Melting point: 178-180°C.

¹H RMN (300 MHz, CDCl₃) δ (ppm): 7.51 (t, *J* = 6.3 Hz, 1H), 6.97 - 6.91 (m, 1H), 6.90 - 6.80 (m, 6H), 4.07 (t, *J* = 7.1 Hz, 2H), 3.94 (s, 2H), 3.80 (s, 5H), 3.57 (t, *J* = 1.0 Hz, 2H), 3.39 (dt, *J* = 12.5, 7.1 Hz, 2H), 3.27 (dt, *J* = 12.3, 7.0 Hz, 2H), 3.19 (td, *J* = 7.0, 6.3 Hz, 2H), 3.01 (t, *J* = 7.1 Hz, 2H), 2.90 (s, 2H), 2.63 (t, *J* = 7.1 Hz, 4H), 2.44 (t, *J* = 7.1 Hz, 2H), 2.12 - 1.82 (m, 4H). HRMS (ESI) calcd. for C₃₀H₄₂N₅O₅ [M + H]⁺: 552.3178, found: 552.3182.

### Example 2.

Compound **2** was obtained by following the procedure described above in General method 1.

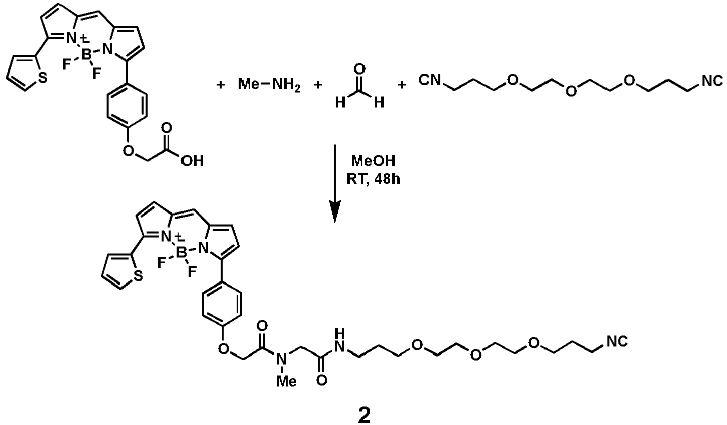

"**RT**" stands for room temperature. Yield: 32%. ¹H NMR (300 MHz, CDCl₃) δ: 8.22 - 8.05 (m, 1H), 8.00 - 7.90 (m, 2H), 7.46 (dd, J = 5.1, J = 1.1 Hz, 1H), 7.21 - 7.12 (m, 2H), 7.10 - 6.98 (m, 4H), 6.81 (d, J = 4.4 Hz, 1H), 6.73 - 6.60 (m, 1H), 6.53 (s, 1H), 4.83 (s, 1H), 4.77 (s, 1H), 4.07 (s, 1H), 4.01 (s, 1H), 3.70 - 3.42 (m, 14H), 3.41 - 3.28 (m, 2H), 3.20 (s, 2H), 3.03 (s, 1H), 1.95 - 1.82 (m, 2H), 1.80 - 1.65 (m, 2H). HRMS (ESI) calcd. for C₃₅H₄₁BF₂N₅O₆S [M + H]⁺ : 708.2833 found: 708.2838

### Example 3.

Compound **3** was obtained by following the procedure described above in General method 1.

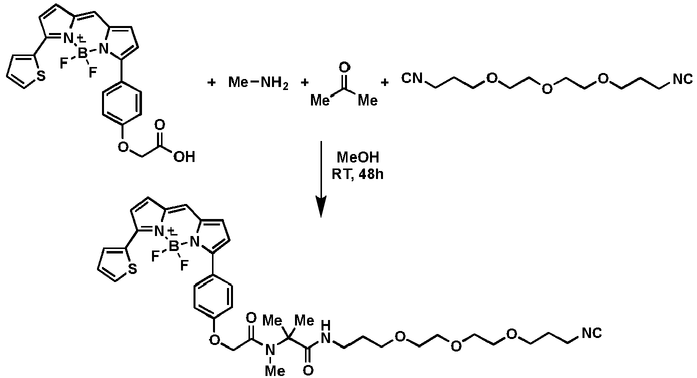

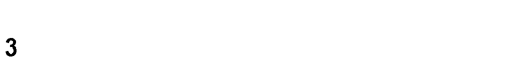

"**RT**" stands for room temperature. Yield: 14%. Melting point: > 190 °C (decomposes).

¹H RMN (300 MHz, CDCl₃) δ (ppm): 8.13 - 8.07 (m, 1H), 7.72 - 7.67 (m, 1H), 7.65 - 7.62 (m, 1H), 7.60 - 7.57 (m, 1H), 7.57 - 7.54 (m, 2H), 7.49 (d, *J* = 7.5 Hz, 1H), 7.44 (t, *J* = 5.8 Hz, 1H), 7.22 (t, *J* = 7.5 Hz, 1H), 7.18 (d, *J* = 7.5 Hz, 1H), 7.10 (d, *J* = 1.3 Hz, 1H), 7.07 - 7.03 (m, 2H), 4.75 (s, 2H), 3.67 - 3.59 (m, 8H), 3.53 (dt, *J* = 17.0, 7.1 Hz, 4H), 3.23 - 3.14 (m, 4H), 2.95 (s, 3H), 2.06 - 1.88 (m, 2H), 1.88 - 1.80 (m, 2H), 1.39 (s, 6H). HRMS (ESI) calcd. for C₃₇H₄₅BF₂N₅O₆S [M + H]⁺: 736.3156, found: 736.3154.

### Example 4.

Compound **4** was obtained by following the procedure described above in General method 1. 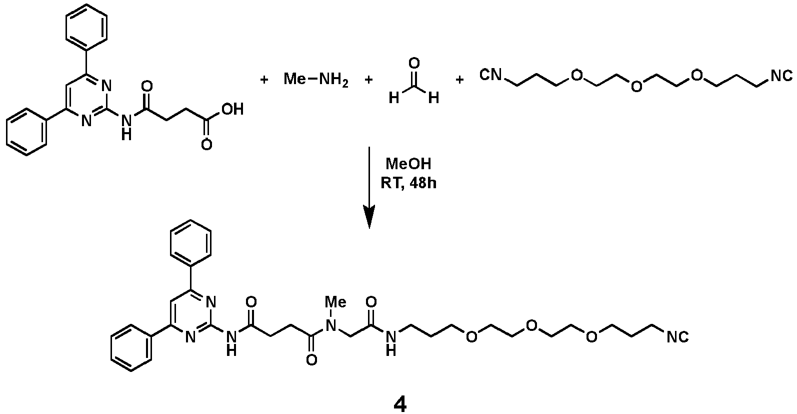 "**RT**" stands for room temperature. Yield: 58%.

¹H NMR (300 MHz, CDCl₃) δ (ppm): 8.51 (s, 1H), 8.20 - 8.02 (m, 4H), 7.82 (s, 1H), 7.63 - 7.45 (m, 6H), 6.91 - 6.83 (m, 1H), 4.09 (s, 2H), 3.71 - 3.42 (m, 14H), 3.39 - 3.28 (m, 2H), 3.21 (s, 3H), 2.85 - 2.61 (m, 4H), 2.01 - 1.72 (m, 4H). HRMS (ESI) calcd. for C₃₄H₄₃N₆O₆ [M+1]⁺: 631.3244, found: 631.3248.

### Example 5.

Compound **5** was obtained by following the procedure described above in General method 1.

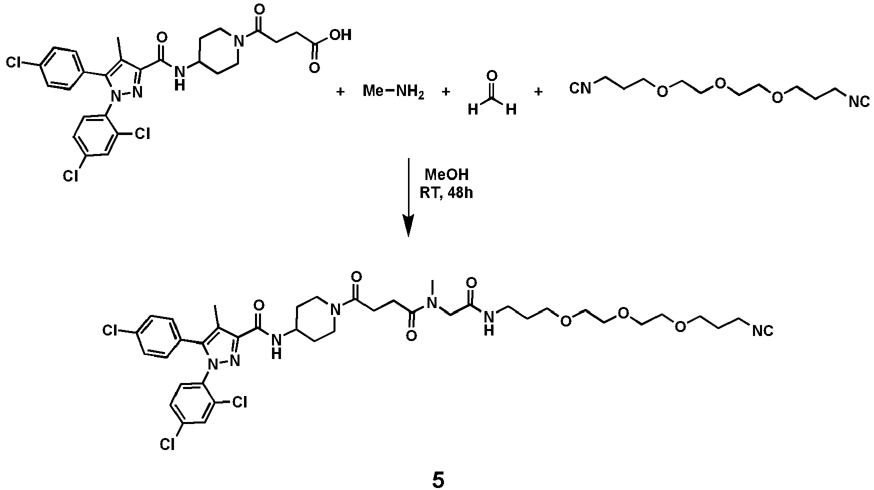

"**RT**" stands for room temperature. Yield: 41%.

¹H NMR (300 MHz, CDCl₃) δ (ppm): 7.83 - 7.79 (m, 1H), 7.54 - 7.50 (m, 1H), 7.46 (s, 1H), 7.39 - 7.31 (m, 4H), 7.18 - 7.15 (m, 2H), 3.96 (s, 2H), 3.90 - 3.81 (m, 1H), 3.72 - 3.47 (m, 14H), 3.41 - 3.34 (m, 2H), 3.22 - 3.15 (m, 4H), 2.93 (s, 3H), 2.62 - 2.52 (m, 4H), 2.34 (s, 3H), 2.05 - 1.96 (m, 2H), 1.93 - 1.79 (m, 6H). HRMS (ESI) calcd. for C₄₀H₅₁Cl₃N₇O₇ [M+1]⁺: 846.2916, found: 846.2920.

### Example 6.

Compound **6** was obtained by following the procedure described above in General method 1.

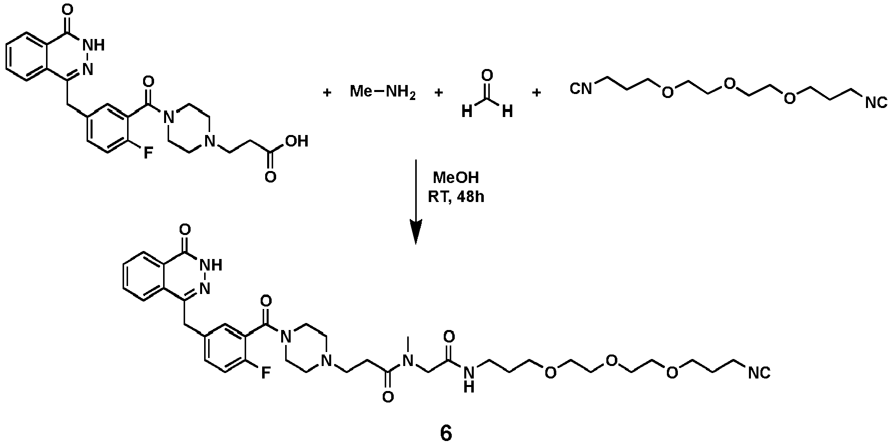

"**RT**" stands for room temperature. Yield: 43%.

¹H NMR (300 MHz, CDCl₃) δ (ppm): 10.76 (s, 1H), 8.51 - 8.37 (m, 1H), 7.83 - 7.64 (m, 3H), 7.37 - 7.19 (m, 3H), 7.01 (t, *J* = 8.8 Hz, 1H), 4.28 (s, 2H), 4.02 - 3.87 (m, 2H), 3.68 - 3.45 (m, 17H), 3.43 - 3.26 (m, 3H), 3.10 (s, 3H), 2.82 - 2.71 (m, 2H), 2.63 - 2.32 (m, 6H), 1.96-1.85 (m, 2H), 1.82-1.71 (m, 2H). HRMS (ESI) calcd. for C₃₇H₄₉FN₇O₇ [M+1]⁺: 722.3678, found: 722.3674.

### Example 7.

Compound **7** was obtained by following the procedure described above in General method 1.

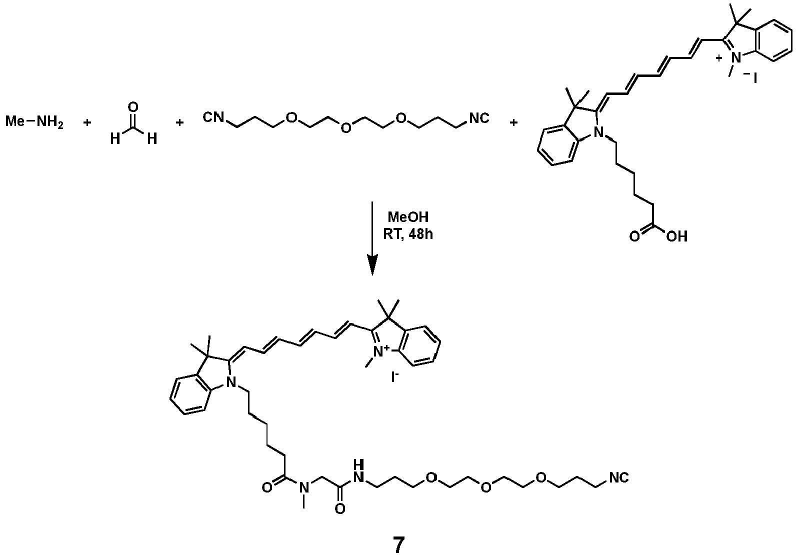

"RT" stands for room temperature. Yield: 16%. Melting point: > 180 °C (decomposes). ¹H RMN (300 MHz, CDCl₃) δ (ppm): 7.88 - 7.48 (m, 3H), 7.44 - 7.27 (m, 4H), 7.25 - 6.99 (m, 4H), 6.92-6.06 (m, 4H), 4.19-3.96 (m, 5H), 3.71-3.45 (m, 16H), 3.43-3.28 (m, 2H), 3.11 (s, 2H), 3.02 (s, 1H), 2.49 (t, J = 7.1 Hz, 1H), 2.39 (t, J = 7.1 Hz, 1H), 1.99 - 1.65 (m, 22H). HRMS (ESI) calcd. for C₄₈H₆₆N₅O₅⁺ [M]⁺: 792.5058, found: 792.5058.

### Example 8.

Compound **8** was obtained by following the procedure described above in General method 1.

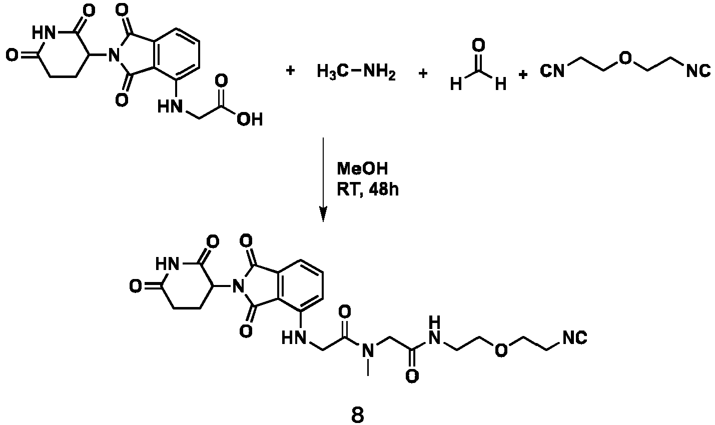

"**RT**" stands for room temperature. Yield: 49%.

¹H NMR (300 MHz, CDCl₃) δ (ppm): 9.50 (s, 1H), 7.69 - 7.50 (m, 3H), 7.18 (d, J = 7.5 Hz, 1H), 7.09 (t, J = 7.6 Hz, 1H), 5.55 - 5.46 (m, 1H), 4.01 - 3.91 (m, 3H), 3.91 - 3.79 (m, 3H), 3.60 - 3.49 (m, 2H), 3.43 - 3.37 (m, 2H), 3.32 - 3.17 (m, 2H), 2.94 (s, 3H), 2.65 - 2.59 (m, 2H), 2.31 - 2.23 (m, 1H), 2.18 - 2.10 (m, 1H). HRMS (ESI) calcd. for C₂₃H₂₇N₆O₇ [M+1]⁺: 499.1941, found: 499.1945.

### Example 9.

Compound **9** was obtained by following the procedure described above in General method 1.

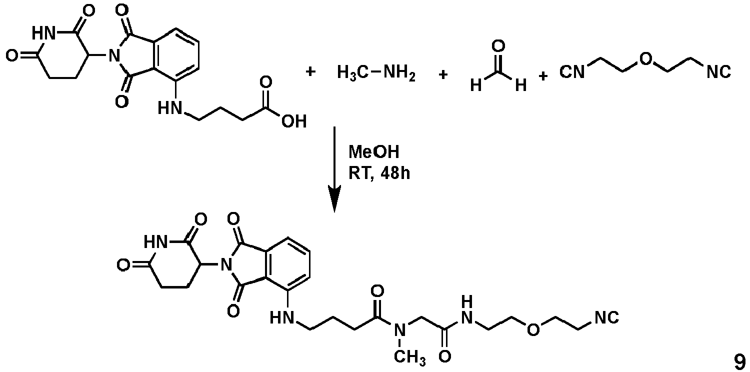

"**RT**" stands for room temperature. Yield: 54%.

¹H NMR (300 MHz, CDCl₃) δ (ppm): 9.35 (s, 1H), 7.80 - 7.71 (m, 1H), 7.68 - 7.57 (m, 2H), 7.57 - 7.48 (m, 1H), 7.17 - 7.09 (m, 1H), 5.54 - 5.46 (m, 1H), 4.00 - 3.94 (m, 2H), 3.87 - 3.78 (m, 2H), 3.60 - 3.49 (m, 2H), 3.44 - 3.34 (m, 4H), 3.32 - 3.17 (m, 2H), 2.96 (s, 3H), 2.67 - 2.57 (m, 2H), 2.45 - 2.03 (m, 4H), 1.92 - 1.71 (m, 2H). HRMS (ESI) calcd. for C₂₅H₃₁N₆O₇ [M+1]⁺: 527.2254, found: 527.2258.

### Example 10.

Compound 10 was obtained by following the procedure described above in General method 1.

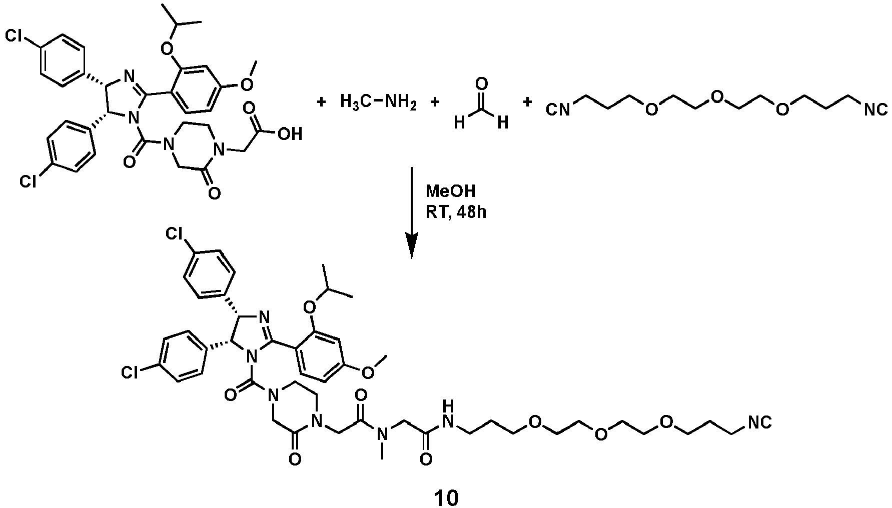

"**RT**" stands for room temperature. Yield: 45%.

¹H NMR (300 MHz, CDCl₃) δ (ppm): 7.77 - 7.73 (m, 1H), 7.54 - 7.48 (m, 1H), 7.41 - 7.35 (m, 2H), 7.34 - 7.25 (m, 6H), 6.71 - 6.66 (m, 1H), 6.61 - 6.58 (m, 1H), 5.81 - 5.77 (m, 1H), 5.52 - 5.47 (m, 1H), 4.68 - 4.58 (m, 1H), 4.07 - 3.91 (m, 6H), 3.80 (s, 3H), 3.77 - 3.49 (m, 15H), 3.47 - 3.39 (m, 1H), 3.27 - 3.10 (m, 4H), 2.92 (s, 3H), 2.07 - 1.94 (m, 3H), 1.90 - 1.79 (m, 1H), 1.37 (d, *J* = 6.9 Hz, 3H), 1.32 (d, *J* = 6.8 Hz, 3H). HRMS (ESI) calcd. for C₄₆H₅₈Cl₂N₇O₉ [M+1]⁺: 922.3674, found: 922.3668.

### Example 11.

Compound **11** was obtained by following the procedure described above in General method 1.

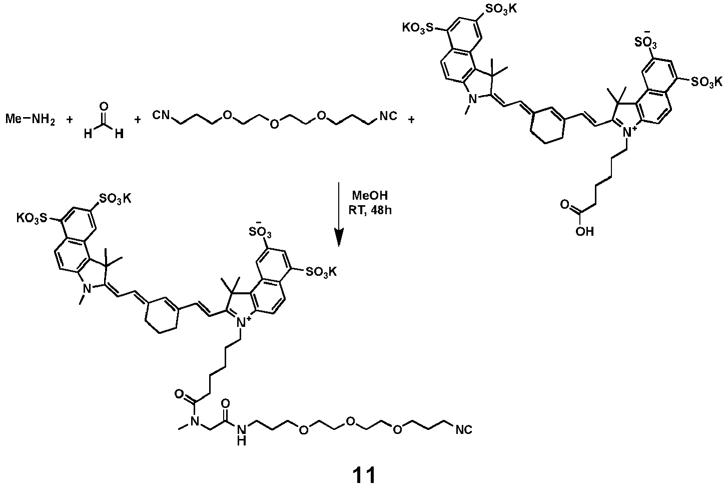

"**RT**" stands for room temperature. Yield: 16%. Melting point: > 190 °C (decomposes).

¹H RMN (300 MHz, CD₃OD) δ: 9.12 - 9.00 (m, 2H), 8.76 (s, 2H), 8.59 (s, 2H), 7.97 - 7.82 (m, 2H), 7.79 - 7.64 (m, 2H), 7.57 (s, 1H), 6.33 - 6.13 (m, 2H), 4.33 - 4.18 (m, 2H), 4.01 - 3.92 (m, 2H), 3.74 (s, 3H), 3.71 - 3.42 (m, 8H), 3.39 - 3.14 (m, 8H), 3.02 (s, 2H), 2.83 (s, 1H), 2.67 - 2.51 (m, 4H), 2.46 (t, J = 7.1 Hz, 1H), 2.35 (t, J = 7.1 Hz, 1H), 2.03 (s, 12H), 1.96 - 1.81 (m, 6H), 1.81 - 1.61 (m, 4H), 1.59 - 1.42 (m, 2H). HRMS (ESI) calcd. for C₅₉H₇₄N₅O₁₇S₄ [M + 4H]⁺: 1252.3957, found: 1252.3954.

### Example 12.

Compound **12** was obtained by following the procedure described above in General method 2.

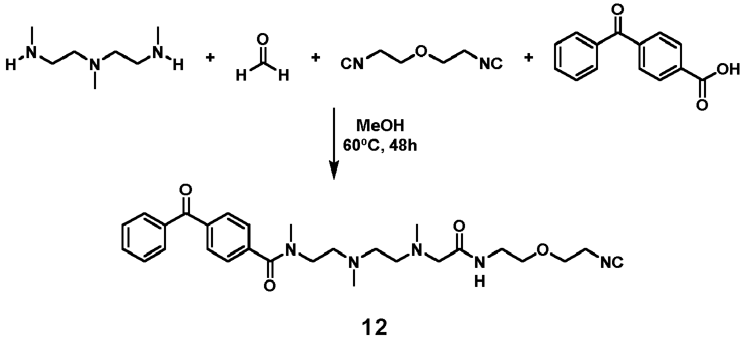

Yield: 64%. Melting point: 167 - 169 °C.

¹H RMN (300 MHz, CDCl₃) δ (ppm): 7.94 - 7.88 (m, 2H), 7.87 - 7.83 (m, 2H), 7.81 - 7.73 (m, 3H), 7.57 - 7.45 (m, 3H), 3.82 (t, *J* = 7.1 Hz, 2H), 3.63 - 3.51 (m, 4H), 3.39 - 3.32 (m, 2H), 3.30 (s, 2H), 3.24 (t, *J* = 7.1 Hz, 2H), 2.90 (s, 3H), 2.72 (t, *J* = 7.1 Hz, 2H), 2.65 - 2.54 (m, 4H), 2.41 (s, 3H), 2.38 (s, 3H). HRMS (ESI) calcd. for C₂₈H₃₈N₅O₄ [M + H]⁺: 508.2928, found: 508.2932.

### Example 13.

Compound **13** was obtained by following the procedure described above in General method 2.

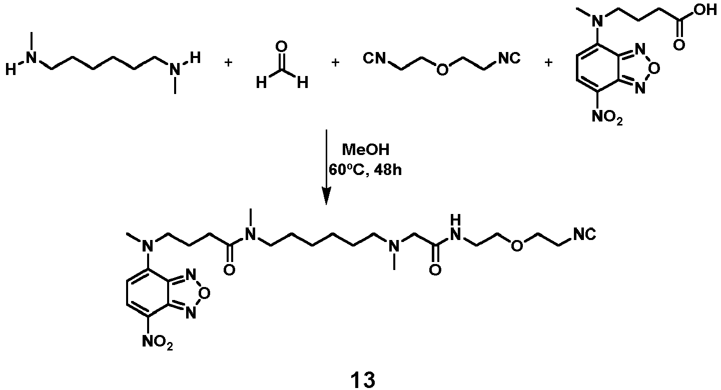

Yield: 52%. Melting point: 177 - 179 °C.

¹H RMN (300 MHz, DMSO-*d*₆) δ (ppm): 8.14 (d, *J* = 7.3 Hz, 1H), 7.71 (t, *J* = 6.9 Hz, 1H), 6.98 (d, *J* = 7.5 Hz, 1H), 3.86 - 3.79 (m, 2H), 3.62 - 3.51 (m, 4H), 3.39 - 3.32 (m, 2H), 3.32 - 3.27 (m, 4H), 3.27 - 3.21 (m, 2H), 3.09 (s, 3H), 2.92 (s, 3H), 2.64 - 2.56 (m, 2H), 2.41 - 2.35 (m, 5H), 1.89 - 1.80 (m, 2H), 1.64 - 1.54 (m, 2H), 1.51 - 1.42 (m, 2H), 1.37 - 1.25 (m, 4H). HRMS (ESI) calcd. for C₂₆H₄₁N₈O₆ [M + H]⁺: 561.3151, found: 561.3147.

### Example 14.

Compound **14** was obtained by following the procedure described above in General method 3.

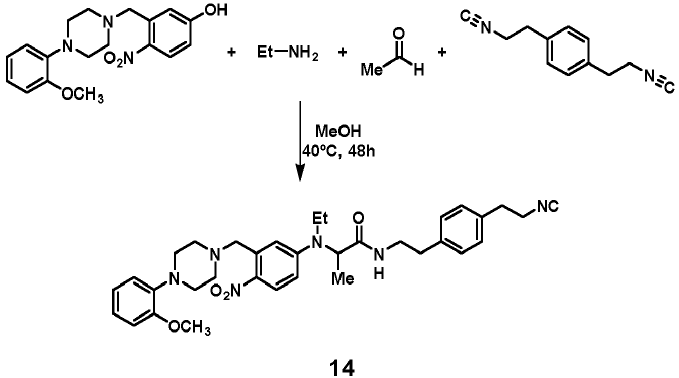

Yield: 60%. Melting point: 192 - 194 °C.

¹H RMN (300 MHz, CDCl₃) δ (ppm): 7.95 (d, *J* = 7.8 Hz, 1H), 7.69 (t, *J* = 6.3 Hz, 1H), 7.15 (dt, *J* = 7.6, 1.0 Hz, 2H), 7.10 (dt, *J* = 7.6, 1.1 Hz, 2H), 7.05 - 7.01 (m, 2H), 6.95 - 6.81 (m, 4H), 4.15 (q, *J* = 6.8 Hz, 1H), 3.86 (d, *J* = 0.9 Hz, 2H), 3.79 (s, 2H), 3.60 - 4.51 (m, 2H), 3.45 - 3.24 (m, 9H), 3.04 - 2.90 (m, 2H), 2.83 - 2.71 (m, 2H), 2.63 (q, *J* = 7.1 Hz, 4H), 1.26 (d, *J* = 6.8 Hz, 3H), 1.20 (t, *J* = 8.0 Hz, 3H). HRMS (ESI) calcd. for C₃₄H₄₃N₆O₄ [M + H]⁺: 599.3344, found: 599.3348.

### Example 15.

Compound **15** was obtained by following the procedure described above in General method 4.

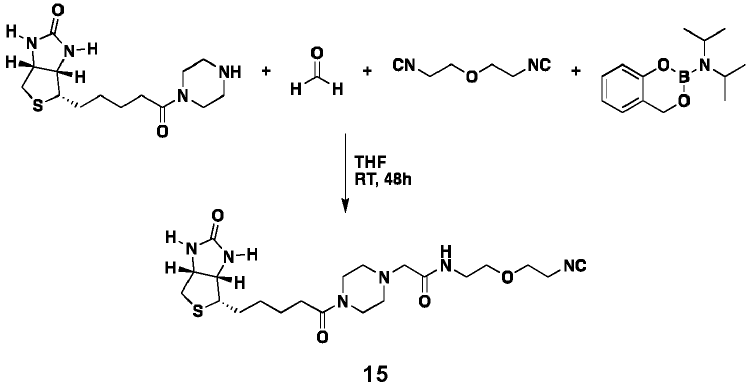

"**RT**" stands for room temperature. Yield: 34%. Melting point: > 120 °C (dec.).

¹H RMN (300 MHz, DMSO-*d*₆) δ (ppm): 6.60 (d, *J* = 7.5 Hz, 1H), 5.80 (d, *J* = 7.1 Hz, 1H), 4.48 - 4.34 (m, 1H), 4.28 - 4.16 (m, 1H), 3.88 - 3.76 (m, 2H), 3.75 - 3.66 (m, 2H), 3.56 - 3.42 (m, 7H), 3.24 - 3.17 (m, 1H), 3.13 - 3.07 (m, 1H), 2.95 - 2.89 (m, 1H), 2.80 - 2.74 (m, 1H), 2.68 - 2.57 (m, 2H), 2.56 - 2.51 (m, 4H), 2.39 - 2.23 (m, 2H), 1.74 - 1.54 (m, 4H), 1.53 - 1.31 (m, 2H). HRMS (EI) calcd. for C₂₁H₃₄N₆O₄ [M]⁺: 466.2358, found: 466.2366.

### Example 16.

Compound **16** was obtained by following the procedure described above in General method 5.

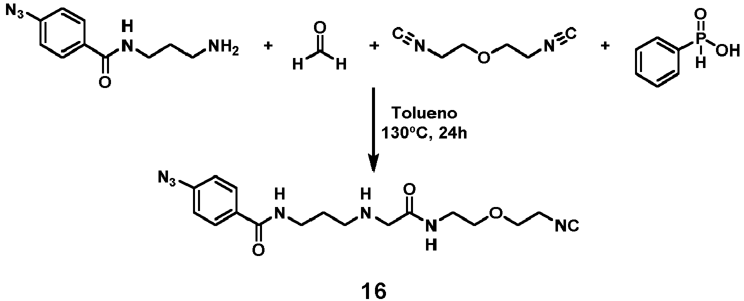

Yield: 28%. Melting point: 153 - 155 °C.

¹H RMN (300 MHz, CDCl₃) δ (ppm): 8.15 (t, *J* = 7.0 Hz, 1H), 7.96 - 7.73 (m, 2H), 7.32 (t, *J* = 6.6 Hz, 1H), 7.21 - 7.03 (m, 2H), 3.74 (t, *J* = 7.1 Hz, 2H), 3.56 (t, *J* = 7.1 Hz, 2H), 3.47 (tt, *J* = 8.4, 6.7 Hz, 1H), 3.31 (q, *J* = 7.0 Hz, 2H), 3.24 - 3.13 (m, 6H), 2.66 (q, *J* = 7.0 Hz, 2H), 1.98 (p, *J* = 7.1 Hz, 2H). HRMS (EI) calcd. for C₁₇H₂₃N₇O₃ [M]⁺: 373.1860, found: 373.1864.

### Example 17.

Compound **17** was obtained by following the procedure described above in General method 5.

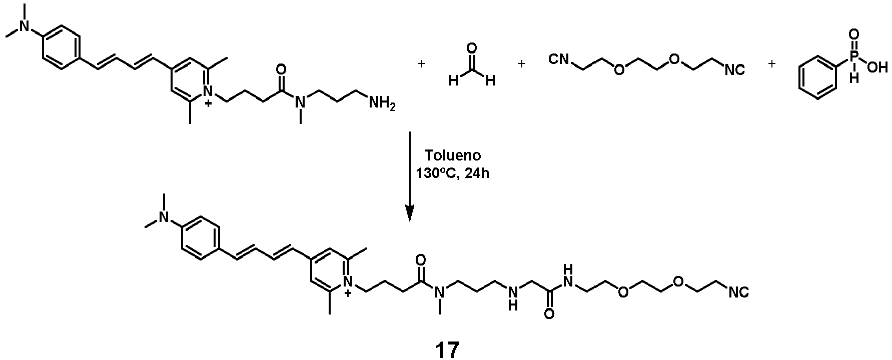

Yield: 19%. Melting point: >190 °C (dec.).

¹H RMN (300 MHz, DMSO-*d*₆) δ (ppm): 7.91 - 7.82 (m, 3H), 7.49 - 7.36 (m, 2H), 7.08 - 6.92 (m, 2H), 6.81 - 6.66 (m, 4H), 4.48 (t, *J* = 7.0 Hz, 2H), 3.84 (t, *J* = 7.1 Hz, 2H), 3.68 - 3.62 (m, 2H), 3.61 - 3.53 (m, 4H), 3.45 - 3.29 (m, 7H), 3.27 - 3.20 (m, 1H), 3.12 - 3.03 (m, 1H), 2.99 (s, 6H), 2.85 (s, 3H), 2.80 - 2.73 (m, 2H), 2.49 (s, 6H), 2.47 - 2.42 (m, 2H), 2.38 - 2.29 (m, 2H), 1.80 - 1.71 (m, 2H). HRMS (ESI) calcd. for C₃₆H₅₃N₆O₄ [M]⁺: 633.4125, found: 633.4121.
found: 373.1864.

### Example 18.

Compound **18** was obtained by following the procedure described above in General method 6.

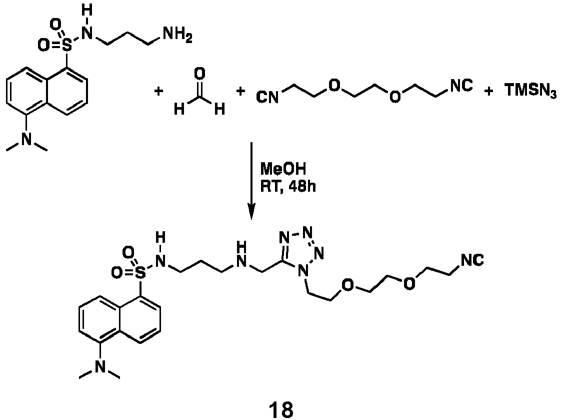

"**RT**" stands for room temperature. Yield: 42%. Melting point: 168 - 170 °C.

¹H RMN (300 MHz, CDCl₃) δ (ppm): 8.32 (d, *J* = 7.5 Hz, 1H), 8.20 (d, *J* = 7.5 Hz, 1H), 7.87 (d, *J* = 7.5 Hz, 1H), 7.70 - 7.60 (m, 1H), 7.57 - 7.46 (m, 2H), 7.33 - 7.26 (m, 1H), 7.17 (d, *J* = 7.5 Hz, 1H), 4.52 - 4.43 (m, 2H), 4.14 (d, *J* = 8.3 Hz, 2H), 3.94 - 3.88 (m, 2H), 3.86 - 3.80 (m, 2H), 3.64 - 3.58 (m, 2H), 3.58 - 3.52 (m, 2H), 3.24 (t, *J* = 7.0 Hz, 2H), 2.96 - 2.84 (m, 4H), 2.82 (s, 6H), 1.92 - 1.80 (m, 2H). HRMS (ESI) calcd. for C₂₄H₃₅N₈O₄S [M + H]⁺: 531,2506, found: 531.2510.

### Example 19.

Compound **19** was obtained by following the procedure described above in General method 6 but the functional element **F** comprised a reactive secondary amine group as the one of general method 4.

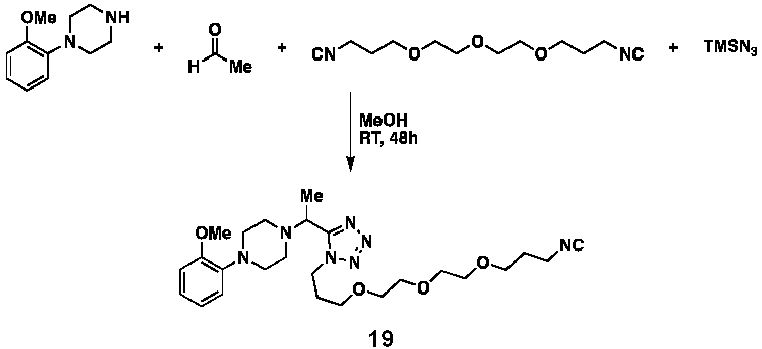

"**RT**" stands for room temperature. Yield: 46%. Melting point: 171 - 172 °C.

¹H RMN (300 MHz, CDCl₄) δ (ppm): 6.98 - 6.79 (m, 4H), 4.86 - 4.76 (m, 1H), 4.45 - 4.32 (m, 2H), 3.79 (s, 3H), 3.77 - 3.46 (m, 12H), 3.34 - 3.25 (m, 4H), 3.13 - 2.88 (m, 6H), 2.24 - 1.97 (m, 3H), 1.96 - 1.85 (m, 1H), 1.56 (d, *J* = 6.8 Hz, 3H). HRMS (ESI) calcd. for C₂₅H₄₀N₇O₄ [M + H]⁺: 502.3148, found: 502.3144.

### Example 20.

Compound **20** was obtained by following the procedure described above in General method 7.

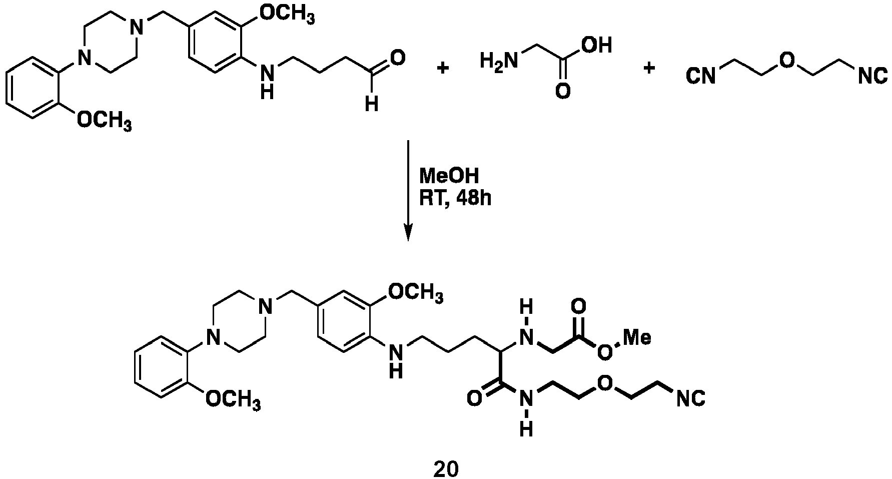

"RT" stands for room temperature. Yield: 48%. Melting point: 184 - 186 °C.

¹H RMN (300 MHz, DMSO-*d*₆) δ (ppm): 7.90 (t, *J* = 6.6 Hz, 1H), 7.03 (d, *J* = 7.5 Hz, 1H), 6.96 - 6.91 (m, 1H), 6.91 - 6.81 (m, 4H), 6.80 (d, *J* = 7.4 Hz, 1H), 6.75 (t, *J* = 6.4 Hz, 1H), 3.90-3.76 (m, 8H), 3.71-3.63 (m, 4H), 3.60-3.16 (m, 17H), 2.68-2.58 (m, 4H), 1.72 - 1.62 (m, 4H). HRMS (ESI) calcd. for C₃₂H₄₇N₆O₆ [M + H]⁺: 611.3561, found: 611.3559.

### Example 21.

Compound **21** was obtained by following the procedure described above in General method 7.

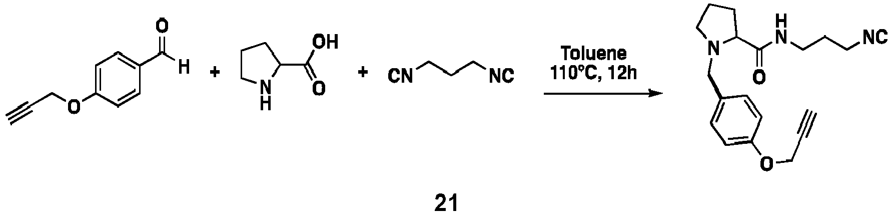

Yield: 39%. Melting point: 156 - 158 °C.

¹H RMN (300 MHz, CDCl₃) δ (ppm): 7.73 - 7.38 (m, 1H), 7.16 - 6.98 (m, 2H), 6.98 - 6.80 (m, 2H), 4.83 (dd, *J* = 12.5, 2.9 Hz, 1H), 4.69 (dd, *J* = 12.5, 3.1 Hz, 1H), 4.41 -4.30 (m, 1H), 3.69 - 3.60 (m, 2H), 3.51 (t, *J* = 3.0 Hz, 1H), 3.26 - 3.05 (m, 4H), 2.05 - 1.94 (m, 1H), 1.94 - 1.79 (m, 5H). HRMS (ESI) calcd. for C₁₈H₂₂N₃O₂ [M + H]⁺: 312.1714, found: 312.1714.

### Example 22.

Compound **22** was obtained by following the procedure described above in General method 1 but wherein the amine compound **R¹**-NH₂ was also an amine-**F** compound.

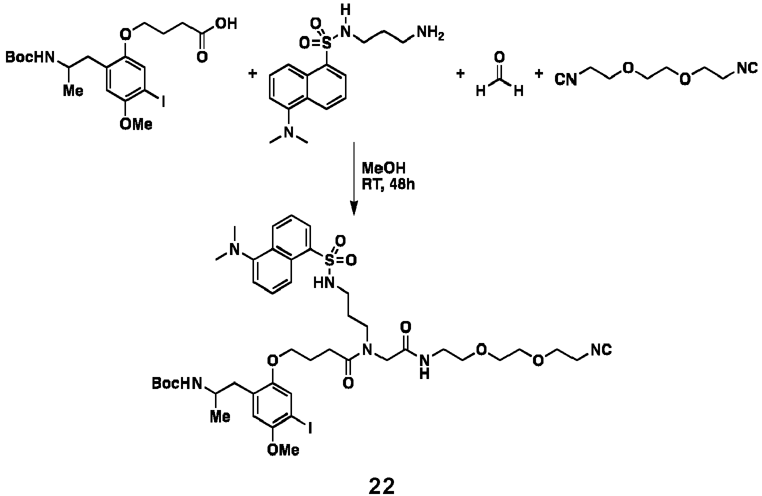

"**RT**" stands for room temperature. Yield: 27%. Melting point: 221 - 223 °C.

¹H RMN (300 MHz, DMSO-*d*₆) δ (ppm): 8.37 (d, *J* = 7.5 Hz, 1H), 8.19 (d, *J* = 7.5 Hz, 1H), 7.95 - 7.87 (m, 2H), 7.60 - 7.53 (m, 1H), 7.52 - 7.47 (m, 1H), 7.33 (t, *J* = 9.5 Hz, 1H), 7.23 (s, 1H), 7.17 (d, *J* = 7.6 Hz, 1H), 6.68-6.64 (m, 1H), 6.52 (d, *J* = 8.8 Hz, 1H), 4.11 - 3.99 (m, 2H), 3.99 - 3.90 (m, 3H), 3.90 - 3.77 (m, 5H), 3.76 - 3.67 (m, 1H), 3.65 - 3.48 (m, 5H), 3.46 - 3.28 (m, 6H), 3.10 - 3.00 (m, 1H), 2.95 - 2.84 (m, 3H), 2.82 (s, 6H), 2.52 - 2.38 (m, 2H), 2.10 - 1.96 (m, 2H), 1.94 - 1.77 (m, 2H), 1.40 (s, 9H), 1.19 (d, *J* = 6.8 Hz, 3H). HRMS (ESI) calcd. for C₄₃H₆₂IN₆O₁₀S [M + H]⁺: 981.3291, found: 981.3297.

### Example 23.

Compound **23** was obtained by following the procedure described above in General method 1 but wherein **R¹** was **F.**

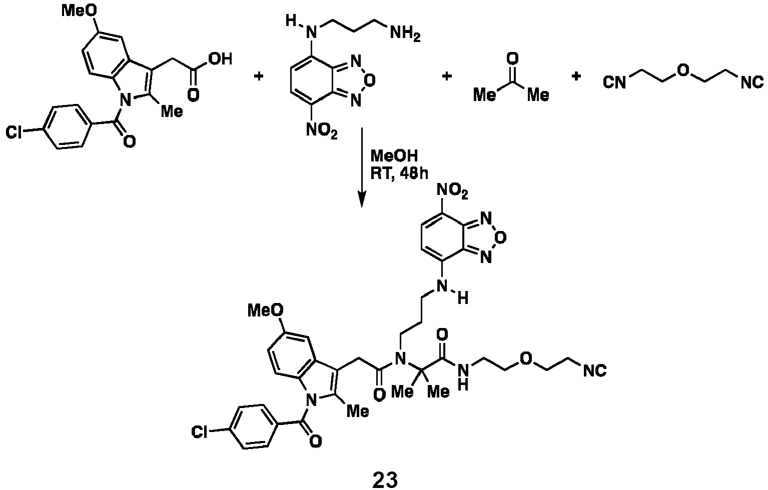

"**RT**" stands for room temperature. Yield: 33%. Melting point: 187 - 189 °C.

¹H RMN (300 MHz, DMSO-*d*₆) δ (ppm): 8.16 (d, *J* = 7.5 Hz, 1H), 7.92 - 7.85 (m, 1H), 7.75 - 7.67 (m, 3H), 7.54 - 7.48 (m, 2H), 7.03 - 6.98 (m, 2H), 6.82 (s, 1H), 6.78 (d, *J* = 7.5 Hz, 1H), 3.86 - 3.81 (m, 2H), 3.79 (s, 3H), 3.75 (s, 2H), 3.57 - 3.50 (m, 2H), 3.45 - 3.33 (m, 7H), 3.29 - 3.20 (m, 1H), 2.40 (s, 3H), 2.06 - 1.92 (m, 2H), 1.48 (s, 6H). HRMS (ESI) calcd. for C₃₇H₄₀ClN₈O₈ [M + H]⁺: 759.2960, found: 759.2962.

### Example 24.

Compound **24** was obtained by following the procedure described above in General method 1 but wherein **R¹** was **F**.

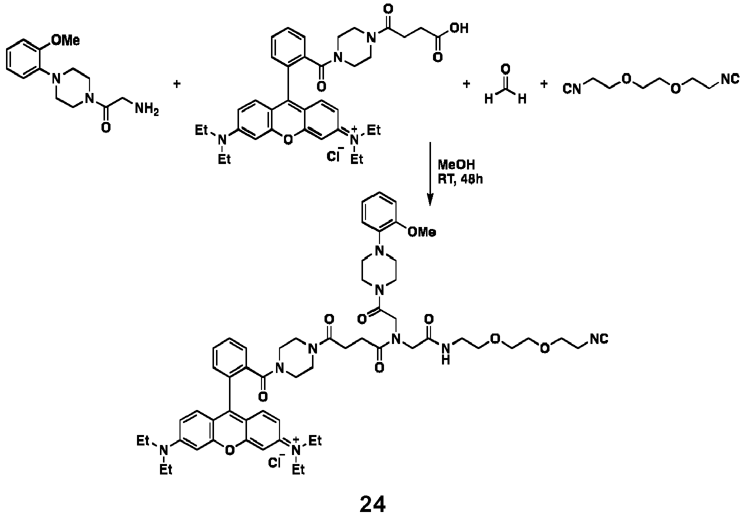

"**RT**" stands for room temperature. Yield: 26%. Melting point: > 200 °C (dec.). ¹H RMN (300 MHz, DMSO-*d*₆) δ (ppm): 7.90 (t, *J* = 6.8 Hz, 1H), 7.74 - 7.70 (m, 1H), 7.68 - 7.61 (m, 2H), 7.58 - 7.54 (m, 1H), 7.31 - 7.26 (m, 1H), 7.24 - 7.19 (m, 1H), 6.99 - 6.91 (m, 3H), 6.91 - 6.81 (m, 5H), 4.00 (s, 2H), 3.97 (s, 2H), 3.84 (t, *J* = 7.1 Hz, 2H), 3.78 (s, 3H), 3.69 - 3.41 (m, 30H), 3.41 - 3.33 (m, 4H), 2.63 - 2.56 (m, 4H), 1.24 (t, *J* = 8.0 Hz, 6H), 1.15 (t, *J* = 8.0 Hz, 6H). HRMS (ESI) calcd. for C₅₈H₇₄N₉O₉ [M]⁺: 1040.5600, found: 1040.5606.

### Example 25.

Compound **25** was obtained by following the procedure described above in General method 1 but wherein **Y** was N-alkyl, substituted with **F.**

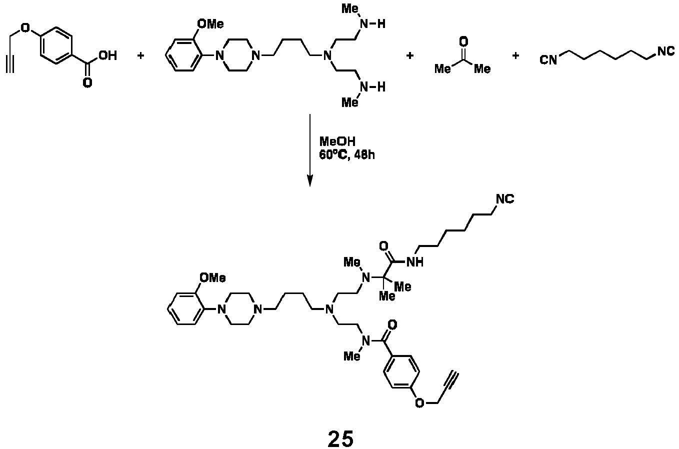

Yield: 34%. Melting point: 175 - 176 °C.

¹H RMN (300 MHz, DMSO-*d*₆) δ (ppm): 7.62 - 7.52 (m, 2H), 7.52 - 7.43 (m, 1H), 7.03 - 6.79 (m, 6H), 4.71 (s, 2H), 3.83 (s, 3H), 3.63 - 3.52 (m, 2H), 3.25 - 3.07 (m, 8H), 2.87 (s, 3H), 2.80 - 2.72 (m, 2H), 2.72 - 2.55 (m, 8H), 2.54 - 2.44 (m, 5H), 2.42 (s, 3H), 1.77 - 1.67 (m, 2H), 1.64 - 1.41 (m, 8H), 1.40 - 1.29 (m, 8H). HRMS (ESI) calcd. for C₄₂H₆₄N₄O₄ [M + H]⁺: 730.5026, found: 730.5028.

### Example 26.

Compound **26** was obtained by following the procedure described above in General method 1 but wherein Y was N-alkyl, substituted with **F.**

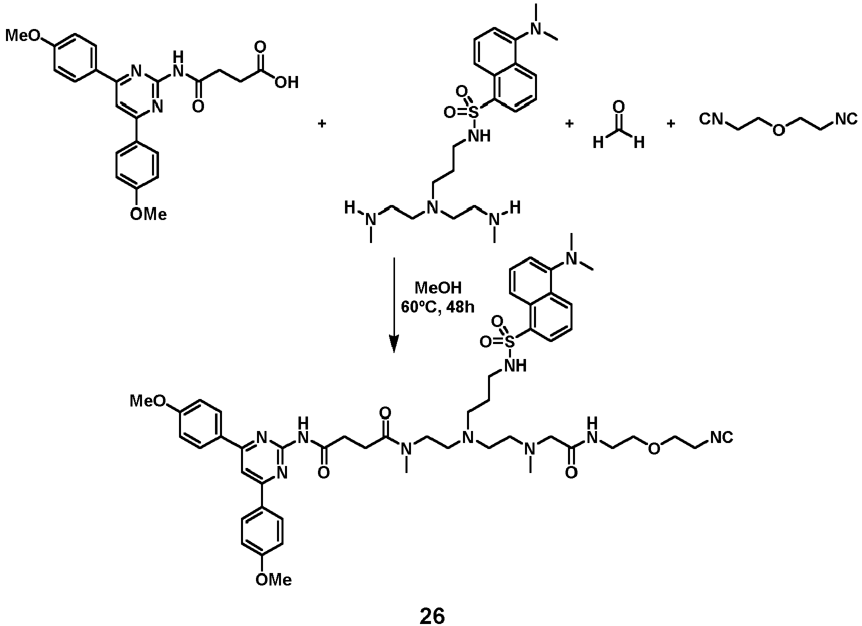

Yield: 31%. Melting point: 207 - 209 °C.

¹H RMN (300 MHz, DMSO-*d*₆) δ (ppm): 8.37 (d, *J* = 7.5 Hz, 1H), 8.18 (d, *J* = 7.5 Hz, 1H), 8.13 (s, 1H), 7.95 - 7.91 (m, 1H), 7.91 - 7.84 (m, 5H), 7.57 (t, *J* = 7.5 Hz, 1H), 7.50 (t, *J* = 7.5 Hz, 1H), 7.29 (t, *J* = 9.5 Hz, 1H), 7.19 - 7.14 (m, 1H), 6.95 - 6.81 (m, 4H), 3.83 (t, *J* = 7.1 Hz, 2H), 3.78 (s, 6H), 3.57 - 3.50 (m, 4H), 3.42 - 3.32 (m, 3H), 3.30 (s, 2H), 3.28 - 3.20 (m, 1H), 3.05 - 2.96 (m, 2H), 2.82 (s, 6H), 2.81 (s, 3H), 2.66 - 2.54 (m, 10H), 2.50 (t, *J=* 7.1 Hz, 2H), 2.39 (s, 3H), 1.88 -1.80 (m, 2H). HRMS (ESI) calcd. forC₅₀H₆₅N₁₀O₈S [M + H]⁺: 965.4706, found: 965.4702.

### Example 27.

Compound **27** was obtained by following the procedure described above in General method 2 but wherein Y was N-alkyl, substituted with **F**.

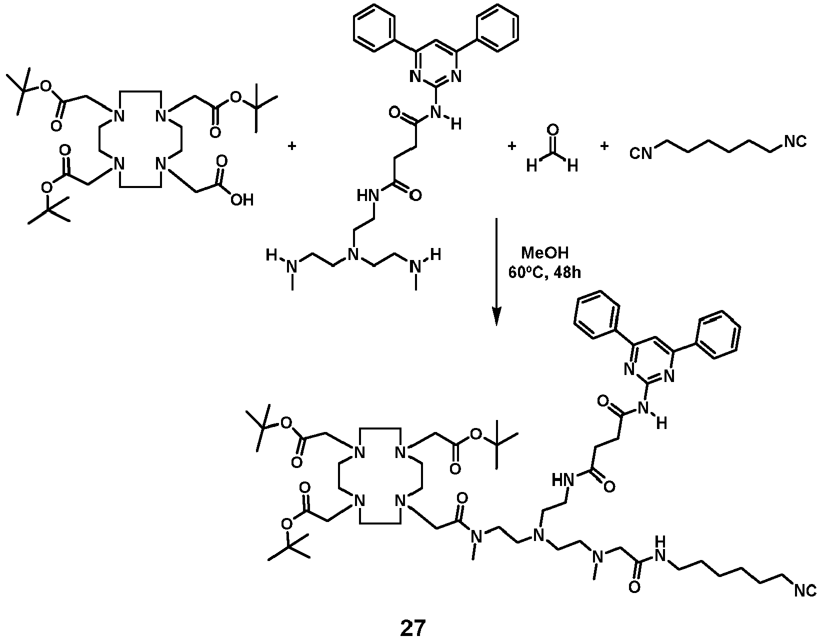

Yield: 21%. Melting point: 189-191 °C.

¹H RMN (300 MHz, DMSO-*d*₆) δ (ppm): 8.17 - 8.12 (m, 4H), 8.02 (s, 1H), 7.60 - 7.55 (m, 1H), 7.52 - 7.45 (m, 5H), 7.44 - 7.39 (m, 2H), 3.54 - 3.49 (m, 2H), 3.42 - 3.31 (m, 8H), 3.31 - 3.25 (m, 2H), 3.14 (s, 2H), 3.13 - 3.07 (m, 4H), 2.88 (s, 3H), 2.77 - 2.69 (m, 4H), 2.67 - 2.54 (m, 21H), 2.50 - 2.43 (m, 5H), 1.76 - 1.68 (m, 2H), 1.43 (s, 33H), 1.38 - 1.29 (m, 2H). HRMS (ESI) calcd. for C₆₅H₁₀₂N₁₃O₁₀ [M + H]⁺: 1224.7871, found: 1224.7876.

### Example 28.

Compound **28** was obtained by following the procedure described above in General method 4 but wherein the reactive secondary amine comprises not one but two functional element **F** groups.

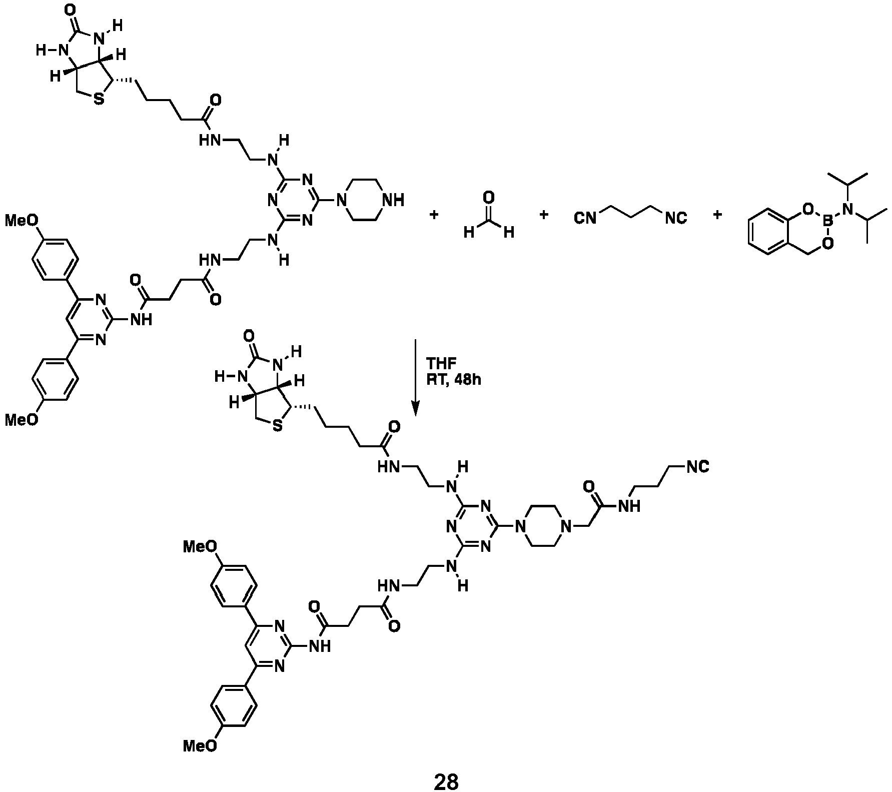

"**RT**" stands for room temperature. Yield: 17%. Melting point: > 200 °C (dec.).

¹H RMN (300 MHz, DMSO-*d*₆) δ (ppm): 8.13 (s, 1H), 7.92 - 7.86 (m, 4H), 7.86 - 7.80 (m, 1H), 7.73 - 7.66 (m, 1H), 7.55 - 7.48 (m, 1H), 6.91 - 6.87 (m, 4H), 6.87 - 6.82 (m, 2H), 6.65 (d, *J* = 7.5 Hz, 1H), 5.91 (d, *J* = 7.1 Hz, 1H), 4.46 - 4.36 (m, 1H), 4.27 - 4.18 (m, 1H), 3.78 (s, 6H), 3.75 - 3.55 (m, 8H), 3.55 - 3.41 (m, 4H), 3.26 - 3.06 (m, 6H), 3.01 - 2.88 (m, 2H), 2.81 - 2.73 (m, 1H), 2.72 - 2.67 (m, 4H), 2.67 - 2.56 (m, 2H), 2.50 - 2.36 (m, 2H), 2.22 - 2.13 (m, 2H), 2.04-1.81 (m, 2H), 1.71 - 1.54 (m, 4H), 1.53 - 1.43 (m, 1H), 1.41 - 1.30 (m, 1H). HRMS (ESI) calcd. for C₄₉H₆₅N₁₆O₇S [M + H]⁺: 1021.4947, found: 1021.4945.

### Example 29.

Compound **29** was obtained by following the procedure described above in General method 1 but wherein **R⁸** was a moiety of formula (**V**) and **L** an isonitrile. Therefore, this example shows a reaction with a tri-isonitrile.

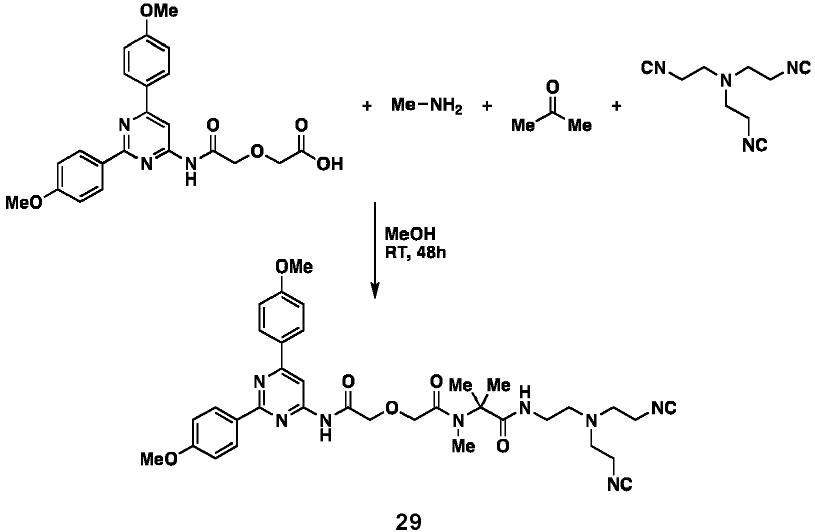

"**RT**" stands for room temperature. Yield: 28%. Melting point: 194 - 196 °C.

¹H RMN (300 MHz, DMSO-*d*₆) δ (ppm): 8.15 (s, 1H), 8.12 - 8.05 (m, 2H), 7.91 - 7.84 (m, 2H), 7.73 - 7.65 (m, 1H), 7.07 - 6.99 (m, 2H), 6.93 - 6.85 (m, 2H), 4.25 (s, 2H), 4.20 (s, 2H), 3.80 (s, 3H), 3.78 (s, 3H), 3.39 - 3.28 (m, 2H), 3.19 - 3.06 (m, 4H), 2.95 (s, 3H), 2.82 -2.71 (m, 4H), 2.61 - 2.50 (m, 2H), 1.40 (s, 6H). HRMS (ESI) calcd. for C₃₅H₄₃N₈O₆ [M + H]⁺: 671.3308, found: 671.3310.

### Example 30.

Compound **30** was obtained by following the procedure described above in General method 1 but wherein **R⁸** was a moiety of formula (**V**) and **L** an isonitrile. Therefore, this example shows a reaction with a tri-isonitrile.

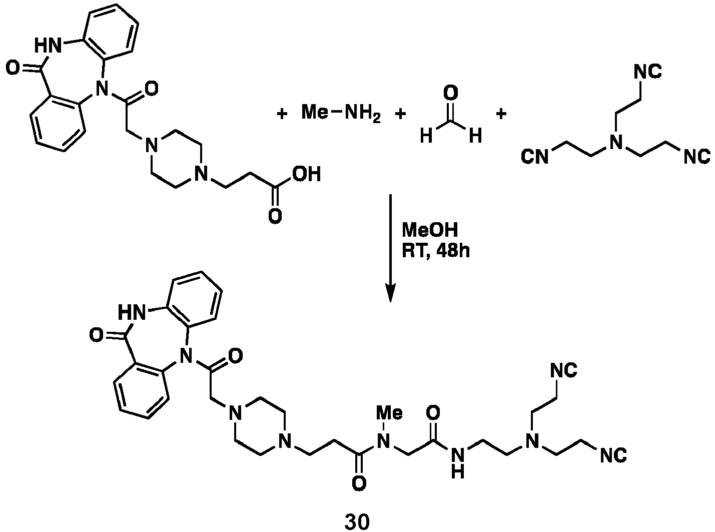

"**RT**" stands for room temperature. Yield: 49%. Melting point: > 170 °C (dec.).

¹H RMN (300 MHz, DMSO-*d*₆) δ (ppm): 9.87 (s, 1H), 8.42 (d, *J* = 7.5 Hz, 1H), 8.29 (d, *J* = 7.4 Hz, 1H), 7.92 (d, *J* = 7.5 Hz, 1H), 7.87 - 7.75 (m, 2H), 7.31 - 7.27 (m, 1H), 7.19 - 7.10 (m, 1H), 6.99 - 6.92 (m, 1H), 3.94 (s, 2H), 3.42 (s, 2H), 3.28 - 3.20 (m, 2H), 3.19 - 3.05 (m, 4H), 3.01 (s, 3H), 2.63 - 2.52 (m, 12H), 2.50 - 2.43 (m, 4H), 2.41 (m, 2H). HRMS (ESI) calcd. for C₃₂H₄₁N₁₀O₄ [M + H]⁺: 629.3315, found: 629.3311.

### Example 31.

Compound **31** was obtained by following the procedure described above in General method 1 but wherein **R⁸** was a moiety of formula (**V**) and **L** an isonitrile.

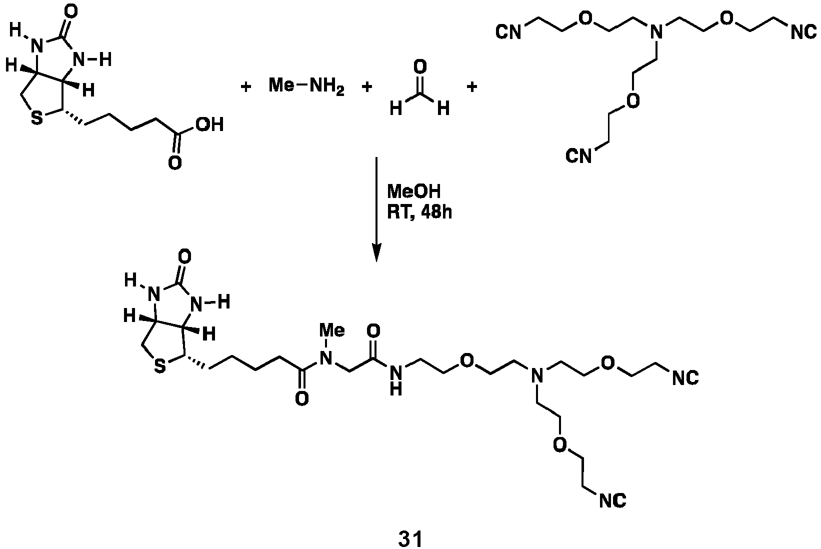

"**RT**" stands for room temperature. Yield: 34%.

¹H NMR (300 MHz, CDCl₃) δ (ppm): 7.65 - 7.56 (m, 1H), 6.70 - 6.62 (m, 1H), 5.92 - 5.85 (m, 1H), 4.43 - 4.35 (m, 1H), 4.26 - 4.20 (m, 1H), 4.01 - 3.92 (m, 2H), 3.87 - 3.74 (m, 4H), 3.64 - 3.32 (m, 12H), 3.25 - 3.03 (m, 7H), 2.96 - 2.89 (m, 4H), 2.86 - 2.71 (m, 3H), 2.40 - 2.26 (m, 2H), 1.72 - 1.57 (m, 4H), 1.53 - 1.32 (m, 2H). HRMS (ESI) calcd. for C₂₇H₄₆N₇O₆S [M+1]⁺: 596.3233, found: 596.3227.

### Example 32.

Compound **32** was obtained by following the procedure described above in General method 1 but wherein the precursor isonitrile was compound **31.**

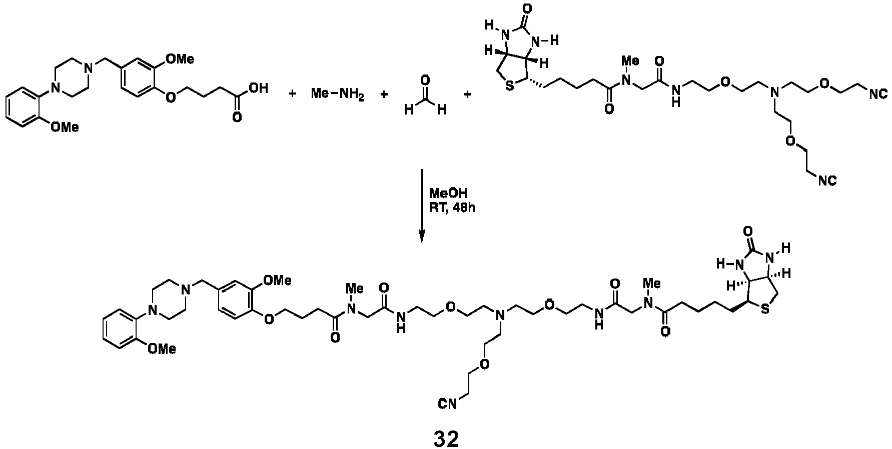

"**RT**" stands for room temperature. Yield: 27%.

¹H NMR (300 MHz, DMSO-*d*₆) δ (ppm): 9.89 - 9.82 (m, 2H), 8.79 - 8.75 (m, 1H), 8.63 - 8.60 (m, 1H), 6.97 - 6.77 (m, 7H), 4.47 - 4.36 (m, 1H), 4.26 - 4.19 (m, 1H), 4.13 - 3.92 (m, 6H), 3.85 - 3.77 (m, 8H), 3.66 - 3.47 (m, 12H), 3.46 - 3.24 (m, 8H), 3.21 - 3.06 (m, 8H), 3.00-2.90 (m, 11H), 2.89 - 2.81 (m, 1H), 2.80-2.73 (m, 1H), 2.52 - 2.41 (m, 2H), 2.39-2.27 (m, 2H), 2.08-2.00 (m, 2H), 1.72-1.57 (m, 4H), 1.52-1.34 (m, 2H). HRMS (ESI) calcd. for C₅₂H₈₁N₁₀O₁₁S [M+1]⁺: 1053.5719, found: 1053.5723.

### Example 33.

Compound **33** was obtained by following the procedure described above in General method 2 but wherein R**⁸ was** a moiety of formula (V) and L an isonitrile.

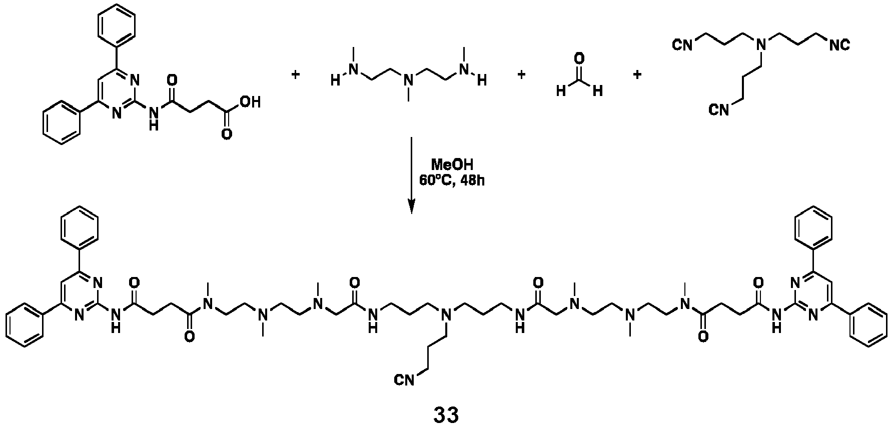

Yield: 37%.

¹H NMR (300 MHz, CDCl₃) δ (ppm): 8.21 - 8.16 (m, 2H), 7.78 - 7.70 (m, 8H), 7.51 - 7.34 (m, 14H), 3.57 - 3.50 (m, 4H), 3.38 - 3.20 (m, 4H), 3.22 - 3.14 (m, 5H), 3.05 - 2.97 (m, 1H), 2.91 - 2.76 (m, 18H), 2.66 - 2.60 (m, 6H), 2.59 - 2.53 (m, 4H), 2.51 - 2.45 (m, 4H), 2.36 (s, 6H), 2.30 (s, 6H), 1.91 - 1.76 (m, 2H), 1.73 - 1.64 (m, 4H). HRMS (ESI) calcd. for C68H91N16O6 [M+1]+: 1227.7310, found: 1227.7306.

### Example 34.

Compound **34** was obtained by following the procedure described above in General method 2 but wherein **R**⁸ was a moiety of formula (**V**) and **L** an isonitrile.

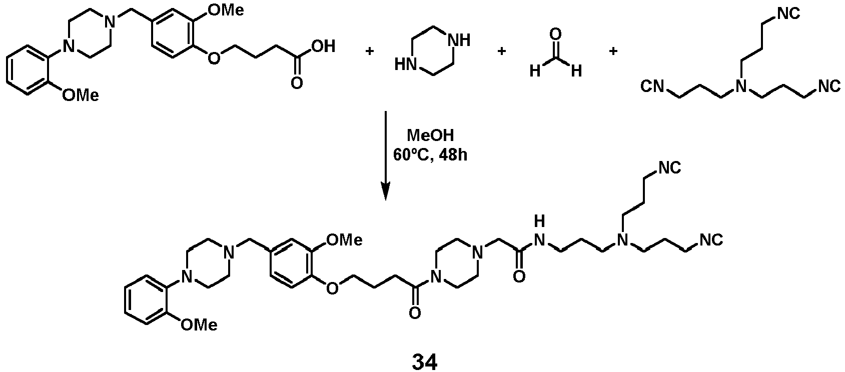

Yield: 38%. Melting point: 168 - 170 °C.

¹H RMN (300 MHz, DMSO-*d*₆) δ (ppm): 7.52 (t, *J* = 6.7 Hz, 1H), 6.96 - 6.91 (m, 1H), 6.91 - 6.85 (m, 4H), 6.85 - 6.81 (m, 2H), 4.08 (t, *J* = 7.1 Hz, 2H), 3.80 (s, 3H), 3.79 (s, 3H), 3.57 (t, *J* = 1.0 Hz, 2H), 3.52 - 3.45 (m, 4H), 3.44 - 3.36 (m, 2H), 3.30 - 3.23 (m, 2H), 3.22 - 3.14 (m, 6H), 3.05 - 2.97 (m, 2H), 2.68 - 2.41 (m, 16H), 2.08 - 1.99 (m, 2H), 1.92 - 1.75 (m, 4H), 1.74 - 1.65 (m, 2H). HRMS (ESI) calcd. for C₄₀H₅₉N₈O₅ [M + H]⁺: 731.4610, found: 731.4614.

### Example 35.

Compound **35** was obtained by following the procedure described above in General method 2 but wherein **R⁸** was a moiety of formula (**V**) and **L** an isonitrile.

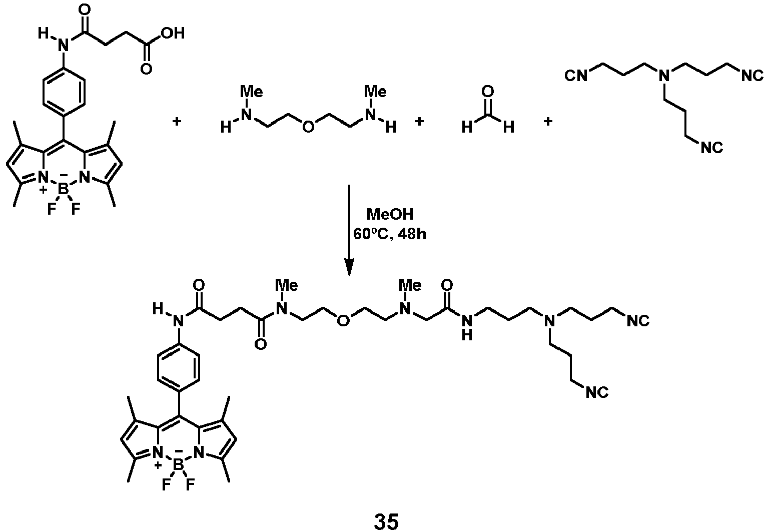

Yield: 42%. Melting point > 170 °C (dec.).

¹H RMN (500 MHz, Methanol-*d*₄) δ 9.55 (s, 1H), 7.81 - 7.75 (m, 2H), 7.59 - 7.53 (m, 3H), 7.50 (t, *J* = 6.7 Hz, 1H), 6.00 (s, 1H), 3.77 - 3.71 (m, 2H), 3.62 - 3.51 (m, 4H), 3.32 (s, 2H), 3.21 - 3.13 (m, 4H), 3.05 - 2.98 (m, 2H), 2.81 (s, 3H), 2.72 (t, *J* = 7.1 Hz, 2H), 2.68 - 2.62 (m, 7H), 2.61 - 2.57 (m, 4H), 2.52 - 2.47 (m, 5H), 2.39 (s, 3H), 2.04 (s, 3H), 2.00 (s, 3H), 1.92 - 1.76 (m, 4H), 1.74 - 1.67 (m, 2H). HRMS (ESI) calcd. for C₄₂H₅₉BF₂N₉O₄ [M + H]⁺: 802.4753, found: 802.4755.

### Example 36.

Compound **36** was obtained by following the procedure described above in General method 4 but wherein **R⁸** was a moiety of formula (V) and L an isonitrile.

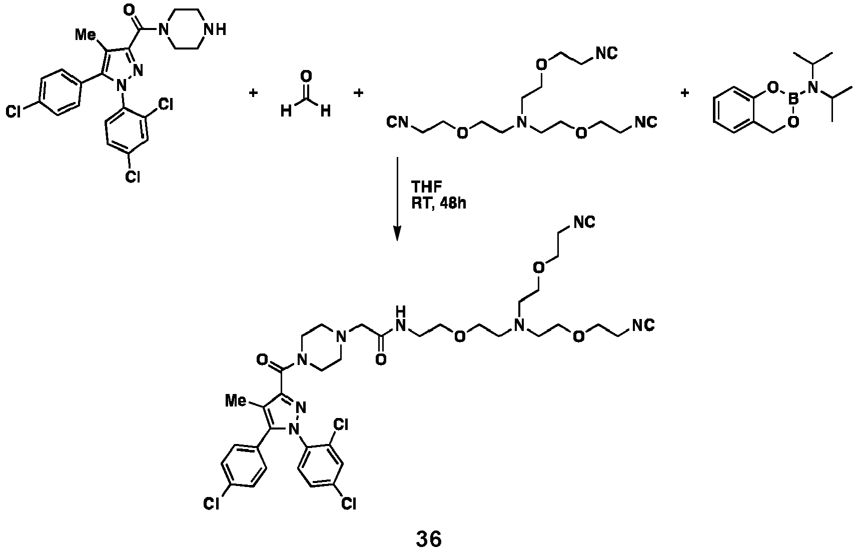

"RT" stands for room temperature. Yield: 39%. Melting point 195 - 197 °C.

¹H RMN (300 MHz, DMSO-*d*₆) δ (ppm): 7.86 (t, *J* = 6.9 Hz, 1H), 7.39 - 7.35 (m, 2H), 7.31 (s, 1H), 7.18 - 7.15 (m, 2H), 7.13 (d, *J* = 7.5 Hz, 1H), 7.04 (d, *J* = 7.5 Hz, 1H), 3.84 - 3.78 (m, 4H), 3.67 - 3.62 (m, 4H), 3.62 - 3.53 (m, 4H), 3.52 - 3.46 (m, 4H), 3.43 - 3.31 (m, 6H), 3.18 (s, 2H), 2.71 (t, *J* = 7.1 Hz, 2H), 2.68 - 2.62 (m, 4H), 2.54 - 2.46 (m, 4H), 2.40 (s, 3H). HRMS (ESI) calcd. for C₃₇H₄₆Cl₃N₈O₅ [M + H]⁺: 787.2655, found: 787.2659.

### Example 37.

Compound **37** was obtained by following the procedure described above in General method 4 but wherein **R⁸** was a moiety of formula (**V**) and **L** an isonitrile.

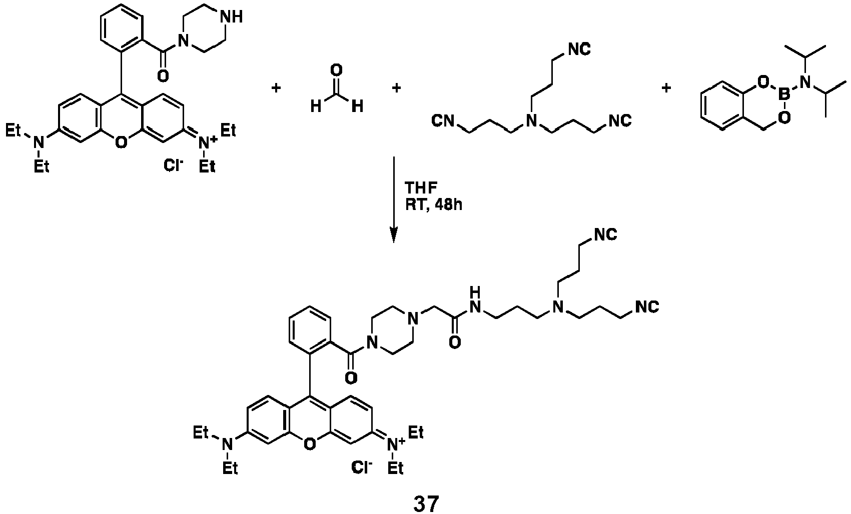

"**RT**" stands for room temperature. Yield: 46%. Melting point > 180 °C (dec.).

¹H RMN (300 MHz, DMSO-*d*₆) δ (ppm): 7.75 - 7.63 (m, 3H), 7.58 - 7.55 (m, 1H), 7.52 (t, *J* = 6.7 Hz, 1H), 7.29 (d, *J* = 7.5 Hz, 1H), 7.22 (d, *J* = 10.8 Hz, 1H), 6.98 - 6.94 (m, 2H), 6.87 (s, 1H), 6.86 (s, 1H), 3.70 - 3.60 (m, 8H), 3.51 - 3.43 (m, 4H), 3.21 - 3.14 (m, 6H), 3.05 - 2.97 (m, 2H), 2.69 - 2.62 (m, 4H), 2.54 - 2.46 (m, 6H), 1.92 - 1.75 (m, 4H), 1.74 - 1.66 (m, 2H), 1.24 (t, *J* = 8.0 Hz, 6H), 1.16 (t, *J* = 8.0 Hz, 6H). HRMS (ESI) calcd. for C₄₅H₅₉N₈O₃ [M]⁺: 759.4703, found: 759.4705.

### Example 38.

Compound **38** was obtained by following the procedure described above in General method 5 but wherein **R⁸** was a moiety of formula (**V**) and **L** an isonitrile.

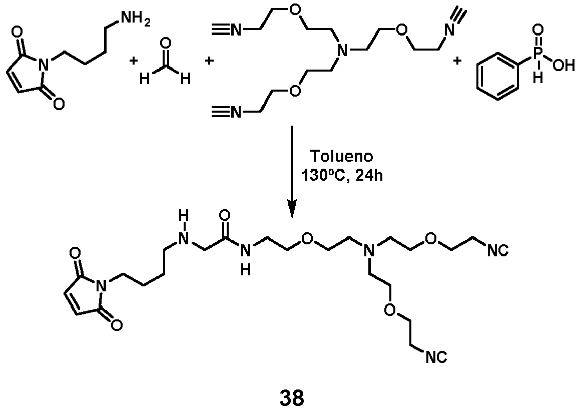

Yield: 37%. Melting point > 150 °C (dec.).

¹H RMN (300 MHz, CDCl₃) δ (ppm): 7.87 (t, *J* = 6.6 Hz, 1H), 6.69 (s, 2H), 3.81 (t, *J* = 7.1 Hz, 4H), 3.75 (t, *J* = 7.0 Hz, 2H), 3.59 (dt, *J* = 12.9, 7.1 Hz, 4H), 3.50 (t, *J* = 7.1 Hz, 4H), 3.42 - 3.32 (m, 4H), 3.24 (t, *J* = 7.1 Hz, 4H), 2.76 - 2.63 (m, 9H), 1.70 - 1.62 (m, 2H), 1.61 - 1.52 (m, 2H). HRMS (ESI) calcd. for C₂₄H₃₉N₆O₆ [M + H]⁺: 507.2933, found: 507.2935.

### Example 39.

Compound **39** was obtained by following the procedure described above in General method 5 but wherein **R⁸** was a moiety of formula (V) and L an isonitrile.

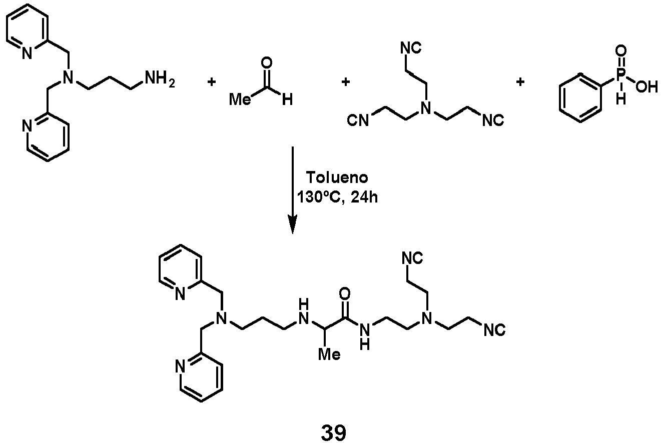

Yield: 41%. Melting point > 160 °C (dec.).

¹H RMN (300 MHz, CDCl₃) δ (ppm): 8.55 (d, *J* = 7.5 Hz, 2H), 7.72 - 7.64 (m, 2H), 7.53 - 7.43 (m, 3H), 7.31 (d, *J* = 7.5 Hz, 2H), 3.94 (s, 2H), 3.89 (s, 2H), 3.86 - 3.82 (m, 1H), 3.72 - 3.62 (m, 1H), 3.42 - 3.31 (m, 4H), 3.14 - 3.05 (m, 2H), 2.96 - 2.76 (m, 6H), 2.75 - 2.67 (m, 2H), 2.59 - 2.52 (m, 2H), 1.78 - 1.67 (m, 2H), 1.27 (d, *J* = 6.8 Hz, 3H). HRMS (ESI) calcd. for C₂₆H₃₇N₈O [M + H]⁺: 477.3088, found: 477.3092.

### Example 40.

Compound **40** was obtained by following the procedure described above in General method 5 but wherein **R⁸** was a moiety of formula (**V**), and **L** was an isonitrile.

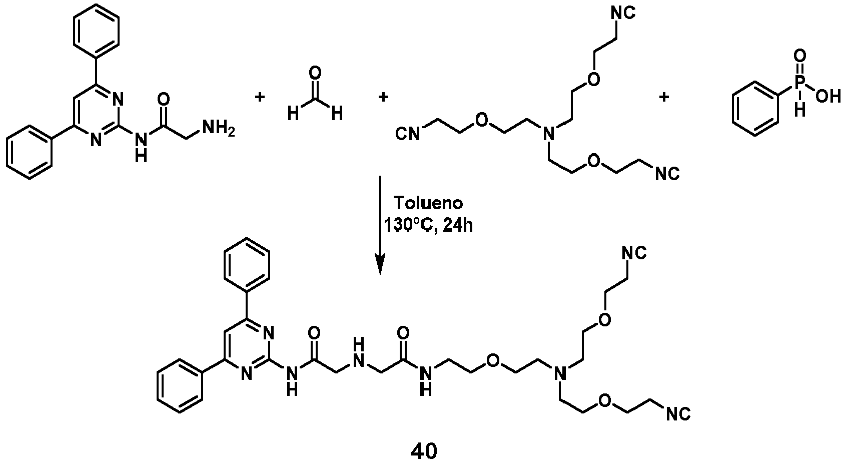

Yield: 34%. Melting point: 170 - 172 °C.

¹H NMR (300 MHz, DMSO-*d*₆) δ (ppm): 8.19 (s, 1H), 8.02-7.98 (m, 4H), 7.92 (t, *J* = 6.6 Hz, 1H), 7.53 - 7.48 (m, 4H), 7.44 - 7.40 (m, 2H), 3.84 - 3.78 (m, 4H), 3.63 - 3.54 (m, 6H), 3.52 - 3.47 (m, 5H), 3.46 - 3.32 (m, 6H), 3.28 - 3.21 (m, 2H), 2.71 (t, *J* = 7.1 Hz, 2H), 2.68 - 2.62 (m, 4H). HRMS (ESI) calcd. for CaaHaaNsOs [M + H]⁺: 643.3358, found: 643.3360.

### Example 41.

Compound **41** was obtained by following the procedure described above in General method 1 but wherein **R¹** was **F**, **R⁸** was a moiety of formula (**V**), and **L** was an isonitrile.

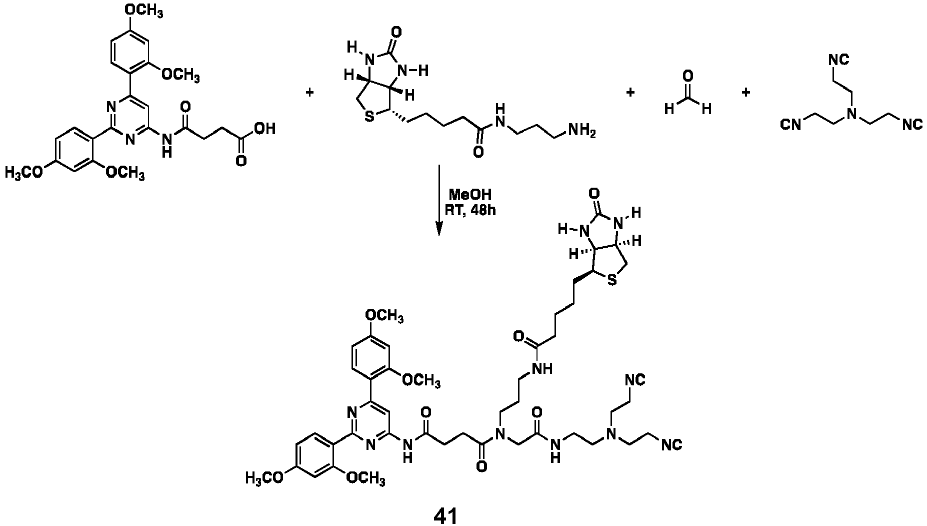

"**RT**" stands for room temperature. Yield: 27%. Melting point: 202 - 203 °C.

¹H NMR (300 MHz, DMSO-*d*₆) δ (ppm): 7.86 (d, *J* = 7.5 Hz, 1H), 7.82 - 7.75 (m, 2H), 7.58 - 7.49 (m, 2H), 6.85 - 6.78 (m, 2H), 6.71 - 6.61 (m, 3H), 5.91 (d, *J* = 7.1 Hz, 1H), 4.45 - 4.38 (m, 1H), 4.26 - 4.19 (m, 1H), 3.99 - 3.89 (m, 2H), 3.86 (s, 6H), 3.79 (s, 3H), 3.79 (s, 3H), 3.40 - 3.24 (m, 5H), 3.23 - 3.04 (m, 7H), 2.95 - 2.88 (m, 1H), 2.84 - 2.68 (m, 5H), 2.66 - 2.58 (m, 5H), 2.55 - 2.48 (m, 1H), 2.23 - 2.13 (m, 2H), 1.91 - 1.81 (m, 1H), 1.80 - 1.71 (m, 1H), 1.71 - 1.54 (m, 4H), 1.54-1.43 (m, 1H), 1.41 - 1.30 (m, 1H). HRMS (ESI) calcd. for C₄₇H₆₂N₁₁O₉S [M + H]⁺: 956.4455, found: 956.4459.

### Example 42.

Compound **42** was obtained by following the procedure described above in General method 1 but wherein **R¹** was **F**, **R⁸** was a moiety of formula (**V**), and **L** was an isonitrile.

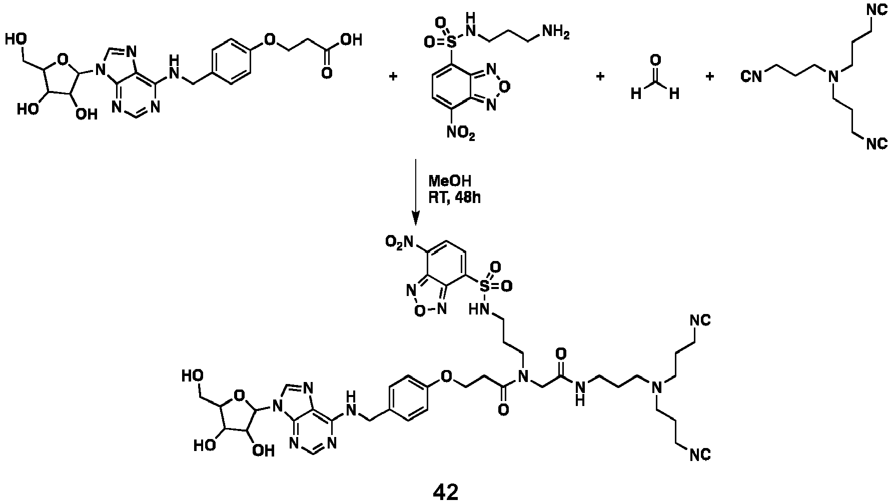

"**RT**" stands for room temperature. Yield: 21%. Melting point: 221 - 223 °C.

¹H NMR (300 MHz, DMSO-*d*₆) δ (ppm): 8.36 (s, 1H), 8.32 - 8.26 (m, 2H), 8.19 (d, *J* = 7.5 Hz, 1H), 8.13 - 8.04 (m, 1H), 7.75 - 7.67 (m, 1H), 7.40 - 7.30 (m, 1H), 7.23 (dt, *J* = 7.5, 1.0 Hz, 2H), 6.84-6.75 (m, 2H), 6.02 - 5.95 (m, 1H), 5.43 (d, *J* = 7.5 Hz, 1H), 5.31 (t, *J* = 7.4 Hz, 1H), 5.15 (d, *J* = 7.0 Hz, 1H), 4.79 - 4.70 (m, 1H), 4.67 - 4.60 (m, 1H), 4.59 - 4.53 (m, 1H), 4.28 - 4.16 (m, 3H), 4.01 - 3.90 (m, 3H), 3.78 - 3.70 (m, 1H), 3.63 - 3.55 (m, 1H), 3.40 - 3.29 (m, 2H), 3.27 - 3.15 (m, 4H), 3.10 - 2.87 (m, 4H), 2.72 - 2.58 (m, 6H), 2.58 - 2.42 (m, 2H), 1.94-1.70 (m, 7H), 1.68 - 1.58 (m, 1H). HRMS (ESI) calcd. for C₄₂H₅₃N₁₄O₂S [M + H]⁺: 977.3686, found: 977.3690.

### Example 43.

Compound **43** was obtained by following the procedure described above in General method 2 but wherein **Y** was alkyl-**F**, **R⁸** was a moiety of formula (**V**), and **L** was an isonitrile.

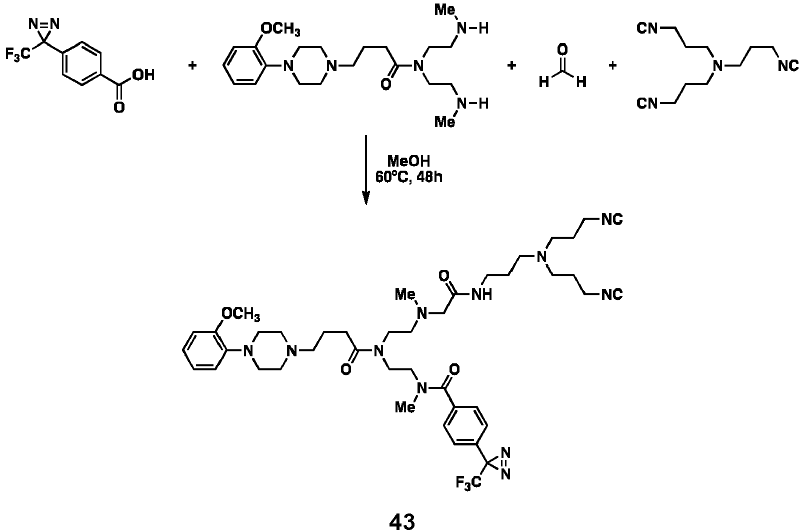

Yield: 23%. Melting point: 194 - 195 °C.

¹H NMR (300 MHz, DMSO-*d*₆) δ (ppm): 7.73 - 7.65 (m, 4H), 7.53 - 7.44 (m, 1H), 7.00 - 6.91 (m, 2H), 6.89 - 6.82 (m, 2H), 3.81 (s, 3H), 3.65 - 3.57 (m, 4H), 3.50 - 3.44 (m, 2H), 3.41 -3.36 (m, 1H), 3.33 - 3.28 (m, 1H), 3.22 - 3.12 (m, 10H), 2.87 (s, 3H), 2.84 - 2.79 (m, 2H), 2.67 - 2.55 (m, 10H), 2.53 - 2.47 (m, 2H), 2.45 (s, 3H), 2.40 - 2.32 (m, 2H), 1.94-1.85 (m, 4H), 1.84 - 1.68 (m, 4H). HRMS (ESI) calcd. for C₄₃H₆₁F₃N₁₁O₄ [M + H]⁺: 852.4862, found: 852.4864.

### Example 44.

Compound **44** was obtained by following the procedure described above in General method 1 but wherein **R**⁸ was a moiety of formula (**V**) and **L** an isonitrile. Therefore, this example shows a reaction with a tri-isonitrile.

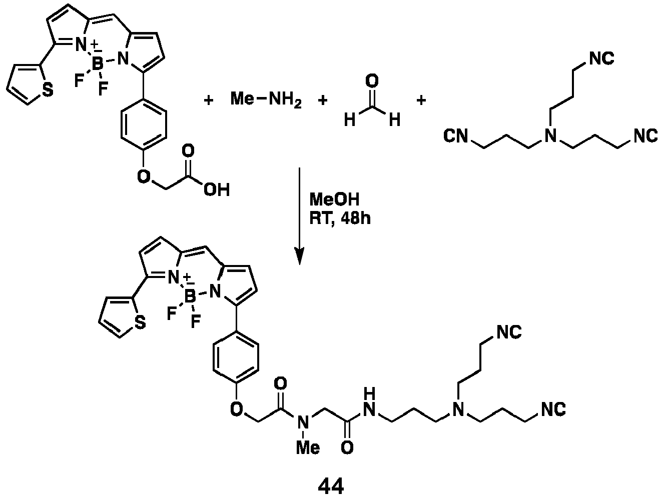

"**RT**" stands for room temperature. Yield: 42%. Melting point: > 160 °C (dec.).

¹H RMN (300 MHz, DMSO-*d*₆) δ (ppm): 8.11 (d, *J* = 10.9 Hz, 1H), 7.65 - 7.59 (m, 2H), 7.27 - 7.22 (m, 1H), 7.20 (s, 1H), 7.17 -7.05 (m, 5H), 7.03 (d, *J* = 7.5 Hz, 2H), 6.51 (m, 1H), 4.80 (s, 2H), 3.99 (s, 2H), 3.23 - 3.20 (m, 2H), 3.17 - 3.03 (m, 4H), 3.01 (s, 3H), 2.63 - 2.49 (m, 6H), 1.88 - 1.72 (m, 6H). HRMS (ESI) calcd. for C₃₅H₃₉BF₂N₇O₃S [M + H]⁺: 686.2893, found: 686.2899.

### Exemplary synthesis of compounds of general formula (I)

### Example 45.

Compound **45** was obtained by following the procedure described above in General method 1 but wherein the isonitrile precursor was compound **4.**

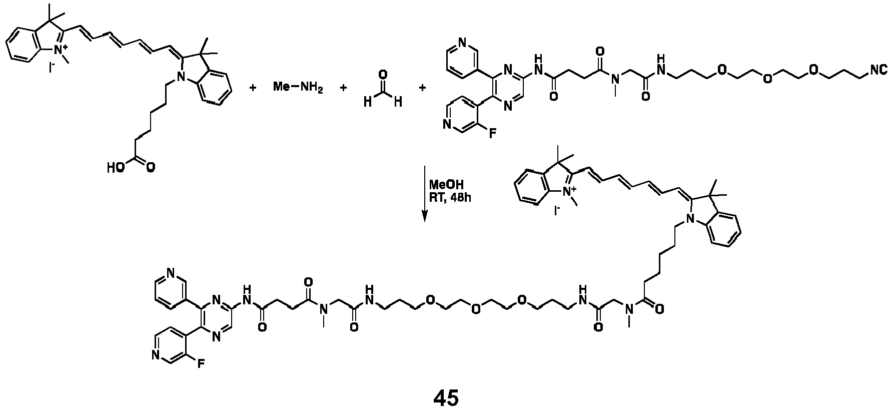

"**RT**" stands for room temperature. Yield: 19%.

¹H NMR (300 MHz, CDCl₃) δ (ppm): 9.30 (s, 1H), 8.72 - 8.63 (m, 2H), 8.57 - 8.45 (m, 2H), 7.96 - 7.87 (m, 2H), 7.81 - 7.75 (m, 1H), 7.62 - 7.35 (m, 7H), 7.22 - 7.16 (m, 1H), 7.13 (d, *J* = 7.5 Hz, 1H), 6.94 - 6.88 (m, 1H), 6.87 - 6.80 (m, 1H), 6.63 - 6.41 (m, 4H), 6.37 - 6.25 (m, 2H), 4.04 - 3.94 (m, 6H), 3.92 (s, 3H), 3.67 - 3.46 (m, 12H), 3.23 - 3.16 (m, 4H), 2.94 (s, 6H), 2.65 - 2.53 (m, 4H), 2.33 (t, *J* = 7.1 Hz, 2H), 1.89 - 1.80 (m, 4H), 1.78 (s, 6H), 1.69 - 1.55 (m, 4H), 1.48 (s, 6H), 1.42 (t, *J* = 7.0 Hz, 2H). HRMS (ESI) calcd. for C₆₈H₈₅FN₁₁O₈⁺ [M]⁺: 1202.6561, found: 1202.6565.

### Example 46.

Compound **46** was obtained by following the procedure described above in General method 1 but wherein the isonitrile precursor was compound **8.**

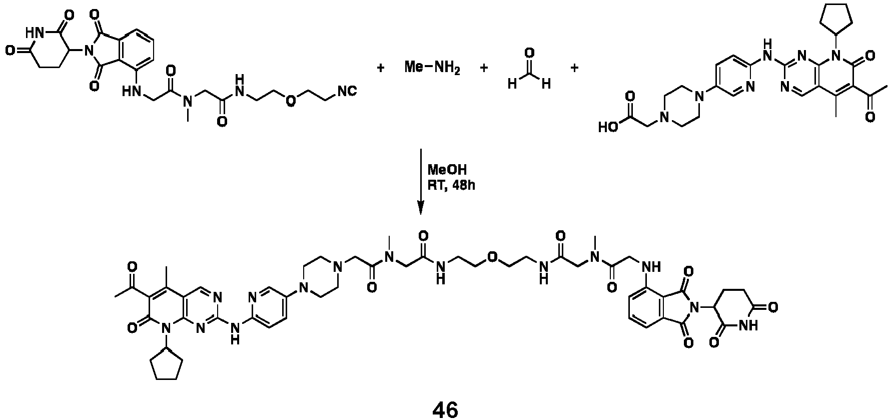

"**RT**" stands for room temperature. Yield: 32%.

¹H NMR (300 MHz, CDCl₃) δ (ppm): 9.34 (s, 1H), 8.90 (s, 1H), 7.90-7.85 (m, 2H), 7.77 - 7.72 (m, 2H), 7.66 - 7.57 (m, 2H), 7.50 - 7.44 (m, 1H), 7.27 (s, 1H), 7.14 - 7.10 (m, 1H), 5.53 - 5.45 (m, 1H), 4.34 - 4.21 (m, 3H), 4.02 - 3.95 (m, 4H), 3.57 - 3.48 (m, 8H), 3.40 - 3.34 (m, 4H), 3.22 (s, 2H), 2.94 - 2.91 (m, 9H), 2.87 - 2.80 (m, 4H), 2.66 - 2.59 (m, 2H), 2.32 (s, 3H), 2.24-2.16 (m, 5H), 2.13-2.06 (m, 1H), 1.67-1.55 (m, 4H).HRMS (ESI) calcd. for C₅₂H₆₃N₁₄O₁₁ [M+1]⁺: 1047.4803, found: 1047.4799.

### Example 47.

Compound **47** was obtained by following the procedure described above in General method 1 but wherein the precursor isonitrile was compound **9.**

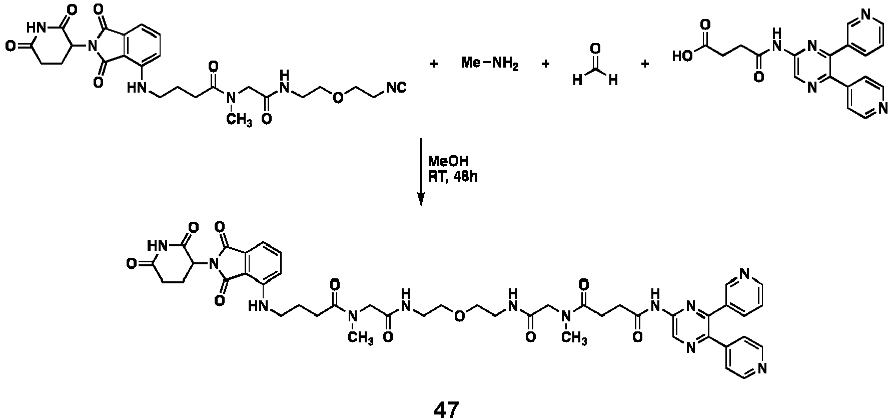

"**RT**" stands for room temperature. Yield: 34%.

¹H NMR (300 MHz, CDCl₃) δ (ppm): 9.32 (s, 1H), 8.99 (s, 1H), 8.72 - 8.63 (m, 3H), 8.53 - 8.46 (m, 1H), 8.17 - 8.11 (m, 2H), 7.93 - 7.80 (m, 4H), 7.60 - 7.51 (m, 1H), 7.47 - 7.40 (m, 1H), 7.26 - 7.20 (m, 1H), 7.11 - 7.05 (m, 1H), 5.55 - 5.44 (m, 1H), 4.04 - 3.93 (m, 4H), 3.63 - 3.50 (m, 4H), 3.46 - 3.32 (m, 6H), 2.99 (s, 6H), 2.67 - 2.49 (m, 6H), 2.43 - 2.29 (m, 2H), 2.23 - 2.07 (m, 2H), 1.89 - 1.71 (m, 2H). HRMS (ESI) calcd. for C₄₅H₅₁N₁₂O₁₀ [M+1]⁺: 919.3851, found: 919.3855.

### Example 48.

Compound **48** was obtained by following the procedure described above in General method 1 but wherein the isonitrile was compound **10.**

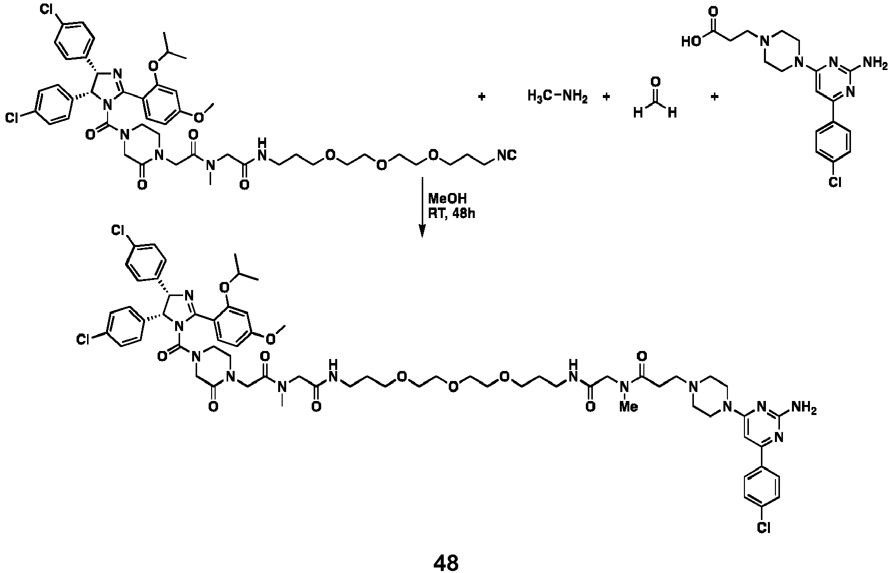

"**RT**" stands for room temperature. Yield: 31%.

¹H NMR (300 MHz, CDCl₃) δ (ppm): 7.77 - 7.71 (m, 3H), 7.61 - 7.51 (m, 2H), 7.40 - 7.35 (m, 4H), 7.33 - 7.26 (m, 6H), 6.71 - 6.66 (m, 1H), 6.61 - 6.54 (m, 2H), 6.19 (s, 2H), 5.82 - 5.77 (m, 1H), 5.52 - 5.47 (m, 1H), 4.67 - 4.58 (m, 1H), 4.06 - 3.92 (m, 8H), 3.80 (s, 3H), 3.77 - 3.67 (m, 6H), 3.67 - 3.48 (m, 12H), 3.47 - 3.38 (m, 1H), 3.27 - 3.11 (m, 4H), 2.95 - 2.92 (m, 6H), 2.84 - 2.62 (m, 8H), 2.08 - 1.98 (m, 1H), 1.90 - 1.79 (m, 3H), 1.38 (d, *J* = 6.8 Hz, 3H), 1.33 (d, *J* = 6.8 Hz, 3H). HRMS (ESI) calcd. for C₆₅H₈₂Cl₃N₁₃O₁₁ [M+1]⁺: 1326.5402, found: 1326.5406.

### Example 49.

Compound **49** was obtained by following the procedure described above in General method 1, but wherein the isonitrile was compound **7**.

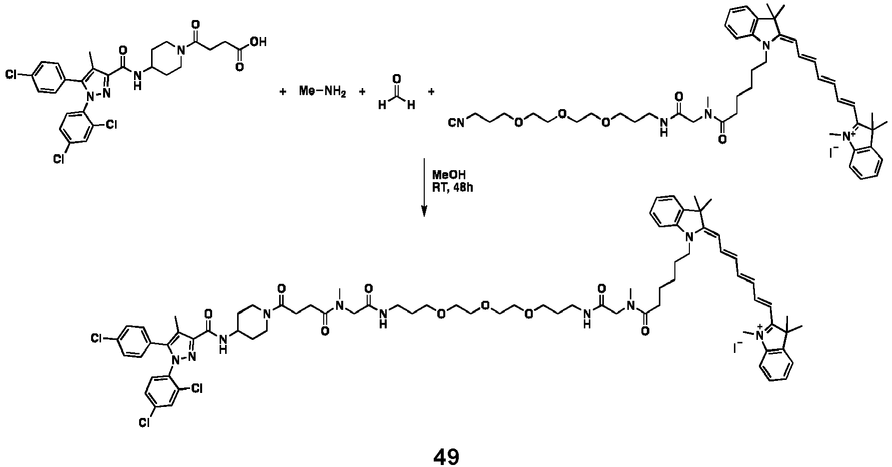

"**RT**" stands for room temperature. Yield: 18%.

¹H NMR (300 MHz, CDCl₃) δ (ppm): 7.83 - 7.76 (m, 2H), 7.60 - 7.50 (m, 4H), 7.47 - 7.42 (m, 2H), 7.41 - 7.30 (m, 5H), 7.21 - 7.11 (m, 4H), 6.91 (d, *J* = 7.5 Hz, 1H), 6.84 (d, *J* = 7.5 Hz, 1H), 6.63 - 6.41 (m, 4H), 6.36 - 6.26 (m, 2H), 4.02 - 3.98 (m, 4H), 3.96 (s, 2H), 3.92 (s, 3H), 3.89 - 3.82 (m, 1H), 3.72 - 3.60 (m, 10H), 3.54 - 3.48 (m, 4H), 3.41 - 3.34 (m, 2H), 3.23 - 3.15 (m, 4H), 2.94 (s, 6H), 2.62 - 2.53 (m, 4H), 2.36 - 2.30 (m, 5H), 1.93 - 1.81 (m, 8H), 1.78 (s, 6H), 1.69 - 1.56 (m, 4H), 1.48 (s, 6H), 1.46 - 1.38 (m, 2H). HRMS (ESI) calcd. for C₇₆H₉₆Cl₃N₁₀O₉⁺ [M]⁺: 1397.6420, found: 1397.6426

### Example 50.

Compound **50** was obtained by following the procedure described above in General method 1, but wherein the isonitrile was compound **7.**

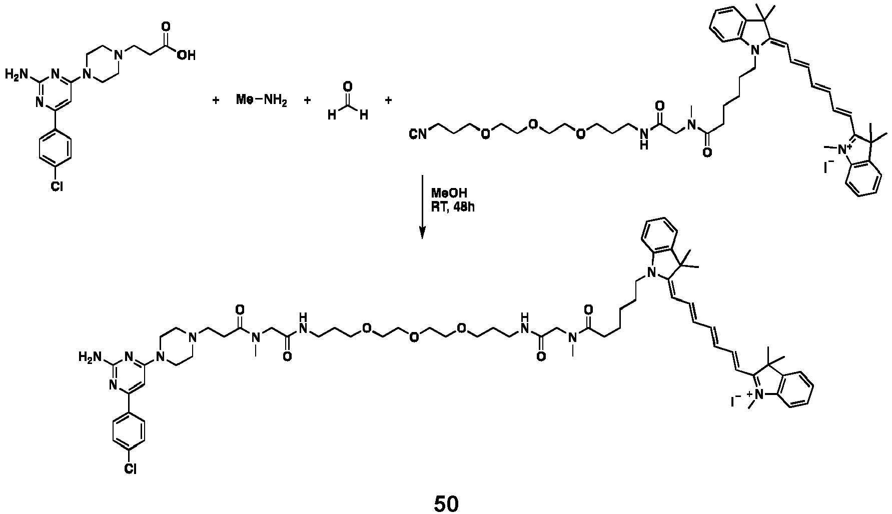

"**RT**" stands for room temperature. Yield: 18%.

¹H NMR (300 MHz, CDCl₃) δ (ppm): 7.81 - 7.70 (m, 3H), 7.61 - 7.49 (m, 4H), 7.45 - 7.35 (m, 4H), 7.22 - 7.16 (m, 1H), 7.14 - 7.11 (m, 1H), 6.91 (d, *J* = 7.5 Hz, 1H), 6.84 (d, *J* = 7.5 Hz, 1H), 6.63 - 6.40 (m, 5H), 6.33 - 6.25 (m, 1H), 6.21 - 6.16 (m, 2H), 6.02 - 5.97 (m, 1H), 4.04 - 3.95 (m, 6H), 3.92 (s, 3H), 3.77 - 3.70 (m, 4H), 3.66 - 3.58 (m, 8H), 3.54 - 3.47 (m, 4H), 3.23 - 3.15 (m, 4H), 2.93 (s, 6H), 2.80 - 2.73 (m, 2H), 2.72 - 2.66 (m, 6H), 2.35-2.30 (m, 2H), 1.89-1.78 (m, 4H), 1.69-1.55 (m, 4H), 1.53 (s, 6H), 1.48 (s, 6H), 1.46 - 1.36 (m, 2H). HRMS (ESI) calcd. for C₆₇H₉₁ClN₁₁O₇⁺ [M]⁺: 1198.6786, found: 1196.6790.

### Example 51.

Compound **51** was obtained by following the procedure described above in General method 2 but wherein the isonitrile precursor was compound **8**.

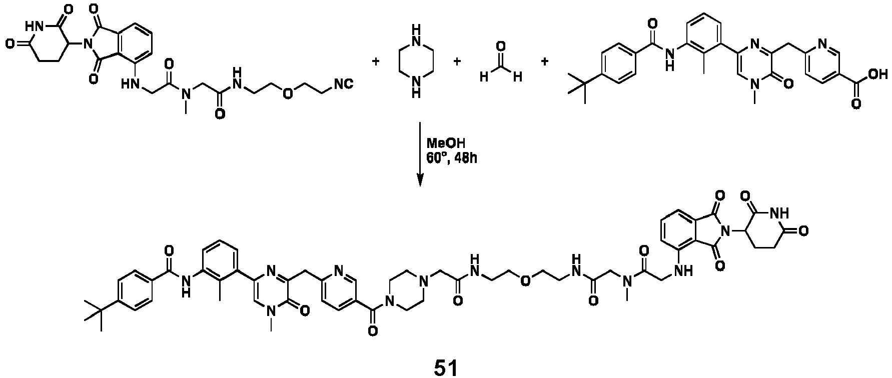

Yield: 29%.

¹H NMR (300 MHz, CDCl₃) δ (ppm): 9.90 (s, 1H), 8.99 (s, 1H), 8.87 (s, 1H), 8.09 - 8.02 (m, 1H), 7.86 - 7.78 (m, 4H), 7.67 - 7.63 (m, 1H), 7.57 - 7.52 (m, 1H), 7.48 - 7.44 (m, 2H), 7.36 - 7.31 (m, 1H), 7.26 - 7.18 (m, 3H), 7.15 (s, 1H), 7.10 - 7.02 (m, 2H), 5.54 - 5.46 (m, 1H), 4.03 - 3.93 (m, 3H), 3.90 - 3.79 (m, 3H), 3.69 - 3.51 (m, 8H), 3.46 - 3.32 (m, 7H), 3.24 - 3.13 (m, 2H), 2.94 (s, 3H), 2.62 (t, *J* = 7.1 Hz, 2H), 2.56 - 2.45 (m, 4H), 2.41 (s, 3H), 2.23 - 2.07 (m, 2H), 1.35 (s, 9H). HRMS (ESI) calcd. for C₅₈H₆₇N₁₂O₁₁ [M+1]⁺: 1107.5050, found: 1107.5054.

### Example 52.

Compound **52** was obtained by following the procedure described above in General method 2 but wherein the precursor isonitrile was compound **9.**

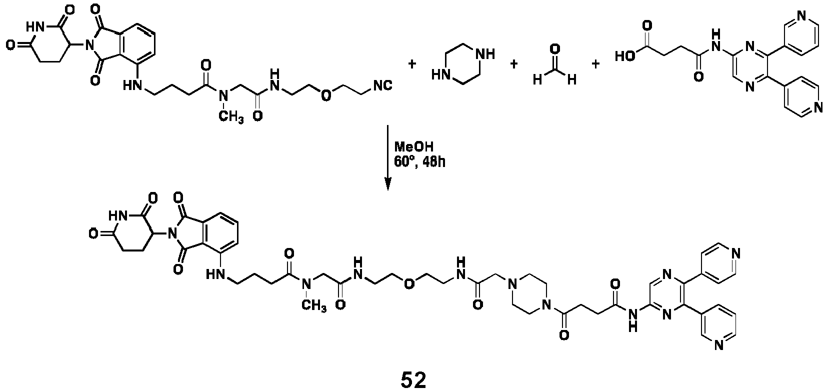

Yield: 31%.

¹H NMR (300 MHz, CDCl₃) δ (ppm): 9.31 (d, *J* = 1.4 Hz, 1H), 8.99 (s, 1H), 8.71 - 8.63 (m, 3H), 8.51 - 8.47 (m, 1H), 8.16 - 8.11 (m, 2H), 7.92 - 7.80 (m, 4H), 7.58 - 7.52 (m, 1H), 7.46 - 7.40 (m, 1H), 7.23 (d, *J* = 7.5 Hz, 1H), 7.09 (d, *J* = 7.5 Hz, 1H), 5.50 (t, *J* = 7.0 Hz, 1H), 4.03 - 3.95 (m, 2H), 3.62 - 3.54 (m, 4H), 3.52 - 3.46 (m, 4H), 3.45 - 3.33 (m, 6H), 3.23 - 3.16 (s, 2H), 2.93 (s, 3H), 2.68 - 2.46 (m, 10H), 2.42 - 2.29 (m, 2H), 2.23 - 2.06 (m, 2H), 1.89 - 1.72 (m, 2H). HRMS (ESI) calcd. for C₄₈H₅₆N₁₃O₁₀ [M+1]⁺: 974.4273, found: 974.4277.

### Example 53.

Compound **53** was obtained by following the procedure described above in General method 2 but wherein the precursor isonitrile was compound **9.**

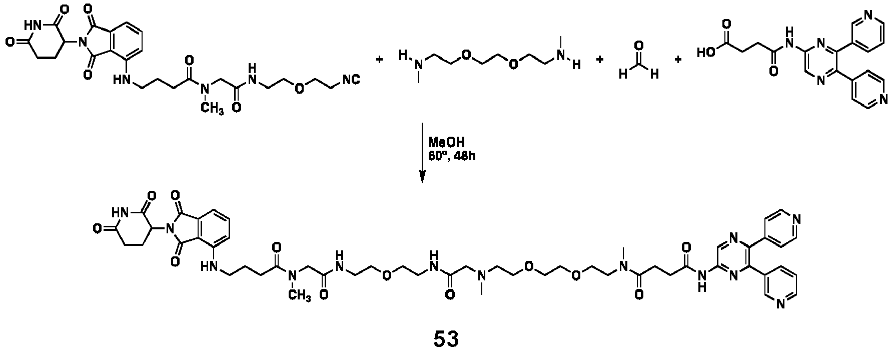

Yield: 36%.

¹H NMR (300 MHz, CDCl₃) δ (ppm): 9.33 (d, *J* = 1.5 Hz, 1H), 8.96 (s, 1H), 8.74 - 8.60 (m, 3H), 8.53 - 8.48 (m, 1H), 8.20 - 8.14 (m, 2H), 7.91 - 7.75 (m, 4H), 7.60 - 7.51 (m, 1H), 7.49 - 7.42 (m, 1H), 7.23 - 7.17 (m, 1H), 7.12 -7.06 (m, 1H), 5.55 - 5.46 (m, 1H), 4.03 - 3.94 (m, 2H), 3.72 - 3.47 (m, 14H), 3.45 - 3.25 (m, 8H), 2.93 (s, 3H), 2.85 (s, 3H), 2.77 (t, *J* = 7.0 Hz, 2H), 2.65 - 2.51 (m, 6H), 2.42 - 2.29 (m, 5H), 2.26 - 2.03 (m, 2H), 1.91 - 1.70 (m, 2H). HRMS (ESI) calcd. for C₅₂H₆₆N₁₃O₁₂ [M+1]⁺: 1064.4956, found: 1064.4960.

### Example 54.

Compound **54** was obtained by following the procedure described above in General method 2 but wherein the precursor isonitrile was compound **9.**

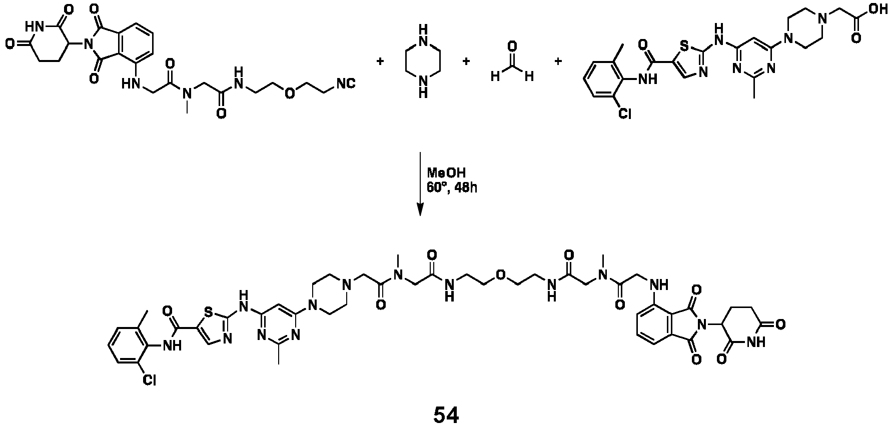

Yield: 35%.

¹H NMR (300 MHz, CDCl₃) δ (ppm): 9.57 (s, 1H), 9.25 (s, 1H), 8.99 (s, 1H), 8.15 (s, 1H), 7.86 - 7.79 (m, 2H), 7.58 - 7.50 (m, 1H), 7.28 - 7.15 (m, 4H), 7.11 - 7.01 (m, 2H), 6.12 (s, 1H), 5.53 - 5.47 (m, 1H), 4.04 - 3.84 (m, 6H), 3.72 - 3.65 (m, 4H), 3.63 - 3.51 (m, 4H), 3.45 - 3.34 (m, 4H), 3.26 - 3.11 (m, 2H), 2.94 (s, 6H), 2.75 - 2.68 (m, 4H), 2.66 - 2.59 (m, 2H), 2.37 (s, 3H), 2.25 (s, 3H), 2.23 - 2.08 (m, 2H). HRMS (ESI) calcd. for C₄₇H₅₆ClN₁₄O₁₀S [M+1]⁺: 1043.3713, found: 1043.3717.

### Example 55.

Compound **55** was obtained by following the procedure described above in General method 2, and wherein the precursor nitrile was compound **2.**

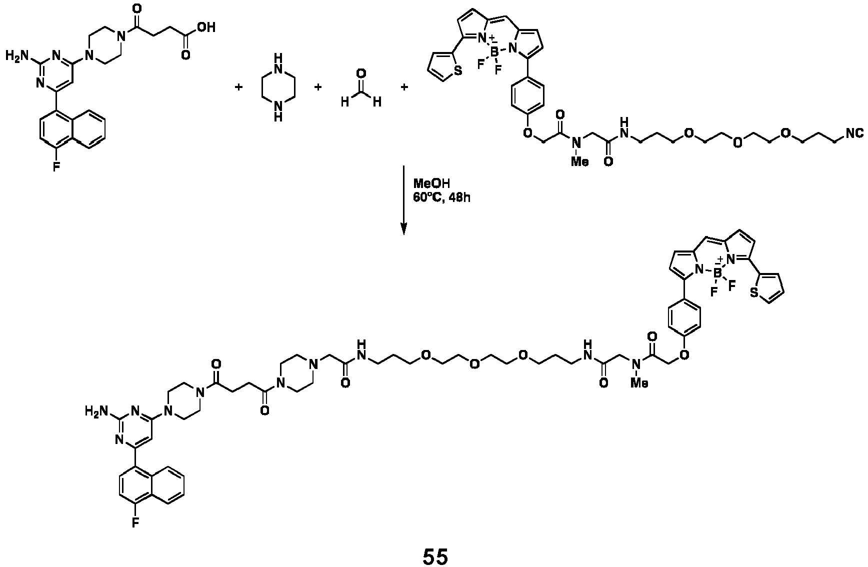

Yield: 39%.

¹H NMR (300 MHz, CDCl₃) δ (ppm): 8.19 (d, *J* = 6.8 Hz, 1H), 7.91 (d, *J* = 6.8 Hz, 1H), 7.85 - 7.80 (m, 1H), 7.60 - 7.45 (m, 9H), 7.37 - 7.30 (m, 2H), 7.17 - 7.05 (m, 4H), 7.03 - 6.96 (m, 2H), 6.77 (s, 1H), 6.32 (s, 2H), 4.69 (s, 2H), 3.96 (s, 2H), 3.74 - 3.63 (m, 12H), 3.61 - 3.53 (m, 4H), 3.53 - 3.41 (m, 8H), 3.20 - 3.08 (m, 6H), 2.93 (s, 3H), 2.65 - 2.60 (m, 4H), 2.52 (s, 4H), 1.89 - 1.79 (m, 4H). HRMS (ESI) calcd. for C₆₂H₇₃BF₃N₁₂O₉S [M+1]⁺: 1229.5387, found: 1229.5291.

### Example 56.

Compound **56** was obtained by following the procedure described above in General method 2, and wherein the precursor nitrile was compound **2.**

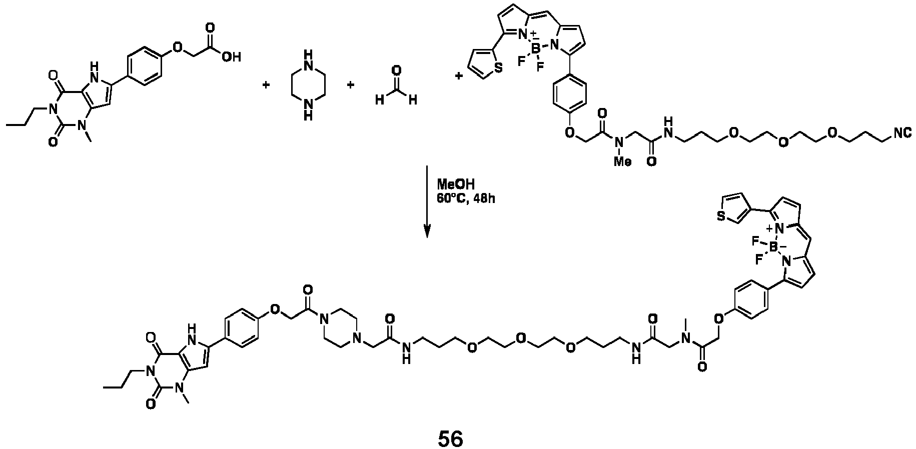

Yield: 16%.

¹H NMR (300 MHz, CDCl₃) δ (ppm): 10.64 (s, 1H), 8.15 - 8.00 (m, 1H), 7.99 - 7.83 (m, 2H), 7.72 - 7.59 (m, 2H), 7.49 - 7.40 (m, 1H), 7.37 - 7.21 (m, 1H), 7.18 - 7.07 (m, 2H), 7.07 - 6.90 (m, 6H), 6.88 - 6.72 (m, 2H), 6.68 - 6.53 (m, 1H), 6.22 - 6.06 (m, 1H), 4.80 (s, 2H), 4.68 (s, 2H), 4.16 - 3.88 (m, 4H), 3.72 - 3.41 (m, 21H), 3.41 - 3.24 (m, 2H), 3.19 (s, 2H), 2.99 (s, 3H), 2.46 (t, *J* = 4.9 Hz, 4H), 1.87 - 1.56 (m, 6H), 0.93 (t, *J* = 7.3 Hz, 3H). HRMS (ESI) calcd. for C₅₈H₇₀BF₂N₁₀O₁₁S [M+1]⁺: 1163.5007, found: 1163.5011

### Example 57.

Compound **57** was obtained by following the procedure described above in General method 2, and wherein the precursor isonitrile was a mono isonitrile similar in structure to (and obtainable as) compound **31.**

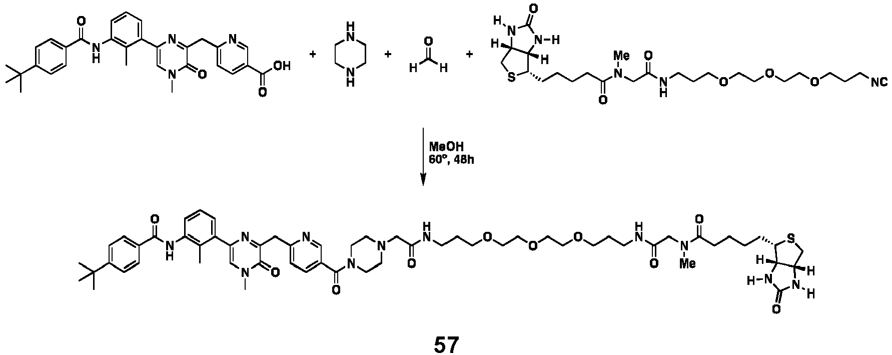

Yield: 29%.

¹H NMR (300 MHz, CDCl₃) δ (ppm): 9.90 (s, 1H), 8.87 (s, 1H), 8.08-8.03 (m, 1H), 7.83 - 7.77 (m, 2H), 7.67 - 7.62 (m, 1H), 7.59 - 7.43 (m, 4H), 7.36 - 7.31 (m, 1H), 7.26 - 7.13 (m, 3H), 6.65 (d, *J* = 7.7 Hz, 1H), 5.93 (d, *J* = 7.1 Hz, 1H), 4.45-4.38 (m, 1H), 4.23 (q, *J* = 7.1 Hz, 1H), 3.98 (s, 2H), 3.88 - 3.80 (m, 2H), 3.77 - 3.48 (m, 15H), 3.47 - 3.39 (m, 1H), 3.34 (s, 3H), 3.28 - 3.07 (m, 7H), 2.95 - 2.88 (m, 4H), 2.80 - 2.74 (m, 1H), 2.56 - 2.45 (m, 4H), 2.41 (s, 3H), 2.40 - 2.27 (m, 2H), 2.08 - 1.98 (m, 1H), 1.90 - 1.80 (m, 3H), 1.72 - 1.58 (m, 4H), 1.52 - 1.32 (m, 11H). HRMS (ESI) calcd. for C₅₉H₈₂N₁₁O₁₀S [M+1]⁺: 1136.5967, found: 1136.5973.

### Example 58.

Compound **58** was obtained by following the procedure described above in General method 2, and wherein the precursor isonitrile was a mono isonitrile similar in structure to (and obtainable as) compound **31.**

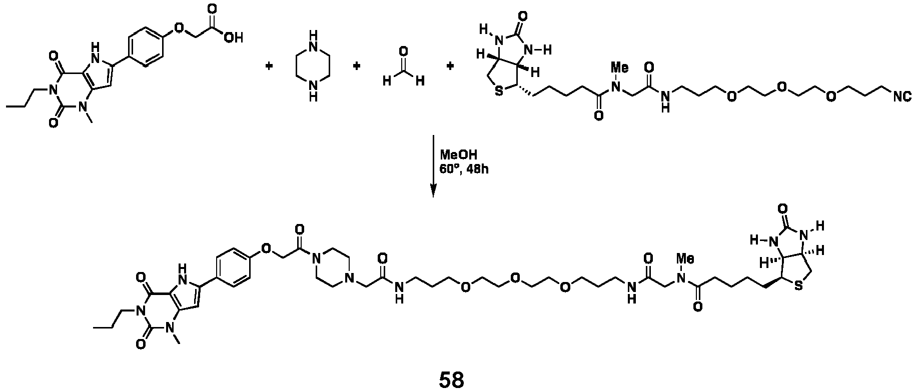

Yield: 39%.

¹H NMR (300 MHz, CDCl₃) δ (ppm): 7.56 (t, *J* = 6.5 Hz, 1H), 7.53 - 7.47 (m, 2H), 7.37 - 7.32 (m, 2H), 7.02 - 6.96 (m, 2H), 6.65 (d, *J* = 7.7 Hz, 1H), 5.93 (d, *J* = 7.1 Hz, 1H), 4.78 (s, 2H), 4.45 - 4.37 (m, 1H), 4.26 - 4.19 (m, 1H), 4.05 - 3.38 (m, 23H), 3.28 - 3.06 (m, 7H), 2.97 - 2.89 (m, 4H), 2.79 - 2.73 (m, 1H), 2.61 - 2.45 (m, 4H), 2.39 - 2.27 (m, 2H), 2.09 - 1.97 (m, 1H), 1.90 - 1.79 (m, 3H), 1.75 - 1.55 (m, 6H), 1.52 - 1.33 (m, 2H), 0.94 (t, *J* = 8.0 Hz, 3H). HRMS (ESI) calcd. for C₄₇H₇₁N₁₀O₁₁S [M+1]⁺: 983.5024, found: 983.5022.

### Example 59.

Compound **59** was obtained by following the procedure described above in General method 1 but wherein **R**⁸ was a moiety of formula (**V**) and **L** an isonitrile and wherein the isonitrile was the di-isonitrile compound **44.**

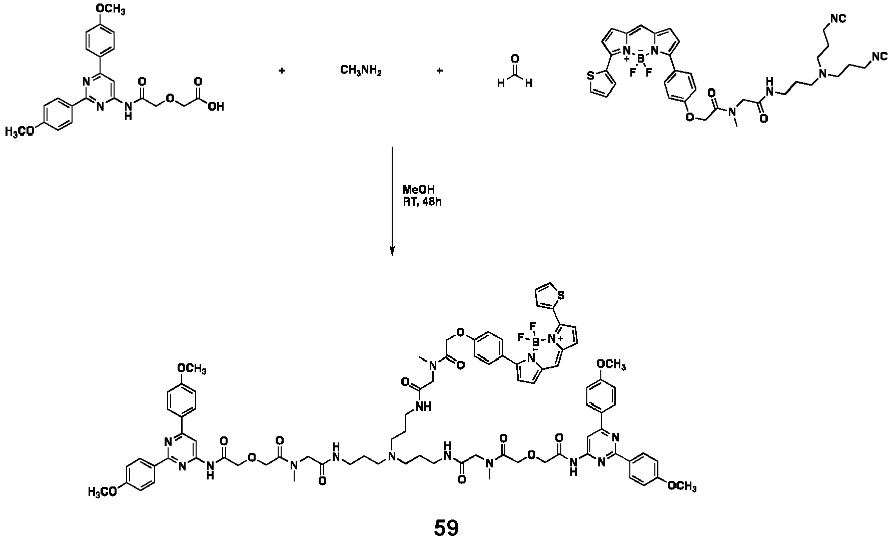

"RT" stands for room temperature. Yield: 19%.

¹H NMR (300 MHz, DMSO-*d*₆) δ (ppm): 8.16 - 8.14 (m, 2H), 8.13-8.04 (m, 5H), 7.76-7.72 (m, 4H), 7.72 - 7.67 (m, 3H), 7.59 - 7.50 (m, 4H), 7.47 - 7.36 (m, 4H), 7.27 - 7.18 (m, 2H), 7.10 - 7.08 (m, 1H), 7.05 - 7.01 (m, 4H), 6.90 - 6.86 (m, 4H), 5.16 - 5.13 (m, 2H), 4.20 - 4.17 (m, 4H), 4.16 - 4.13 (m, 4H), 3.98 - 3.96 (m, 4H), 3.93 - 3.91 (m, 2H), 3.80 - 3.77 (m, 12H), 3.21 - 3.14 (m, 6H), 2.98 - 2.95 (m, 6H), 2.93 (s, 3H), 2.51 - 2.45 (m, 6H), 1.72 - 1.63 (m, 6H). HRMS (ESI) calcd. for C₈₃H₉₁BF₂N₁₅O₁₅S [M+1]⁺: 1618.6610, found: 1618.6607.

### Example 60.

Compound **60** was obtained by following the procedure described above in General method 1 but wherein the precursor isonitrile was compound **32.**

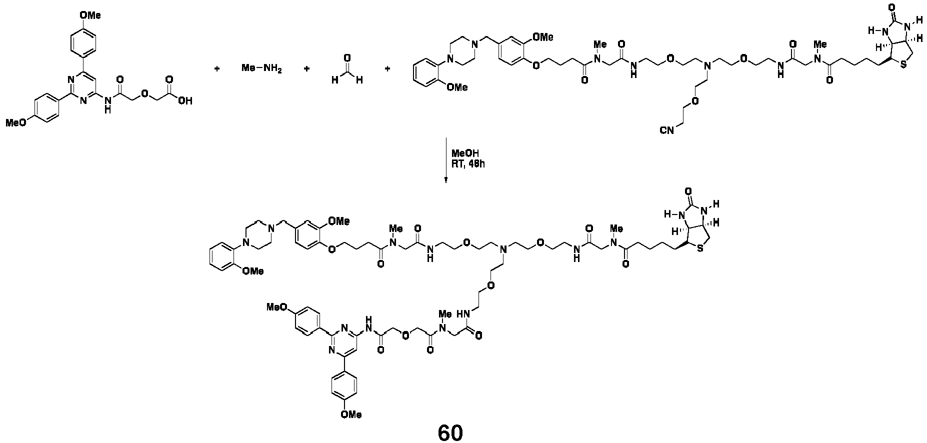

"**RT**" stands for room temperature. Yield: 16%.

¹H NMR (300 MHz, DMSO-*d*₆) δ (ppm): 9.90 - 9.85 (m, 3H), 8.76 - 8.71 (m, 1H), 8.61 - 8.57 (m, 1H), 8.16 - 8.14 (m, 1H), 8.09 - 8.05 (m, 2H), 7.76 - 7.72 (m, 2H), 7.05 - 7.00 (m, 2H), 6.97-6.80 (m, 9H), 4.46 - 4.31 (m, 1H), 4.26 - 4.15 (m, 4H), 4.14 - 3.93 (m, 9H), 3.84 - 3.77 (m, 12H), 3.65 - 3.47 (m, 14H), 3.45 - 3.33 (m, 6H), 3.33 - 3.07 (m, 10H), 3.00 - 2.89 (m, 14H), 2.88 - 2.73 (m, 2H), 2.52 - 2.41 (m, 2H), 2.40 - 2.28 (m, 2H), 2.08 - 2.00 (m, 2H), 1.73 - 1.56 (m, 4H), 1.52 - 1.32 (m, 2H). HRMS (ESI) calcd. for C₇₆H₁₀₇N₁₄O₁₇S [M+1]+: 1519.7651, found: 1519.7657.

### Example 61.

Compound **61** was obtained by following the procedure described above in General method 2 but wherein the precursor isonitrile was compound **33.**

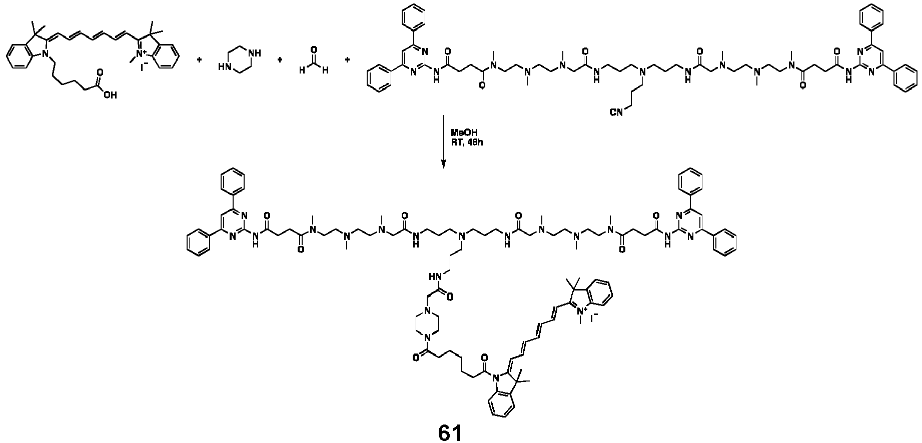

"**RT**" stands for room temperature. Yield: 16%.

¹H NMR (300 MHz, DMSO-*d₆*) δ (ppm): 9.92 - 9.87 (m, 3H), 8.28 - 8.21 (m, 2H), 7.89-7.81 (m, 1H), 7.61 - 7.50 (m, 10H), 7.45 - 7.31 (m, 15H), 7.29 - 7.19 (m, 2H), 6.57 - 6.51 (m, 2H), 6.31 - 6.14 (m, 5H), 3.67 - 3.59 (m, 7H), 3.53 - 3.47 (m, 4H), 3.41 - 3.29 (m, 4H), 3.23 - 3.14 (m, 8H), 2.99 - 2.89 (m, 8H), 2.88 - 2.80 (m, 10H), 2.63 - 2.45 (m, 20H), 2.39 (s, 6H), 2.37 - 2.32 (m, 2H), 2.29 (s, 6H), 1.72 - 1.57 (m, 10H), 1.51 (s, 6H), 1.46 (s, 6H), 1.44-1.37 (m, 2H). HRMS (ESI) calcd. for C₁₀₈H₁₄₁N₂₀O₉⁺ [M]+: 1862.1183, found: 1862.1177.

### Example 62.

Compound **62** was obtained by following the procedure described above in General method 1 but wherein the isonitrile was compound **41.**

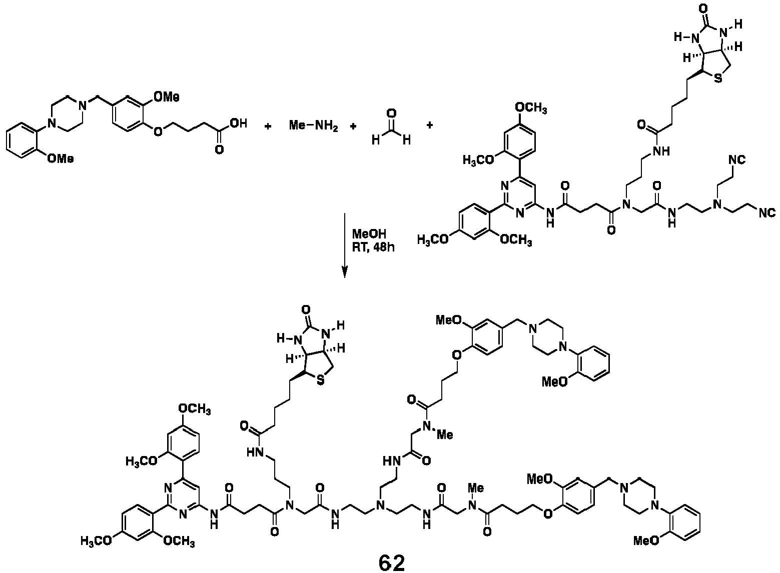

"**RT**" stands for room temperature. Yield: 12%.

¹H NMR (300 MHz, DMSO-*d*₆) δ (ppm): 7.98 - 7.94 (m, 1H), 7.87 - 7.83 (m, 1H), 7.65 - 7.59 (m, 3H), 7.55 - 7.51 (m, 2H), 7.03 - 6.99 (m, 2H), 6.99 - 6.92 (m, 4H), 6.88 - 6.79 (m, 10H), 6.69 - 6.65 (m, 2H), 6.62 - 6.60 (m, 1H), 5.86 - 5.82 (m, 1H), 4.42 - 4.36 (m, 1H), 4.23 - 4.18 (m, 1H), 4.12 - 4.02 (m, 4H), 4.01 - 3.91 (m, 6H), 3.88 - 3.84 (m, 6H), 3.83 - 3.76 (m, 18H), 3.64 - 3.60 (m, 4H), 3.39 - 3.30 (m, 7H), 3.26 - 3.16 (m, 10H), 3.15 - 3.08 (m, 2H), 2.89 (s, 6H), 2.85 - 2.82 (m, 2H), 2.71 - 2.58 (m, 18H), 2.49 - 2.41 (m, 4H), 2.24 - 2.14 (m, 2H), 2.09 - 1.98 (m, 4H), 1.85 - 1.75 (m, 2H), 1.69 - 1.52 (m, 4H), 1.49 - 1.37 (m, 2H). HRMS (ESI) calcd. for C₉₈H₁₃₂N₁₇O₁₉S [M+1]+: 1870.9606, found: 1870.9612.

### Example 62. Biological activity of the exemplary compounds of general formula (I)

The adenosinergic (affinity and selectivity) profile of compound, 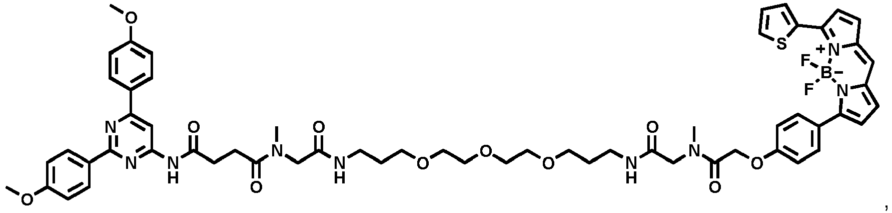 was evaluated *in vitro* using radioligand binding assays following a previously described protocol (J. Med. Chem. 2019, 62, 9315-9330). Human ARs were expressed in transfected cell lines [e.g. chinese hamster ovary (CHO) cells (A₁AR), human epithelial carcinoma (HeLa) cells (A_{2A}AR and A₃AR) and human embryonic kidney (HEK-293) cells (A_{2B}AR)]. [³H]DPCPX for A₁AR and A_{2B}AR, [³H]NECA for A₃AR, and [³H]ZM241385 for A_{2A}AR were employed as radioligands. The binding data is presented as K*ᵢ* ± SEM (nM, n = 3) or as specific binding inhibition percentage at 1 µM (n = 2, average) for those compounds that did not completely displace the radioligand. Using Prism 5.0 software (GraphPad, San Diego, CA) K*ᵢ* values were acquired by fitting the data by non-linear regression. Results are the average of three experiments, each of them performed in duplicate. Radioligand binding competition assays were performed *in vitro* using human ARs expressed in transfected HeLa [*h*A_{2A}AR (9 pmol/mg protein) and *h*A₃AR (3 pmol/mg protein)], HEK-293 [*h*A_{2B}AR (1.5 pmol/mg protein)] and CHO [*h*A₁AR (1.5 pmol/mg protein)] cells. A brief description is given below. A₁AR competition binding experiments were carried out in membranes from CHO-A₁ cells labelled with 1 nM [³H]DPCPX (K_{D} = 0.7 nM). Non-specific binding was determined in the presence of 10 µM R-PIA. The reaction mixture was incubated at 25 °C for 60 min. A_{2A}AR competition binding experiments were carried out in membranes from HeLa-A_{2A}, cells labelled with 3 nM [³H]ZM241385 (K_{D} = 2 nM). Non-specific binding was determined in the presence of 50 µM NECA. The reaction mixture was incubated at 25 °C for 30 min. A_{2B}AR competition binding experiments were carried out in membranes from HEK-293-A_{2B} cells (Euroscreen, Gosselies, Belgium) labelled with 25 nM [³H]DPCPX (K_{D} = 21 nM). Non-specific binding was determined in the presence of 400 µM NECA. The reaction mixture was incubated at 25 °C for 30 min. A₃AR competition binding experiments were carried out in membranes from HeLa-As cells labelled with 10 nM [³H]NECA (K_{D} = 8.7 nM). Non-specific binding was determined in the presence of 100 µM R-PIA. The reaction mixture was incubated at 25 °C for 180 min. After the incubation time, membranes were washed and filtered, radioactivity was detected in a Microbeta Trilux reader (PerkinElmer). Binding data: **A₃ K*ᵢ* = 6.13 nM**, A₁ (1mM) = 2%, A_{2A} (1mM) = 2%, A_{2B} (1mM) = 1%

The dopaminergic (affinity and selectivity) profile of compound, 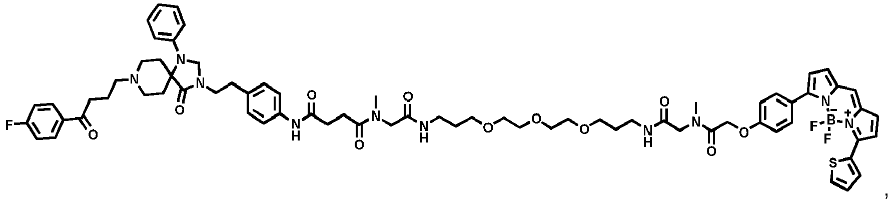 was evaluated *in vitro* using radioligand binding assays following a previously described protocol (J. Med. Chem., 2020, 63, 613-620, Eur. J. Med. Chem., 2019, 180, 673-689). Dopamine D₂ receptor competition binding experiments were carried out in a polypropilene 96-well plate. In each well was incubated 10 µg of membranes from CHO-D2 #S20 cell line prepared in Biofarma laboratory (Lot: A002/22-07-2015, protein concentration=4185 µg/ml), 0.15 nM [3H]-Spiperone (80.2 Ci/mmol, 1 mCi/ml, Perkin Elmer NET1187001MC) and compounds studied and standard. Non-specific binding was determined in the presence of Sulpiride 10µM (Sigma S112). The reaction mixture (Vt: 250 µl/well) was incubated at 25°C for 120 min, 200 µL was trasnfered to GF/C 96-well plate (Millipore, Madrid, Spain) pretreated with 0.5% of PEI and treated with binding buffer (Tris-HCl 50mM, EDTA 1mM, MgCl2 5mM, KCI 5mM, NaCl 120mM, pH=7.4), after was filtered and washed four times with 250 µl wash buffer (Tris-HCl 50mM, NaCl 0.9%, pH=7.4), before measuring in a microplate beta scintillation counter (Microbeta Trilux, PerkinElmer). Dopamine D₃ receptor competition binding experiments were carried out in a polypropilene 96-well plate. In each well was incubated 2 µg of membranes from D3 cell line (Perkin Elmer, ES-173M400UA, protein concentration=1000 µg/ml), 1 nM [3H]-Spiperone (68 Ci/mmol, 1 mCi/ml, Perkin Elmer NET1187001MC) and compounds studied and standard. Non-specific binding was determined in the presence of Haloperidol 1µM (Sigma H1512). The reaction mixture (Vt: 250 µl/well) was incubated at 25°C for 60 min, 200 µL was transfered to GF/C 96-well plate (Millipore, Madrid, Spain) pretreated with 0.5% of PEI and treated with binding buffer (Tris-HCl 50mM, MgCl2 5mM, pH=7.4), after was filtered and washed four times with 250 µl wash buffer (Tris-HCl 50mM, pH=7.4), before measuring in a microplate beta scintillation counter (Microbeta Trilux, PerkinElmer, Madrid, Spain).

Binding data: **D₂ K*ᵢ* = 1.06 nM**, D₃ **K*ᵢ*** = 148 nM.

The dopaminergic and adenosinergic (affinity and selectivity) profile of the compound, 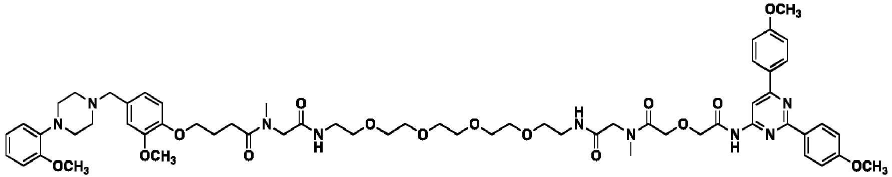 was evaluated *in vitro* using radioligand binding assays following the above mentioned described protocols.

Binding data: D₂ K*ᵢ* = 30 nM, D₃ K*ᵢ* = 2.6 µM, A₃ K*ᵢ* = 10 nM, A₁ (1µM) = 8%, A_{2A} (1µM) = 5%, A_{2B} (1µM) = 4%

The adenosinergic (affinity and selectivity) profile of the compound, 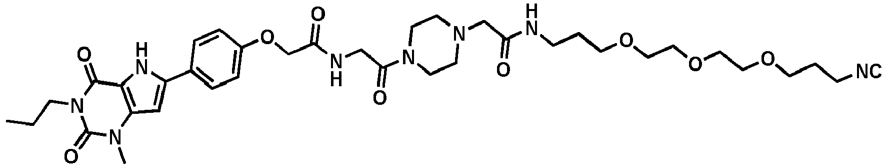 was evaluated *in vitro* using radioligand binding assays following the above mentioned described protocols.

Binding data: A₁ K*ᵢ* = 100.5 nM, A_{2A} (1µM) = 13%, A_{2B} K*ᵢ* = 78.6 nM, A₃ K*ᵢ* = 195.1 nM.

The dopaminergic (affinity and selectivity) profile of the compound, 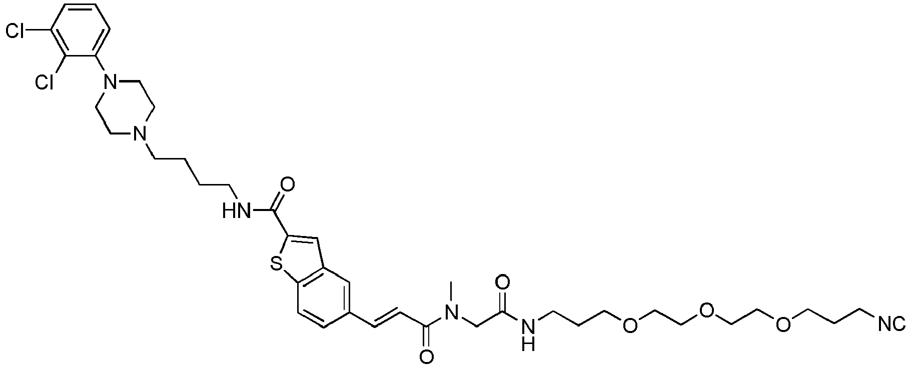 was evaluated *in vitro* using radioligand binding assays following the above mentioned described protocols.

Binding data: D₂ K*ᵢ* = 1.1 nM, D₃ K*ᵢ* = 1 nM, D₄ K*ᵢ* = 500 nM

### Example 63. Effect of spacer and G moieties on the biological activity of the exemplary compounds of the invention

The adenosinergic (affinity and selectivity) profile of several compounds of the invention was evaluated according to the above examples. The binding data is presented as K*ᵢ* ± SEM (nM, n = 3) or as specific binding inhibition percentage at 1 µM (n = 2, average) for those compounds that did not completely displace the radioligand

| | | | |
|---|---|---|---|
| 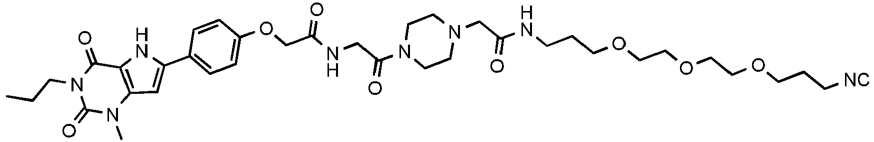 | | | |
| A₁ | A_{2A} | A_{2B} | A₃ |
| 100.5 nM | 13 % | 78.6 nM | 195.1 nM |

| | | | |
|---|---|---|---|
| 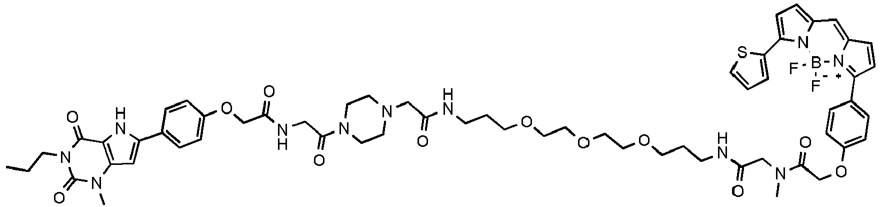 | | | |
| A₁ | A_{2A} | A_{2B} | A₃ |
| 470.9 nM | 23% | 39.6 nM | 42.7 nM |

| | | | |
|---|---|---|---|
| 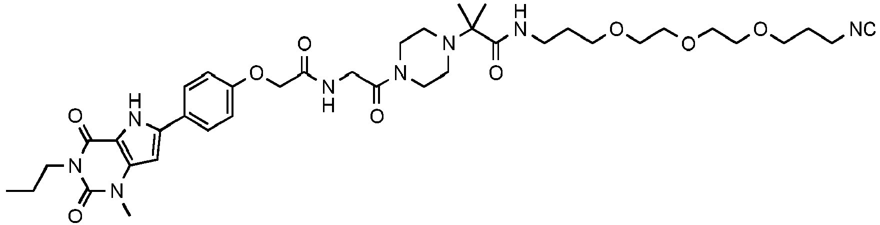 | | | |
| A₁ | A_{2A} | A_{2B} | A₃ |
| 61 % | 13 % | 180 nM | 57 % |

| | | | |
|---|---|---|---|
| 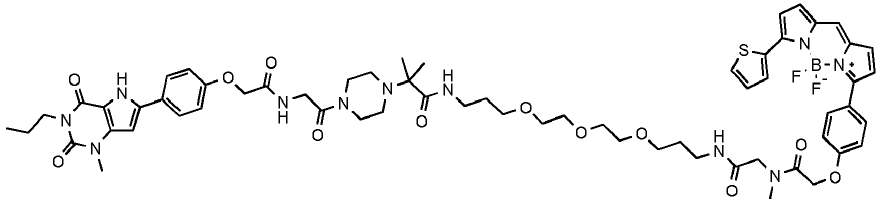 | | | |
| A₁ | A_{2A} | A_{2B} | A₃ |
| 46 % | 1% | 250 nM | 37 % |

| | | | |
|---|---|---|---|
| 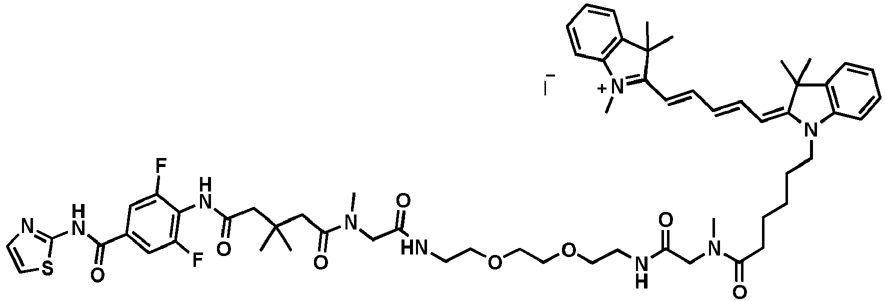 | | | |
| A₁ | A_{2A} | A_{2B} | A₃ |
| 33% | 420 nM | 59 % | 54 % |

| | | | |
|---|---|---|---|
| 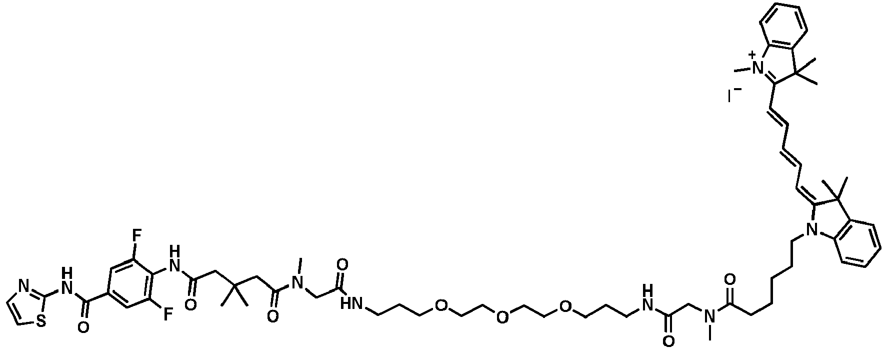 | | | |
| A₁ | A_{2A} | A_{2B} | A₃ |
| 31 % | 8.35 nM | 18 % | 37 % |

| | | | |
|---|---|---|---|
| 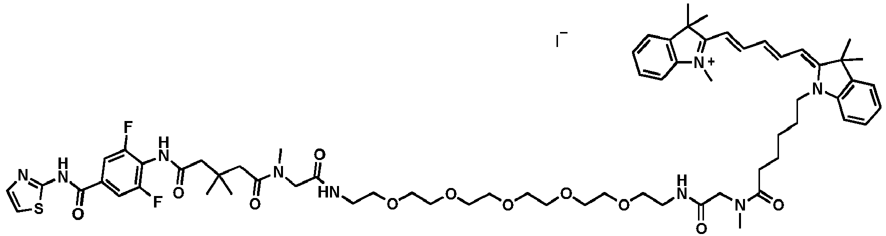 | | | |
| A₁ | A_{2A} | A_{2B} | A₃ |
| 1 % | 62 % | 24% | 44 % |

The data shows that the selectivity profile of the compounds can be improved by tuning the **G** moiety, as defined above, as well as the length of the **S'** chain.

## Claims

1. A compound of formula (**I**), 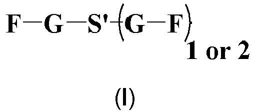 or a salt or solvate thereof,
wherein each occurrence of,
- **F** is independently selected from a moiety comprising a photoactive or a biologically active moiety; and
- **G** is independently selected from a moiety of formula (**II**) or (**III**), 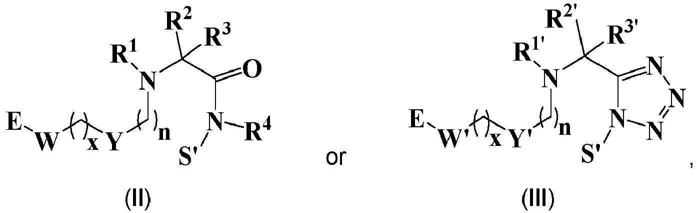 wherein each occurrence of,
- **R¹, R^{1'}, R², R^{2'}, R³, R^{3'}** or **R⁴** is independently selected from H, -COO**R⁹**,-C(=O)H, -CS**R⁹**, -C₁₋₁₂ alkyl, -C₁₋₆ alkyl-O-C₁₋₆ alkyl, **F,** -C₁₋₁₂ alkyl-**F**, -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, -C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkyl heteroaryl, heterocyclyl and alkylheterocyclyl groups are optionally substituted with 1, 2 or 3 substituents each independently selected from the group consisting of halogen, (=O), -O**R⁹**, -COO**R**⁹,-OC(=O)**R⁹**, -C(=O)H, -CN, -S**R⁹**, -N(**R⁹**)₂, -N(**R⁹**)C(=O)H, -C(=O)N(**R⁹**)₂,-ON(**R⁹**)₂, and (5- to 10-membered)-C₂₋₉ heterocyclyl with at least one N atom;
- **n** and **x** are integers independently selected from 0 to 6;
- **Y** and **Y'** are independently selected from single bond, -CH(COO**R⁹**)-, -C(=O)-, -O-, -N(**R⁵**)- or optionally substituted phenyl;
- **R⁵** is independently selected from H, -COO**R**⁹, -C(=O)H, -CS**R⁹**, -C₁₋₁₂ alkyl, -C₁₋₆ alkyl-O-C₁₋₆ alkyl, **-F,** -C₁₋₁₂ alkyl-**F**, -C₁₋₆ alkyl-O-C₁₋₆ alkyl-**F**, -C₃₋₁₀ cycloalkyl, -C₁₋₄ alkyl-C₃₋₁₀ cycloalkyl, -C₆₋₁₂ aryl, -C₁₋₄ alkyl-C₆₋₁₀ aryl, (5- to 10-membered)-C₁₋₉ heteroaryl, -C₁₋₄ alkyl-(5- to 10-membered)-C₁₋₉ heteroaryl, (5- to 10-membered)-C₂₋₉ heterocyclyl, or -C₁₋₄ alkyl-(5- to 10-membered)-C₂₋₉ heterocyclyl, wherein said alkyl, cycloalkyl, alkylcycloalkyl, aryl, alkylaryl, heteroaryl, alkylheteroaryl, heterocyclyl and alkylheterocyclyl groups are optionally substituted with 1, 2 or 3 substituents each independently selected from the group consisting of halogen, (=O), -O**R⁹**, -COO**R⁹**, -OC(=O)**R⁹**,-C(=O)H, -CN, -S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom;
- **W** is independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁷**)-, -OC(=O)(**R⁷**)-, -C(=O)CH₂O-,-CH₂OC(=O)-, -C(=O)-, -O-, -C₆₋₁₂ aryl-, or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), -O**R⁹**, -COO**R**⁹,-OC(=O)**R⁹**, -C(=O)H, -CN, -S**R⁹**, -N(**R⁹**)₂, -N(**R⁹**)C(=O)H, -C(=O)N(**R⁹**)₂,-ON(**R⁹**)₂, and (5- to 10-membered)-C₂₋₉ heterocyclyl with at least one N atom;
- **W'** is independently selected from single bond, -N(**R⁶**)-, -N(**R⁶**)C(=O)-, -N(**R⁶**)-CH₂O-, -ON(**R⁶**)-, -C(=O)N(**R⁶**)-, -C(=O)O(**R⁶**)-, -OC(=O)(**R⁶**)-, -C(=O)-,-C(=O)O-, -O-, -C₆₋₁₂ aryl- or -(5- to 10-membered)-C₂₋₉ heterocyclyl with at least one N atom-, wherein said aryl and heterocyclyl groups are optionally substituted with 1, 2 or 3 substituents each independently selected from the group consisting of halogen, (=O), -O**R⁹**, -COO**R⁹**, -OC(=O)**R⁹**, -C(=O)H, -CN,-S**R⁹**, -N(**R⁹**)₂, -N(**R⁹**)C(=O)H, -C(=O)N(**R⁹**)₂, -ON(**R⁹**)₂, and (5- to 10-membered)-C₂₋₉ heterocyclyl with at least one N atom;
- **R⁶** is independently selected from H, -C₁₋₆ alkyl or -C₆₋₁₂ aryl, wherein said alkyl and aryl groups are optionally substituted with 1, 2 or 3 substituents each independently selected from the group consisting of halogen, (=O), -O**R⁹**,-COO**R⁹**, -OC(=O)**R⁹**, -C(=O)H, -CN, -S**R⁹**, -N(**R⁹**)₂, -N(**R⁹**)C(=O)H,-C(=O)N(**R⁹**)₂, -ON(**R⁹**)₂, and -(5- to 10-membered)-C₂₋₉ heterocyclyl with at least one N atom;
- **R⁷** is independently selected from -C₂-C₁₂ alkyl, optionally substituted with 1, 2 or 3 substituents each independently selected from the group consisting of halogen, (=O), -O**R⁹**, -COOR⁹, -OC(=O)**R⁹**, -C(=O)H, -CN, -S**R⁹**, -N(**R⁹**)₂,-N(**R⁹**)C(=O)H, -C(=O)N(**R⁹**)₂, -ON(**R⁹**)₂, and -(5- to 10-membered)-C₂₋₉ heterocyclyl with at least one N atom;
- **E** is independently selected from **-R⁶** or **-F**;
- **R⁹** is independently selected from H or C₁-C₄ alkyl; and
wherein,
- **R¹** and **R²**, or **R^{1'}** and **R^{2'},** together with the nitrogen and carbon atoms to which they are attached, can form an optionally substituted 5- to 10-membered heterocyclic ring, wherein said heterocyclic ring optionally contains 1, 2, or 3 additional heteroatoms selected from the group consisting of N, S, or O;
- **R¹**, or **R^{1'},** and **R⁵**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O, as long as **W** or **W'** is not a bond; and
- **R¹**, or **R^{1'},** and **R⁶**, together with the nitrogen atoms to which they are attached, can form an optionally substituted 5- to 10-membered heterocyclic ring, wherein said heterocyclic ring optionally contains 1, 2, or 3 additional heteroatoms selected from the group consisting of N, S, or O;
and wherein **S'** is a moiety of formula **(IV),** 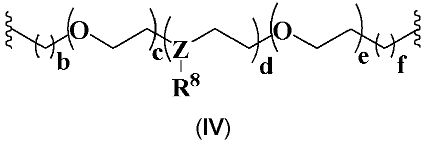 wherein,
**b** is an integer comprised between 1 and 3;
**c** is an integer comprised between 0 and 20;
**d** is an integer comprised between 0 and 1;
**e** is an integer comprised between 0 and 20;
**f** is an integer comprised between 0 and 3;
**Z** is selected from C, N, -C₃-C₁₀ cycloalkyl, (5- to 10-membered)-C₂₋₉ heterocyclyl, - C₆-C₁₂ aryl, or (5- to 10-membered)-C₁₋₉ heteroaryl; and
**R⁸** is selected from H, C₁-C₆ optionally substituted alkyl, or a moiety of formula (V) 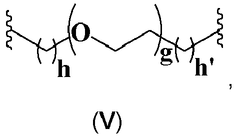 wherein,
- **g** is selected from an integer comprised between 0 and 20; and
- **h and h'** are each independently selected from an integer comprised between 0 and 3;
with the proviso that the following conditions are excluded when the compounds comprise only two biologically active **F** moieties, which are identical, and **G** = formula (**II**),
**R²** = methyl, **R¹** = **R³** = **R⁴** = H, and **n** = **x** = 0, and **Y** = CO and **W** = bond;
**R²** = -CH₂COOH, **R¹** = **R³** = **R⁴** = H, and **n** = **x** = 0, and **Y** = **W** = bond;
**R¹** forms a 2-oxopiperazine ring with **R⁵** or **R⁶**;
**R²** = diphenylmethyl, unsubstituted phenyl or CH₂-OH; or
**R²** = **R³** = **R⁴** = H, **R¹** forms a piperazine ring with **R⁶** and **F** = quinolone.

2. The compound according to claim 1, wherein **c + e** is at least 1, preferably at least 2.

3. The compound according to any one of claims 1 or 2, wherein at least one F moiety is a biologically active moiety.

4. The compound according to any one of claims 1 to 3, wherein at least one F moiety is a biologically active moiety with the proviso that said at least one biologically active moiety does not consist of an amino acid sequence comprising from 1 to 3 aminoacids, preferably from 2 to 6 aminoacids.

5. The compound according to any one of claims 1 to 4, comprising at least two different **F** moieties wherein at least one is a biologically active moiety.

6. The compound according to any one of claims 1 to 5, wherein at least one biologically active moiety is selected from the group consisting of adenosine antagonists, and agonists and derivatives therefrom, dopamine antagonists and agonists and derivatives therefrom, serotonin antagonists and agonists and derivatives therefrom, cannabinoid antagonists and agonists and derivatives therefrom, muscarinic antagonists and agonists and derivatives therefrom, protein-binding moieties capable of engaging an E3 ubiquitin ligase, and formulae **F¹** to **F¹²⁰**, wherein formulae **F¹** to **F¹²⁰** are 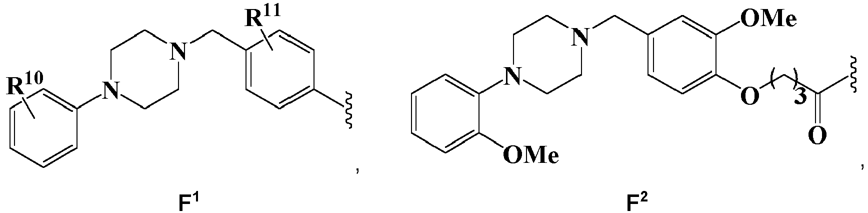 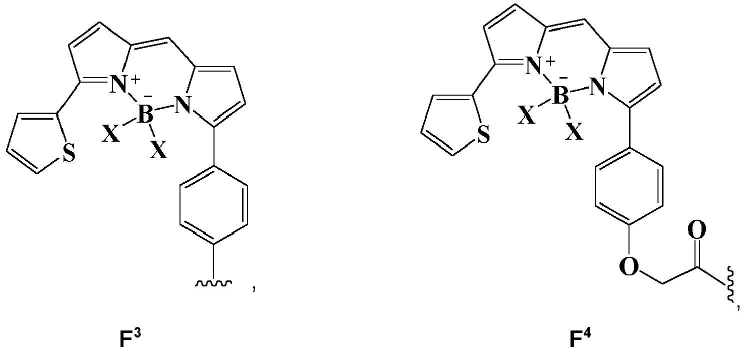 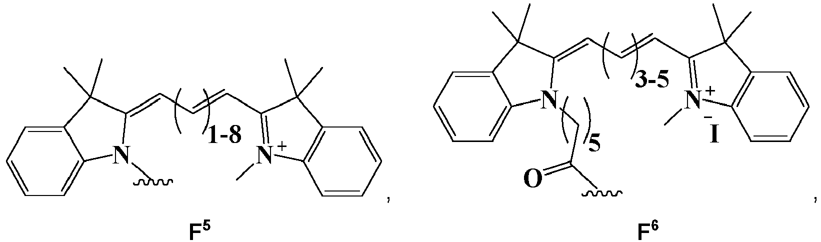 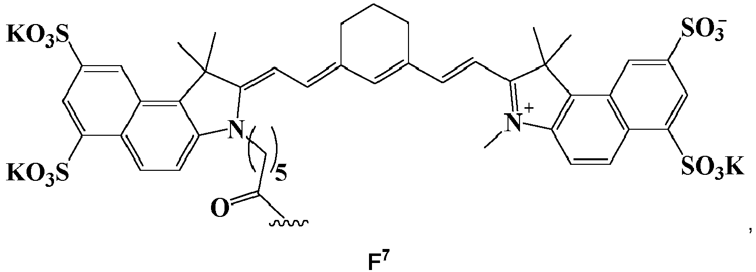 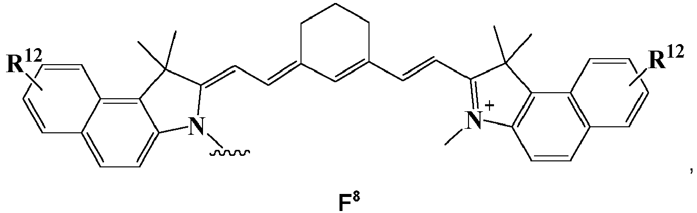 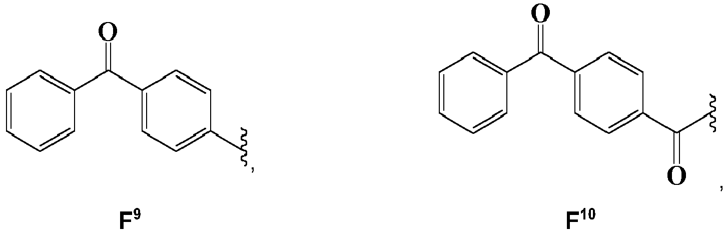 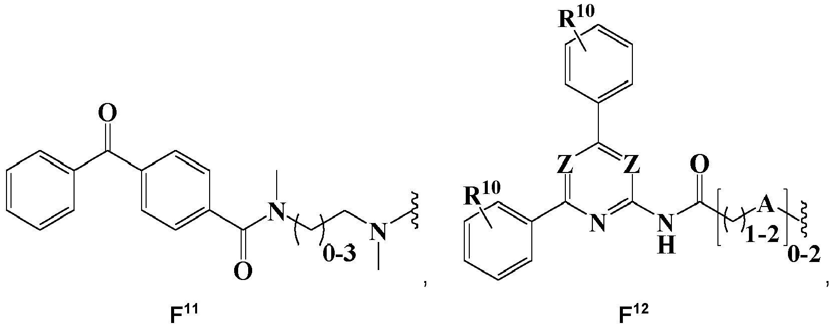 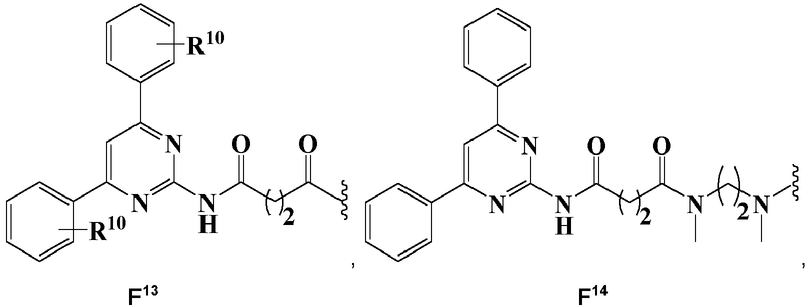 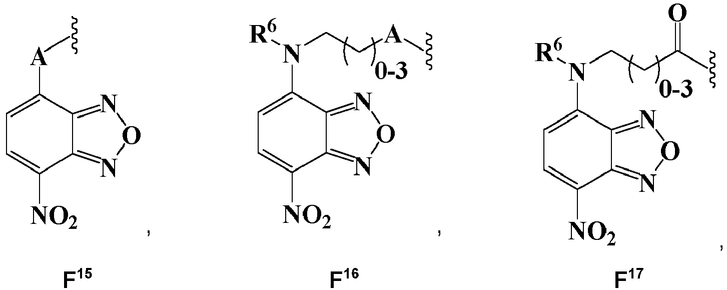 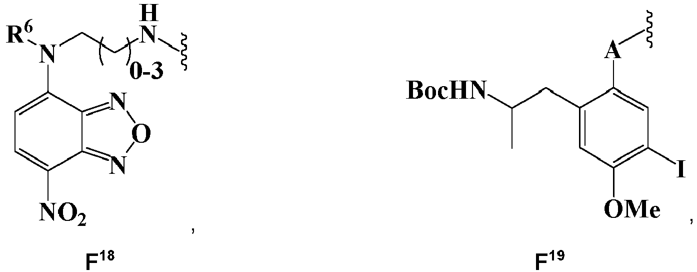 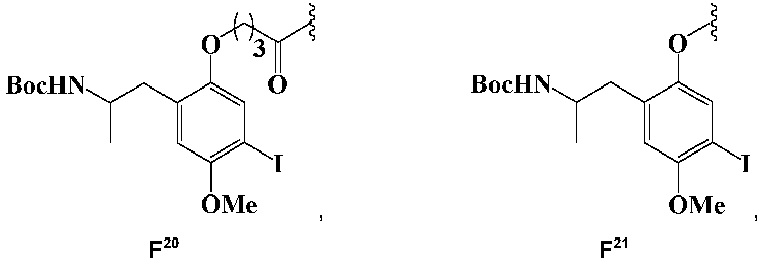 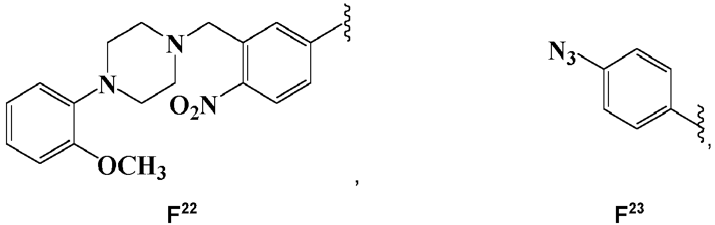 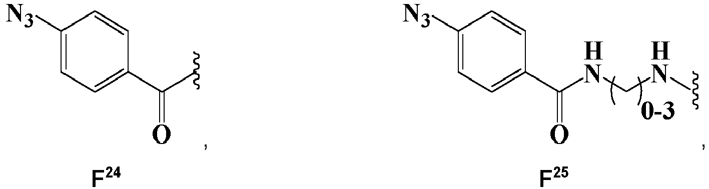 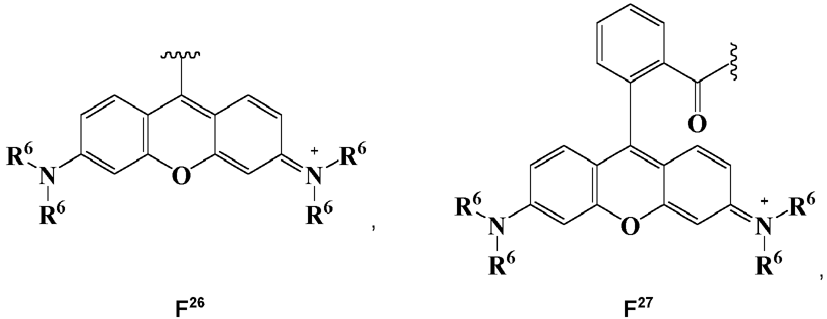 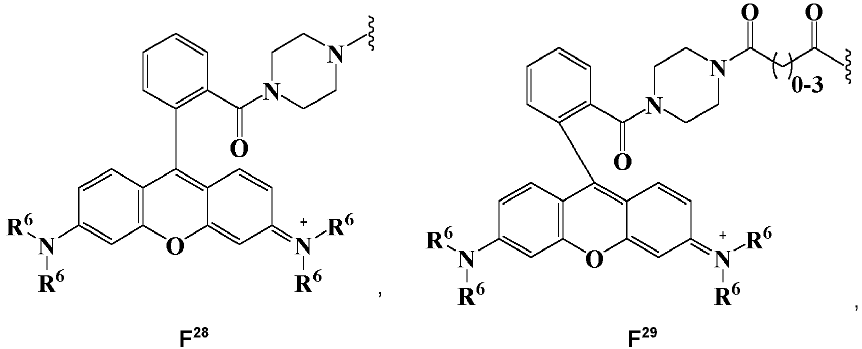 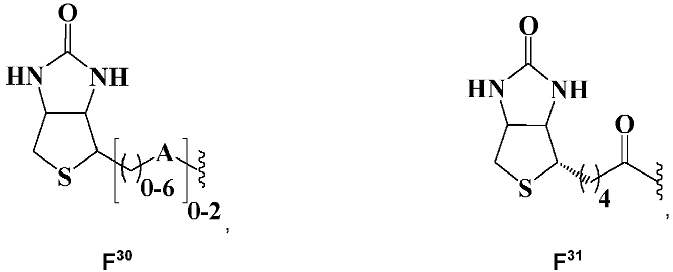 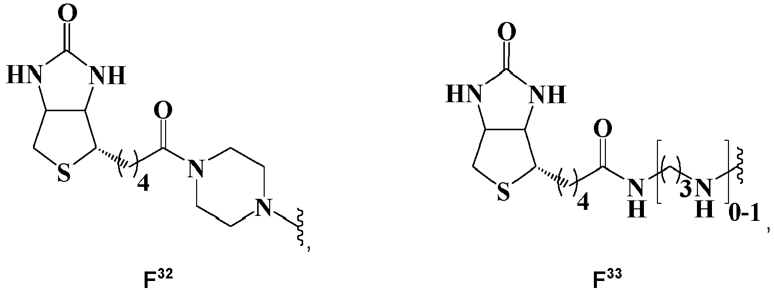 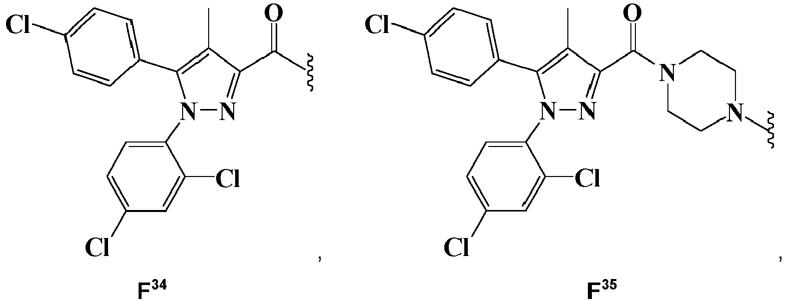 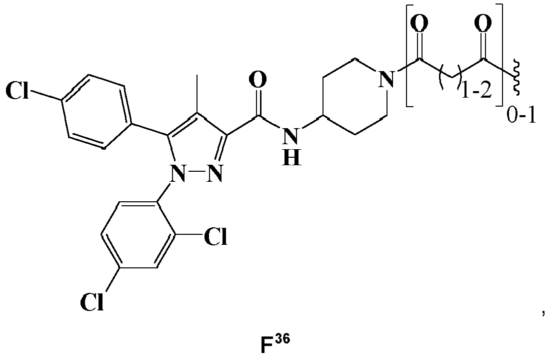 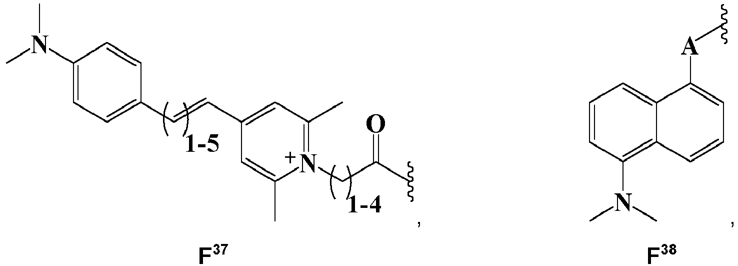 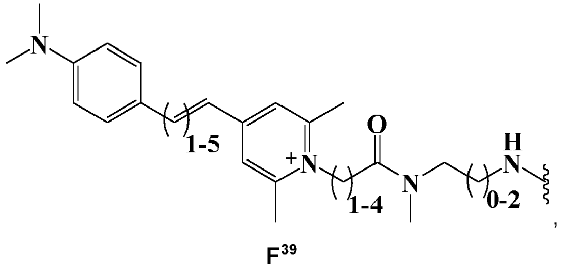 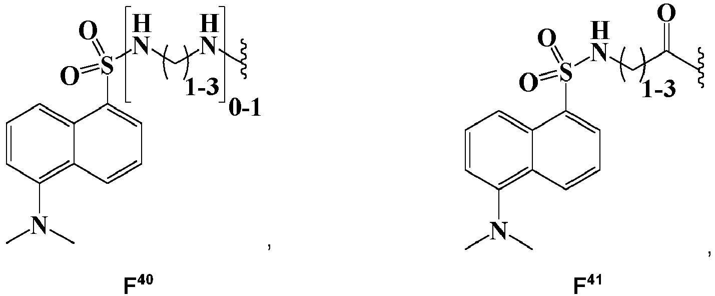 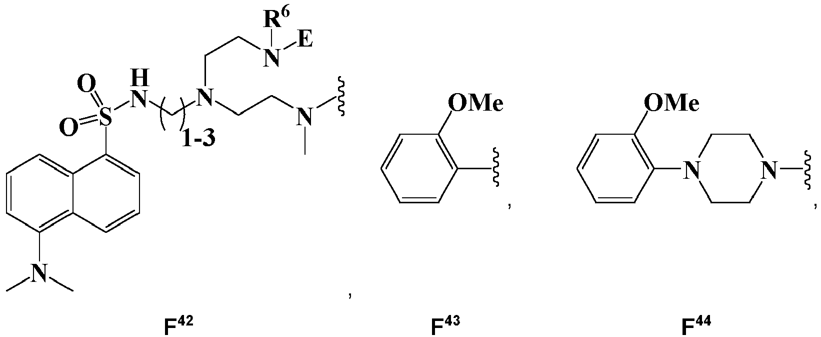 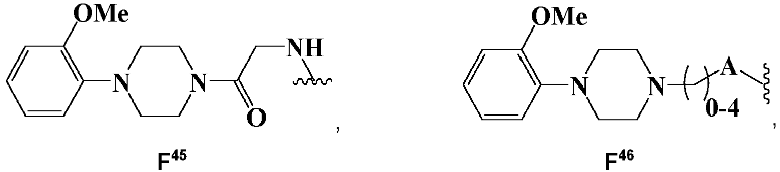 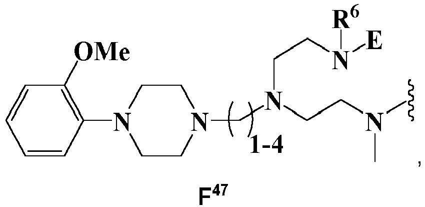 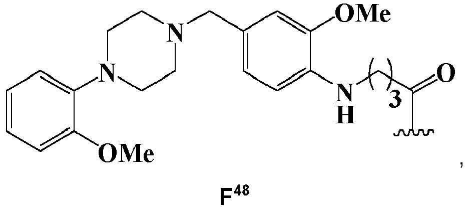 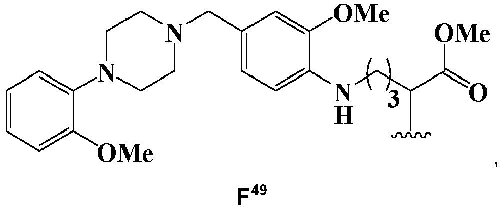 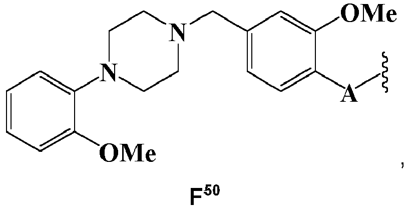 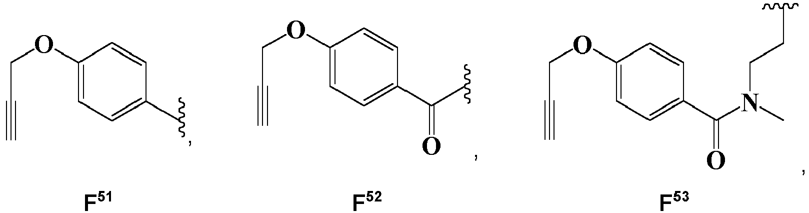 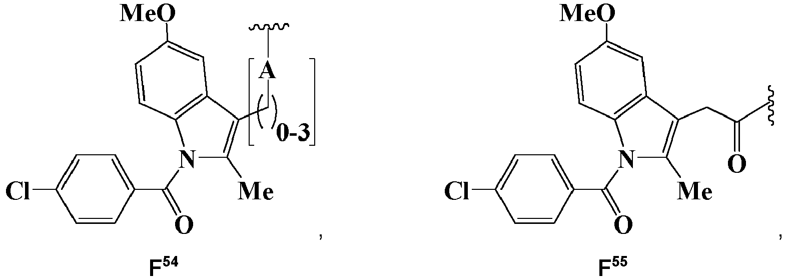 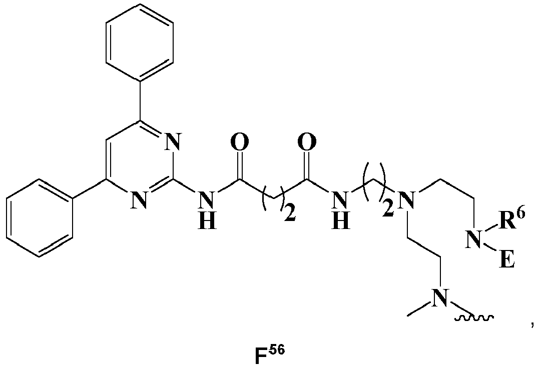 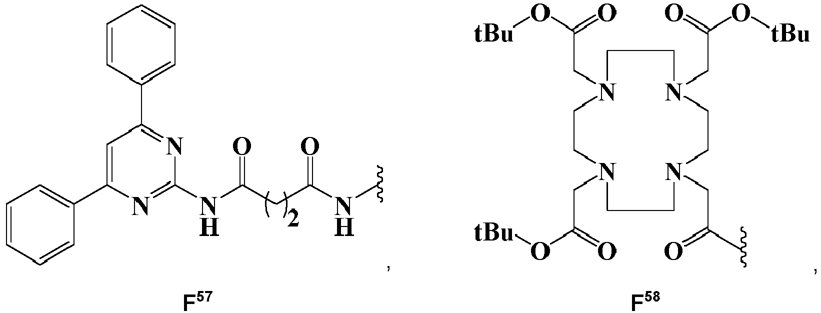 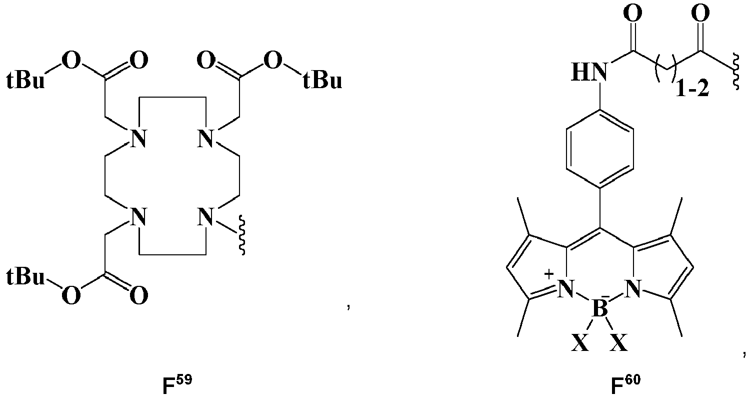 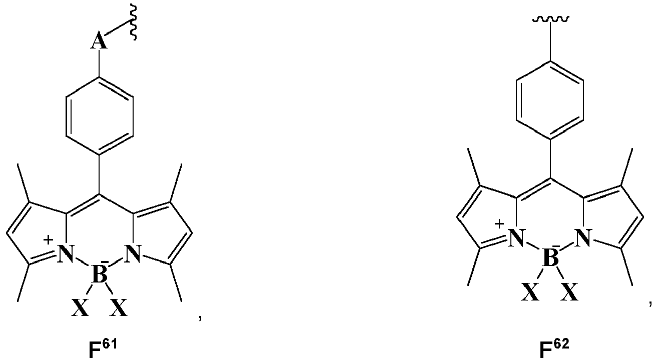 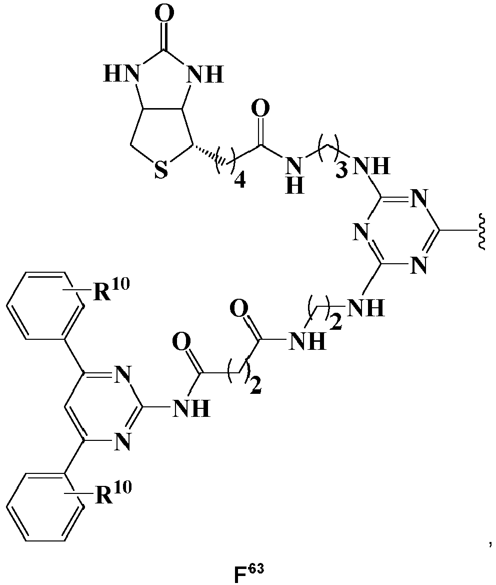 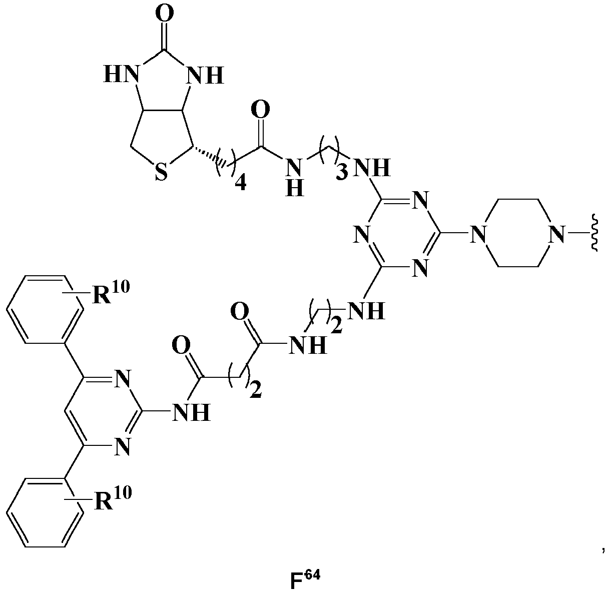 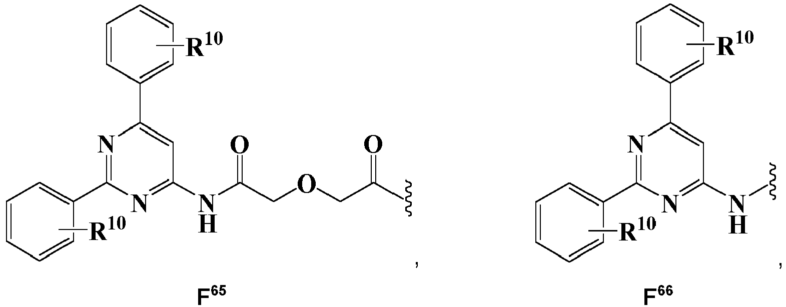 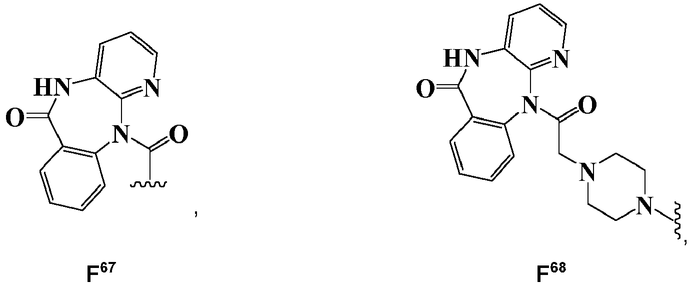 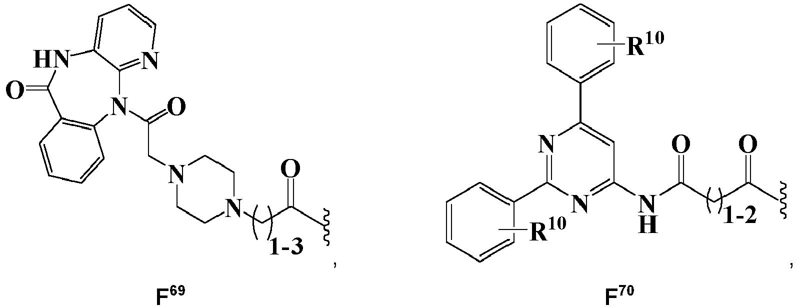 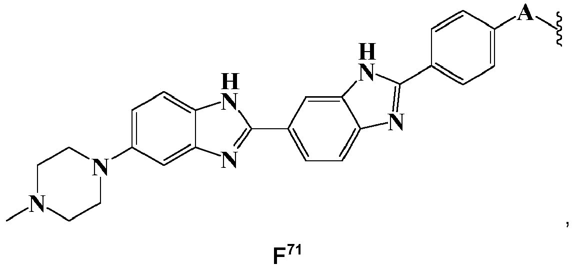 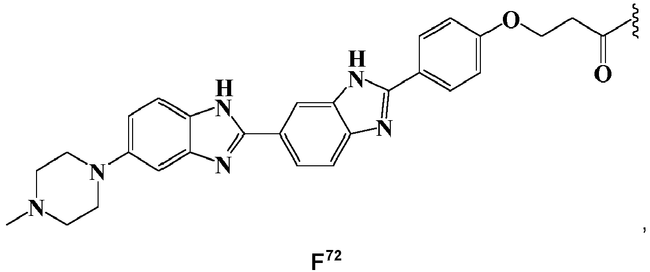 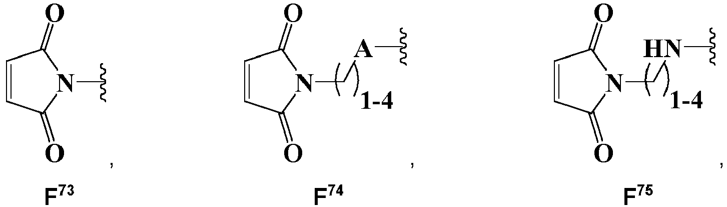 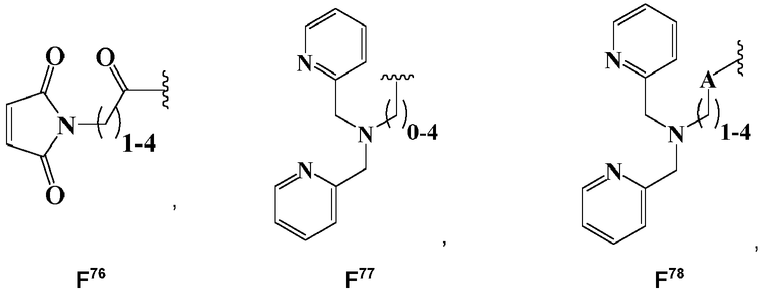 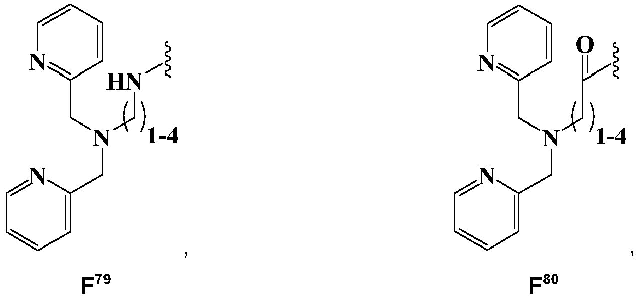 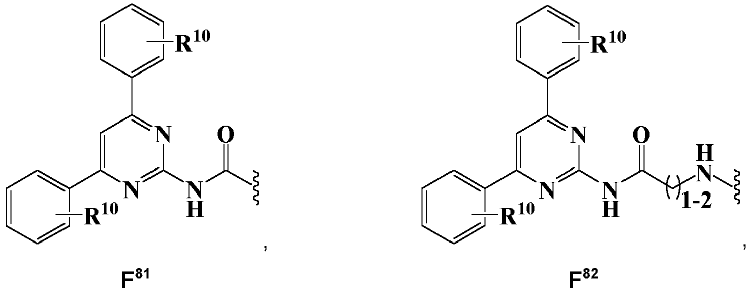 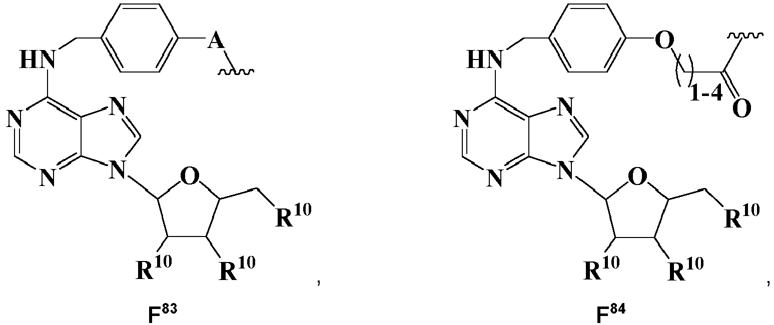 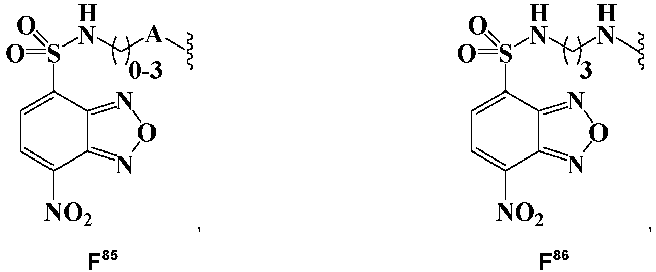 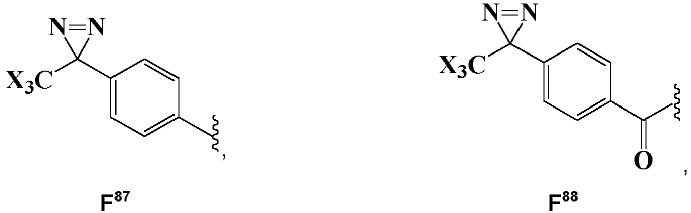 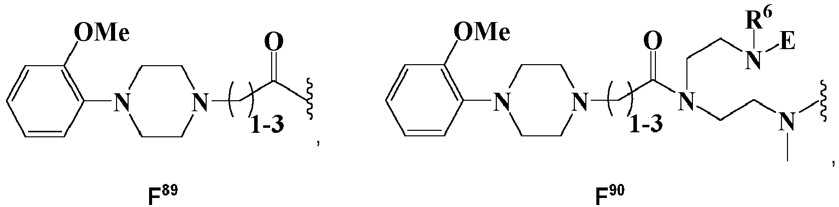 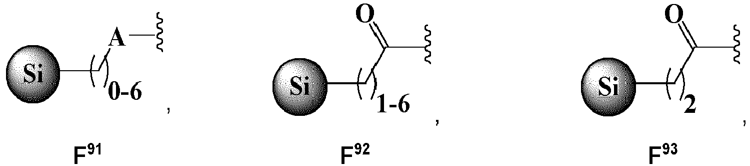 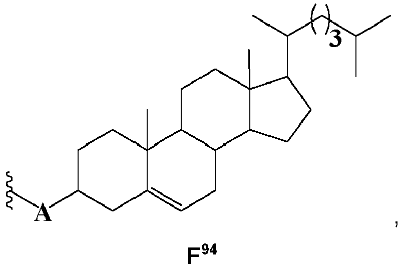 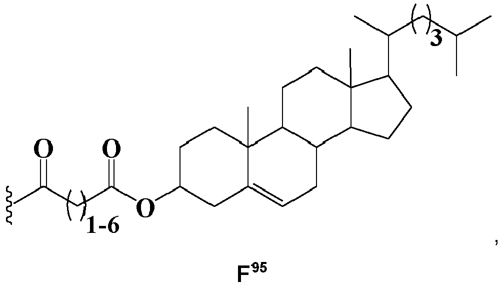 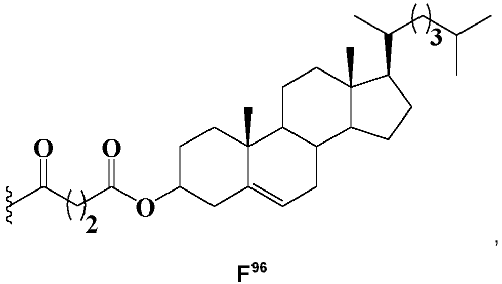 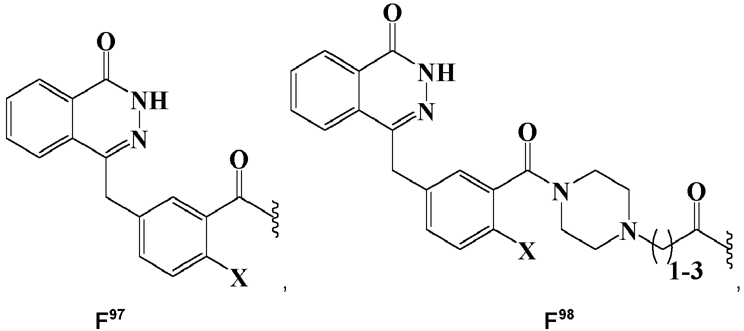 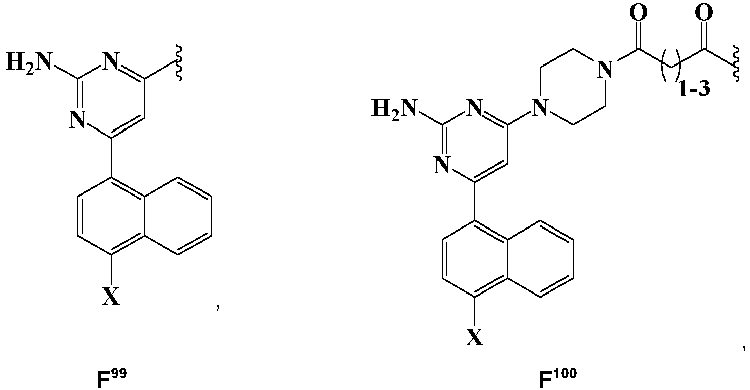 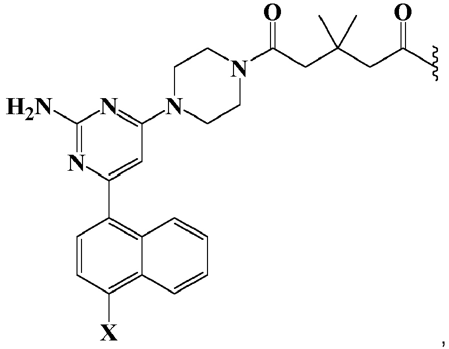 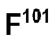 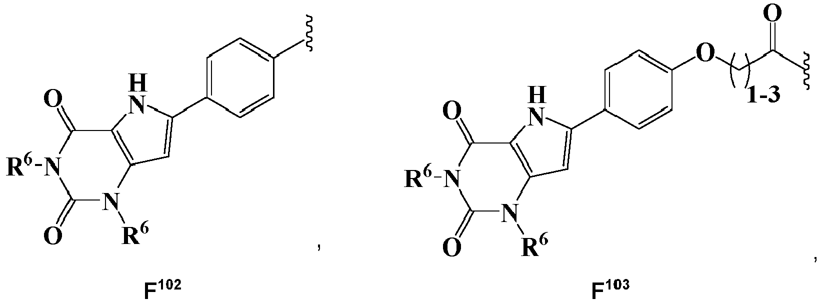 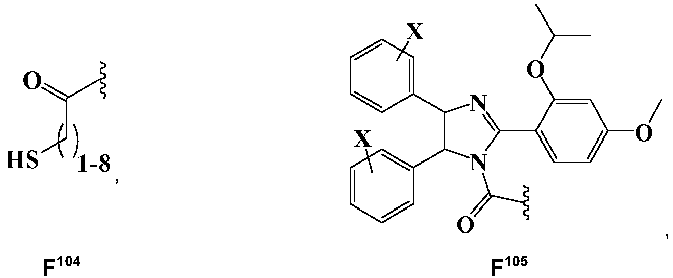 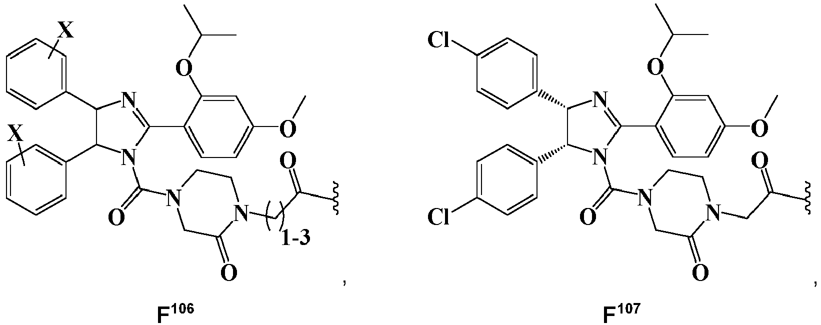 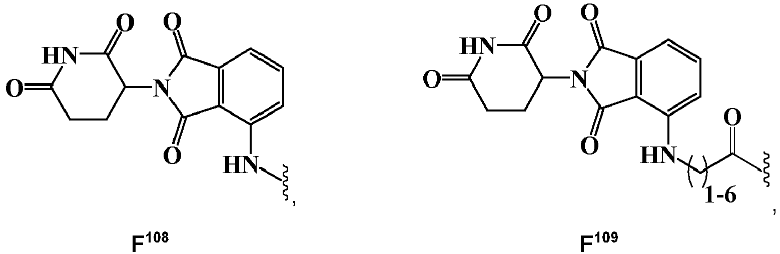 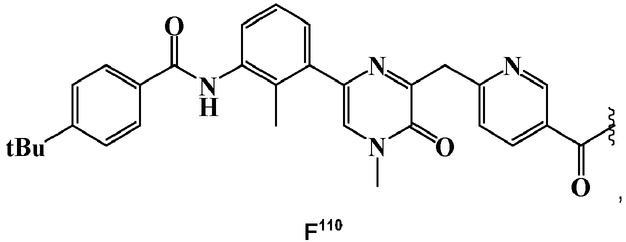 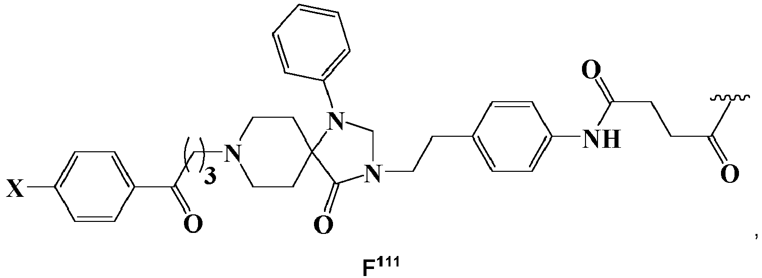 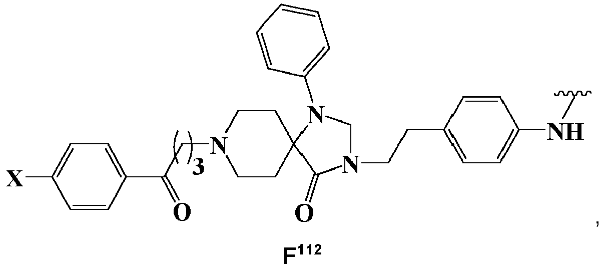 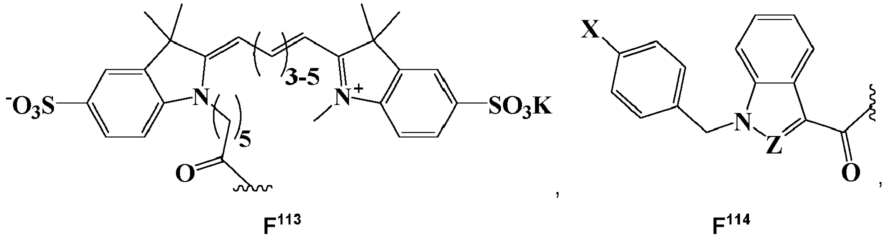 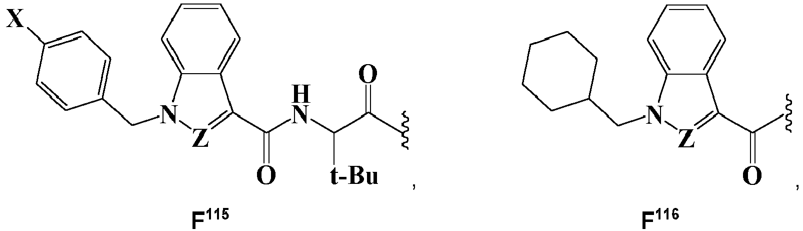 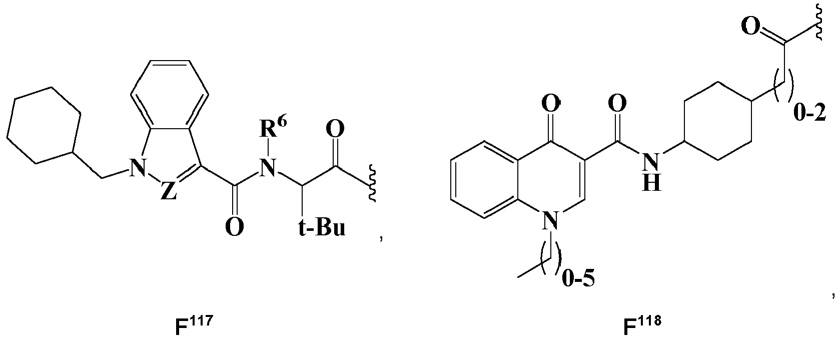 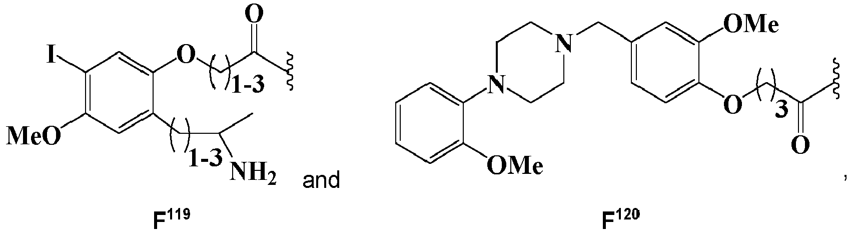 wherein
**R⁶** is independently selected from H, -C₁₋₆ alkyl or-C₆₋₁₂ aryl, wherein said alkyl and aryl groups are optionally substituted with 1, 2 or 3 substituents each independently selected from the group consisting of halogen, (=O), -O**R⁹**, -COO**R⁹**, -OC(=O)**R⁹**, -C(=O)H, -CN,-S**R⁹**, -N(**R⁹**)₂, -N(**R⁹**)C(=O)H, -C(=O)N(**R⁹**)₂, -ON(**R⁹**)₂, and -(5- to 10-membered)-C₂₋₉ heterocyclyl with at least one N atom, **R⁶** being preferably selected from H, methyl or ethyl;
**R⁹** is independently selected from H or C₁-C₄ alkyl;
**R¹⁰** represents at least one substituent, each independently selected from H, -OH or - OMe;
**R¹¹** represents at least one substituent, each independently selected from H, -OMe or-NO₂;
**R¹²** represents at least one substituent, each independently selected from H or -SO₃⁻;
**X** is a halogen atom, preferably a chlorine or a fluorine atom;
**Z** is each independently selected from C or N atoms;
**A** is each independently selected from the group consisting of -O-, -OCH₂C(=O)-,-C(=O)-, -NH-, -N(CH₃)-, -C(=O)NH-, -NHC(=O)- and -S(O)₂-;
**E** is each independently selected from H, C₁-C₃ alkyl or **-F,** preferably selected from H, methyl, ethyl or **-F;** and
**Si** represents any silicon-based support.

7. The compound according to claim 6, wherein said protein-binding moieties capable of engaging an E3 ubiquitin ligase are selected from the group consisting of pVHL, Mdm2, beta-TrCP1, cereblon, c-IAP1, pomalidomide, thalidomide, lenalidomide and derivatives therefrom.

8. The compound according to any one of claims 1 to 7, wherein **R¹** or **R^{1'}** is each independently selected from H, -C₁-C₃ alkyl, -C₁₋₃ alkyl-**F**, 5-membered heterocyclic ring with **R²** or **R^{2'},** respectively, 6- to 7-membered heterocyclic ring with **R⁵**, respectively, or optionally substituted 6- to 7-membered heterocyclic ring with R6.

9. The compound according to any one of claims 24 to 37, wherein **R²** or **R^{2'}** is each independently selected from H, -C₁-C₃ optionally substituted alkyl, -C₁₋₃ alkyl**-F**, **-F,** or 5-membered heterocyclic ring with **R¹** or **R^{1'},** respectively, preferably H, -C₁-C₃ alkyl or 5-membered heterocyclic ring with **R¹** or **R^{1'},** respectively.

10. The compound according to any one of claims 1 to 9, wherein each occurrence of **G** is independently selected from the group consisting of formulae **G¹** to **G²⁷**: 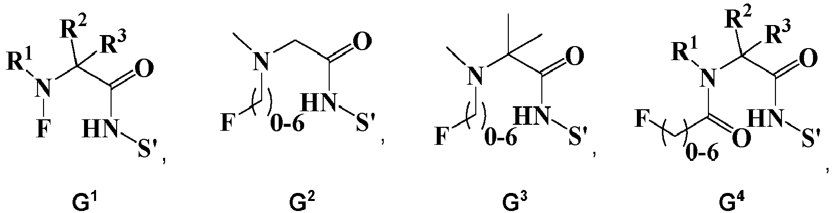 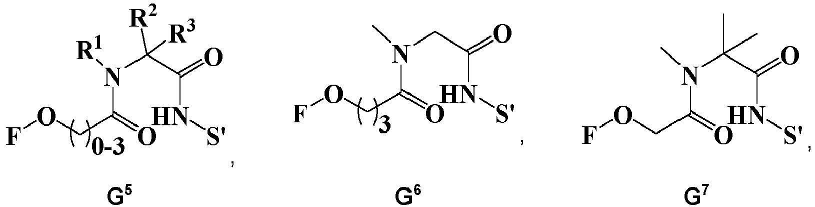 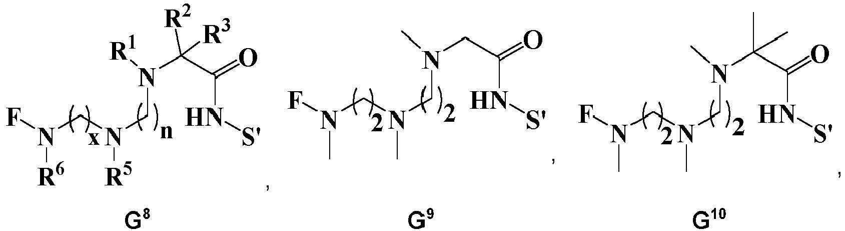 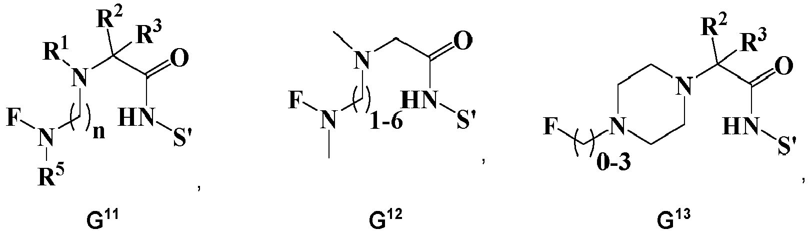 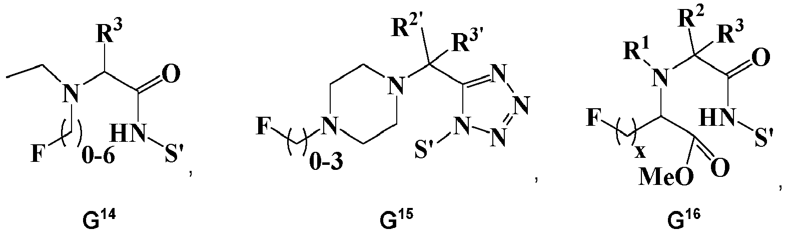 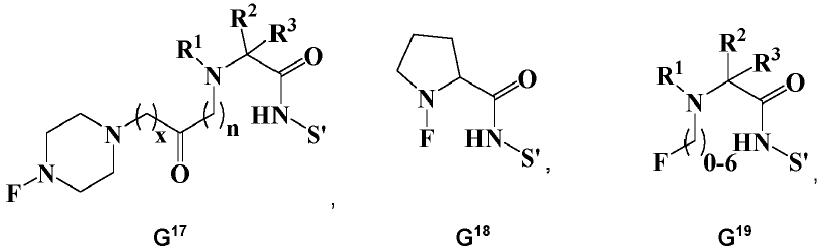 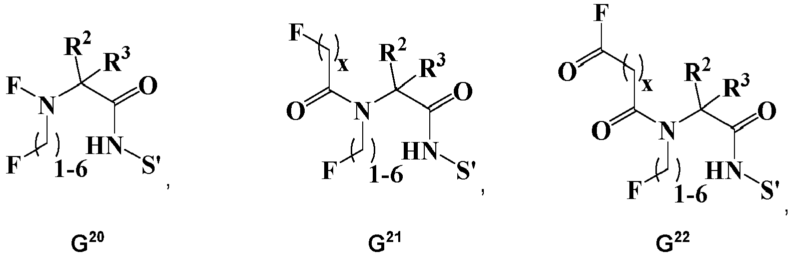 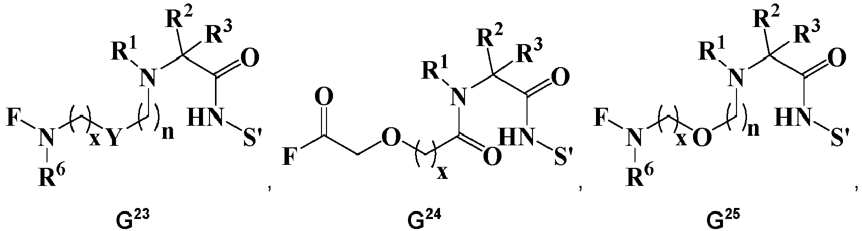 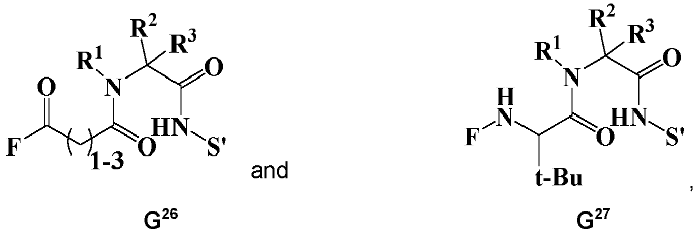 wherein,
**R¹**, **R³** and **R^{3'}** are as defined in any one of claims 1 to 9, preferably each is independently selected from the group consisting of H, methyl and ethyl;
**R²** and **R^{2'}** are as defined in any one of claims 1 to 9, preferably each is independently selected from the group consisting of H, methyl, ethyl, isopropyl and tert-butyl;
**R⁵** is as defined in any one of claims 1 to 9, preferably each is independently selected from the group consisting of H, methyl, **-F** and -C₁₋₆ alkyl**-**F;
**R⁶** is as defined in any one of claims 1 to 9, preferably each is independently selected from the group consisting of H and methyl; and
**x** and **n** are as defined in any one of claims 1 to 9.

11. A method for the preparation of a compound of formula (**I**), or a salt or solvate thereof, according to any one of claims 1 to 10, comprising the mixing of at least three precursors selected from:
a) at least one compound of general formula **(G1)** 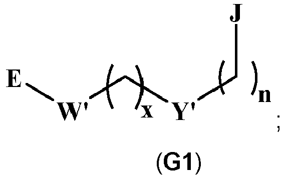 and/or a combination of at least one compound of general formula **(G2)** 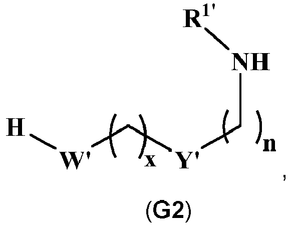 and at least one compound comprising a moiety **F** and at least one reactive group, wherein said at least one reactive group is preferably selected from an amine, hydroxyl, aldehyde or acid reactive group;
b) optionally, at least one compound of formula **R²**-C(=O)-**R³** or **R^{1'}**-NH-(CH₂)₀₋₆-C(**R^{2'}**)(**R^{3'}**)-COOH; and
c) at least one compound of general formula (**Ia**) 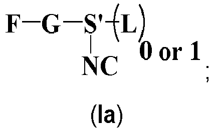 wherein
**J** is COOH or NH**R¹**,
**E, F, W', x, Y', n, R^{1'}, R^{2'}** and **R^{3'}**, **G** and **S'** are as defined in any one of the preceding claims and wherein **L,** when present, is **G-F.**

12. A pharmaceutical composition comprising a compound of formula (**I**) as defined in any one of claims 1 to 10 and a pharmaceutically acceptable excipient.

13. Use of a compound of formula (**I**) as defined in any one of claims 1 to 10, in sensors, diagnostic methods, bioanalytical methods, chemical tests and as chelating agents, wherein the use is not practiced on the human or animal body.

14. A compound of formula (**I**) as defined in any one of claims 1 to 10, or a pharmaceutical composition as defined in claim 12, for use as a medicament.

15. A compound of formula (**I**) as defined in any one of claims 1 to 10, or a pharmaceutical composition as defined in claim 12, for use in the treatment and/or prevention of a heavy metal poisoning, cardiovascular diseases, neurodevelopment disorders, immune system disorders, metabolic diseases or disorders, neurodegenerative diseases, diabetes, respiratory diseases and cancer.
